(19) Europäisches Patentamt
European Patent Office
Office européen des brevets

(11) EP 1 789 535 B1

(12) # EUROPEAN PATENT SPECIFICATION

(45) Date of publication and mention of the grant of the patent:
**07.10.2009 Bulletin 2009/41**

(21) Application number: **05778088.4**

(22) Date of filing: **09.08.2005**

(51) Int Cl.:
***C12N 5/06*** *(2006.01)*

(86) International application number:
**PCT/EP2005/008653**

(87) International publication number:
**WO 2006/015853 (16.02.2006 Gazette 2007/06)**

(54) **USE OF PAX4 IN PANCREATIC CELL PROLIFERATION**

VERWENDUNG VON PAX4 ZUR PROLIFERATION VON PANKREASZELLEN

UTILISATION DU PAX4 DANS LA PROLIFÉRATION DE CELLULES PANCRÉATIQUES

(84) Designated Contracting States:
**AT BE BG CH CY CZ DE DK EE ES FI FR GB GR HU IE IS IT LI LT LU LV MC NL PL PT RO SE SI SK TR**

(30) Priority: **11.08.2004 US 600704 P**
**15.12.2004 EP 04029743**

(43) Date of publication of application:
**30.05.2007 Bulletin 2007/22**

(73) Proprietor: **UNIVERSITE DE GENEVE**
**1211 Genève 4 (CH)**

(72) Inventors:
- **GAUTHIER, Benoit, Raymond**
**1202 Genève (CH)**
- **BRUN, Thierry**
**1234 Vessy (CH)**
- **WOLLHEIM, Claes, Benedict**
**1234 Vessy (CH)**
- **WEHR, Roland**
**37075 Göttingen (DE)**

(74) Representative: **Vossius & Partner**
**Siebertstraße 4**
**81675 München (DE)**

(56) References cited:
**WO-A-98/29566**
**WO-A-02/086107**
**WO-A-03/087349**

- **SHAPIRO A M ET AL: "Islet transplantation in seven patients with type 1 diabetes mellitus using a glucocorticoid-free immunosuppressive regimen" NEW ENGLAND JOURNAL OF MEDICINE, THE, MASSACHUSETTS MEDICAL SOCIETY, WALTHAM, MA, US, vol. 343, no. 4, 27 July 2000 (2000-07-27), pages 230-238, XP002220559 ISSN: 0028-4793 cited in the application**
- **BRUN T ET AL: "The diabetes-linked transcription factor PAX4 promotes [beta]-cell proliferation and survival in rat and human islets" JOURNAL OF CELL BIOLOGY, ROCKEFELLER UNIVERSITY PRESS, NEW YORK, US, US, vol. 167, no. 6, 13 December 2004 (2004-12-13), pages 1123-1135, XP002350953 ISSN: 0021-9525**
- **SOSA-PINEDA B ET AL: "THE PAX4 GENE IS ESSENTIAL FOR DIFFERENTIATION OF INSULIN-PRODUCINGBETA CELLS IN THE MAMMALIAN PANCREAS" NATURE, NATURE PUBLISHING GROUP, LONDON, GB, vol. 386, 27 March 1997 (1997-03-27), pages 399-402, XP002066645 ISSN: 0028-0836 cited in the application**
- **COLMAN ALAN: "Making new beta cells from stem cells" SEMINARS IN CELL & DEVELOPMENTAL BIOLOGY, vol. 15, no. 3, June 2004 (2004-06), pages 337-345, XP002365752 ISSN: 1084-9521**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 789 535 B1

## Description

## BACKGROUND OF THE INVENTION

The present invention relates to an *in vitro* method for the generation and isolation of pancreatic β-cells, comprising the steps of (a) contacting an adult cell derived from mammalian pancreatic islets or an explant culture of adult pancreatic islets with functional, wild-type Pax4; and (b) detecting and isolating, from said adult cell or explant culture, β-cells that proliferate in response to the contact with Pax4.

Plasma glucose levels are regulated by the action of insulin, a hormone that is produced and secreted by the pancreatic β-cells in response to nutrients. Diabetes mellitus, which comprises a heterogeneous group of hyperglycemic disorders, results from inadequate mass and function of pancreatic β-cells. Worldwide prevalence figures give an estimate of 151 million cases in 2000 and an extrapolated number of 221 million in 2010 (Zimmet, 2001). There are two forms of diabetes: type 1 diabetes is linked to selective autoimmune destruction of pancreatic β-cells while type 2 diabetes is a severe disease of intermediary metabolism usually caused by both β-cell dysfunction and resistance to the biological actions of insulin on its main target tissues (liver, muscle and fat) (Bell and Polonsky, 2001; Saltiel and Kahn, 2001). Uncontrolled elevated plasma levels of glucose as well as dyslipidemia increase the risk for diabetic complications such as cardiovascular and cerebrovascular disease, kidney disease, neuropathy and blindness. Current therapy for type 2 diabetes includes modification of life style, such as diet and exercise and the use of pharmacological agents that stimulate insulin secretion, decrease hepatic glucose production and increase sensitivity of target tissues to insulin. Nonetheless often type 2 diabetics, like type 1 patients, require treatment with insulin. secretion, decrease hepatic glucose production and increase sensitivity of target tissues to insulin. Nonetheless often type 2 diabetics, like type 1 patients, require treatment with insulin.

The susceptibility for type 2 diabetes is inherited but single diabetes-linked genes have only been identified in about 5% of cases. These monogenic subforms are maturity onset diabetes of the young (MODY) and mitochondrial diabetes (MD). The latter is due to mutations in the mitochondrial genome (Maassen, 2001; Maechler and Wollheim, 2001) and is associated with a selective impairment of glucose-stimulated insulin secretion (Brandle, 2001). The MODY phenotype is characterized by autosomal dominant transmission with early onset (< 25 years) and primary β-cell dysfunction. Mutations in glucokinase, the rate-limiting enzyme for glucose metabolism in the β-cell, lead to MODY2 (Froguel, 1993). Interestingly, all of the additional 5 MODY subforms have been linked to mutations in genes encoding transcription factors: MODY1, *hnf-4*α; MODY3, *hnf-1*α; MODY4, *ipfl/pdx1*; MODY5, *hnf-1*β; MODY6, *Beta2/NeuroD* (Kristinsson, 2001; Malecki, 1999; Ryffel, 2001; Stoffers, 1998; Mitchell, 2002). A limited number of studies have also implicated HNF-1α and Ipf1/Pdx1 in the polygenic, late onset type 2 diabetes (Hansen, 2000; Weng, 2001). Consistent with the importance of transcription factors in the development of type 2 diabetes, several recent studies have identified the paired box homeodomain family member, Pax4, as an additional predisposing gene for type 2 diabetes in the Japanese population. Homozygocity for the Pax4 mutation, located in the paired DNA binding domain, resulted in severe diabetes while heterozygous subjects were glucose intolerant (Kanatsuka, 2002; Shimajiri, 2001; Shimajiri, 2003). In contrast, no evidence of linkage between mutations in the *pax4* gene and type 2 diabetes was apparent in either Ashkenazi Jews or in the French population (Dupont, 1999; Tao, 1998). The discrepancies among these studies also encountered for NeuroD may be derived from ethnic or racial differences and will require further clarification. Unfortunately, the reports segregating NeuroD with diabetes give no information on Pax4 function, which as been suggested to be controlled by NeuroD (Smith, 2000). In addition to its potential implication in type 2 diabetes, several Pax4 haplotypes have recently been associated with type 1 diabetes in Scandinavian families (Holm *et al.,* 2004).

Increased insulin requirements in pregnancy, obesity and other insulin resistant states are compensated by β-cell hyperplasia and hypertrophy. This β-cell plasticity reaches remarkable levels in animal models such as fa/fa rats, ob/ob mice and in the liver insulin receptor null mice (Chan, 1999; Hellman, 1965; Michael, 2000). An increase in β-cell mass is also observed in human obesity (Butler, 2003; Kloppel, 1985). Up to 20% of such individuals develop type 2 diabetes probably caused by defective β-cell adaptation due to increased sensitivity to harmful environmental factors such as free fatty acids combined with predisposing genetic factors (Kashyap, 2003). There is however only limited information regarding factors controlling β-cell plasticity.

The mechanism, by which Pax4 affects β-cell function, and thus its potential implication in type 2 diabetes, is still poorly understood. In contrast to the widespread embryonic expression of other *pax* family members, expression of Pax4 is tightly regulated during development. Pax4 mRNA is detected in the pancreatic bud as early as mouse embryonic day 10.5 (E10.5), but expression becomes progressively restricted to the β- and δ- cells of the islet of Langerhans, producing respectively insulin and somatostatin. Although initial investigations suggested that Pax4 was not expressed in mature endocrine cells, more recent studies have detected mRNA for the transcription factor in adult human, rat and mouse pancreatic islets (Dohrmann, 2000; Heremans, 2002; Kojima, 2003; Zalzman, 2003; Zhang, 2001). Consistent with its tissue and cell-specific expression pattern, targeted disruption of the *pax4* gene in mice results in the absence of mature pancreatic β- and δ-cells with a commensurate increase in the glucagon-producing α-cells (Sosa-Pineda, 1997; Wang, 2004). This increase was recently attributed to the α-cell specific transcription factor Arx that is repressed by Pax4 during development (Collombat, 2003). Normally, the earliest insulin-producing precursor cells are detected at E8.5-9 (Gittes and Rutter, 1992). Similarly, in mouse mutant Pax4 embryos, insulin-staining cells are apparent at this stage indicating that Pax4 expression is not mandatory for the generation of β-cell precursors. However, the onset of Pax4 gene expression in the pancreas around E10.5 and the absence of mature β cells in islets of Pax4 mutant newborn mice strongly suggest a critical role of this factor in the proliferation and/or survival of these early committed insulin-producing cells (Sosa-Pineda, 1997). Consistent with this hypothesis, the peak of Pax4 expression in mice was recently shown to reside between E13.5 and E15.5, a period coinciding with differentiation of β-cells, the so-called secondary transition (Wang, 2004).

In contrast to primary β-cells, elevated expression levels of Pax4 are found in human insulinomas. The same study reported the presence of a novel spliced variant of Pax4 which lacks the carboxy-terminal end of the protein involved in mediating repression of gene transcription (Miyamoto, 2001). It is presently unclear whether or not Pax4 expression is directly link to the proliferative phenotype of the insulinoma. However, ablation of the repressor domain may alleviate protein-protein interactions with potential negative regulators leading to the constitutive activation of Pax4 and ultimately cell replication. Consistent with a role of Pax4 in cell proliferation, an oncogenic function has been attributed to other closely related pax genes, *e.g.*, the involvement of both Pax3 and Pax7 in the genesis of alveolar rhabdomyosarcoma, the responsibility of aberrant Pax5 expression for the formation of medulloblastoma and the dependence of ovarian and bladder cancer cell lines on Pax2 for survival (Davis, 1994; Galili, 1993; Kozmik, 1995; Muratovska, 2003). Interestingly suppression of Pax2 or Pax7 in tumour cell lines resulted in programmed cell death or apoptosis (Margue, 2000; Muratovska, 2003). It is noteworthy that regulation of β-cell mass during the neonatal period also implicates apoptosis (Bonner-Weir, 2000). Pax4 has indeed been shown to be down regulated in islets from newborn mice as compared to embryos (Sosa-Pineda, 1997). The role of Pax4 in determining the choice between proliferation and apoptosis is thus possible but has not yet been demonstrated. Taken together, these studies clearly demonstrate the critical role of Pax family members in cell growth and survival. Activin A, a member of the transforming growth factor β family, has independently been shown to induce Pax4 gene expression in pancreatic β cell lines (Ueda, 1996) and to stimulate growth and differentiation of human foetal pancreatic cells in combination with betacellulin (Demeterco, 2000). It will be of interest to determine whether glucose and/or the gut hormone glucagon-like peptide 1 (GLP-1) and its more stable analogue exendin-4, which also stimulate both β-cell replication and neogenesis, can modulate Pax4 gene expression (Drucker, 2001; Paris, 2003; Xu, 1999). It is noteworthy that the action of GLP-1 may be mediated by the activation of betacellulin (Buteau, 2003).

To understand the mechanisms that control the expression of Pax4 and thereby elucidate its impact on endocrine cell type determination, several studies have mapped and characterized the regulatory regions of both the human and mouse *pax4* gene (Brink, 2001; Smith, 2000; Xu and Murphy, 2000). One of the most striking aspects of the *pax4* gene is its capacity for auto-repression. If Pax4 functions to maintain and proliferate either β or δ-cell lineage during pancreas development, then persistent expression may be detrimental due to sustained cell division. Auto-repression will however terminate expression of Pax4 and allow expanded cells to proceed towards either a β- or δ-cell phenotype. In contrast, Pax4 expression was shown to be dependent on the concerted action of the transcription factors Panl, Beta2/NeuroD, HNF-1α, HNF-4α and Pdx1 interacting with the promoter. A further increase in transcription was observed when Beta2/NeuroD was substituted by the early pancreatic committing transcription factor Ngn3 (Smith, 2004; Smith, 2000). The action of Beta2/NeuroD, HNF-1α, HNF-4α and Pdx1 in Pax4 gene expression is interesting given the role of these transcription factors in the development of type 2 diabetes. Thus, Pax4 may be a downstream target of these MODY-related genes. Consistent with this hypothesis, mice overexpressing a dominant negative form of HNF-1α (SM6) have morphologically normal islets at birth but gradually lose β-cell mass and developed diabetes within 6 weeks (Hagenfeldt-Johansson, 2001). In another transgenic mouse model in which a human HNF-1α mutation was targeted to the β-cells, there was already a decreased β-cell mass at birth and the adult animals displayed reduced β-cell replication (Yamagata, 2002). It is tempting to speculate that deregulation of Pax4 expression due to the absence of a functional HNF-1α effects the formation and expansion of new β-cells. The initial β-cell mass observed at birth would potentially be generated during development due to the presence of Ngn3, which could compensate for the lack of HNF-1α. Interestingly, no apparent alterations in Pax4 mRNA levels were detected in a transgenic mouse harbouring the targeted null mutation of HNF-1α (Shih, 2001). However, these studies were conducted in newborn mice, which express only low levels of Pax4 (Brink, 2001; Sosa-Pineda, 1997). Studies in mice carrying targeted mutations in MODY-genes have indicated altered expression of genes involved in glucose sensing. These alterations may underlie the insulin secretory defects observed in patients, although impaired β-cell development and proliferation could also contribute to the eventual β-cell deficiency.

The prior art, as shown above has suggested that Pax4 may be involved in differentiation of pancreatic cells during embryogenesis. Dor (2004) *loc. cit.* has also documented that no new islets are formed during adult life, and concludes that pre-existing β-cells are the major source for new β-cells during adult life, as well as during regeneration from (partial) pancreatectomy.

WO 98/29566 provides for a method for testing the differentiation status in/of pancreatic cells in a mammal and discloses various wild-type alleles of Pax4 (human and mouse). Pax4 is taught as a molecule involved in β-cell differentiation during embryogenesis and as an expression factor for the promotion of genes specific for insulin-producing β-cells.

Similarly, Blysczuk (2003) teaches that Pax4 expression promotes differentiation of embryonic stem cells to insulin-producing cells. Kahan (2003) uses Pax4 expression as a marker for pancreatic differentiation. Wang (2004) *loc. cit.*, teaches that Pax4 is expressed in differentiating endocrine cells and proposes that Pax4 and NKx2.2 are two key components for initating pancreatic β-cells differentation.

Treatment of diabetes and other pancreatic disorders is still a challenging task. Although intensive exogenous insulin therapy can approach the physiological control of blood glucose, and delay or prevent the onset of chronic complications, such treatment remains cumbersome. It is also associated with increased risk for hypoglycaemia with brain damage. The ultimate goal is treating Type 1 diabetes by the replacement of destroyed β-cells by insulin-producing cells capable of restoring glucose homeostasis in the organism.

The concept of transplantation, first attempted in the seventies, recently gained attention with the advent of the Edmonton protocol in which patients were transplanted with human islets of Langerhans isolated from brain-dead subjects. This approach has been improved by the introduction a novel immunosuppressive therapy less aggressive to the transplanted tissue (Shapiro, 2000). The largest most recent update on the clinical outcomes of the Edmonton experience was published in 2002. The authors reported 54 islet infusions in 30 patients with type 1 diabetes and provided a detailed analysis on 17 patients all of whom became insulin-independent (Ryan, 2002). However, a significant problem in human islet allotransplantation is the requirement of two to three cadaver donors to treat a single patient with diabetes (Ryan, 2001). Thus, the dramatic shortage of islets has been a barrier to the use of islet transplantation on a larger scale.

Animal donors such as pigs could provide an alternative supply of pancreas for islet isolation and transplantation. Pigs are ideal sources of xenotransplants because they are available in large numbers and because their organs are similar in size and nature to those of humans. However, pigs contain several copies of porcine endogenous retroviruses (PERV) which have been shown to infect human cells lines *in vitro* (Van der Laan; 2000). Thus the promise of xenotransplantation is offset by possible public health risk of a cross species infection.

The pressure to create an adequate supply of islets has led to extensive research into the potential use of islet surrogates. This include establishment of human pancreatic β-cell lines. However, research completed to date has demonstrated that human islets (Maedler, 2001) and β-cells are particularly susceptible to apoptosis (cell death) following their purification and subsequent maintenance in culture. Furthermore, conditional immortalisation of human β-cells using lentivirus technology has failed to yield stable and differentiated human cell lines. An alternative approach to generate insulin-producing cells has been the use of embryonic or adult stem cells (Weir, 2004; Street, 2004). These offer several theoretical advantages including unlimited supply, multipotency, and the possibility of being non-immunogenic. Although some progress has been achieved in generating insulin-producing cells, problems in directing homogenous differentiation have curtailed advances.

Accordingly, the problem of the present invention is the provision of islet material to be used in medical settings for the prevention, amelioration and/or treatment of pancreatic disorders, in particular in the treatment of diabetes. The problem is solved by the provision of the embodiments of the invention as characterized in the claims and as described herein.

The present invention relates to an *in vitro* method for the generation and isolation of pancreatic β-cells, comprising the steps of:

(a) providing an adult cell derived from mammalian pancreatic islets or an explant culture of adult pancreatic islets with functional, wild-type Pax4; and

(b) detecting and isolating, from said adult cell or explant culture, β-cells that proliferate in response to the contact with (functional, wild-type) Pax4.

The above described "providing an adult cell derived from mammalian pancreatic islets or an explant culture of adult pancreatic islets with functional, wild-type Pax4" is not limited to the contact of a functional wild-type Pax4 to a single cell but also comprises the contact of whole islets which may, *inter alia*, be transplated.

The "detection and isolation, from said adult cell or explant culture, β-cells that proliferate in response to the contact with (functional, wild-type) Pax4" as carried out in the method of the invention may comprse the visualization of proliferative β-cells by methods which comprise microscopical means. Proliferative β-cells may also be detected by methods which comprise cell counts. Corresponding examples and further embodiments are given in the experimental part of this invention. Said "isolation" does not always comprise the physical separation from the originally used islet (islet cells) as employed in step (a) of the method of the present invention.

The term " adult cell" relates to a cell which is not in an embryonic or foetal stage and, most preferably is not in a post-natal stage.

The term "functional, wild-type Pax4" relates to Pax4 molecules which do not comprise a mutation, ion particular no mutation which leads to a disorder or a disease, like diabetes. Such wild-type Pax4 molecules are known in the art and, inter alia, described herein in SEQ ID NOS: 1-6. Said "functional, wild-type Pax4" also comprises, however, genetic variants as well as allelic variants of the wild-type Pax4 molecules described herein. As documented in the appended examples (employing non functional or detrimental mutants), the person skilled in the art is readily in a position to deduce whether a given Pax4 molecule is a "functional, wild-type molecule". Corresponding assays are shown in the appended examples.

In a particular embodiment of the method of the invention, the functional wild-type Pax4 is administered to the pancreatic cell, explant culture or isolated islet in form of a nucleic acid molecule. Yet, it is also envisaged that said functional wild-type Pax4 is administered to said cell, explant culture or isolated islet in form of a Pax4 gene expression product or a functional fragment thereof. Said Pax4 gene expression product may be an mRNA or a protein.

Most particularly, when said Pax4 is to be administered to the Pax4 to said cell, explant culture or isolated islet in form of a nucleic acid molecule, it is administered in form of a nucleic acid molecule comprised in a vector, whereby said vector is most particularly a viral vector. Said viral vector may, *inter alia*, be selected from the group from the group consisting of a retroviral vector, a lentiviral vector and an adenoviral vector. Also comprised is the use of vial particles, (like adenovial particles) or empty (adenoviral) capsids in the transfer of functional, wild-type Pax4 as desired herein. Corresponding virus-mediated co-internalization processes are known in the art and described for example in U.S. Patent 5,928,944. Further envisaged are AAV, Herpex simplex virus, pox virus, measles virus, vaccinia virus or Semliki forest virus. Also "bacterial shuttles" are envisaged in accordance with this invention. Nonlimiting examples of such shuttles correspondingly engineered *Salmonella typhimutium* and *Listeria monocytogenes.*

The methods described herein are not limited to the vectors comprising a nucleic acid molecule encoding wild-type Pax4. It is also envisaged that other means for gene transfer, for example device-mediated or chemically mediated gene delivery, like gene gun methods, ultrasound or lipofections are employed. Also "naked" wild-type encoding Pax4 molecules may be employed in accordance with the methods and uses of this invention. Such methods and uses comprise, *inter alia*, the use of plasmid DNA or RNA-transfer methods known in the art. Further methods to be employed comprise porlymer-based gene delivery systems, like, *e.g.*, polyethylenimines, histidin/lysine polymers (HK) and the like. In a particular embodiment of the invention, the Pax4 is employed in the method of this invention in form of a nucleic acid molecule comprised in a viral vector, particularly in an adenovial vector. The adenovial vectors presently used in gene therapy protocols lack most of the E1 region which renders the virus replication deficient. Most of the adenoviral vectors use din medical settings are also E3 deleted. The feasibility of gene transfer using these vectors have successfully shown in a variety of tissues *in vivo* and *in vitro*; U.S. Patent 6,099, 831 or U.S. Patent 6,013,638. As documented in the appended examples, adenoviral vector which may be employed in context of this invention are AdCMV or pHVAd2 as described in detail herein. Also RNA-inhibiting/RNA-interference approaches have been carried out in order to document the surprising and direct contribution of Pax4 on the survival of insulin-producing cells.

The following drawings form part of the present specification and are included to further demonstrate certain aspects of the present invention. The invention may be better understood by reference to one or more of these drawings in combination with the detailed description of specific embodiments presented herein.

**FIGS. 1A-D: Activin A and betacellulin increase Pax4 mRNA levels in a dose dependent manner in mature rat pancreatic islets.** (FIG. 1A) Low levels of Pax4 are expressed in adult rat islets. Quantitative real time RT-PCR using RNA purified from freshly isolated rat islets and INS-1E cells. Data are presented as percent of Pax4 mRNA levels relative to INS-1E. Each value (n = 6 independent experiments) represents the mean ± SE. Quantitative real-time RT-PCR analysis of PAX4 and insulin mRNA steady state levels in isolated rat islets treated with increasing doses of activin A (FIG. 1B), betacellulin (FIG. 1C) or TGF-β1 (FIG. 1D) for 24 h. Total RNA from 50 islets was extracted and reverse transcribed into cDNA as described in "experimental procedures". For each sample (20 ng), three distinct amplifications were performed in parallel and mean values for PAX4 and insulin were normalized to the mean value of the reference housekeeping gene *cyclophilin.* Results are expressed as the relative fold increase of the stimulated over the control group. Each value represents the mean ± SE of at least 4 independent experiments. *, $P< 0.05$.

**FIGS. 2A-B: Activin A and betacellulin increase β-cell proliferation in mature rat islets.** (FIG. 2A) Immunofluorescent detection of BrdU (first row), insulin (second row) and merged image including nuclear DAPI staining (third row) in dispersed islet cells 48 h after incubation with the indicated growth factors. The proliferation was measured using the BrdU incorporation assay as in FIGS. 4A-C. Magnification X 400. (FIG. 2B) β-cells immunostained for both insulin and BrdU were counted and results are expressed as a percentage of BrdU/insulin positive cells over the total number of insulin positive cells. Calculated values are depicted below the graph. Data represent the mean ± SE of 4 independent experiments, comprising more than 900 cells per condition. Statistical significance was tested between control islets and islets incubated with the various growth factors by Student's t test. **, $P< 0.01$

**FIGS. 3A-B: Pax4 is overexpressed in isolated rat pancreatic islets after infection with the recombinant adenovirus AdCMVPaX4IRESGFP.** (FIG. 3A) Immunofluorescent detection of EGFP (first row), insulin (second row) as well as nuclei staining with DAPI (third row) in dispersed islet cells 48 h after infection with the indicated doses of adenovirus. Pax4 overexpression is identified via the reporter co-translated EGFP in insulin positive cells. Of note, the viral infection does not induce nuclear damage as assessed by DAPI staining. Magnification X 400. (FIG. 3B) EMSA using a radiolabeled G3 element of the glucagon gene promoter and full-length mouse Pax4, produced *in vitro* with the coupled TNT system (lanes 1, 2, 3) as well as nuclear protein extracts (6 μg) from infected rat islets (lanes 4 to 8). Infection for 48 h with the indicated amounts of the adenovirus increased Pax4 DNA binding activity to the G3 element in a dose-dependent manner (lanes 5, 6, 7). The asterisk * delineates the formation of a supershift complex due to the addition of anti-Pax4 serum (lanes 2 and 8).

**FIGS.4A-C: Overexpression of Pax4 induces β-cell proliferation in AdCMVPaXIRESGFP-transduced rat islets.** (FIG. 4A) Immunocytochemical detection of BrdU (first row) and insulin (second row) in dispersed islet cells 48 h after infection with various recombinant adenoviruses (2.4 X $10^7$ pfu/ml). The merged image also includes DAPI staining (third row) to highlight nuclei. Cells were labeled with 10 pM BrdU for the last seven h of incubation and proliferation was visualized using the BrdU incorporation assay. Magnification X 400. (FIG. 4B) Merged image of BrdU (green in-colour print), insulin (red in colour print) and DAPI (blue in colour print) of intact islets infected with either AdCaLacZ or AdCMVPax4IRESGFP. (FIG. 4C) β-cells immunostained for both insulin and BrdU were counted under a fluorescent microscope. Results are expressed as a percentage of BrdU/insulin positive cells over the total amount of insulin positive cells and values are depicted below the graph. Data show the mean ± SE of 4 independent experiments, each representing more than 1000 cells per condition. Statistical significance was tested between LacZ- and the transcription factors Pax4-, Pax6- and neurogenin3 (Ngn3)-overexpressing islets by Student's t test **, P< 0.01

**FIGS. 5A-C: Time-dependent gene expression profiling of Pax4 overexpressing rat islets.** (FIG. 5A) EMSA using nuclear protein extracts (6 μg) from AdCMVPax4IRESGFP transduced rat islets cultured in RPMI 1640 medium over a period of 6 days. Pax4 DNA binding activity to the G3 element is maximal one-day post infection. The asterisk * represents the supershifted complex in the presence of anti-Pax4 serum. (FIGS. 5B-C Quantitative real-time RT-PCR analysis performed on RNA isolated from AdCaLacZ (LacZ)- (•) and AdCMVPaxIRESGFP (PAX4)- (■) infected islets (2.4 X $10^7$ pfu/ml). Transcript levels were grouped into 4 categories; proliferative genes comprising c-myc and Id2; apoptotic genes composed of Bcl-xL, Bcl-2 and caspase-3; the transcription factor Pdx-1 and finally endocrine hormone genes comprising insulin, glucagon and somatostatin. Expression patterns were measured over a period of 6 days. Each value represents mean ± SE of 3 independent experiments. Statistical significance was tested between LacZ and PAX4 infected islets by unpaired Student's t test. *, P< 0.05 **, P< 0.01.

**FIGS. 6A-C: Analysis of the expression and function of Pax4 wt and its mutant R129W.** (FIG. 6A) Immunofluorescent detection of the myc-tagged Pax4 or synaptotagmin VII proteins (first row) and nuclei staining with DAPI (second row) in INS1E cells 48 h after transfection with the indicated constructs. The transcription factor Pax4 and synaptotagmin VII were detected via the myc epitope in the nuclei and cytoplasm of INS1E cells, respectively; the R129W mutation does not affect Pax4 nuclear localisation. Magnification X 400. (FIG. 6B) EMSA using a radiolabeled G3 element of the glucagon gene promoter and the recombinant proteins Pax4-myc wt (lanes 1 and 2), and Pax4-myc R129W (lanes 3 and 4). An equal amount of protein, produced *in vitro* with the coupled TNT system, was applied in each lane. Wild-type Pax4 bound to the G3 element (lane 1) whereas the R129W mutation strongly decreased the DNA binding activity to the probe (lane 3). The asterisk * delineates the formation of a supershift complex due to the addition of anti-myc epitope antibody (lanes 2 and 4). (FIG. 6C) Effects of Pax4-myc wt (■) and its mutant R129W (□) on the human c-myc promoter and murine Bcl-xL promoter. Transient cotransfection studies using BHK-21 cells were performed with increasing amounts of wt and mutant Pax4. The telomerase promoter construct (▲) was used as an internal control in Pax4-myc wt-transfected BHK-21 cells. Data are presented as fold induction of basal luciferase activity and expressed as the mean ± SEM of 4-5 independent experiments. Pax4-myc transcriptionally activated the c-myc and Bcl-xL promoters whereas the mutant showed a significant reduction of transcriptional activation indicated by * (P< 0.05).

**FIGS.7A-B: Effects of Pax4 overexpression on insulin secretion and glucose oxidation in isolated rat islets.** (FIG. 7A) Glucose-induced insulin secretion was inhibited by AdCMVPax4IRESGFP in a dose dependent manner. Two days after infection, islet hormone secretion was assayed as described in experimental procedures. Data are expressed as the mean ± SEM of 4 independent experiments. ** = P< 0.01 as analysed unpaired Student's t-test. (FIG. 7B) Two days post infection with 2.4 X $10^7$ pfu/ml of indicated adenoviruses, islet $CO_2$ generation was measured in the presence of 2.5 mM or 16.7 mM glucose to assess glucose oxidation rate as described in the experimental procedures. Data represent the mean ± SEM of 5 independent experiments.

**FIGS. 8A-C: Both cellular ATP and mitochondrial calcium levels are increased in AdCMVPax4IRESGFP-infected** islets. (FIG. 8A) Total cellular ATP levels were measured in islets overexpressing either β-galactosidase or PAX4 (2.4 X $10^7$ pfu/ml) and maintained in 1 mM glucose for 10 min. Results represent the means ± SE. ***P*<0.01. (FIG. 8B) Cytosolic ATP production in response to 2.5 or 16.5 mM glucose was also determined over a period of 20 min using the ATP-sensitive bioluminescence probe luciferase. Luminescence was recorded in a FLUOstar Optima apparatus. Islets were equilibrated in KRBH buffer for 30 min prior to initiation of recording. Glucose and azide were added at indicated times (arrows). Results are the mean ± SE of at least 5 experiments performed in duplicates. (FIG. 8C) Mitochondrial calcium was monitored in LacZ or PAX4 overexpressing islets using β-cell specific/mitochondrial-targeted aeqourin. Islets infected with rAdRIPmAQ (4.8 X $10^8$ pfu/ml) and either AdCaLacZ or AdCMVPax4IRESGFP (2.4 X $10^7$ pfu/ml) were cultured for 65 h prior to experimentation. After the establishment of baseline luminescence (30 min; LacZ = 210 ± 49 nM and Pax4 = 387 ± 46 nM), islets were superfused for 5 min in basal conditions (0 glucose) before stimulation with glucose (16.7 mM), and then KC1 (60 mM), for 5 min intervals each, as shown (inset). The induced increases in $[Ca^{2+}]_m$ were evaluated on the basis of peak height and area under the peak (AUP). The elapsed time from the addition of glucose to the start of the calcium response (reaction time), and basal calcium (at 200-300 s) was also compared. Results for Pax4 overexpressing islets are expressed as a percentage of those for LacZ controls (100%). Each value represents the mean ± SEM of a minimum of 6 separate experiments. *= p<0.02, **=p<0.055.

**FIG. 9: Proposed model of Pax4-induced β-cell proliferation.** Based on these results, the inventors propose that mitogens such as activin A and betacellulin activate Pax4, which will stimulate c-myc and Bc1-xL gene transcription. c-myc will then promote Id2 gene expression and activate the cell cycle replication program. Bcl-xL increased expression will promote proliferation by preventing mitochondria from initiating the apoptotic program. However, cells become refractory to glucose-evoked insulin secretion due to altered ATP production and handling as well as calcium homeostasis.

**FIG. 10: Insulin and glucagon protein contents in Pax4 overexpressing islets.** Total insulin and glucagon protein contents were quantified by radioimmunoassay 48 h post infection and results were expressed as ng of protein per islet. Data show the mean ± SEM of 3 independent experiments.

**FIGS. 11A-B: Mitogens as well as Pax4 overexpression promotes human islet β-cell replication** (FIG. 11A) Quantitative real-time RT-PCR analysis of PAX4 mRNA steady state levels in isolated human islets treated with 11 or 25 mM glucose for 24 and 48 hours as well as with increasing doses of activin A, betacellulin or TGF-β1 for 24 h. Total RNA from 100 islets was extracted and reverse transcribed into cDNA. For each sample (20 ng), three distinct amplifications were performed in parallel and mean values for PAX4 were normalized to the mean value of the reference housekeeping gene *cyclophilin.* Results are expressed as the relative fold increase of the stimulated over the 5.5 mM glucose control group. (FIG. 11B) Immunocytochemical detection of BrdU (first row) in dispersed human islet cells 48 h after infection with various recombinant adenoviruses. The merged image includes DAPI staining (second row) to highlight nuclei. Magnification X 400.

**FIGS. 12A-B: Construction and analysis of the expression of mouse Pax4-myc wild-type (WT) and its mutant R129W.** (FIG. 12A) The full-length mouse Pax4 cDNA (represented schematically) was amplified by polymerase chain reaction (PCR) and subcloned into the expression vector pcDNA3.1/c-myc/6-His (Invitrogen). The myc-tagged was incorporated in order to detect Pax4 by immunofluorescence since available antibodies fail to recognise the transcription factor. The Pax4-myc wild-type (WT) was subjected to mutagenesis to substitute an arginine to a tryptophan at position 129 (Pax4-myc R129W). This mutation, located in the paired DNA binding domain, corresponds to the human Pax4 mutation R121W which has been associated with late onset type 2 diabetes. (FIG. 12B) EMSA was subsequently performed using recombinant Pax4-myc WT or R129W proteins generated *in vitro* in the presence of the glucagon promoter element G3, a *bona fide* Pax4 binding site. Wild-type Pax4 bound to the G3 element (lane 1) whereas the R129W mutation strongly decreased the DNA binding activity to the probe (lane 3). The asterisk * delineates the formation of a supershift complex due to the addition of anti-myc epitope antibody (lanes 2 and 4). Lane 5, c-myc epitope antibody.

**FIGS. 13A-B: Expression of Pax4 induces human islet cell proliferation in an adenoviral-mediated inducible expression system.** (FIG. 13A) Immunofluorescent detection of the myc-tagged Pax4 protein (Pax4 in first row), nuclei staining with DAPI (DAPI in second row) and merged image in dispersed human islet cells 48 hrs after infection with the recombinant adenovirus Ad-mPax4-myc WT (2.4 X $10^7$ pfu/ml) and the adenoviral construct harbouring the tetracycline transcriptional activator Ad-X Tet-On (1.2 X $10^7$ pfu/ml). Infected cells were cultured in the absence or presence of the inducer doxycycline (0.5 μg/ml). The transcription factor Pax4 was detected, via the c-myc epitope, in the nuclei of approximately 70% of human islet cells cultured in the presence of doxycycline, while no basal induction of Pax4 was observed in the absence of doxycycline. Nuclear fragmentation was absent in transduced cells indicating that viral infection did not affect cell viability or apoptosis. Magnification X 400. (FIG. 13B) Immunocytochemical detection of BrdU incorporation (first row), nuclei staining (second row) and merged image in human islet cells 48 hrs after infection with the adenoviral-mediated inducible expression system (see above) and cultured in the absence or presence of doxycycline (0.5 μg/ml). Cells were labeled with 10 μM BrdU for 48 hrs of incubation and proliferation was visualized using the BrdU incorporation assay. Forced expression of Pax4 induced proliferation in human islet cells. The merged image confirms the nuclear incorporation of BrdU. Magnification X 400.

**FIGS. 14A-B: Induction of mutant Pax4 R129W had no effect on human islet cell proliferation.** (FIG. 14A) Immunofluorescent detection of the c-myc-tagged mutant Pax4 R129W protein (Pax4 first row), nuclear DAPI staining (second row) and merged image in dispersed human islet cells 48 hrs after infection with the recombinant adenovirus Ad-mPax4-myc R129W (2.4 X $10^7$ pfu/ml) and the adenovirus Ad-X Tet-On (1.2 X $10^7$ pfu/ml). Infected cells were cultured in the absence or presence of doxycycline (0.5 μg/ml). The R129W mutation does not affect Pax4 nuclear localisation in infected human islet cells cultured in the presence of doxycycline. Magnification X 400. (FIG. 14B) Immunocytochemical detection of BrdU incorporation (green), nuclei staining (DAPI in blue) and merged image in human islet cells 48 hrs after infection with the adenoviral-mediated inducible expression system (see above) and cultured in the absence or presence of doxycycline (0.5 μg/ml). The proliferation was measured using the BrdU incorporation assay as in FIG. 2B. Magnification X 400.

**Fig. 15: Doxycycline-sfimulated Pax4 expression completely protects human islets from cytokine-induced apoptosis. Human** islets were infected with the recombinant adenovirus Ad-mPax4-myc WT and the adenovirus Ad-X Tet-On and cultured for 24 h in the absence or presence of the indicated concentrations of doxycycline. Islets were subsequently treated for 24h with IFN-γ, IL-1β and TNF-α(2ng/ml each) to promote apoptosis. (A) Immunocytochemical detection of apoptotic cells (first row) using TUNEL assay, nuclei staining (second row) and merged image in human islet cells 48 hrs after infection with the adenoviral-mediated inducible expression system and cultured in the absence or presence of doxycycline (0.5 μglml) in the presence of cytokines. The merged image confirms the nuclear incorporation of fluorescein in apoptotic cells. Magnification X 400. (B) Cell death was measured by the TUNEL assay and results were expressed as a percentage of fluorescein-labeled nuclei (TUNEL-positive cells) over the total amount of islet cells (nuclei staining by DAPI). Data show the mean ± SEM of four independent experiments, each representing more than 700 cells per condition *, $p<0.05$ and ** $p<0.01$.

**Fig. 16: Induction of mutant Pax4 R129W partially protects human islets from cytokine-induced apoptosis.** Human islets were infected with the recombinant adenovirus Ad-mPax4-myc R129W and the adenovirus Ad-X Tet-On as described in Materials and Methods and cultured for 24 h in the absence or presence of the indicated concentrations of doxycycline. Islets were subsequently treated for 24h with IFN-γ, IL-1β and TNF-α(2 ng/ml each) to promote apoptosis. (A) Immunocytochemical detection of apoptotic cells (first row) using TUNEL assay, nuclei staining (second row) and merged image in human islet cells 48 hrs after infection with the adenoviral-mediated inducible expression system and cultured in the absence or presence of doxycycline (0.5 μg/ml) in the presence of cytokines. The merged image confirms the nuclear incorporation of fluorescein in apoptotic cells. Magnification X 400. (B) Cell death was measured by the TUNEL assay and results were expressed as a percentage of fluorescein-labeled nuclei (TUNEL-positive cells) over the total amount of islet cells (nuclei staining by DAPI). Data show the mean ± SEM of four independent experiments, each representing more than 700 cells per condition *, $p<0.05$ and ** $p<0.01$.

**Fig. 17: Activin A and betacellulin increase endogenous Pax4 mRNA levels in human islets.** Pax4 mRNA levels in human islets treated with 0.5 nM of activin A, betacellulin or TGF-β1 in the presence of 5.5 or 11 mM glucose for 24 hours as indicated in the figure. Relative Pax4 mRNA abundance levels were measured by quantitative real-time RT-PCR and normalized to the transcript cyclophilin. Data represent the mean of 3-5 independent experiments.

**Fig. 18: Development of a RNA interference strategy (RNAi) and its impact an the expression of endogenous rat Pax4, Pdxl and Bcl-xL gene expression in INS-1E cells. (A) Schematic representation** of Pax4 protein structure depicting the two highly conserved DNA binding domains (paired and homeo domains) as well as a repressor/activator domain. The three Pax4 siRNA structures (two targeted to the paired domain, siPD21 and siPD29 and one to the homeo domain, siHD21) are represented with bars. Quantitative real-time RT-PCR analysis of rat Pax4 (B), PDX1 (C) and Bel-xL-(D) mRNA steady state levels in INS-1E cells co-transfected with an empty pDLDU6 vector (U6, control) or with a pDLDU6 vector containing the different Pax4 siRNA (siPD21, siPD29 or siHD21) along with an expression vector for GFP. Seventy two hours post-transfection, $GFP^-$ (white bars) and $GFP^+$ (black bars) cells were purified by FACS, RNA was extracted and Pax4, PDX1 as well as Bcl-xL mRNA steady state levels were evaluated by real time RT-PCR and normalized to the transcript cyclophilin. Results are the mean of 2 independent experiments performed in triplicates and are expressed as fold changes as compared to the $GFP^-$ cells.

**Fig. 19: INS-1E cells transfected with either siPD21, siPD29 or siHD21 are more sensitive to cytokine-induced cell death.** INS-1E cells were co-tranfected with the control vector (U6) or with the different Pax4 siRNA (siPD21, siPD29 or siHD21) along with a phogrin-GFP plasmid and were cultured for 48 hours. INS-1E cells were subsequently treated for 24h with IFN-γ, IL-1β and TNF-α (1 ng/ml each) to promote apoptosis. Immunocytochemical detection of apoptotic cells using TUNEL assay and phogrin-GFP expressing cells and merged image with nuclei staining. The merged image confirms the nuclear incorporation of rhodamin in apoptotic cells. Magnification X 400.

In accordance with the present invention it has been surprisingly found that Pax4, in contrast to the teaching of the prior art, is not only involved in β-cell differentiation during regeneration of β-cells or during embryogenesis, but that it can also successfully be used to initiate cell proliferation of pre-existing, particularly adult pancreatic β-cells. The advantage of the method of the present invention is the provision of larger quantities of pancreatic β-cells which may be used in particular transplantation approaches for the medical intervention in pancreatic diseases, in particular in diabetes. Results presented in the experimental part of this invention show that Pax4 promotes β-cell proliferation and is capable of preventing cell death, in particular by increased expression of the Bcl-xL gene. This documents that terminally differentiated adult β-cells retain a proliferative capacity and can be exploited as an alternative source for cell- or tissue-based therapy.

Polymorphisms in the *pax4* gene have been associated with Type 1 diabetes while point mutations have been linked to Type 2 diabetes, implicating Pax4 in mature β-cell function and/or regeneration. As documented in the appended examples, induction of endogenous Pax4 gene expression coincides with β-cell proliferation induced by the mitogens activin A and betacellulin in adult rat islets. Consistent with a proliferative role of Pax4, we also demonstrated using recombinant adenoviruses, that rat and human β-cells overexpressing Pax4 displayed greater replication rates as compared to control-infected islets. In context of the present invention the impact of mitogens on endogenous Pax4 expression both at the transcript and protein level in human islets was evaluated. Furthermore, in order to evaluate the direct contribution of Pax4 on β-cell plasticity, a RNA interference (RNAi) strategy to suppress Pax4 activation in response to mitogens; see also Example 11.

Immunofluorescence studies were performed on partially trypsinized human islets for the transcription factors Pax4, Pdx1, Nkx6.1, Isl1, Ngn3 and insulin. Isolated human islets were exposed to either 5.5 or 11 mM glucose for 24 and 48 hours. Islets were also treated with 0.5 nM of activin A, betacellulin or TGF-β1 for 24 hours. Steady state mRNA levels for Pax4 were quantified by quantitative real-time RT-PCR and normalized to cyclophilin. A 21-nucleotide Pax4 hairpin RNA structures (siPax4) was cloned into the pDLDU6 vector and transfected into the rat insulinoma cell line INS1E along with GFP using lipofectamine. Subsequent to cell sorting using GFP (72 hours post-transfection), the effects of RNAi on endogenous Pax4 transcript levels were quantified by real time RT-PCR.

A comparative profile of transcription factors in human islets was performed initially to establish relative expression levels of Pax4. Low but consistent levels of Pax4 mRNA and protein were detected in isolated human islets as compared to Nkx6.1, Pdx1 and Isl1. In contrast, Ngn3 was undetectable. In parallel, it was found that exposure to 11 mM glucose for 48 hours resulted in a 3.6-fold increase in Pax4 mRNA levels as compared to control 5.5 mM glucose. Treatment with either 0.5 nM activin A or betacellulin for 24 hours resulted in a 3.5-and 8 fold increase in Pax4 transcript, respectively. In contrast, TGF-β1 was ineffective. Accordingly, Pax4 activity is regulated by physiological stimuli in human islets. The insulinoma INS1E cells expressed high levels of Pax4 mRNA. Pax4 steady state mRNA levels were lowered by 80% in INSIE cells co-expressing GFP and an interfering/inhibiting RNA, namely siPax4 (see also Example 11). Repression was specific since mRNA levels for PDX1 and insulin remained constant

Therefore, Pax4 is induced by mitogens known to promote pancreatic islet cell proliferation.

As will be detailed herein below, in particular *ex vivo* gene delivery of Pax4 into terminally differentiated adult β-cells can be employed to expand β-cells in culture and to reduce the number of human islets required for successful transplantation.

### I. Pax4 Proteins and Nucleic Acids

The functional, wild-type Pax4 to be particularly used in context of this invention is the wild-type Pax4 of mouse, rat or human. Most particularly, the functional, wild-type Pax4 to be employed is human Pax4. Corresponding moleceules (*i.e.*, nucleotide sequences and amino acid sequences) are known in the art and also shown herein below. Accordingly, in a particular embodiment of the invention, the functional, wild-type Pax4 to be employed in the method and uses provided herein is encoded by

(a) a nucleic acid molecule comprising a nucleic acid molecule encoding the polypeptide having the amino acid sequence as shown in SEQ ID NO: 2 (rat), 4 (mouse), or 6 (human);
(b) a nucleic acid molecule comprising a nucleic acid molecule having the DNA sequence as shown in SEQ ID NO: 1 (rat), 3 (mouse), or 5 (human);
(c) a nucleic acid molecule hybridizing to the complementary strand of nucleic acid molecules of (a) or (b) and encoding a functional wild-type Pax4; and
(d) a nucleic acid molecule being degenerate as a result of the genetic code to the nucleotide sequence of the nucleic acid molecule as defined in (c).

In accordance with this invention, also Pax4 molecules may be employed which are highly homologous to the functional, wild-type Pax4 molecules known in the art and disclosed herein. Yet, this molecules have to be functional in the sense that these molecules do stimulate the proliferation of β-cells as documented herein and that these functional molecules do not provoke detrimental effects as shown with detrimental mutant forms presented in the experimental part of this invention. Accordingly, the invention also provides for a "read-out system" whether a give Pax4 molecule is "functional", *i.e.*, capable of stimulating β-cell proliferation of adult (islet) cells. Such "functional, wild-type Pax4 molecules are known in the art, and comprise, but are not limited to the sequences provided herein or disclosed in U.S. Patent 6,071,697. Further "functional, wild-type Pax4" molecules are encoded by nucleic acid molecules as provided in the GeneBank under accession numbers: (rat Pax4) NM_031799 (mousePax4) NM_011038 and (humanPax4): AF043978 or NM_006193.

In order to determine whether a nucleic acid sequence has a certain degree of identity to the nucleic acid sequence encoding a wild-type Pax4 as defined above, the skilled person can use means and methods well-known in the art, e.g., alignments, either manually or by using computer programs such as those mentioned further down below in connection with the definition of the term "hybridization" and degrees of homology.

For example, BLAST2.0, which stands for Basic Local Alignment Search Tool (Altschul, 1997; Altschul, 1993); Altschul, 1990), can be used to search for local sequence alignments. BLAST produces alignments of both nucleotide and amino acid sequences to determine sequence similarity. Because of the local nature of the alignments, BLAST is especially useful in determining exact matches or in identifying similar sequences. The fundamental unit of BLAST algorithm output is the High-scoring Segment Pair (HSP). An HSP consists of two sequence fragments of arbitrary but equal lengths whose alignment is locally maximal and for which the alignment score meets or exceeds a threshold or cutoff score set by the user. The BLAST approach is to look for HSPs between a query sequence and a database sequence, to evaluate the statistical significance of any matches found, and to report only those matches which satisfy the user-selected threshold of significance. The parameter E establishes the statistically significant threshold for reporting database sequence matches. E is interpreted as the upper bound of the expected frequency of chance occurrence of an HSP (or set of HSPs) within the context of the entire database search. Any database sequence whose match satisfies E is reported in the program output

Analogous computer techniques using BLAST (Altschul (1997), *loc. cit.*; Altschul (1993), loc. cit.; Altschul (1990), loc. cit.) are used to search for identical or related molecules in nucleotide databases such as GenBank or EMBL. This analysis is much faster than multiple membrane-based hybridizations. In addition, the sensitivity of the computer search can be modified to determine whether any particular match is categorized as exact or similar. The basis of the search is the product score which is defined as:

$$\frac{\%\text{sequence identity x } \%\text{ maximum BLAST score}}{100}$$

and it takes into account both the degree of similarity between two sequences and the length of the sequence match. For example, with a product score of 40, the match will be exact within a 1-2% error; and at 70, the match will be exact. Similar molecules are usually identified by selecting those which show product scores between 15 and 40, although lower scores may identify related molecules.

The present invention also uses in the inventive method wild-type Pax4 nucleic acid molecules which hybridize to one of the above described nucleic acid molecules and which encode a functional Pax4 molecule as described herein, *i.e.*, a Pax4 molecules which does not lead to disorders or diseases, like diabetes.

The term "hybridizes" as used in accordance with the present invention may relate to hybridization under stringent or non-stringent conditions. If not further specified, the conditions are particularly non-stringent. Said hybridization conditions may be established according to conventional protocols described, for example, in Sambrook, (2001); Ausubel, (1996), or Higgins and Hames, (1985). The setting of conditions is well within the skill of the artisan and can be determined according to protocols described in the art. Thus, the detection of only specifically hybridizing sequences will usually require stringent hybridization and washing conditions such as 0.1xSSC, 0.1% SDS at 65°C. Non-stringent hybridization conditions for the detection of homologous or not exactly complementary sequences may be set at 6xSSC, 1% SDS at 65°C. As is well known, the length of the probe and the composition of the nucleic acid to be determined constitute further parameters of the hybridization conditions. Note that variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations.

The inclusion of specific blocking reagents may require modification of the hybridization conditions described above, due to problems with compatibility. Hybridizing nucleic acid molecules also comprise fragments of the above described molecules. Such fragments may represent nucleic acid sequences which encode wild-type Pax4 which lacks the repressor region, as inter alia, encoded by the nucleic acid molecule shown in SEQ ID No. 13. Accordingly, envisaged in context of this invention is a wild-type Pax4 molecule which lacks the repressor domain. Such a wild-type Pax4 molecule may be encoded by a (human) Pax4 nucleic acid molecule as shown below (SEQ ID NO. 13) lacking the repressor domain. Such a construct may show high proliferative capacity on human islet β-cells.

Furthermore, nucleic acid molecules which hybridize with any of the aforementioned nucleic acid molecules also include complementary fragments, derivatives and allelic variants of these molecules as long as these molecules are capable of inducing β-cell proliferation of in adult pancreatic cells and do not lead to disorders like diabetes. Additionally, a hybridization complex refers to a complex between two nucleic acid sequences by virtue of the formation of hydrogen bonds between complementary G and C bases and between complementary A and T bases; these hydrogen bonds may be further stabilized by base stacking interactions. The two complementary nucleic acid sequences hydrogen bond in an antiparallel configuration. A hybridization complex may be formed in solution (*e.g.*, Cot or Rot analysis) or between one nucleic acid sequence present in solution and another nucleic acid sequence immobilized on a solid support (*e.g.*, membranes, filters, chips, pins or glass slides to which, *e.g.*, cells have been fixed). The terms complementary or complementarity refer to the natural binding of polynucleotides under permissive salt and temperature conditions by base-pairing. For example, the sequence "A-G-T" binds to the complementary sequence "T-C-A". Complementarity between two single-stranded molecules may be "partial", in which only some of the nucleic acids bind, or it may be complete when total complementarity exists between single-stranded molecules. The degree of complementartity between nucleic acid strands has significant effects on the efficiency and strength of hybridization between nucleic acid strands. This is of particular importance in amplification reactions, which depend upon binding between nucleic acids strands.

The term "hybridizing sequences" particularly refers to sequences which display a sequence identity of at least 40%, particularly at least 50%, more particularly at least 60%, even more particularly at least 70%, particularly at least 80%, more particularly at least 90%, even more particularly at least 95%, 97% or 98% and most particularly at least 99% identity with a nucleic acid sequence as described above encoding a wild-type Pax4.

In accordance with the present invention, the term "identical" or "percent identity" in the context of two or more nucleic acid or amino acid sequences, refers to two or more sequences or subsequences that are the same, or that have a specified percentage of amino acid residues or nucleotides that are the same (*e.g.*, 70-95% identity, more particularly at least 95%, 97%, 98% or 99% identity), when compared and aligned for maximum correspondence over a window of comparison, or over a designated region as measured using a sequence comparison algorithm as known in the art, or by manual alignment and visual inspection. Sequences having, for example, 95% or greater sequence identity are considered to be substantially identical. Such a definition also applies to the complement of a test sequence. Those having skill in the art will know how to determine percent identity between/among sequences using, for example, algorithms such as those based on CLUSTALW computer program (Thompson, 1994; or FASTDB (Brutlag, 1990, as known in the art.

Recombinant vectors form important further aspects of the present invention. The term "expression vector or construct" means any type of genetic construct containing a nucleic acid coding for a gene product in which part or all of the nucleic acid encoding sequence is capable of being transcribed. Particularly useful vectors are contemplated to be those vectors in which the coding portion of the DNA segment, whether encoding a full length protein or smaller peptide, is positioned under the transcriptional control of a promoter. A "promoter" refers to a DNA sequence recognized by the synthetic machinery of the cell, or introduced synthetic machinery, required to initiate the specific transcription of a gene. The phrases "operatively positioned", "under control" or "under transcriptional control" means that the promoter is in the correct location and orientation in relation to the nucleic acid to control RNA polymerase initiation and expression of the gene.

The promoter may be in the form of the promoter that is naturally associated with a particular gene, as may be obtained by isolating the 5' non-coding sequences located upstream of the coding segment or exon, for example, using recombinant cloning and/or polymerase chain reaction (PCR™) technology, in connection with the compositions disclosed herein (PCR technology is disclosed in U.S. Patent 4,683,202 and U.S. Patent 4,682,195, each incorporated herein by reference).

In other embodiments, it is contemplated that certain advantages will be gained by positioning the coding DNA segment under the control of a recombinant, or heterologous, promoter. As used herein, a recombinant or heterologous promoter is intended to refer to a promoter that is not normally associated with a particular gene in its natural environment. Such promoters may include promoters normally associated with other genes, and/or promoters isolated from any other bacterial, viral, eukaryotic, or mammalian cells.

Naturally, it will be important to employ a promoter that effectively directs the expression of the DNA segment in the cell type chosen for expression. The use of promoter and cell type combinations for protein expression is generally known to those of skill in the art of molecular biology, for example, see Sambrook *et al.* (2001), incorporated herein by reference. The promoters employed may be constitutive, or inducible, and can be used under the appropriate conditions to direct high level expression of the introduced DNA segment, such as is advantageous in the large-scale production of recombinant proteins or peptides.

### A. Promoters

The promoter is required to express the transforming genetic construct to a degree sufficient to effect transformation of a target cell type amongst a population of different cell types such that the transformed target cell results in the generation of a stable human regulated secretory cell. Promoters can be classified into two groups, ubiquitous and tissue- or cell-specific. Ubiquitous promoters activate transcription in all or most tissues and cell types. Examples of ubiquitous promoters are cellular promoters like the histone promoters, promoters for many metabolic enzyme genes such as hexokinase I and glyceraldehyde-3-phosphate dehydrogenase, and many viral promoters such as the cytomegalovirus promoter-(CMVp) and the Rous sarcoma virus promoter (RSVp). In certain aspects of the present invention, these promoters are appropriate for use with the immortalizing constructs described herein, as well as finding use in additional aspects of the present invention.

Tissue- or cell-specific promoters activate transcription in a restricted set of tissues or cell types or, in some cases, only in a single cell type of a particular tissue. Examples of stringent cell-specific promoters are the insulin gene promoters which are expressed in only a single cell type (pancreatic β-cells) while remaining silent in all other cell types, and the immunoglobulin gene promoters which are expressed only in cell types of the immune system.

The promoter may also be "context specific" in that it will be expressed only in the desired cell type and not in other cell types that are likely to be present in the population of target cells, *e.g.*, it will be expressed in β-cells, but not in α- or δ-cells, when introduced into intact human islets. For example, an insulin promoter targets the expression of a linked transforming oncogene selectively to β-cells of a human islet preparation even though many other contaminating cell types exist in the preparation.

#### 1. β-Cell-Specific Promoters

It has been documented that the two rat insulin gene promoters, RIP1 (GenBank accession number J00747) and RIP2 (GenBank accession number J00748), as well as the human insulin promoter (HIP; GenBank accession number V00565), direct stringent cell-specific expression of the insulin gene in rodent β-cell insulinoma lines (German *et al.*, 1990), primary islet cells (Melloul *et al.,* 1993), and in β-cells of transgenic mice (Efrat *et al.,* 1988).

As the sequence and position of the functional promoter elements are well conserved between HIP, RIP1 and RIP2, the transcription factors that interact with these elements are likely to be conserved across species. In fact, HIP can direct cell-specific expression of linked genes in rodent β-cell lines and rat primary islets, albeit, at a somewhat lower level than that observed for RIP1 (Melloul *et al.,* 1993). Melloul *et al.* (1993) demonstrated that the isolated 50-bp RIP1 FAR/FLAT minienhancer (FF), an essential promoter element for RIP1 activity, could express a linked reporter gene in both adult rat and human islet cells. Furthermore, FF activity could be substantially induced by increased concentrations of glucose in both species of adult islets. Additional results from gel-shift studies strongly suggested that the same or similar β-cell-specific transcription factor(s) from both adult rat and human islet cell nuclear extracts bound to conserved sequences contained within both the RIP1 FF and the analogous region of HIP.

#### 2. Modified Promoters

Promoters can be modified in a number of ways to increase their transcriptional activity. Multiple copies of a given promoter can be linked in tandem, mutations which increase activity may be introduced, single or multiple copies of individual promoter elements may be attached, parts of unrelated promoters may be fused together, or some combination of all of the above can be employed to generate highly active promoters. All such methods are contemplated for use in connection with the present invention.

German *et al.* (1992a) mutated three nucleotides in the transcriptionally important FLAT E box of the rat insulin I gene promoter (RIP), resulting in a three- to four-fold increase in transcriptional activity of the mutated RIP compared to that of a nonmutated RIP as assayed in transiently transfected HIT cells. Also, the introduction of multiple copies of a promoter element from the *E. coli* tetracycline resistance operon promoter were introduced into the CMV promoter, significantly increasing the activity of this already very potent promoter (Liang *et al.*, 1996). Additionally, part of the CMV promoter, which has high but short-lived transcriptional activity in dog myoblasts, was linked to the muscle-specific creatine kinase promoter (MCKp), which has weak but sustained expression in dog myoblasts, resulting in a hybrid promoter that sustained high-level expression for extended periods in dog myoblasts.

### 3. Multimerized Promoters

Several modified rat insulin promoters (modRIP) containing multimerized enhancer elements have been engineered. The currently preferred modRIP contains six multimerized repeats of a 50 base pair region of the *cis* acting enhancer of RIP, placed upstream of an intact copy of RIP. These novel promoters have been shown to direct expression of transgenes in stably engineered β-cell lines at levels above those attained with unmodified insulin promoters and, in some cases, approaching the levels achieved with the cytomegalovirus promoter (CMVp). CMVp is one of the strongest activating promoters known, but in a very non-tissue specific manner.

## B. DNA Delivery

In certain embodiments of the invention, the nucleic acid encoding the one or more product(s) of interest may be integreated into the host cell's genome. In yet further embodiments, the nucleic acid may be stably maintained in the cell as a separate, episomal segment of DNA. Such nucleic acid segments or "episomes" encode sequences sufficient to permit maintenance and replication independent of or in synchronization with the host cell cycle. All delivery methods are contemplated for use in the context of the present invention, although certain methods are preferred, as outlined below.

### 1. Transfection

In order to effect expression, the construct must be delivered into a cell. As described below, the preferred mechanism for delivery is via viral infection, where the construct is encapsidated in an infectious viral particle. However, several non-viral methods for the transfer of one or more immortalizing or other expression constructs into cultured mammalian cells also are contemplated by the present invention. In one embodiment of the present invention, the expression construct may consist only of naked recombinant DNA or plasmids. Transfer of the construct may be performed by any of the methods mentioned which physically or chemically permeabilize the cell membrane.

In a further embodiment of the invention, the expression construct may be entrapped in a liposome. Liposomes are vesicular structures characterized by a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo self-rearrangement before the formation of closed structures and entrap water and dissolved solutes between the lipid bilayers (Ghosh and Bachhawat, 1991). Also contemplated is an expression construct complexed with Lipofectamine (Gibco BRL).

Liposome-mediated nucleic acid delivery and expression of foreign DNA *in vitro* has been very successful (Nicolau and Sene, 1982; Fraley *et al.,* 1979; Nicolau *et al.,* 1987). Wong *et al.* (1980) demonstrated the feasibility of liposome-mediated delivery and expression of foreign DNA in cultured chick embryo, HeLa and hepatoma cells.

In certain embodiments of the method of the present invention, the liposome may be complexed with a hemagglutinating virus (HVJ). This has been shown to facilitate fusion with the cell membrane and promote cell entry of liposome-encapsulated DNA (Kaneda *et al.,* 1989). In other embodiments, the liposome may be complexed or employed in conjunction with nuclear non-histone chromosomal proteins (HMG-1) (Kato *et al.*, 1991). In yet further embodiments, the liposome may be complexed or employed in conjunction with both HVJ and HMG-1.

Melloul *et al.* (1993) demonstrated transfection of both rat and human islet cells using liposomes made from the cationic lipid DOTAP, and Gainer *et al.* (1996) transfected mouse islets using Lipofectamine-DNA complexes.

In certain embodiments of the method of the present invention, the expression construct is introduced into the cell via electroporation. Electroporation involves the exposure of a suspension of cells and DNA to a high-voltage electric discharge.

Transfection of eukaryotic cells using electroporation has been quite successful. Mouse pre-B lymphocytes have been transfected with human kappa-immunoglobulin genes (Potter *et al.*, 1984), and rat hepatocytes have been transfected with the chloramphenicol acetyltransferase gene (Tur-Kaspa *et al.,* 1986) in this manner. Examples of electroporation of islets include Soldevila *et al.* (1991) and PCT application WO 91/09939.

In other embodiments of the method of the present invention, the expression construct is introduced to the cells using calcium phosphate precipitation. Human KB cells have been transfected with adenovirus 5 DNA (Graham and Van Der Eb, 1973) using this technique. Also in this manner, mouse L(A9), mouse C127, CHO, CV-1, BHK, NIH3T3 and HeLa cells were transfected with a neomycin marker gene (Chen and Okayama, 1987), and rat hepatocytes were transfected with a variety of marker genes (Rippe *et al*., 1990).

In another embodiment, the expression construct is delivered into the cell using DEAE-dextran followed by polyethylene glycol. In this manner, reporter plasmids were introduced into mouse myeloma and erythroleukemia cells (Gopal, 1985).

Another embodiment of the method of the present invention for transferring one or more naked DNA immortalizing or other expression construct into cells may involve particle bombardment. This method depends on the ability to accelerate DNA-coated microprojectiles to a high velocity allowing them to pierce cell membranes and enter cells without killing them (Klein *et al.,* 1987). Several devices for accelerating small particles have been developed. One such device relies on a high voltage discharge to generate an electrical current, which in turn provides the motive force (Yang *et al*., 1990). The microprojectiles used have consisted of biologically inert substances such as tungsten or gold beads.

Gainer *et al.* (1996) have transfected mouse islets with a luciferase gene/human immediate early promoter reporter construct, using biolistic particles accelerated by helium pressure.

Further embodiments of the method of the present invention include the introduction of the expression construct by direct microinjection or sonication loading. Direct microinjection has been used to introduce nucleic acid constructs into *Xenopus* oocytes (Harland and Weintraub, 1985), and LTK$^-$ fibroblasts have been transfected with the thymidine kinase gene by sonication loading (Fechheimer *et al.,* 1987).

In certain embodiments of the method of the present invention, the expression construct is introduced into the cell using adenovirus assisted transfection. Increased transfection efficiencies have been reported in cell systems using adenovirus coupled systems (Kelleher and Vos, 1994; Cotten *et al.,* 1992; Curiel, 1994), and the inventors contemplate using the same technique to increase transfection efficiencies into human islets.

Still further constructs that may be employed to deliver the one or more immortalizing or other expression construct to the target cells are receptor-mediated delivery vehicles. These take advantage of the selective uptake of macromolecules by receptor-mediated endocytosis that will be occurring in the target cells. In view of the cell type-specific distribution of various receptors, this delivery method adds another degree of specificity to the present invention. Specific delivery in the context of another mammalian cell type is described by Wu and Wu (1993); incorporated herein by reference).

Certain receptor-mediated gene targeting vehicles comprise a cell receptor-specific ligand and a DNA-binding agent Others comprise a cell receptor-specific ligand to which the DNA construct to be delivered has been operatively attached. Several ligands have been used for receptor-mediated gene transfer (Wu and Wu, 1987, 1988; Wagner *et al*., 1990; Ferkol *et al.,* 1993; Perales *et al*., 1994; Myers, EPO 0273085), which establishes the operability of the technique. In the context of the present invention, the ligand will be chosen to correspond to a receptor specifically expressed on the neuroendocrine target cell population.

In other embodiments, the DNA delivery vehicle component of a cell-specific gene targeting vehicle may comprise a specific binding ligand in combination with a liposome. The nucleic acids to be delivered are housed within the liposome and the specific binding ligand is functionally incorporated into the liposome membrane. The liposome will thus specifically bind to the receptors of the target cell and deliver the contents to the cell. Such systems have been shown to be functional using systems in which, for example, epidermal growth factor (EGF) is used in the receptor-mediated delivery of a nucleic acid to cells that exhibit upregulation of the EGF receptor.

In still further embodiments, the DNA delivery vehicle component of the targeted delivery vehicles may be a liposome itself, which will preferably comprise one or more lipids or glycoproteins that direct cell-specific binding. For example, Nicolau *et al.* (1987) employed lactosyl-ceramide, a galactose-terminal asialganglioside, incorporated into liposomes and observed an increase in the uptake of the insulin gene by hepatocytes. It is contemplated that the one or more immortalizing or other expression constructs of the present invention can be specifically delivered into the target cells in a similar manner.

### 2. Viral Infection

The vector expressing functional wild-type Pax4 to be used in an *in vitro* method of the invention or to be used in a pharmaceutical composition or a method of treatment in accordance with this invention, is particularly is a viral vector. Yet, also other systems for gene transfer as described above are envisaged in context of this invention. The viral vector may be a vector as discussed above but is particularly selected from the group consisting of an adenoviral vector, a retroviral vector or an lentiviral vector.

One of the preferred methods for delivery of expression constructs involves the use of an adenovirus expression vector. Although adenovirus vectors are known to have a low capacity for integration into genomic DNA, this feature is counterbalanced by the high efficiency of gene transfer afforded by these vectors. "Adenovirus expression vector" is meant to include those constructs containing adenovirus sequences sufficient to (a) support packaging of the construct and (b) to ultimately express a tissue-specific transforming construct that has been cloned therein.

The expression vector comprises a genetically engineered form of adenovirus. Knowledge of the genetic organization of adenovirus, a 36 kb, linear, double-stranded DNA virus, allows substitution of large pieces of adenoviral DNA with foreign sequences up to 7 kb (Grunhaus and Horwitz, 1992). In contrast to retrovirus, the adenoviral infection of host cells does not result in chromosomal integration because adenoviral DNA can replicate in an episomal manner without potential genotoxicity. Also, adenoviruses are structurally stable, and no genome rearrangement has been detected after extensive amplification.

Adenovirus is particularly suitable for use as a gene transfer vector because of its mid-sized genome, ease of manipulation, high titer, wide target-cell range and high infectivity. Both ends of the viral genome contain 100-200 base pair inverted repeats (ITRs), which are *cis* elements necessary for viral DNA replication and packaging. The early (E) and late (L) regions of the genome contain different transcription units that are divided by the onset of viral DNA replication. The E1 region (E1A and E1B) encodes proteins responsible for the regulation of transcription of the viral genome and a few cellular genes. The expression of the E2 region (E2A and E2B) results in the synthesis of the proteins for viral DNA replication. These proteins are involved in DNA replication, late gene expression and -host cell shut-off (Renan, 1990). The products of the late genes, including the majority of the viral capsid proteins, are expressed only after significant processing of a single primary transcript issued by the major late promoter (MLP). The MLP (located at 16.8 m.u.) is particularly efficient during the late phase of infection, and all the mRNA's issued from this promoter possess a 5'-tripartite leader (TPL) sequence which makes them preferred mRNA's for translation.

In a current system, recombinant adenovirus is generated from homologous recombination between shuttle vector and provirus vector. Due to the possible recombination between two proviral vectors, wild-type adenovirus may be generated from this process. Therefore, it is critical to isolate a single clone of virus from an individual plaque and examine its genomic structure.

Generation and propagation of the current adenovirus vectors, which are replication deficient, depend on a unique helper cell line, designated 293, which was transformed from human embryonic kidney cells by Ad5 DNA fragments and constitutively expresses E1 proteins (Graham *et al.*, 1977). Since the E3 region is dispensable from the adenovirus genome (Jones and Shenk, 1978), the current adenovirus vectors, with the help of 293 cells, carry foreign DNA in either the E1, the E3, or both the E1 and E3 regions (Graham and Prevec, 1991). In nature, adenovirus can package approximately 105% of the wild-type genome (Ghosh-Choudhury *et al.*, 1987), providing capacity for about 2 kb of extra DNA. Combined with the approximately 5.5 kb of DNA that is replaceable in the E1 and E3 regions, the maximum capacity of the current adenovirus vector is under 7.5 kb, or about 15% of the total length of the vector. More than 80% of the adenovirus viral genome remains in the vector backbone.

Helper cell lines may be derived from human cells such as human embryonic kidney cells, muscle cells, hematopoietic cells or other human embryonic mesenchymal or epithelial cells. Alternatively, the helper cells may be derived from the cells of other mammalian species that are permissive for human adenovirus. Such cells include, *e.g.,* Vero cells or other monkey embryonic mesenchymal or epithelial cells. As stated above, the preferred helper cell line is 293.

Racher *et al.* (1995) disclosed improved methods for culturing 293 cells and propagating adenovirus. In one format, natural cell aggregates are grown by inoculating individual cells into 1 liter siliconized spinner flasks (Techne, Cambridge, UK) containing 100-200 ml of media. Following stirring at 40 rpm, the cell viability is estimated with trypan blue. In another format, Fibra-Cel microcarriers (Bibby Sterlin, Stone, UK) (5 g/l) is employed as follows. A cell inoculum, resuspended in 5 ml of media, is added to the carrier (50 ml) in a 250 ml Erlenmeyer flask and left stationary, with occasional agitation, for 1 to 4 h. The media is then replaced with 50 ml of fresh media and shaking initiated. For virus production, cells are allowed to grow to about 80% confluence, after which time the media is replaced (to 25% of the final volume) and adenovirus added at an MOI of 0.05. Cultures are left stationary overnight, following which the volume is increased to 100% and shaking commenced for another 72 h.

Other than the requirement that the adenovirus vector be replication defective, or at least conditionally defective, the nature of the adenovirus vector is not believed to be crucial to the successful practice of the invention. The adenovirus may be of any of the 42 different known serotypes or subgroups A-F. Adenovirus type 5 of subgroup C is the preferred starting material in order to obtain the conditional replication-defective adenovirus vector preferred for use in the present invention. This is because Adenovirus type 5 is a human adenovirus about which a great deal of biochemical and genetic information is known, and it has historically been used for most constructions employing adenovirus as a vector.

As stated above, the typical vector according to the present invention is replication defective and will not have an adenovirus E1 region. Thus, it will be most convenient to introduce the transforming construct at the position from which the E1-coding sequences have been removed. However, the position of insertion of the construct within the adenovirus sequences is not critical to the invention. The polynucleotide encoding the gene of interest may also be inserted in lieu of the deleted E3 region in E3 replacement vectors as described by Karlsson *et al.* (1986) or in the E4 region where a helper cell line or helper virus complements the E4 defect.

Adenovirus growth and manipulation is known to those of skill in the art, and exhibits broad host range *in vitro* and *in vivo*. This group of viruses can be obtained in high titers, *e.g.*, $10^9$-$10^{11}$ plaque-forming units per ml, and they are highly infective. The life cycle of adenovirus does not require integration into the host cell genome. The foreign genes delivered by adenovirus vectors are episomal and, therefore, have low genotoxicity to host cells. No side effects have been reported in studies of vaccination with wild-type adenovirus (Couch *et al.,* 1963; Top *et al.*, 1971), demonstrating their safety and therapeutic potential as *in vivo* gene transfer vectors.

Adenovirus vectors have been used in eukaryotic gene expression (Levrero *et al.*, 1991; Gomez-Foix *et al.*, 1992) and vaccine development (Grunhaus and Horwitz, 1992; Graham and Prevec, 1992). Recently, animal studies suggested that recombinant adenovirus could be used for gene therapy (Stratford-Perricaudet and Perncaudet, 1991; Stratford-Perricaudet *et al.*, 1990; Rich *et al.*, 1993). Studies in administering recombinant adenovirus to different tissues include trachea instillation (Rosenfeld *et al.,* 1991; Rosenfeld *et al.,* 1992), muscle injection (Ragot *et al.,* 1993), peripheral intravenous injections (Herz and Gerard, 1993) and stereotactic inoculation into the brain (Le Gal La Salle *et al.,* 1993).

Recombinant adenovirus and adeno-associated virus (see below) can both infect and transduce non-dividing human primary cells. Gene transfer efficiencies of approximately 70% for isolated rat islets have been demonstrated by the inventors (Becker *et al.*, 1994a; Becker *et al.,* 1994b; Becker *et al.*, 1996) as well as by other investigators (Gainer *et al.,* 1996).

Known adenoviral vector which are routinely employed in medical settings compriseAd2 or Ad5. Herein below are adenoviral vectors described which may, *inter alia,* be used in accordance with this invention. Such vectors are, *inter alia,* adenoviral vector comprising a DNA as shown in SEQ ID NOS: 12 or 14. Accordingly, the invention also provides for an adenoviral vector system expressing function, wild-type Pax4, wherein said adenoviral vector system has the nucleic acid sequence as shown in SEQ ID NO: 15, which is a Ad2-vecor expressing human wild-type Pax4 (pHP_Ad2_Pax4; human) or as shown in SEQ ID NO. 16, which is a further adenoviral vector expressing human wild-type Pax4. Both adenoviral vectors also express a myc-tag. Additional "tags" are particularly useful in the vector systems provided herein since they allow for screening of cells and or tissues which have successfully been contacted with exogenous wild-type Pax4.

Adeno-associated virus (AAV) is an attractive vector system for use in the human cell transformation of the present invention as it has a high frequency of integration and it can infect nondividing cells, thus making it useful for delivery of genes into mammalian cells in tissue culture (Muzyczka, 1992). AAV has a broad host range for infectivity (Tratschin, *et al.*, 1984; Laughlin, *et al.*, 1986; Lebkowski, *et al.,* 1988; McLaughlin, *et al.,* 1988), which means it is applicable for use with human neuroendocrine cells, however, the tissue-specific promoter aspect of the present invention will ensure specific expression of the transforming construct in aspects of the invention where this is desired or required. Details concerning the generation and use of rAAV vectors are described in U.S. Patent 5,139,941 and U.S. Patent 4,797,368, each incorporated herein by reference.

Studies demonstrating the use of AAV in gene delivery include LaFace *et al.* (1988); Zhou *et al.* (1993); Flotte *et al.* (1993); and Walsh *et al.* (1994). Recombinant AAV vectors have been used successfully for *in vitro* and *in vivo* transduction of marker genes (Kaplitt, *et al.,* 1994; Lebkowski, *et al.*, 1988; Samulski, *et al.,* 1989; Shelling and Smith, 1994; Yoder, *et al.*, 1994; Zhou, *et al.*, 1994; Hermonat and Muzyczka, 1984; Tratschin, *et al.*, 1985; McLaughlin, *et al.*, 1988) and genes involved in human diseases (Flotte, *et al.,* 1992; Luo, *et al.*, 1996; Ohi, *et al.,* 1990; Walsh, *et al.,* 1994; Wei, *et al.,* 1994). Recently, an AAV vector has been approved for phase I human trials for the treatment of cystic fibrosis.

AAV is a dependent parvovirus in that it requires coinfection with another virus (either adenovirus or a member of the herpes virus family) to undergo a productive infection in cultured cells (Muzyczka, 1992). In the absence of coinfection with helper virus, the wild type AAV genome integrates through its ends into human chromosome 19 where it resides in a latent state as a provirus (Kotin *et al.*, 1990; Samulski *et al.,* 1991). rAAV, however, is not restricted to chromosome 19 for integration unless the AAV Rep protein is also expressed (Shelling and Smith, 1994). When a cell carrying an AAV provirus is superinfected with a helper virus, the AAV genome is "rescued" from the chromosome or from a recombinant plasmid, and a normal productive infection is established (Samulski, *et al.*, 1989; McLaughlin, *et al.,* 1988; Kotin, *et al.*, 1990; Muzyczka, 1992).

Typically, recombinant AAV (rAAV) virus is made by cotransfecting a plasmid containing the gene of interest flanked by the two AAV terminal repeats (McLaughlin *et al.*, 1988; Samulski *et al.,* 1989; each incorporated herein by reference) and an expression plasmid containing the wild type AAV coding sequences without the terminal repeats, for example pIM45 (McCarty *et al.*, 1991; incorporated herein by reference). The cells are also infected or transfected with adenovirus or plasmids carrying the adenovirus genes required for AAV helper function. rAAV virus stocks made in such fashion are contaminated with adenovirus which must be physically separated from the rAAV particles (for example, by cesium chloride density centrifugation). Alternatively, adenovirus vectors containing the AAV coding regions or cell lines containing the AAV coding regions and some or all of the adenovirus helper genes could be used (Yang *et al.,* 1994a; Clark *et al.,* 1995). Cell lines carrying the rAAV DNA as an integrated provirus can also be used (Flotte *et al.,* 1995).

The retroviruses are a group of single-stranded RNA viruses characterized by an ability to convert their RNA to double-stranded DNA in infected cells by a process of reverse-transcription (Coffin, 1990). The resulting DNA then stably integrates into cellular chromosomes as a provirus and directs synthesis of viral proteins. The integration results in the retention of the viral gene sequences in the recipient cell and its descendants. The retroviral genome contains three genes, gag, pol, and env that code for capsid proteins, polymerase enzyme, and envelope components, respectively. A sequence found upstream from the gag gene contains a signal for packaging of the genome into virions. Two long terminal repeat (LTR) sequences are present at the 5' and 3' ends of the viral genome. These contain strong promoter and enhancer sequences and are also required for integration in the host cell genome (Coffin, 1990).

In order to construct a retroviral vector, a nucleic acid encoding a gene of interest is inserted into the viral genome in the place of certain viral sequences to produce a virus that is replication-defective. In order to produce virions, a packaging cell line containing the gag, pol, and env genes but without the LTR and packaging components is constructed (Mann *et al.,* 1983). When a recombinant plasmid containing a cDNA, together with the retroviral LTR and packaging sequences is introduced into this cell line (by calcium phosphate precipitation for example), the packaging sequence allows the RNA transcript of the recombinant plasmid to be packaged into viral particles, which are then secreted into the culture media (Nicolas and Rubenstein, 1988; Temin, 1986; Mann *et al.,* 1983). The media containing the recombinant retroviruses is then collected, optionally concentrated, and used for gene transfer. Retroviral vectors are able to infect a broad variety of cell types. However, integration and stable expression require the division of host cells (Paskind *et al*., 1975). Additional retroviral vectors contemplated for use in the present invention have been described (Osborne *et al.,* 1990; Flowers *et al.,* 1990; Stockschlaeder *et al.,* 1991; Kiem *et al*., 1994; Bauer *et al*., 1995, Miller and Rosman, 1989; Miller, 1992; Miller *et al*., 1993; each incorporated herein by reference).

Concern with the use of defective retrovirus vectors is the potential appearance of wild-type replication-competent virus in the packaging cells. This can result from recombination events in which the intact sequence from the recombinant virus inserts upstream from the gag, pol, env sequence integrated in the host cell genome. However, new packaging cell lines are now available that should greatly decrease the likelihood of recombination (Markowitz *et al*., 1988; Hersdorffer *et al*., 1990). A preferred cell line is the PA317 cell line (Osborne *et al.,* 1990).

A major determinant of virus titer is the number of packagable RNA transcripts per producer cell, which is dependent on the integrated proviral DNA copy number. Packaging cell lines are coated with viral envelope glycoproteins and are thus resistant to infection by virus of the same host range, but not virus of a different host range. This process is called interference. Therefore, recombinant retroviruses can shuttle back and forth between amphotropic and ecotropic packaging cell lines in a mixed culture (referred to as ping-ponging), thus leading to an increase in proviral DNA copy number and virus titer (Bestwick *et al.,* 1988). Some drawbacks to the ping-pong process are that transfer of packaging functions between ecotropic and anphotropic lines can lead eventually to generation of replication-competent helper virus. Also, increasing numbers of cells express both ecotropic and amphotropic envelope proteins and are therefore resistant to further infection. Moreover, cells with large numbers of proviruses are unhealthy. Thus, there is an optimum period during the ping-pong process when virus titer is high and helper virus is absent. This time period is empirically determined and is relatively constant for a given ecotropic plus amphotropic packaging line combination.

Other viral vectors may be employed as expression constructs in the present invention. Vectors derived from viruses such as vaccinia virus (Ridgeway, 1988; Baichwal and Sugden, 1986; Coupar *et al.,* 1988) and herpesviruses may be employed. They offer several attractive features for various mammalian cells (Friedmann, 1989; Ridgeway, 1988; Baichwal and Sugden, 1986; Coupar *et al.,* 1988; Horwich *et al.,* 1990). Lentivirus vectors are also contemplated for use in the present invention (Gallichan *et al.,* 1998; Miyoshi *et al.,* 1998; Kafri *et al*., 1999).

With the recent recognition of defective hepatitis B viruses, new insight was gained into the structure-function relationship of different viral sequences. *In vitro* studies showed that the virus could retain the ability for helper-dependent packaging and reverse transcription despite the deletion of up to 80% of its genome (Horwich *et al.,* 1990). This suggested that large portions of the genome could be replaced with foreign genetic material. Chang *et al.* recently introduced the chloramphenicol acetyltransferase (CAT) gene into duck hepatitis B virus genome in the place of the polymerase, surface, and pre-surface coding sequences. It was cotransfected with wild-type virus into an avian hepatoma cell line. Culture media containing high titers of the recombinant virus were used to infect primary duckling hepatocytes. Stable CAT gene expression was detected for at least 24 days after transfection (Chang *et al*., 1991).

In still further embodiments of the method of the present invention, the nucleic acids to be delivered are housed within an infective virus that has been engineered to express a specific binding ligand. The virus particle will thus bind specifically to the cognate receptors of the target cell and deliver the contents to the cell. A novel approach designed to allow specific targeting of retrovirus vectors was recently developed based on the chemical modification of a retrovirus by the chemical addition of lactose residues to the viral envelope. This modification can permit the specific infection of hepatocytes *via* sialoglycoprotein receptors.

Another approach to targeting of recombinant retroviruses was designed in which biotinylated antibodies against a retroviral envelope protein and against a specific cell receptor were used.

The antibodies were coupled via the biotin components by using streptavidin (Roux *et al.,* 1989). Using antibodies against major histocompatibility complex class I and class II antigens, they demonstrated the infection of a variety of human cells that bore those surface antigens with an ecotropic virus *in vitro* (Roux *et al.,* 1989).

## II. β-Cells and Cell Culturing

The invention also describes a pancreatic β-cell obtained by the *in vitro* method described herein and documented in the appended examples. The pancreatic β-cells as obtained in accordance with the present invention are particularly useful in medical and pharmaceutical settings. Accordingly, the invention also describes a pharmaceutical composition comprising a pancreatic β-cell as obtained by the *in vitro* method described herein or a pancreatic β-cell obtained by the method of the present invention. The β-cell obtained by the method of the present invention is, *inter alia,* **characterized in that** it is an adult β-cell which proliferates after contact with the herein described exogenous wild-type Pax4. Said β-cell may also comprise an expressed "tag", like a myc-tag as provided, *inter alia,* by the adenoviral vectors described herein. These expressed "tags" are particularly useful in detection of β-cells which have successfully been contacted with exogenous wild-type Pax4 as described herein.

The (proliferating) pancreatic β-cell as obtained by the *in vitro* method of the invention is particularly used for the preparation of a pharmaceutical composition for transplantation and/or tissue replacement. Accordingly, the present invention also describes the (medical/pharmaceutical) use of functional, wild-type Pax4 (as defined herein) for the preparation of a pharmaceutical composition for transplantation and/or tissue replacement. Said transplantation or tissue replacement is particularly to be carried out on a patient suffering from a pancreatic disease, most particularly from diabetes. The adult, proliferating β-cells as obtained by the method of the present invention may be employed in particular in transplantations and tissue gratings. It is of note that these cells may be used in devices, like encapsulation devices or immunobarrier devices (for example semi-permeable membrane devices). Accordingly, the β-cells as obtained by the method of the invention may also be comprised in therapy systems or devices to be implanted into in subject in need of treatment, amelioration and/or prevention of a disorder/disease. These "micro-encapsulations" of the β-cells obtained by the method of the present invention may particularly be useful in the treatment, amelioration and/or prevention of a pancreatic disorder, particularly of diabetes.

More generally, culture conditions may involve manipulating the following cell culture variables: media growth/survival factors (such as IGF-1, growth hormone, prolactin, PDGF, hepatocyte growth factor, and transferrin), media differentiation factors (such as TGF-β), media lipids, media metabolites (such as glucose, pyruvate, galactose, and amino acids), media serum (percentage serum, serum fraction, species of serum), gaseous exchange (ratio atmospheric $O_2$:$CO_2$, and media volume), physical form of the islets prior to plating (whole, dispersed, or cell sorted islet cells), and extracellular substrate for cellular attachment (such as laminin, collagen, matrigel, and HTB-9 bladder carcinoma derived matrix).

Media comprising one or more growth factors that stimulate the growth of the target cell and do not substantially stimulate growth of distinct cells in the cell population; *i.e.*, act to induce preferential growth of the target cells rather than faster-growing, more hardy cells in the population, may be used to deplete fibroblasts. Examples include defined serum free conditions used for β-cells (Clark *et al.*, 1990; incorporated herein by reference), or inclusion of growth or differentiation factors known to allow preferential growth of β-cells (WO95/29989; PCT/US99/00553; incorporated herein by reference). A commercially available medium, EGM™ Endothelial Cell Medium (Cambrex) is a preferred defined media.

Cells also may be induced to proliferate by initial infection with adenovirus or adeno-associated virus (AAV) comprising a gene that induces cellular proliferation, the gene being under the control of a promoter specific for the regulated secretory cell. The cells may alternatively be induced to proliferate by growth on a stimulatory cell matrix.

## III. Pharmaceutical Compositions

The herein described pharmaceutical compositions comprising the β-cells as obtained by the method of the invention may be used in a method for treating a disorder characterized by insufficient pancreatic function in a subject. Methods of treatment may comprise the introduction of the pharmaceutical composition described herein into a subject in need of such a treatment. Particularly, the subject is a human and the disorder to be treated is diabetes.

### A. Pharmaceutically Acceptable Formulations

Where clinical applications are contemplated, it will be necessary to prepare pharmaceutical compositions of the cells in a form appropriate for transplant. The cells will generally be prepared as a composition that is essentially free of pyrogens, as well as other impurities that could be harmful to humans or animals.

One will generally desire to employ appropriate salts and buffers to render stable cells suitable for introduction into a patient Aqueous compositions of the present invention comprise an effective amount of stable cells dispersed in a pharmaceutically acceptable carrier or aqueous medium, and preferably encapsulated.

The phrase "pharmaceutically or pharmacologically acceptable" refer to molecular entities and compositions that do not produce adverse, allergic, or other untoward reactions when administered to an animal or a human. As used herein, "pharmaceutically acceptable carrier" includes any and all solvents, dispersion media, coatings, antibacterial and antifungal agents, isotonic and absorption delaying agents and the like. As used herein, this term is particularly intended to include biocompatible implantable devices and encapsulated cell populations. The use of such media and agents for pharmaceutically active substances is well know in the art. Except insofar as any conventional media or agent is incompatible with the vectors or cells of the present invention, its use in therapeutic compositions is contemplated. Supplementary active ingredients also can be incorporated into the compositions.

Under ordinary conditions of storage and use, the cell preparations may further contain a preservative to prevent growth of microorganisms. Intravenous vehicles include fluid and nutrient replenishers. Preservatives include antimicrobial agents, anti-oxidants, chelating agents and inert gases. The pH and exact concentration of the various components in the pharmaceutical are adjusted according to well-known parameters.

**B. Cell-Based Delivery and Devices**

The engineered cells as obtained by the method of the present invention may be introduced into animals, including human subjects, with certain needs, such as patients with insulin-dependent diabetes. It should be pointed out that the studies of Madsen and coworkers have shown that implantation of poorly differentiated rat insulinoma cells into animals results in a return to a more differentiated state, marked by enhanced insulin secretion in response to metabolic fuels (Madsen *et al*., 1988). These studies suggest that exposure of engineered cell lines to the *in vivo* milieu may have some effects on their response(s) to secretagogues.

As discussed above, one method of administration involves the encapsulation of the engineered cells in a biocompatible coating. In this approach, the cells are entrapped in a capsular coating that protects the contents from immunological responses. One encapsulation technique involves encapsulation with alginate-polylysine-alginate. Capsules made employing this technique generally have a diameter of approximately 1 mm and should contain several hundred cells.

Cells may thus be implanted using the alginate-polylysine encapsulation technique of O'Shea and Sun (1986), with modifications, as later described by Fritschy *et al*. (1991; both references incorporated herein by reference). The engineered cells are suspended in 1.3% sodium alginate and encapsulated by extrusion of drops of the cell/alginate suspension through a syringe into $CaCl_2$. After several washing steps, the droplets are suspended in polylysine and rewashed. The alginate within the capsules is then reliquified by suspension in 1 mM EGTA and then rewashed with Krebs balanced salt buffer.

An alternative approach is to seed Amicon fibers with stable cells of the present invention. The cells become enmeshed in the fibers, which are semipermeable, and are thus protected in a manner similar to the micro encapsulates (Altman *et al.,* 1986; incorporated herein by reference). After successful encapsulation or fiber seeding, the cells may be implanted intraperitoneally, usually by injection into the peritoneal cavity through a large gauge needle (23 gauge).

A variety of other encapsulation technologies have been developed that are applicable to the practice of the present invention (see, *e.g*., Lacy *et al.,* 1991; Sullivan *et al.,* 1991; WO 91/10470; WO 91/10425; WO 90/15637; WO 90/02580; U.S. Patent 5,011,472; U.S. Patent 4,892,538; and WO 89/01967; each of the foregoing being incorporated by reference).

Lacy *et. al*. (1991) encapsulated rat islets in hollow acrylic fibers and immobilized these in alginate hydrogel. Following intraperitoneal transplantation of the encapsulated islets into diabetic mice, normoglycemia was reportedly restored. Similar results were also obtained using subcutaneous implants that had an appropriately constructed outer surface on the fibers. It is therefore contemplated that engineered cells of the present invention may also be straightforwardly "transplanted" into a mammal by similar subcutaneous injection.

Sullivan *et. al.* (1991) reported the development of a biohybrid perfused "artificial pancreas," which encapsulates islet tissue in a selectively permeable membrane. In these studies, a tubular semi-permeable membrane was coiled inside a protective housing to provide a compartment for the islet cells. Each end of the membrane was then connected to an arterial polytetrafluoroethylene (PTFE) graft that extended beyond the housing and joined the device to the vascular system as an arteriovenous shunt. The implantation of such a device containing islet allografts into pancreatectomized dogs was reported to result in the control of fasting glucose levels in 6/10 animals. Grafts of this type encapsulating engineered cells could also be used in accordance with the present invention.

The company Cytotherapeutics has developed encapsulation technologies that are now commercially available that are envisioned for use in the application of the present invention. A vascular device has also been developed by Biohybrid, of Shrewsbury, Mass., that can be used with the technology of the present invention. Other implantable containment apparati contemplated for use with in the application of the present invention are described in U.S. Patents 5,626,561, 5,787,900 and 5,843,069, each of which are incorporated herein by reference.

Implantation employing such encapsulation techniques provides various advantages. For example, transplantation of islets into animal models of diabetes by this method has been shown to significantly increase the period of normal glycemic control, by prolonging xenograft survival compared to unencapsulated islets (O'Shea and Sun, 1986; Fritschy *et al.,* 1991). Also, encapsulation will prevent uncontrolled proliferation of clonal cells. Capsules containing cells are implanted (approximately 1,000-10,000/animal) intraperitoneally and blood samples taken daily for monitoring of blood glucose and insulin.

An alternate approach to encapsulation is to simply inject glucose-sensing cells into the scapular region or peritoneal cavity (Sato *et al.,* 1962). Implantation by this approach may circumvent problems with viability or function, at least for the short term, that may be encountered with the encapsulation strategy.

### IV. Treatments

The term "treatment" as used herein also comprises the amelioration or even prevention of a disorder/disease, e.g., a pancreatic disorder, in particular diabetes. The terms "treatment", "treating" and the like are used herein to generally mean obtaining a desired pharmacological and/or physiological effect. The effect may be prophylactic in terms of completely or partially preventing a disease or symptom thereof and/or may be therapeutic in terms of partially or completely curing a disease and/or adverse effect attributed to the disease. The term "treatment" as used herein covers any treatment of a disease in a mammal, particularly a human, and includes: (a) preventing the disease from occurring in a subject which may be predisposed to the disease but has not yet been diagnosed as having it; (b) inhibiting the disease, *i.e.*, arresting its development; or (c) relieving the disease, *i.e.*, causing regression of the disease. Patients with medical conditions relating to a disorder of the pancreas may be treated with cells as obtained by the method of this invention. Accordingly, a treatment can involve preventing, inhibiting or relieving any medical condition related to pancreatic disorders, particularly diabetes. The "treatment" may in particular be achieved by tissue engineering (of pancreatic cells or islets) and transplantation approaches. Said transplantations comprise, but are not limited to, cell and tissue grafting, islet transplantations and tissue replacement therapies. As discussed herein, the transplantation is not limited to human-human transplantations (homo-transplantations) but also comprises animal-human transplantations (xenotransplantations). Particularly are, however, human-human transplantations and most particularly, the method of the invention is employed to *in vitro* proliferate adult β-cells obtained from the patient to be treated and to re-implant the proliferated β-cells obtained by the inventive method. Most particularly, the transplantation is carried out in accordance with the Edmonton protocol. It is envisaged that wild-type Pax4 encoding nucleic acid molecules or expressed wild-type Pax4 (*e.g.*, mRNA or protein) be employed to stimulate proliferation of pancreatic cells, *e.g.*, β-cells *ex vivo*.

An effective amount of the stable cells is determined based on the intended goal. The term "unit dose" refers to a physically discrete unit suitable for use in a subject, each unit containing a predetermined quantity of the cell composition calculated to produce the desired response in association with its administration, i.e., the appropriate route and treatment regimen. The quantity to be administered, both according to number of treatments and unit dose, depends on the subject to be treated, the state of the subject, and the protection desired. Precise amounts of the therapeutic composition also depend on the judgment of the practitioner and are peculiar to each individual.

### V. Adjunct Therapies and Procedures

It may prove advantageous to combine the methods disclosed herein with adjunct therapies or procedures to enhance the overall therapeutic effect. Such therapies and procedures are set forth in general, below. A skilled physician will be apprised of the most appropriate fashion in which these therapies and procedures may be employed.

#### A. Supplemental Insulin Therapy

Generated and inventive β-cells as obtained by the method of this invention may work well in combination with traditional insulin supplementation. Such therapies should be tailored specifically for the individual patient given their current clinical situation, and particularly in light of the extent to which transplanted cells can provide insulin. The following are general guidelines for typical a "monotherapy" using insulin supplementation by injection.

Insulin can be injected in the thighs, abdomen, upper arms or gluteal region. In children, the thighs or the abdomen are preferred. These offer a large area for frequent site rotation and are easily accessible for self-injection. Insulin injected in the abdomen is absorbed rapidly while from the thigh it is absorbed more slowly. Hence, patients should not switch from one area to the other at random. The abdomen should be used for the time of the day when a short interval between injection and meal is desired (usually pre-breakfast when the child may be in a hurry to go to school) and the thigh when the patient can wait 30 minutes after injection for his meal (usually pre-dinner). Within the selected area systematic site rotation must be practiced so that not more than one or two injections a month are given at any single spot. If site rotation is not practiced, fatty lumps known as lipohypertrophy may develop at frequently injected sites. These lumps are cosmetically unacceptable and, what is more important, insulin absorption from these regions is highly erratic.

Before injecting insulin, the selected site should be cleaned with alcohol. Injecting before the spirit evaporates can prove to be quite painful. The syringe is held like a pen in one hand, pinching up the skin between the thumb and index finger of the other hand, and inserting the needle through the skin at an angle of 45-90° to the surface. The piston is pushed down to inject insulin into the subcutaneous space (the space between the skin and muscle), then one waits for a few seconds after which release the pinched up skin before withdrawing the needle. The injection site should not be massaged.

For day-to-day management of diabetes, a combination of short acting and intermediate acting insulin is used. Some children in the first year after onset of diabetes may remain well controlled on a single injection of insulin each day. However, most diabetic children will require 2,3 or even 4 shots of insulin a day for good control. A doctor should decide which regimen is best suited.

One injection regimen: A single injection comprising a mix of short acting and intermediate acting insulin (mixed in the same syringe) in 1:3 or 1:4 proportion is taken 20 to 30 minutes before breakfast. The usual total starting dose is 0.5 to 1.0 units/kg body weight per day. This regimen has three disadvantages: (1) all meals must be consumed at fixed times; (2) since the entire quantity of insulin is given at one time, a single large peak of insulin action is seen during the late and early evening hours making one prone to hyopglycemia at this time; (3) as the action of intermediate acting insulin rarely lasts beyond 16-18 hours, the patient's body remains underinsulinized during the early morning hours, the period during which insulin requirement in the body is actually the highest.

Two-injection regimen: This regimen is fairly popular. Two shots of insulin are taken - one before breakfast (2/3 of the total dose) and the other before dinner (1/3 of the total dose). Each is a combination of short acting and intermediate acting insulin in the ratio of 1:2 or 1:3 for the morning dose, and 1:2 or 1:1 for the evening dose. With this regimen the disadvantages of the single injection regimen are partly rectified. Some flexibility is possible for the evening meal. Further, as the total days' insulin is split, single large peaks of insulin action do not occur hence risk of hypoglycemia is reduced and one remains more or less evenly insulinized throughout the day. On this regimen, if the pre-breakfast blood glucose is high, while the 3 a.m. level is low, then the evening dose may need to be split so as to provide short acting insulin before dinner and intermediate acting insulin at bedtime.

Multi-dose insulin regimens: The body normally produces insulin in a basal-bolus manner, *i.e.*, there is a constant basal secretion unrelated to meal intake and superimposed on this there is bolus insulin release in response to each meal. Multi-dose insulin regimens were devised to mimic this physiological pattern of insulin production. Short acting insulin is taken before each major meal (breakfast, lunch and dinner) to provide "bolus insulin" and intermediate acting insulin is administered once or twice a day for "basal insulin." Usually bolus insulin comprises 60% of the total dose and basal insulin makes up the remaining 40%. With this regimen you have a lot of flexibility. Both the timing as well as the quantity of each meal can be altered as desired by making appropriate alterations in the bolus insulin doses. To take maximum advantage of this regimen, one should learn "carbohydrate counting" and work out carbohydrate:insulin ratio - the number of grams of carbohydrate for which the body needs 1 unit of insulin.

### B. Immunosuppressive Therapy

It may prove necessary to deliver an immunosuppressive therapy to a transplant recipient to prevent graft rejection. A general approach to transplant immunosuppression is to combine agents in small doses so as to get an added immunosuppressive effect, but without individual side effects of the different drugs. Commonly used agents include azathioprine, corticosteroids and cyclosporin are combined in a variety of protocols. Each has different side effects: corticosteroids stunt growth and cause a round face and impair the healing of wounds; azathioprine can inhibit the bone marrow and cause anaemia and a low white cell count; cyclosporin can cause increase in growth of hair and damage the kidney. However, when these three agents are used together in reduced doses, the patient can generally tolerate the immunosuppression quite well.

Unfortunately, acute rejection crises can still occur and are usually treated with a short course of high dose steroids or anti-lymphocyte globulin preparations. This powerful immunosuppression for rejection may lead to infection particularly with viruses, causing severe cold sores and may activate cytomegalic virus infection which can cause a temperature and specific bad effects on a variety of different organs. All immunosuppression predisposes a patient to infection and tumour formation.

Despite these difficulties, most patients tolerate organ grafts and, after a time, can be maintained with relatively low dosage of immunosuppression. With few exceptions however, stopping immunosuppression usually leads to acute rejection and chronic rejection which is difficult to detect and can occur insidiously after years of good function of a graft. Thus, the search for new immunosuppressives such as FK506, celcept mycophenolate mofital and rapamycin is important.

### C. Pro-Angiogenic Therapy

Pro-angiogenic therapy, also known as "therapeutic angiogenesis," uses angiogenic growth factors or gene therapy to stimulate blood vessel growth in tissues that require an improved blood supply. While angiogenesis is normally activated by hypoxia (decreased oxygen), many afflicted tissues are unable to respond adequately to reverse the disease processes and prevent tissue damage.

Currently, a variety of angiogenesis-stimulating modalities are being tested in clinical trials sponsored by biotechnology and pharmaceutical companies, medical centers, and the National Institutes of Health. The most prevalently discussed pro-angiogenic therapy is the use of VEGF. Another involves a topical gel of recombinant platelet-derived growth factor (rhPDGF-BB).

### D. Monitoring Glucose Levels

Any person suffering from diabetes will be very familiar with the need to regularly measure blood glucose levels. Blood glucose level is the amount of glucose, or sugar, in the blood. It is also is referred to as "serum glucose level." Normally, blood glucose levels stay within fairly narrow limits throughout the day (4 to 8 mmol/l), but are often higher after meals and usually lowest in the morning. Unfortuantely, when a person has diabetes, their blood glucose level sometimes moves outside these limits. Thus, much of a diabetic's challenge is to When one suffers from diabetes, it is important that glucose level be as near normal as possible. Stable blood glucose significantly reduces the risk of developing late-stage diabetic complications, which start to appear 10 to 15 years after diagnosis with Type 1 diabetes, and often less than 10 years after diagnosis with Type 2 diabetes.

Blood glucose levels can be measured very simply and quickly with a home blood glucose level testing kit, consisting of a measuring device itself and a test strip. To check blood glucose level, a small amount of blood is placed on the test strip, which is then placed into the device. After about 30 seconds, the device displays the blood glucose level. The best way to take a blood sample is by pricking the finger with a lancet. Ideal values are (a) 4 to 7 mmol/l before meals, (b) less than 10 mmol/l one-and-a-half hours after meals; and (c) around 8 mmol/l at bedtime.

People who have Type 1 diabetes should measure their blood glucose level once a day, either in the morning before breakfast or at bedtime. In addition, a 24-hour profile should be performed a couple of times a week (measuring blood glucose levels before each meal and before bed). People who have Type 2 diabetes and are being treated with insulin should also follow the schedule above. People who have Type 2 diabetes and who are being treated with tablets or a special diet should measure their blood glucose levels once or twice a week, either before meals or one-and-a-half hours after a meal. They should also perform a 24-hour profile once or twice a month.

The main advantage for measuring blood glucose levels of insulin-treated diabetics in the morning is that adjusted amounts of insulin can be taken if the blood glucose level is high or low, thereby reducing the risk of developing late-stage diabetic complications. Similarly, the blood glucose level at bedtime should be between 7 and 10 mmol/l. If blood glucose is very low or very high at bedtime, there may be a need to adjust food intake or insulin dose. Blood glucose should also be measured any time the patient does not feel well, or think blood glucose is either too high or too low. People who have Type 1 diabetes with a high level of glucose in their blood (more than 20 mmol/l), in addition to sugar traces in the urine, should check for ketone bodies in their urine, using a urine strip. If ketone bodies are present, it is a warning signal that they either have, or may develop, diabetic acidosis.

## VI. Kits

Also described herein is a kit comprising a vector system as discussed above or a gene delivery device, wherein said vector system or gene delivery device leads to the expression of functional, wild-type Pax4. Particularly, said kit may comprise an adenoviral vector as defined above. The kit may include buffers and substrates for reporter genes that may be present in recombinant Pax4 molecules or vectors. The kit may advantageously be used for carrying out any one of the methods of the invention and could be, *inter alia,* employed in a variety of applications referred to herein, *e.g.*, in the medical field, the pharmaceutical field or as research tool. The parts of the kit can be packaged individually in vials or in combination in containers or multicontainer units. Manufacture of the kit follows particularly standard procedures which are known to the person skilled in the art.

## VII. Examples

The following examples are included to demonstrate preferred embodiments of the invention. It should be appreciated by those of skill in the art that the techniques disclosed in the examples which follow represent techniques discovered by the inventors to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the scope of the invention.

**Example 1: Material and Methods used in this study**

***Rat islet isolation and culture.*** 7-week-old male Wistar rats (250 g) were purchased from Elevage Janvier (Le Genest-St-Isle, France). Pancreatic islets were isolated by collagenase digestion, handpicked and maintained in 11.5 mM glucose/RPMI-1640 (Invitrogen, Basel, Switzerland) supplemented with 10% fetal calf serum (FCS; Brunschwig AG, Basel, Switzerland), 100 Units/ml penicillin, 100 μg/ml streptomycin and 100 μg/ml gentamycin (Sigma Basel, Switzerland). In several instances, islets were exposed to 0.1, 0.5 and 2 nM of activin A, betacellulin and TGFβ1 (Sigma, Basel, Switzerland) for 24 h.

***Human isolation and culture.*** Freshly isolated human islets, obtained from the Cell Isolation and Transplantation Laboratory in Geneva, were maintained in CMRL-1066 supplemented with 10% FCS, 100 Units/ml penicillin, 100 μg/ml streptomycin and 100 μg/ml gentamycin (Fournier, 1996; Janjic, 1996). Islets were treated with 11 mM or 25 mM glucose, 0.5 nM betacellulin, activin A or TGF-β1 for 24 and 48 hours. Total RNA was isolated using RNeasy mini kit as described by the manufacturer (Quiagen). Pax4 and cyclophilin RNA levels were assessed by quantitative RT-PCR using an ABI 7000 Sequence Detection System (Applera Europe). Alternatively, human islets were transduced with either AdCaLacZ or AdCMVPax4IRESGFP and proliferation was assessed by BrdU incorporation.

***Plasmid construction.*** The full-length mouse Pax4 cDNA was amplified by polymerase chain reaction (PCR) and the PCR product was subcloned into an expression vector pGEM-T Easy (Promega). An *EcoRI* fragment containing Pax4 was then transferred into the expression vector pcDNA3.1/myc-His (Invitrogen). The inventors used the myc-tagged fusion protein for immunofluorescent detection of Pax4, because anti-Pax4-specific antibodies failed to detect the transcription factor. The Pax4-myc wild-type (wt) was subjected to mutagenesis using the oligonucleotides 5'-CGAGTACTTTGGGCACTTC-3' (SEQ ID NO:17) and 5'-GAAGTGCCCAAAGTACTCG-3' (SEQ ID NO:18), the Turbo *Pfu* polymerase (Stratagene) and the restriction enzyme *DpnI* to generate mutant Pax4-myc R129W (arginine at codon 129 to tryptophan). The mouse mutant R129W of Pax4 gene was described to correspond to the human mutation R121 W (Shimajiri, 2001).

***Adenoviral infection of rat islets.*** The recombinant adenoviruses AdCMVPax4IRESGFP, AdCMVNgn3IRESGFP and AdCMVPax6 were generated using standart methods; see also Fig. 6. AdCAlacZ, containing the bacterial β-galactosidase cDNA, was used as control (Ishihara, 1999). Twelve hours post-isolation, islets were infected with various amounts of recombinant adenoviruses (as indicated in FIGS.) for 90 min, washed and cultured in RPMI 1640 medium supplemented with 10% FCS. The overexpression of Pax4 in infected islets was monitored daily by EGFP expression visualized by fluorescent microscopy.

***Generation of further adenoviral vectors comprising wildtype and mutant Pax4.*** The mouse Pax4 cDNA was amplified by PCR and cloned into the pcDNA-3 vector (Invitrogen) in frame with the c-myc/6-Histidine (6-HIS) tag sequence. This carboxy-terminal end tag allowed for the detection of the recombinant protein by immunofluorescence using an antibody against the c-myc epitope. Site directed mutagenesis was performed on this construct to generate the R129W mutant. This mutation located in the paired DNA binding domain corresponds to the human R121W mutation which has been associated with type 2 diabetes. The wild-type and mutant Pax4/c-myc/6-HIS cDNAs were then subcloned into the pTRE-Shuttle2 vector harbouring a tetracycline-inducible cytomegalovirus (CMV) promoter (Clontech). The inducible cassettes were subsequently transferred into the Adeno-X viral DNA to generate recombinant adenoviruses (Ad-mPax4-myc WT and Ad-mPax4-myc R129W). Further adenoviral vectors comprise the pHVAd2-Pax4 vector, generated as follows: Pax4 cDNA has been isolated by standard molecular biology cloning techniques and cloned into Bam HI and Hind will restriction sites of the first generation adenoviral vector pACCMV.pLpA (Becker, 1994) which contains the CMV promoter and the SV40 poly-adenylation (polA) signal. In a second step the CMV-Pax4-SV40 polA transgene has been isolated by Not I restriction digest, blunted and cloned into the Eco RV restriction site of pHVAd2 (provided by DeveloGen Berlin).

The herein provided Ad-Pax4-myc construct was generated as follows. The Myc-His tag added at the end of mouse Pax4 nucleic acid sequence plus linker sequences, leading to the following construct:

ATG....ctcaaactgg cca) pax4 – (atc act agt gaa ttc tgc aga tat cca gca cag tgg cgg ccg ctc gag tct aga ggg ccc ttc) Linker – (GAA CAA AAA CTC ATC TCA GAA GAG GAT CTG) (SEQ ID NO:19) myc epitope – (AAT ATG CAT ACC GGT CAT CAT CAC CAT CAC CAT) (SEQ ID NO:20) PolyHIS tag

This cassette was cloned into position 787 of pTRE-Shuttle2 (EcoRV site). This construct was digested with enzymes CeuI (position 18 of vector) and SceI (position 1608 of vector) and subcloned into CeuI and SceI predigested pAdeno-X viral DNA (position 21 and 57 respectively)

***Infection studies on isolated human islets.*** Freshly isolated human islets, obtained from the Cell Isolation and Transplantation Laboratory in Geneva, were maintained for 48 hours in CMRL-1066 supplemented with 10% FCS, 100 Units/ml penicillin, 100 μg/ml streptomycin and 100 μg/ml gentamycin. Partially trypsinized islets were then infected with either Ad-mPax4-myc WT or Ad-mPax4-myc R129W along with the adenoviral construct harbouring the tetracycline transcriptional activator (Ad-X Tet-On). Cells were rinsed 90 minutes post infection and replenished with fresh media supplemented with or without doxycycline (1μg/ml) and BrdU (10 μM). Doxycycline-dependent activation of PAX4 as well as cell proliferation was then assessed 48 hours later by immunohistochemistry using antibodies raised against the c-myc epitope and BrdU, respectively.

***Quantitative real-time RT-PCR.*** Total RNA from 50 islets was extracted using the Trizol reagent (Invitrogen) and 2 μg were converted into cDNA as previously described (Gauthier *et al*., 1999b). Primers for cyclophilin, somatostatin, glucagon, insulin, Pdx1, c-myc, Id2, Bcl-xL, Bcl-2, Pax4 and caspase 3 were designed using the Primer Express Software (Applera Europe, Rotkreuz, Switzerland) and sequences can be obtained upon request. Quantitative real-time PCR was performed using an ABI 7000 Sequence Detection System (Applera Europe) and PCR products were quantified fluorometrically using the SYBR Green Core Reagent kit. Three distinct amplifications derived from 4 independent experiments were performed in duplicate for each transcript and mean values were normalized to the mean value of the reference mRNA cyclophilin. An RNA control sample was used with each real time PCR experiment containing cyclophilin primers to control for genomic DNA contamination. Quantification was achieved by the standard curve method as described by Applera Europe.

***Transient transfection assays.*** The c-myc (pDEL-1-Luc), Bcl-xL (Bc1-xL/515) and telomerase (pTERT-luc) gene promoter luciferase reporter constructs were kindly provided by Dr B. Vogelstein (The Johns Hopkins Oncology Center, Baltimore, MD), Dr B Schaefer (University of Zurich, Zurich, CH) and Dr R Dalla-Favera (Columbia University, New York, NY), respectively. The BHK-21 cell line was transiently transfected using the calcium phosphate precipitation technique as described previously (Gauthier, 1999a). The pSV-β-Galactosidase control vector (Promega) was used as internal control to normalize for transfection efficiency. Values presented for each set of experiments correspond to the mean and standard deviation of at least three individual transfections performed in duplicates. Results are presented as fold induction of the control sample obtained from cells transfected with empty expression vector.

***Nuclear extract preparation and electrophoretic mobility shift assay (EMSA).*** Nuclear protein extracts were prepared from AdCaLacZ or AdCMVPax4IRESGFP-infected islets according to the protocol of Schreiber *et al.* (Schreiber, 1988). DNA binding assays using a radiolabeled glucagon promoter G3 element were performed as described previously (Gauthier, 2002). Recombinant Pax4 as well as Pax6 were prepared using an *in vitro* transcription and translation system as described by the manufacturer (Promega Inc.). Antibodies generated against Pax4 and Pax6 were kindly provided by Dr M.S. German (UCSF, San Francisco CA, USA) and Dr S. Saule (Institut Curie, Orsay Cedex, France), respectively.

***Hormone radioimmunoassays.*** Islets were washed in Krebs-Ringer-bicarbonate-HEPES buffer (KRBH) and incubated at 37°C for 60 min in the same buffer supplemented with 2.5 mM glucose. Subsequently, insulin secretion from 15 matched-size islets/condition was measured over a period of 30 min in KRBH containing the indicated stimulators. Total hormone content was extracted using acid ethanol. Total and secreted insulin was detected by radioimmunoassay as described previously (Maechler and Wollheim, 1999). Secreted insulin was expressed as a percentage of total cellular insulin content. For glucagon radioimmunoassays, a protocol was adapted from that of Salehi *et al.* (Salehi, 1999) as follows: 50 μl samples of diluted (1:400) acid ethanol extracts were kept for 24 h at 4 °C in the presence of an equal volume of anti-glucagon (Dako Diagnostics AG, Zug, Switzerland; diluted 1:150 in PBS-EDTA containing 0.5% BSA). Glucagon $I^{125}$ tracer (50 μl) was then added and assays were maintained for a further 48 h at 4 °C. Subsequently, PBS-EDTA containing 4% BSA (100 μl) was included and the reaction was kept at 4 °C for an additional 45 min. Free Glucagon $I^{125}$ was then separated from bound tracer through precipitation with charcoal as described for insulin (Maechler and Wollheim, 1999). A standard curve was generated in parallel using purified rat glucagon 1-29 (Bachem AG, Switzerland) diluted in the range of 25-400 pg.

***Glucose oxidation***. Carbon dioxide production derived from glucose oxidation was measured using the multiwell $^{14}CO_2$-capture assay developed by Collins and colleagues (Collins, 1998). Radioactive carbon dioxide trapped in filter paper was measured using a Beckman LS6500 Scintillation counter (Beckman Coulter International, Nyon, Switzerland) and results were expressed as nmole of glucose/mg protein/hour.

***ATP measurements***. Total cellular ATP was measured after 10 min incubation in 2.5 mM glucose using a bioluminescence assay kit according to the manufacturer's recommendations (HS II, Roche Diagnostic, Rotkreuz, Switzerland). Alterations in cytosolic ATP levels in response to 16.5 mM glucose were monitored using the adenoviral construct AdCAGLuc encoding the ATP-sensitive bioluminescence probe luciferase (Ishihara, 2003). Changes in ATP were then recorded using a FLUOstar Optima apparatus (BMG Labtechnologies) as previously described by Merglen *et al.* (Merglen, 2004).

***Mitochondrial calcium measurements.*** Islets were infected with rAdRIPmAQ (4.8 X $10^8$ pfu/ml) and either AdCaLacZ or AdCMVPax4IRESGFP (2.4 X $10^7$ Pfu/ml) for 90 min. Islets were then washed and immediately seeded onto A431 extracellular matrix-coated 15-mm diameter Thermanox™ coverslips (Nunc), at a density of approx 80 islets per coverslip, and cultured in complete medium for 65 hr to maximise adherence and allow for the expression of recombinant protein (Ishihara, 2003). The mtAq was reconstituted with coelenterazine (5 μM, Molecular Probes) in glucose/ serum-free RPMI medium for 2-4 h immediately prior to the experiment Cover slips were then placed in a sealed, thermostatted (37°C) chamber, 5 mm from a photonmultiplier, which was used to detect emitted luminescence, as previously described (Kennedy, 1996). Islets were superfused (1.0 ml $min^{-1}$) with KRBH supplemented with either glucose (16.7 mM) or KCl (60 mM) where indicated. Luminesence output was recorded every second using a photon-counting board (EMI C660) after a 30 min equilibration period to establish the baseline. Recorded counts were converted to $[Ca^{2+}]_m$ as published elsewhere (Challet, 2001).

***Immunohistochemistry and cell proliferation assay.*** Isolated rat islets were infected with recombinant adenoviruses and subsequently dissociated by trypsin-EDTA treatment. Single cells were cultured on polyornithine-treated glass cover slips for two days, washed with PBS and fixed in 4% paraformaldehyde in PBS for 20 min at room temperature. Immunochemical detection of β-cells was performed as previously described (Ishihara, 2003) using a polyclonal guinea pig anti-insulin antibody (dilution 1:1000; Sigma). The antibody was visualized using a Texas Red dye-conjugated anti-guinea pig IgG (Milan analytica AG, La Roche, Switzerland; dilution 1:400). Nuclei were then stained with 4',6-diamidino-2-phenylindole (DAPI, 10 μg/ml; Sigma) for 3 min. Cover slips were rinsed with PBS and mounted using DAKO fluorescent mounting medium. Images were obtained using a Zeiss Axiophot I equipped with an Axiocam color CCD camera.

Cellular proliferation was estimated using an immunohistochemical assay kit as described by the manufacturer (Roche Diagnostics, Rotkreuz, Switzerland). Seven h prior to fixation with ethanol, infected or mitogen treated-cells were labeled with 10 μM BrdU. Cells were co-stained for insulin, as described above, and for BrdU using a monoclonal anti-BrdU mouse IgG (Sigma). BrdU positive cells were visualized after incubation with fluorescein isothiocyanate (FITC)-conjugated anti mouse IgG. β-cells immunostained for both insulin and BrdU were counted using a Zeiss microscope under high magnification (X400). Results are expressed as a percentage of BrdU/insulin positive cells over the total amount of insulin positive cells for each condition. Recombinant Pax4 WT or R129W myc tagged proteins were visualized using an anti-myc antibody (Invitrogen).

***Statistical analysis.*** Results are expressed as mean+/- SE. Where indicated, the statistical significance of the differences between groups was estimated by Student's unpaired *t*-test. * and ** indicate statistical significance with $p<0.05$ and $p<0.01$, respectively.

**Example 2: Activin A and betacellulin increase Pax4 gene transcription as well as β-cell proliferation in rat islets.**

As an initial step towards understanding factors that may influence Pax4 expression and thereby impact endocrine cell mass, the inventors investigated the response of the pax4 gene to mitogens such as activin A and betacellulin in rat islets (Demeterco, 2000). The inventors first established the relative basal mRNA expression level of the transcription factor in rat islets and INS-1E cells. Similar to other insulinomas, the INS-1E cell line expresses Pax4 and therefore provides a suitable reference to measure relative levels of the transcript (Miyamoto, 2001). Pax4 mRNA was detected in rat islets at levels 80% lower than those found in INS-1E cells thus confirming expression of the transcription factor in adult tissue (FIG. 1A). Treatment of islets for 24 h with a range of concentrations of either activin A or betacellulin resulted in a dose-dependent increase of Pax4 mRNA levels. Maximal induction was observed with 0.5 nM of activin A or betacellulin that elicited a 3.5- and 4-fold increase in Pax4 mRNA, respectively (FIGS. 1B and C). In contrast, the related factor TGF-β1 had no significant effect on Pax4 in islets (Ueda, 2000) (FIG. 1D). Of note, insulin mRNA levels were unaffected by both treatments (FIGS. 1B-D) (Ueda, 2000). In parallel, activin A and betacellulin-induced proliferation was measured by BrdU incorporation. This approach was selected because of its ability to discriminate between β-cells proliferation and that of other cell type, in contrast to the [$^3$H] thymidine incorporation method FIG. 2A illustrates a representative experiment in which insulin and BrdU were immune-detected and co-stained in dispersed islet cells. Since Pax4 is predominantly localized to β-cells, only insulin-positive cells were included in the BrdU labelling index calculation. Both growth factors were found to increase β-cell proliferation by approximately 2-fold (control, 1.41±0.2; activin A, 4.46±0.3; betacellulin, 4.99±0.4) while TGF-β1-treated islets remained quiescent (1.33±0.3) (FIG. 2B). Taken together, these results suggest that stimulation of Pax4 gene expression by activin A and betacellulin correlates with islet proliferation induced by the two mitogens.

**Example 3: Adenovirus-mediated Pax4 overexpression in rat islets induces β-cell proliferation.**

To evaluate the importance of Pax4 in β-cell replication, isolated rat pancreatic islets were infected with a CMV promoter driven Pax4/GFP-expressing adenovirus (AdCMVPax4IRESGFP) or control adenovirus (AdCAlacZ). Since the antibody against Pax4 is unable to detect the protein by immunohistochemistry or by Western blotting (data not shown), the inventors monitored its overexpression via the reporter green fluorescent protein (GFP) co-translated from a bi-cistronic transcript containing an internal ribosomal entry sequence (IRES). Immunohistochemical studies revealed that approximately 25% and 50% of β-cells expressed GFP 48 h after infection with 1 and 2.4 X $10^7$ pfu/ml of AdCMVPax4IRESGFP, respectively (FIG. 3A). Nuclear fragmentation was absent in transduced cells indicating that viral infection did not affect viability or apoptosis. Consistent with GFP expression, Pax4 transcript was estimated to reach levels 22 $\pm$ 5-fold higher (n=3) than those found in control AdCALacZ-infected islets (data not shown). In order to confirm the production of a functional protein, the inventors performed electromobility shift assays (EMSA) using a radiolabeled G3 element of the glucagon gene promoter, which has previously been shown to interact with both Pax4 and Pax6 (Ritz-Laser et al., 2002). A single predominant complex corresponding to Pax6 was detected in nuclear extracts derived from AdCaLacZ-infected islets (FIG. 3B, lane 4). A second complex of similar migration pattern to that produced by recombinant Pax4 was generated in islet infected with increasing amounts of AdCMVPax4IRESGFP (FIG. 3B lanes 1, 5, 6 and 7). The addition of Pax4 antiserum specifically abolished this complex confirming the binding of Pax4 to this site (FIG. 3B lane 2 and 8). The inventors next evaluated the capacity of Pax4 to promote islet proliferation by measuring BrdU incorporation in either dispersed cells (FIG. 4A) or whole islets (FIG. 4B). Quantification revealed a 3.5-fold increase in BrdU labelling of β-cells expressing Pax4 as compared to AdCALacZ-transduced islets (8.67$\pm$0.8% versus 2.42$\pm$0.5%, FIG. 4C). To confirm the specificity of Pax4-associated β-cell mitogenesis, the inventors examined the effect of alternative transcription factors, Pax6 and neurogenin-3 (Ngn3), both of which are implicated in islet development (Schwitzgebel, 2001). β-cell replication was unaffected by overexpression of Pax6 and Ngn3 (FIG. 4C). In summary, forced expression of Pax4 in islets specifically induced DNA synthesis in β-cells recapitulating the effect observed with both activin A and betacellulin.

**Example 4: Pax4 overexpression in islets induces genes implicated in proliferation.**

To gain further insight into the potential mechanism by which Pax4 stimulates β-cell replication, the inventors performed a temporal expression profiling of the *c-myc* proto-oncogene in islets infected for up to six days with either AdCMVPax4IRESGFP or AdCaLacZ. This transcription factor has previously been shown to regulate β-cell mass in mouse islets (Pelengaris *et al.,* 2002). Nuclear protein extracts isolated from AdCMVPax4IRESGFP-transduced islets revealed a transient Pax4 DNA binding activity to the G3 element reaching maximal levels around 1 day post infection (FIG. 5A). Expression levels of c-myc mRNA levels were rapidly induced in AdCMVPax4IRESGFP-transduced islets attaining levels 4-fold higher than those found in control islets 24 h and 48 h after infection (FIG. 5B). These further increased, reaching a maximum of 8-fold above control values at day 4 before returning to basal levels at day 6. Since c-myc stimulates proliferation through activation of the Id2 cell cycle progression regulator, the inventors explored whether this pathway was triggered in Pax4 overexpressing islets (Lasorella et al., 2000). Interestingly, AdCMVPax4IRESGFP-transduced islets expressed Id2 mRNA levels 4-fold higher to those of control islets only 4 days after infection indicating a delayed activation of Id2 gene expression by c-myc (FIG. 5B). Bcl-xL has recently been shown to prevent c-myc-induced islet β-cell apoptosis and to promote proliferation by suppressing the mitochondrial apoptotic pathway (Pelengaris et al., 2002). Consistent with these findings, expression levels of Bcl-xL were found to be 2- to 2.5-fold higher in Pax4-expressing cells while caspase-3 RNA level remained relatively constant for the 6 day duration of the experiment (FIG. 5B). In addition, Bcl-2 mRNA levels were transiently induced. Taken together, these results suggest that Pax4 stimulates β-cell proliferation through the coordinated activation of the c-myc-Id2 pathway and Bcl-xL gene expression.

**Example 5: Hormone expression profiling of AdCMVPax4IRESGFP-infected islets.**

Since Pax4 has been reported to inhibit expression of reporter constructs harbouring either the insulin or glucagon gene promoter in various β and a cell lines (Campbell et al., 1999; Fujitani et al., 1999; Ritz-Laser et al., 2002), the inventors investigated whether endogenous levels of these hormones, as well as somatostatin, were repressed in AdCMVPax4IRESGFP-infected islets by quantitative RT-PCR. In order to evaluate potential time-dependent alterations in gene expression, mRNA levels were measured over a period of 6 days. Surprisingly, insulin, glucagon and somatostatin transcripts remained relatively constant throughout the experiment (FIG. 5C). Consistent with these findings, mRNA levels for the transcription factor Pdx1, which is a major stimulator of insulin and somatostatin gene transcription, also remained stable (FIG. 5C). To confirm the quantitative RT-PCR results, glucagon and insulin protein contents were measured by radioimmunoassay 48 h post infection. Interestingly, a small increase, rather than a decrease, in insulin protein content was measured in islets transduced with the highest concentration of AdCMVPax4IRESGFP while glucagon levels remained stable (FIG. 10, Table 1). Therefore, these results suggest that Pax4 does not function as a transcriptional repressor of insulin and glucagon in mature islet cells.

**Example 6: Pax4 transactivates both the c-myc and Bcl-xL gene promoter.**

To examine whether Pax4 was directly involved in the regulation of Bcl-xL and c-myc transcription, transient transfection assays were performed in BHK-21 cells with luciferase reporter constructs harbouring either gene promoters along with increasing amounts of Pax4. The impact of the type 2 diabetes associated Pax4 mutation R129W, located in the paired DNA binding domain, was also evaluated. To this end, the inventors generated two expression vectors containing either a wild-type (Pax4-myc wt) or mutant Pax4 (Pax4-myc R129W) cDNA fused to the myc epitope. The inventors first validated expression and localization of the proteins encoded by the two constructs in rat insulinoma INS1E cells. Immunofluorescence studies using a myc antibody revealed nuclear localisation of both wild-type and mutant Pax4 in transfected cells (FIG. 6A). In contrast, transfection with a recombinant chimera composed of the vesicular protein; synaptotagmin VII and the c-myc tag resulted in cytoplasmic staining indicating that the tagged peptide does not interfere with proper compartmentalization (FIG. 6A bottom panel). Furthermore, EMSA using equal amounts of *in vitro* transcribed and translated recombinant proteins (confirmed by Western blotting, data not shown) and the G3 element of the glucagon promoter confirmed the binding activity of the myc-fused wild-type and mutant Pax4 proteins (FIG. 6B, lanes 1 and 3). The specificity of the complexes was demonstrated by supershift assay using the myc antibody (FIG. 6B, lanes 2 and 4). Interestingly, although the recombinant Pax4-myc R129W protein was able to interact with the G3 element, its binding affinity was weaker than the recombinant Pax4-myc wt. Transient transfections in the heterologous BHK-21 cell system revealed that increasing amounts of the Pax4-myc wt expression vector dose dependently stimulated luciferase activity of the c-myc and Bc1-xL gene promoter constructs reaching up to 3.5-fold and 2.7-fold, respectively (FIG. 6C). However, Pax4-myc R129W was less efficient in transactivating both constructs, reaching maximal induction levels of only 2.1-fold and 1.5-fold for the c-myc and Bcl-xL reporter constructs, respectively (FIG. 6C). Transactivation was promoter specific since Pax4 was unable to induce the telomerase promoter Tert-Luc (FIG. 6C). These results clearly demonstrate that Pax4 regulates c-myc and Bcl-xL transcription while the mutant form is less efficient in transactivating both genes.

**Example 7: Pax4 overexpression attenuates insulin secretion in islets.**

Although increased Bcl-xL expression plays a protective role against c-myc-induced apoptosis, high levels of this mitochondrially-targeted protein were also found to impair insulin secretion (Zhou et al., 2000). The inventors thus examined whether glucose-evoked insulin secretion was altered in Pax4 overexpressing islets. The inventors found that glucose-stimulated insulin secretion was attenuated by 50% in the presence of $2.4 \times 10^7$ pfu/ml of AdCMVPax4IRESGFP 48 h after infection while β-galactosidase-expressing islets and non-infected controls exhibited an expected 3-fold increase in hormone release (FIG. 7A). Interestingly, this level of inhibition corresponds to the percentage of Pax4 infected islet cells. However, inhibition was transient as glucose-induced insulin secretion was restored 6 days post infection at which time Pax4 expression has diminished considerably (data not shown, see also FIG. 5A). Inclusion of 1 μM forskolin/ 100 μM IBMX, which potentiates the effect of glucose on secretion by rising cAMP levels, restored glucose-induced insulin exocytosis indicating that events downstream of membrane depolarisation are functional in Pax4 expressing cells. The intracellular signalling events that couple metabolism to insulin secretion are triggered by glucose oxidation which stimulates ATP production, closure of $K_{ATP}$ channels, membrane depolarisation, influx of calcium and ultimately exocytosis (Maechler and Wollheim, 2001). To evaluate whether Pax4 curtails this cascade, glucose metabolism as well as ATP levels and mitochondrial calcium concentrations ($\{Ca^{2+}]_m$) were measured in transfected islets. The rate of glucose oxidation was estimated by measuring the conversion of D-[$^{14}C$(U)] to $^{14}CO_2$ in islets. Formation of $^{14}CO_2$ from 15 mM D-[U-$^{14}C$] glucose was equally efficient in both control and transduced-islets (4 fold increase, FIG. 7B). However, the inventors found that total cellular ATP levels were 4-fold higher in islets expressing Pax4 as compared to control LacZ islets (FIG. 8A). Total cellular ATP largely reflects sequestered pools in organelles in particular the mitochondria (Detimary et al., 1995). These results prompted us to investigate whether glucose was able to raise cytosolic ATP levels in Pax4 overexpressing islets using targeted expression of the ATP-sensitive bioluminescence probe luciferase (Ishihara et al., 2003). Addition of 16.5 mM glucose to control/LacZ islets resulted in a 23% increase of cytosolic ATP, which was sustained until the injection of azide, a compound that dissipates the mitochondrial membrane potential and thus interrupts ATP formation (FIG. 8B). Cytosolic ATP from islets maintained in 2.5 mM glucose gradually decreased to levels 80% of those at time of glucose injection consistent with low sustained energy consumption. Unexpectedly, basal cytosolic ATP in AdCMVPax4IRESGFP-infected islets was 30% of that measured in control islets. In addition, no significant increase in ATP production was detected in Pax4 islets exposed to 16.5 mM glucose (FIG. 8B). Mitochondrial calcium changes reflect those of the cytosol (Ishihara et al., 2003; Kennedy et al., 1996). Mitochondria targeted-expression of aequorin revealed that resting $[Ca^{2+}]_m$ was also elevated in the β-cells of Pax4-transduced islets, nearly 2-fold higher than controls (FIG. 8C). High concentrations of extracellular potassium trigger calcium influx across the plasma membrane independently of ATP production. The potassium-induced rise in $[Ca^{2+}]_m$ was normal in transduced islets, as assessed by both the maximum $[Ca^{2+}]_m$ response attained (peak height) and the total increase in $[Ca^{2+}]_m$ (area under the peak;

AUP). However, the glucose-induced increase in $[Ca^{2+}]_m$ (AUP) was blunted in Pax4 expressing islets (40±5% less than controls) although the initial peak response was unchanged. The observed elevation in resting levels of both total cellular ATP and mitochondrial calcium in AdCMVPax4IRBSGFP-transduced islets may have caused the attenuated ATP and $[Ca^{2+}]_m$ responses to glucose. These deficient responses in turn underlie blunted glucose-induced insulin secretion in Pax4 overexpressing islets.

**Example 8: Pax4 expression correlates with human islet β-cell replication**

Data presented in examples 2 to 7 suggest that Pax4 is implicated in rat islet β-cell proliferation through the coordinated induction of the c-myc-Id2 replication pathway and expression of the anti-apoptotic Blc-xL gene; see FIG. 9. In this example it was determined that exposure of human isletes to either mitogens or Pax4 overexpression induces β-cell replication.

Exposure to 11 mM glucose for 48 hours resulted in an 11-fold increase in Pax4 mRNA levels as compared to control islets cultured in 5.5 mM glucose for 24 hours (FIGS. 11A-B). No significant increase in Pax4 transcript was detected in islets maintained in either 11 or 25 mM glucose for 24 hours. Treatment of islets with either 0.5 nM betacellulin or activin A for 24 hours induced Pax4 mRNA levels by 8 and 4-fold, respectively, as compared to control (FIG. 11A). Pax4 transcript in TGF-β1-treated islets was unaltered. These results suggests that similar to rat islets, Pax4 expression in human islets is induced by factors known to promote β-cell proliferation.

To evaluate the impact of Pax4 on β-cell replication, human islets were infected with an adenoviral construct harbouring the Pax4 cDNA under the control of the CMV promoter (AdCMVPax4IRESGFP). Islets transduced with AdCMVPax4IRESGFP displayed significant BrdU incorporation as compared to control AdCaLacZ-infected islets (FIG. 11B). These results document that human β-cells show the same response pattern in terms of Pax4 expression and mitogenic effect as rat islets.

**Example 9: Assessment of Pax4 wild-type and loss-of-function mutant in human islet β-cell replication using a novel adenoviral inducing system**

By using an adenoviral-mediated inducible expression system which allows for the regulated production of Pax4 (ad-mPax4-myc WT in co-infection with Ad-X Tet-On) it could further be confirmed that Pax4 leads to proliferation of adult pancreatic cells.

To assess the contribution of Pax4 and its mutant variant on human islet proliferation, the inventors constructed inducible recombinant adenoviruses engineered to express these proteins. In this system, a second adenoviral construct bearing the tet-activator cDNA (Ad-X Tet-On) is co-infected along with the Pax4 recombinant adenoviral constructs (Ad-mPax4-myc WT or Ad-mPax4-myc R129W) in islets. Addition of the inducer, doxycycline (a tetracycline analog), activates the constitutively expressed reverse tet-activator protein (rtTA) that then binds to promoter sequences upstream of the adenoviral Pax4 cDNAs to initiate transcription. In order to detect the proteins after induction by doxycycline, a c-myc/6-HIS tag was integrated at the carboxy terminal end of the two polypeptides; see also example 6. Addition of the c-myc epitope antibody resulted in the supershift of both complexes (FIG. 12B) indicating functional fusion proteins. Primary human islets were then transduced with either Ad-mPax4-myc WT or Ad-mPax4-myc R129W along with Ad-X Tet-On at the optimal ratio of 2:1. In the absence of doxycycline, the immunoreactive c-myc epitope could not be detected in islet cells (FIG. 13A and 14A). However, addition of doxycycline, resulted in the induction of either mPax4-myc WT or R129W in the nuclei of approximately 70 % of islets cells (FIG. 13B and 14B). Concomitant with doxycycline-induced mPax4 WT expression, approximately 10% of cells incorporated BrdU indicative of cell proliferation (FIG. 13B). In contrast, stimulation of cell replication as assessed by BrdU could not be detected in cells expressing mPax4-myc R129W (FIG. 14B). Importantly, in the absence of doyxycline there was no visible β-cell proliferation (FIGS. 13A-B and 14A-B). These results clearly indicate that Pax4 is an important factor involved in islet cell replication and that the R129W mutation abrogates the function of the transcription factor.

**Example 10: Pax4 protects human islets from cell death.**

As documented above, stimulation of the transcription factor Pax4, either by mitogens or forced expression, promotes rat and human islet β-cell proliferation through c-myc activation while potentially protecting from apoptosis through Bcl-xL gene expression. The impact of Pax4 and its loss-of function mutant on human islet survival using the adenoviral-mediated inducible expression system described above is assess in this example.

Freshly isolated human islets, obtained from the Cell Isolation and Transplantation Laboratory in Geneva, were maintained for 48 hours in CMRL-1066 medium supplemented with 10% FCS, 100 Units/ml penicillin, 100 μg/ml streptomycin and 100 μg/ml gentamycin. Partially trypsinized islets were then infected with either Ad-mPax4-myc WT or Ad-mPax4-myc R129W (2.4 X $10^7$ pfu/ml) along with the adenoviral construct harbouring the tetracycline transcriptional activator (Ad-X Tet-On, 1.2 X $10^7$ pfu/ml). Cells were rinsed 90 minutes post infection and replenished with fresh media supplemented with or without doxycycline (0.25 and 0.5μg/ml) for 24 h post infection. Cells were then incubated for 24h in the presence of IFN-γ, IL-1β and TNF-α (2 ng/ml each) to induce apoptosis. Cell death was measured by direct TUNEL (terminal deoxynucleotidyl transferase (TdT)-mediated dUTP nick end labelling) assay. Briefly, DNA strand breaks (free 3'OH ends in genomic DNA) caused by cytokine-induced apoptosis were labelled with fluorescein-dUTP using an optimised TdT. Incorporated fluorescein-dUTP in apoptotic cells was then detected and quantified directly under a fluorescence microscope.

As shown above, Pax4 is an important factor involved in islet cell replication and an mutation, namely the R129W mutation abrogates the function of the transcription factor. In this experiment transduced human islets were exposed to a cocktail of cytokines to induce apoptosis. Adenoviral-infected islets in the absence of both doxycycline and cytokines exhibited a 1.3 fold increase in apoptosis as compared to control non-infected islets (Figure 15 and 16, B). Treatment with cytokines provoked a further 4.4 fold increase in cell death. In contrast, Pax4 wt expression (induced by the two concentrations of doxycycline) conferred complete protection against apoptosis (Figure 15A and B). The R129W mutation showed an attenuate protection against cytokine-induced cell death (Figure 16A and B).

Accordingly, Pax4 promotes survival in human islets. With the help of doxycycline inducible adenoviral vectors it was shown that the wild type Pax4 upon drug stimulation shielded human islet cells from cytokine-induced apoptosis while the mutant was less efficient Furthermore, Pax4 levels were maintained close to physiological ranges indicating a specific effect of the transcription factor on survival.

These results show that Pax4 promotes human islet cell replication, and, most importantly also confers survival through the activation of Bcl-xL. The diabetes-linked mutant form of Pax4 attenuates these cellular processes. It is of note that affected subjects suffer from β-cell death.

Three major conclusions arise as a result of our studies: 1) an adenoviral system has been developed that allows doxycycline-dependent expression of either wild-type or mutant Pax4 in primary human islets; 2) inducible expression of wild-type Pax4 stimulated proliferation while 3) induction of mutant R129W variant had no effect on cell division. These results strongly indicate that Pax4 is a key regulator of islet cell proliferation and that loss-of-function mutants impair β-cell replenishment. Such mutations may lead to reduced β-cell mass and thus could be causally related to the development of both type 1 and type 2 diabetes. A practical consequence of our findings would be to expand the β-cell mass *in vitro* by the regulated expression of Pax4 prior to islet transplantation for the treatment of diabetic patients. The controlled and transient expression of Pax4 avoids prolonged expression of a mitogenic protein that may have untoward effects on islet function.

**Example 11: RNA interference of Pax4 reveals a direct contribution of the transcription factor on survival of insulin-producing cells.**

As discussed above, the paired and homeo domain-containing transcription factor Pax4 plays an important role in β-cell development during embryogenesis while its function in mature islets is still controversial (Sosa-Pineda, 2004, Mol Cells 18, 289-94). Polymorphisms as well as mutations in this gene have been associated with both type 1 and type 2 diabetes suggesting a fundamental role of Pax4 in islet function (Biason-Lauber, 2005, Diabetologia 48, 900-905; Holm, 2004, Diabetes 53, 1584-91; Mauvais-Jarvis, 2004, Hum. Mol. Genet. 13, 3151-3159; Shimajiri, 2003, Biochem. Biophys. Res. Commun. 282, 34-40). Herein above, it was demonstrated that increased Pax4 expression, either by mitogens or forced expression, promotes β-cell proliferation in mature rat islets by simultaneously inducing the c-myc-Id2 pathway and the anti-apoptotic gene Bcl-xL. The impact of Pax4 and its diabetes linked variant R129W on human islet proliferation and survival was also assessed using novel doxycycline inducible recombinant adenoviruses engineered to express these proteins tagged to the myc epitope (Ad-mPax4-myc wt or Ad-mPax4-myc R129W). Induction of Pax4 wt expression stimulated β-cell replication and conferred complete protection against cytokine-induced apoptosis. In contrast, the R129W mutation showed an attenuated protection against cell death. Thus Pax4 promotes rat and human islet cell replication as well as conferring survival potentially through the activation of Bcl-xL. The diabetes-linked mutant form of Pax4 is incapable of reproducing these effects.

Furthermore, the impact of the mitogens activin A and betacellulin on endogenous Pax4 gene expression in human islets at high and low glucose concentrations was evaluated. In addition, a RNA interference (RNAi) strategy to suppress Pax4 in order to evaluate the direct contribution of the transcription factor on β-cell plasticity.

For this experiment, isolated human islets were exposed to 5.5 or 11 mM glucose complemented with 0.5 nM of activin A, betacellulin or TGF-β 1 for 24 hours. Steady state mRNA levels for Pax4 were quantified by real-time RT-PCR and normalized to cyclophilin. Two 21-nucleotide Pax4 hairpin RNA structures targeted to either the paired domain (siPD21; 5'-GCA GGC AAG AGA AGC TGA AAT-3'; SEQ ID NO: 21) or the homeodomain (siHD21; 5'-GGC TCG AAT TGC CCA GCT AAA-3'; SEQ ID NO: 22) of Pax4 were cloned into the pDLDU6 vector (Gauthier, 2003, Diabetologia 46, A47) (see Figure 18A). An extended siRNA of 29 nucleotides targeted to the same sequence of the paired domain was also generated and cloned into pDLDU6 (siPD29; 5'-GGC TCG AAT TGC CCA GCT AAA GGA TGA GT-3'; SEQ ID NO: 23). Constructs were transfected into the rat insulinoma cell line INS-1E along with GFP using lipofectamine. Subsequent to cell sorting using GFP (72 hours post-transfection), RNA was isolated and the effect of siPD21, siPD29 and siHD21 on endogenous Pax4, Pdx1 and Bcl-xL transcript levels was quantified by real time RT-PCR. Alternatively, 48 hours post transfection, cells were incubated with a cocktail of cytokines (1ng/ml of each Il-1, TNFα and INFγ) for an additional 24 hours and cell death was estimated using the TUNEL assay (Roche, CH).

Low but consistent Pax4 mRNA levels were detected in isolated human islets. Treatment with either 0.5 nM activin A or betacellulin in the presence or 5.5 mM glucose resulted in a 7- and 8-fold increase in the Pax4 transcript, respectively (Figure 17). In contrast, TGF-B 1 was ineffective. Interestingly, 11 mM glucose did not stimulate Pax4 gene expression and abrogated the effect of betacellulin and activin A (Figure 17). These results provide evidence that Pax4 activity is regulated by physiological stimuli in human islets.

However, glucose would appear to have an inhibitory effect on activin A and betacellulin-mediated induction of Pax4 gene expression in human islets. It was next evaluated the impact of Pax4 on β-cell survival by RNAi. The transformed INS-1E cell line was employed. Said cell line is characterized by uncontrolled cellular proliferation while retaining high levels of insulin and exhibiting normal stimulus-secretion coupling (Asfari, 1992, Endocrinology 130, 167-78; Merglen, 2004, Endocrinology 148, 667-78). Like human insulinomas (Miyamoto, 2001, Biochem. Biophys. Res. Commun. 282, 34-40), INS-1E cells express high levels of Pax4 mRNA and provide a useful tool to investigate the impact of the transcription factor on cell proliferation and survival. Pax4 steady state mRNA levels were lowered by 40, 55 and 60% in INS-1E cells co-expressing siPD21, siPD29 or siHD21, respectively along with GFP (Figure 18B). A 29-mer was more proficient in suppressing the Pax4 transcript as compared to a 21-mer directed against the same sequence within the paired domain of Pax4 (Siolas, 2005, Nat Biotechnol. 23, 227-31). Repression was specific since mRNA levels for Pdx1 remained constant in cells expressing either siPD21 or siHD21 (Figure 18C). It was also monitored STAT1 mRNA levels, which increase in response to induction of the interferon system (Sledz, 2003, Nat. Cell Biol. 5, 834-9). Similarly to Pdxl, STAT1 transcripts were unaltered indicating that the RNAi did not provoke an interferon response (data not shown). In contrast, mRNA levels for the Pax4 target gene Bcl-xL were suppressed by 40% using either siPD21 or siHD21 (Figure 18D). Inhibition of the anti-apoptotic gene did not promote INS-1E cell death under control conditions (data not shown). However, when challenged with a cocktail of cytokines, cells co-expressing phogrin-GFP along with siPD21, siPD29 or siHD21 displayed increased TUNEL as compared to INS-1E co-transfected with phogrin-GFP and U6 (Figure 19). Taken together, these results clearly indicate that suppression of Pax4 results in decreased Bcl-xL mRNA levels rendering INS-1E cells more susceptible to cytokine-induced apoptosis.

All of the compositions and methods disclosed and claimed herein can be made and executed without undue experimentation in light of the present disclosure. While the compositions and methods of this invention have been described in terms of preferred embodiments, it will be apparent to those of skill in the art that variations may be applied to the compositions and methods and in the steps or in the sequence of steps of the methods described herein without departing from the scope of the invention.

## X. Further References

The following references provide exemplary procedural or other details supplementary to those set forth herein.


U.S. Patent 4,683,202
U.S. Patent 4,797,368
U.S. Patent 4,892,538
U.S. Patent 5,139,941
U.S. Patent 5,626,561
U.S. Patent 5,787,900
U.S. Patent 5,843,069
U.S. Patent 5,928,944
U.S. Patent 6,013,638
U.S. Patent 6,071,697
U.S. Patent 6,099, 831
U.S. Patent 4,682,195
U.S. Patent 5,011,472
Altman et al., Diabetes, 35(6):625-633, 1986.
Altschul, J. Mol. Biol., 215:403-410, 1990.
Altschul, J. Mol. Evol., 36:290-300, 1993.
Altschul, Nucl. Acids Res., 25:3389-3402, 1997.
Ausubel et al., In: Current Protocols in Molecular Biology, John, Wiley & Sons, Inc, New York, 1996.
Baichwal and Sugden, In: Gene Transfer, Kucherlapai (Ed.), NY, Plenum Press, 117-148,1986.

Bauer et al., Blood, 86(6):2379-2387, 1995.
Becker et al., Meth. Cell. Biol., 43:161-189,1994.
Becker et al., Methods Cell Biol., 43(A)::161-189, 1994.
Becker et al., J. Biol. Chem., 269(33):21234-212338, 1994a.
Becker et al., J. Biol. Chem., 271(1):390-394, 1996.
Becker et al., Methods Cell Biol., 43(A):161-189, 1994b.
Bell, Nature,. 414:788-791, 2001.
Bestwick et al., Proc. Natl. Acad. Sci. USA, 85(15):5404-5408, 1988.
Blysczuk, PNAS, 100:908-1003, 2003.
Bonner-Weir, Endocrinology, 141:1926-1929, 2000.
Bonner-Weir, Trends Endocrinol Metab, 11:375-378, 2000.
Brandle, Diabetes Care, 24:1253-1258, 2001.
Brink, Mech. Dev., 100:37-43, 2001.
Brutlag, Comp. App. Biosci., 6:237-245, 1990.
Buteau, Diabetes, 52:124-132, 2003.
Butler, Diabetes, 52:102-110, 2003.
Campell, FEBS, 463:53-57, 1999.
Challet, J. Biol. Chem., 276:3791-3797, 2001.
Chan, Diabetes, 48:997-1005, 1999.
Chang et al., Hepatology, 14:134A, 1991.
Chen and Okayama, Mol. Cell Biol., 7(8):2745-2752, 1987.
Clark et al., Hum. Gene Ther., 6(10):1329-1341, 1995.
Clark et al., Am. J. Clin. Pathol., 93(1):58-69, 1990.
Coffin, In: Virology, Fields et al. (Eds.), Raven Press, NY, 1437-1500, 1990.
Collins, Biotechniques, 24:803-808, 1998.
Collombat, Genes Dev., 17:2591-2603, 2003.
Cotten et al., Proc. Natl. Acad. Sci. USA, 89(13):6094-6098, 1992.
Couch et al., Am. Rev. Resp. Dis., 88:394-403, 1963.
Coupar et al., Gene, 68:1-10,1988.
Curiel, Nat. Immun., 13(2-3):141-164, 1994.
Davis, Cancer Res., 54:2869-2872, 1994.
Demeterco, J. Clin. Endocrinol. Metab., 85:3892-3896, 2000.
Detimary, J. Clin. Invest., 96:1738-1745, 1995.
Dohrmann, Mech. Dev., 92:47-54, 2000.
Dor, Nature, 429:41-46,2004.
Drucker, Endocrinology, 142:521-527, 2001.
Dunbar, Int. J. Biochem. Cell Biol., 32:805-815, 2000.
Dupont, Diabetologica, 42:480-484, 1999.
Efrat et al., Proc. Natl. Acad. Sci. USA, 85(23):9037-9041, 1988. EPO 0273085
Fechheimer, et al., Proc Natl. Acad Sci. USA, 84:8463-8467, 1987.
Ferkol et al., FASEB J., 7:1081-1091, 1993.
Flotte and Carter, Gene Ther., 2(6):357-62, 1995.
Flotte et al., Am. J. Respir. Cell Mol. Biol., 7(3):349-356, 1992.
Flotte et al., Proc. Natl. Acad. Sci. USA, 90(22):10613-10617, 1993.
Fournier, Transplant Proc., 28:2866-2868, 1996.
Fraley et al., Proc. Natl. Acad. Sci. USA, 76:3348-3352, 1979.
Friedmann, Science, 244:1275-1281, 1989.
Fritschy et al. Diabetes, 40(1):37-43, 1991.
Froguel, N. Engl. J. Med., 328:697-702, 1993.
Fujitani, Mol. Cell Biol., 19:8281-8291, 1999.
Gainer et al., Transplantation, 61(11):1567-1571, 1996.
Galili, Nat. Genet., 5:230-235, 1993.
Gallichan et al., Hum. Gene Ther., 9(18):2717-2726, 1998.
Gauthier, Atherosclerosis, 142:301-307, 1999b.
Gauthier, J. Lipid Res., 40:1284-1293, 1999a.
Gauthier, Mol. Endocrinol., 16:170-183, 2002.
German et al., Mol. Cell Biol., 12(4):1777-1788, 1992.
German *et al.*, 1990

Ghosh and Bachhawat, In: Liver Diseases, Targeted Diagnosis and Therapy Using Specific Receptors and Ligands, Wu et al. (Eds.), Marcel Dekker, NY, 87-104,1991.
Ghosh-Choudhury et al., EMBO J., 6:1733-1739, 1987.
Gittes, Proc. Natl. Acad. Sci. USA, 89:1128-1132,1992.
Gomez-Foix et al., J. Biol. Chem., 267:25129-25134, 1992.
Gopal, Mol. Cell Biol., 5:1188-1190, 1985.
Graham and Prevec, In: Methods in Molecular Biology: Gene Transfer and Expression Protocol, Murray (Ed.), Humana Press, Clifton, NJ, 7:109-128, 1991.
Graham and Van Der Eb, Virology, 52:456-467, 1973.
Graham et al, J. General Virology, 36:59-74,1977.
Grunhaus and Horwitz, Seminar in Virology, 3:237-252, 1992.
Hagenfeldt-Johansson, Endocrinology, 142:5311-5320, 2001.
Hansen, J. Clin. Endocrinol. Metab., 85:323-1326, 2000.
Harland and Weintraub, J. Cell Biol., 101(3):1094-1099, 1985.
Hellman, Ann. NY Acad. Sci., 131:541-558, 1965.
Heremans, J. Cell Biol., 159:303-312,2002.
Hermonat and Muzycska, Proc. Natl. Acad. Sci. USA, 81:6466-6470, 1984.
Hersdorffer et al., DNA Cell Biol., 9:713-723, 1990.
Herz and Gerard, Proc. Natl. Acad. Sci. USA, 90:2812-2816, 1993.
Higgins and Hames, In: Nucleic acid hybridization, a practical approach, IRL Press Oxford, Washington DC, 1985.
Holm et al., Diabetes, 53:1584-1591, 2004.
Horwich et al. J. Virol., 64:642-650, 1990.
Ishihara, J. Clin. Invest., 104:1621-1629, 1999.
Ishihara, Nat. Cell Biol., 5:330-335, 2003.
Janjic, Pancreas, 13:166-172, 1996.
Jones and Shenk, Cell, 13:181-188, 1978.
Kafri et al., J. Virol., 73(1):576-584, 1999.
Kahan, Diabetes, 52:2016-2024,2003.
Kanatsuka, Metabolism, 51:1161-1165,2002.
Kaneda et al., Science, 243:375-378, 1989.
Kaplitt et al., Nat Genet., 8(2):148-154, 1994.
Karlsson et al., EMBO J., 5:2377-2385, 1986.
Kashyap, Diabetes, 52:2461-2474, 2003.
Kato et al, J. Biol. Chem., 266:3361-3364, 1991.
Kelleher and Vos, Biotechniques, 17(6):1110-7, 1994.
Kennedy, J. Clin. Invest., 98:2524-2538, 1996.
Kiem et al., Blood, 83(6):1467-1473, 1994.
Klein et al., Nature, 327:70-73, 1987.
Kloppel, Surv. Synth. Pathol. Res., 4:110-125, 1985.
Kojima, Nat. Med., 9:596-603, 2003.
Kotin et al., Proc. Natl. Acad. Sci. USA, 87(6):2211-2215, 1990.
Kozmik, Proc. Natl. Acad. Sci. USA, 92:5709-5713. 1995.
Kristinsson, Diabetologia, 44:2098-2103, 2001.
Lacy et al., Science, 254(5039):1782-1784, 1991.
LaFace et al., Virology, 162(2):483-486, 1988.
Lasorella, Nature, 407:592-598, 2000.
Laughlin et al., J. Virol., 60(2):515-524, 1986.
Le Gal La Salle et al., Science, 259:988-990, 1993.
Lebkowski et al., Mol. Cell. Biol., 8(10):3988-3996, 1988.
Levrero et al., Gene, 101:195-202, 1991.
Liang *et al.*, 1996
Luo et al., Proc. Natl. Acad. Sci. USA, 93:8907-8912, 1996.
Maassen, Exp. Clin. Endocrinol. Diabetes, 109:127-134,2001.
Madsen et al., Proc. Natl. Acad. Sci. USA, 85(18):6652-6656, 1988.
Maechler, Nature, 402:685-689, 1999.
Maechler, Nature, 414:807-812, 2001.
Maedler, Diabeetes, 50:1683-1690, 2001.
Malecki, Nature Genet., 23:323-328, 1999.

Mann et al., Cell, 33:153-159, 1983.
Margue, Oncogene, 19:2921-2929,2000.
Markowitz et al., J. Viro/., 62:1120-1124, 1988.
McCarty et al., J. Virol., 65(6):2936-2945, 1991.
McLaughlin et al., J. Virol., 62(6):1963-1973, 1988.
Melloul et al., Proc. Natl. Acad Sci. USA, 90(9):3865-3869, 1993.
Merglen, Endocrinology, 145:667-678, 2004.
Michael, Mol. Cell, 6:87-97, 2000.
Miller and Rosman, Biotechniques, 7(9):980-982, 984-986, 989-990, 1989.
Miller et al., Am. J. Clin. Oncol., 15(3):216-221, 1992.
Miller et al., J. Pharmacol. Exp. Ther., 264:11-16, 1993.
Mitchell, Mol. Genet. Metab., 77:35-43, 2002.
Miyamoto, Biochem. Biophys. Res. Commun., 282:34-40, 2001.
Miyoshi et al., J Virol., 72(10):8150-8157, 1998.
Muratovska, Oncogene, 22:6045-6053, 2003.
Muzyczka, Curr. Topics Microbiol. Immunol., 158:97-129, 1992.
Nicolas and Rubenstein, In: Vectors: A survey of molecular cloning vectors and their uses, Rodriguez and Denhardt (Eds.), Stoneham: Butterworth, 494-513, 1988.
Nicolau and Sene, Biochim. Biophys. Acta, 721:185-190,1982.
Nicolau et al., Methods Enzymol., 149:157-176, 1987.
Ohi et al., Gene, 89(2):279-282, 1990.
Osborne et al., Hum. Gene Ther., 1(1):31-41, 1990.
O'Shea and Sun, Diabetes, 35(8):943-946, 1986.
Paris, Endocrinology, 144:2717-2727, 2003.
Paskind et al., Virology, 67:242-248, 1975.
PCT Appln. PCT/US99/00553
PCT Appln. WO 89/01967
PCT Appln. WO 90/02580
PCT Appln. WO 90/15637
PCT Appln. WO 91/09939
PCT Appln. WO 91/10425
PCT Appln. WO 91/10470
PCT Appln. WO 98/29566
PCT Appln. WO95/29989
Pelengaris, Cell, 109:321-334,2002.
Perales et al., Proc. Natl. Acad. Sci. USA, 91:4086-4090,1994.
Potter et al., Proc. Natl. Acad Sci. USA, 81:7161-7165, 1984.
Racher et al., Biotechnology Techniques, 9:169-174, 1995.
Ragot et al., Nature, 361:647-650, 1993.
Renan, Radiother. Oncol., 19:197-218, 1990.
Rich et al., Hum. Gene Ther., 4:461-476, 1993.
Ridgeway, In: Vectors: A survey of molecular cloning vectors and their uses, Rodriguez and Denhardt (Eds.), Stoneham:Butterworth, 467-492, 1988.
Rippe et al., Mol. Cell Biol., 10:689-695, 1990.
Ritz-Laser, Diabetologia, 45:97-107, 2002.
Rosenfeld et al., Science, 252:431-434, 1991.
Rosenfeld, et al., Cell, 68:143-155, 1992.
Roux et al., Proc. Natl. Acad. Sci. USA, 86:9079-9083, 1989.
Ryan, Diabetes, 50:710-719, 2001.
Ryan, Diabetes, 51:2148-2157, 2002.
Ryffel, J. Mol. Endocrinol., 27:11-29, 2001.
Salehi, J. Physiol., 15:579-591, 1999.
Saltiel, Nature, 414 :99-806, 2001.
Sambrook et al., In: Molecular cloning, Cold Spring Harbor Laboratory Press, Cold Spring Harbor, NY, 2001.
Samulski et al., EMBO J., 10:3941-3950, 1991.
Samulski et al., J. Virol, 63:3822-3828, 1989.
Samulski et al., J. Virol, 63:3822-3828, 1989.
Sato et al., Proc. Natl. Acad. Sci. U.S.A. 48:1184-1190, 1962.

Schreiber, EMBO J., 7:4221-4229, 1988.
Schwitzgebel, Mol. Cell Endocrinol., 185:99-108, 2001.
Shapiro, N. Engl. J. Med., 343:230-238, 2000.
Shelling and Smith, Gene Therapy, 1:165-169, 1994.
Shih, Diabetes, 50:2472-2480, 2001.
Shimajiri, Biochem. Biophys. Res. Commun., 302:342-344, 2003.
Shimajiri, Diabetes, 50:2864-2869, 2001.
Smith, J. Biol. Chem., 275:36910-36919, 2000.
Smith, Mol. Endocrinol., 18:142-149, 2004.
Soldevila et al. J. Autoimmun., 4(2):291-306,1991.
Sosa-Pineda, Nature, 386:399-402, 1997.
Stockschlaeder et al., Hum. Gene Ther., 2(1):33-39, 1991.
Stoffers, J. Clin. Invest., 102:232-241, 1998.
Stratford-Perricaudet and Perricaudet, In: Human Gene Transfer, Eds, Cohen-Haguenauer and Boiron, John Libbey Eurotext, France, 51-61,1991.
Stratford-Perricaudet et al., Hum. Gene. Ther., 1:241-256, 1990.
Street, Int. J. Biochem. Cell Biol., 3 6:667-683, 2004.
Sullivan et al., J. Infect. Dis., 164(4):781-784, 1991.
Tao, Diabetes, 47:1650-1653, 1998.
Temin, In: Gene Transfer, Kucherlapati (Ed.), NY, Plenum Press, 149-188, 1986.
Thompson, Nucl. Acids Res., 2:4673-4680, 1994.
Top et al., J. Infect. Dis., 124:155-160, 1971.
Tratschin et al., Mol. Cell. Biol., 4:2072-2081, 1984.
Tratschin et al., Mol. Cell. Biol., 5:3258-3260, 1985.
Tur-Kaspa et al., Mol. Cell Biol., 6:716-718, 1986.
Ueda, FEBS Lett., 480:101-105, 2000.
Ueda, J. Virol., 70:4714-472, 1996.
Van der Laan et al., Nature, 407:90-94, 2000.
Wagner et al., Proc. Natl. Acad. Sci. USA 87(9):3410-3414, 1990.
Walsh et al., J. Clin. Invest, 94:1440-1448, 1994.
Walsh et al., J. Clin. Invest, 94:1440-1448, 1994.
Wang, Dev. Biol., 266:178-189, 2400.
Wei et al., Gene Therapy, 1:261-268, 1994.
Weir, Semin. Cell. Dev. Biol., 15:347-357,2004.
Weng, Diabetologia, 44:249-258,2001.
Wong et al., Gene, 10:87-94,1980.
Wu and Wu, Adv. Drug Delivery Rev., 12:159-167, 1993.
Wu and Wu, Biochemistry, 27:887-892,1988.
Wu and Wu, J. Biol. Chem., 262:4429-4432, 1987.
Xu, Diabetes, 48:2270-2276,1999.
Xu, Mol. Cell Endocrinol., 170:79-89, 2000.
Yamagata, Diabetes, 51:114-123, 2002.
Yang et al., J. Virol., 68:4847-4856, 1994.
Yang et al., Proc. Natl. Acad Sci. USA, 87:9568-9572, 1990.
Yoder et al., Blood, 82 (Supp.): 1:347A, 1994.
Zalzman, Proc. Natl. Acad. Sci. USA, 100:2426-2431, 2003.
Zhang, Diabetes, 50(1):S10-14, 2001.
Zhou et al., Exp. Hematol, 21:928-933, 1993.
Zhou et al., J. Exp. Med., 179:1867-1875, 1994.
Zhou, Am. J. Physiol Endocrinol. Metab., 278:E340-351, 2000.
Zimmet, Nature, 414:782-787, 2001.

## Claims

**1.** An in vitro method for the generation and isolation of pancreatic β-cells, comprising the steps of:

(a) providing an adult cell derived from mammalian pancreatic islets or an explant culture of adult pancreatic islets with functional, wild-type Pax4; and
(b) detecting and isolating, from said adult cell or explant culture, β-cells that proliferate in response to the contact with Pax4.

**2.** The method of claim 1, wherein said functional wild-type Pax4 is administered to said cell or explant culture in form of a nucleic acid molecule.

**3.** The method of claim 1, wherein said functional wild-type Pax4 is provided to said cell or explant culture in form of a Pax4 gene expression product or a functional fragment thereof.

**4.** The method of claim 3, wherein the Pax4 gene expression product is an mRNA or a protein.

**5.** The method of claim 1 or 2, wherein said Pax4 is provided as a nucleic acid molecule comprised in a vector.

**6.** The method of claim 5, wherein said vector is a viral vector.

**7.** The method of claim 6, wherein said viral vector is selected from the group consisting of a retroviral vector, a lentiviral vector and an adenoviral vector.

**8.** The method of claim 7, wherein said adenoviral vector is AdCMV or pHVAd2.

**9.** The method of any one of claims 1 to 8, wherein the functional, wild-type Pax4 comprises the wild-type Pax4 of mouse, rat or human.

**10.** The method of any one of claims 1 to 9, wherein said functional, wild-type Pax4 is encoded by

(a) a nucleic acid molecule comprising a nucleic acid molecule encoding the polypeptide having the amino acid sequence as shown in SEQ ID NO: 2, 4, or 6;
(b) a nucleic acid molecule comprising a nucleic acid molecule having the DNA sequence as shown in SEQ ID NO: 1, 3, or 5;
(c) a nucleic acid molecule hybridizing to the complementary strand of nucleic acid molecules of (a) or (b) and encoding a functional wild-type Pax4; and
(d) a nucleic acid molecule being degenerate as a result of the genetic code to the nucleotide sequence of the nucleic acid molecule as defined in (c).

## Patentansprüche

**1.** *In vitro*-Verfahren für die Erzeugung und Isolierung von β-Zellen des Pankreas, umfassend die Schritte:

(a) Bereitstellen einer von Säugerpankreas-Inseln stammenden adulten Zelle oder einer Explantatkultur von adulten Pankreas-Inseln mit funktionellem Wildtyp-Pax4;und
(b) Nachweisen und Isolieren von β-Zellen von der adulten Zelle oder Explantatkultur, die als Antwort auf das Inkontaktbringen mit Pax4 proliferieren.

**2.** Verfahren nach Anspruch 1, wobei das funktionelle Wildtyp-Pax4 der Zelle oder Explantatkultur in Form eines Nucleinsäuremoleküls verabreicht wird.

**3.** Verfahren nach Anspruch 1, wobei das funktionelle Wildtyp-Pax4 der Zelle oder Explantatkultur in Form eines Pax4-Gen-Expressionsprodukts oder eines funktionellen Fragments davon zugeführt wird.

**4.** Verfahren nach Anspruch 3, wobei das Pax4-Gen-Expressionsprodukt eine mRNA oder ein Protein ist.

**5.** Verfahren nach Anspruch 1 oder 2, wobei Pax4 als ein in einem Vektor enthaltenes Nucleinsäuremolekül bereitgestellt wird.

**6.** Verfahren nach Anspruch 5, wobei der Vektor ein viraler Vektor ist.

**7.** Verfahren nach Anspruch 6, wobei der virale Vektor ausgewählt ist aus der Gruppe bestehend aus einem retroviralen Vektor, einem lentiviralen Vektor und einem adenoviralen Vektor.

**8.** Verfahren nach Anspruch 7, wobei der adenovirale Vektor AdCMV oder pHVAd2 ist.

**9.** Verfahren nach einem der Ansprüche 1 bis 8, wobei das funktionelle Wildtyp-Pax4 das Wiltyp-Pax4 von Maus, Ratte oder Mensch umfasst.

**10.** Verfahren nach einem der Ansprüche 1 bis 9, wobei das funktionelle Wildtyp-Pax4 codiert wird durch

(a) ein Nucleinsäuremolekül, umfassend ein Nucleinsäuremolekül, das das Polypeptid mit der in SEQ ID NO: 2, 4 oder 6 dargestellten Aminosäuresequenz codiert;
(b) ein Nucleinsäuremolekül, umfassend ein Nucleinsäuremolekül mit der in SEQ ID NO: 1, 3 oder 5 dargestellten DNA-Sequenz;
(c) ein Nucleinsäuremolekül, das an den komplementären Strang der Nucleinsäuremoleküle von (a) oder (b) hybridisiert und das ein funktionelles Wildtyp-Pax4 codiert; und
(d) ein Nucleinsäuremolekül, das als Ergebnis des genetischen Codes zu der Nucleotidsequenz des Nucleinsäuremoleküls wie in (c) definiert, degeneriert ist.

**Revendications**

**1.** Procédé in vitro pour la production et l'isolement de cellules pancréatiques β, comprenant les étapes de :

(a) fourniture d'une cellule adulte dérivée d'îlots pancréatiques de mammifère ou d'une culture d'explant d'îlots pancréatiques adultes avec Pax4 de type sauvage, fonctionnel ; et
(b) détection et isolement, à partir de ladite cellule adulte ou culture d'explant, de cellules β qui prolifèrent en réponse au contact avec Pax4.

**2.** Procédé selon la revendication 1, dans lequel ledit Pax4 de type sauvage fonctionnel est administré à ladite cellule ou culture d'explant sous la forme d'une molécule d'acide nucléique.

**3.** Procédé selon la revendication 1, dans lequel ledit Pax4 de type sauvage fonctionnel est fourni à ladite cellule ou culture d'explant sous la forme d'un produit d'expression du gène Pax4 ou d'un fragment fonctionnel de celui-ci.

**4.** Procédé selon la revendication 3, dans lequel le produit d'expression du gène Pax4 est un ARNm ou une protéine.

**5.** Procédé selon la revendication 1 ou 2, dans lequel ledit Pax4 est fourni sous la forme d'une molécule d'acide nucléique comprise dans un vecteur.

**6.** Procédé selon la revendication 5, dans lequel ledit vecteur est un vecteur viral.

**7.** Procédé selon la revendication 6, dans lequel ledit vecteur viral est choisi dans le groupe constitué d'un vecteur rétroviral, un vecteur lentiviral et un vecteur adénoviral.

**8.** Procédé selon la revendication 7, dans lequel ledit vecteur adénoviral est AdCMV ou pHVAd2.

**9.** Procédé selon l'une quelconque des revendications 1 à 8, dans lequel Pax4 de type sauvage, fonctionnel, comprend le Pax4 de type sauvage de souris, de rat ou d'humain.

**10.** Procédé selon l'une quelconque des revendications 1 à 9, dans lequel ledit Pax4 de type sauvage, fonctionnel est codé par

(a) une molécule d'acide nucléique comprenant une molécule d'acide nucléique codant pour le polypeptide ayant la séquence d'acides aminés telle qu'indiquée dans SEQ ID NO: 2, 4, ou 6 ;
(b) une molécule d'acide nucléique comprenant une molécule d'acide nucléique ayant la séquence d'ADN telle qu'indiquée dans SEQ ID NO: 1, 3, ou 5 ;
(c) une molécule d'acide nucléique s'hybridant au brin complémentaire des molécules d'acide nucléique de (a) ou (b) et codant pour un Pax4 de type sauvage fonctionnel ; et
(d) une molécule d'acide nucléique qui est dégénérée, du fait du code génétique, par rapport à la séquence nucléotidique de la molécule d'acide nucléique telle que définie en (c).

# Figure 1

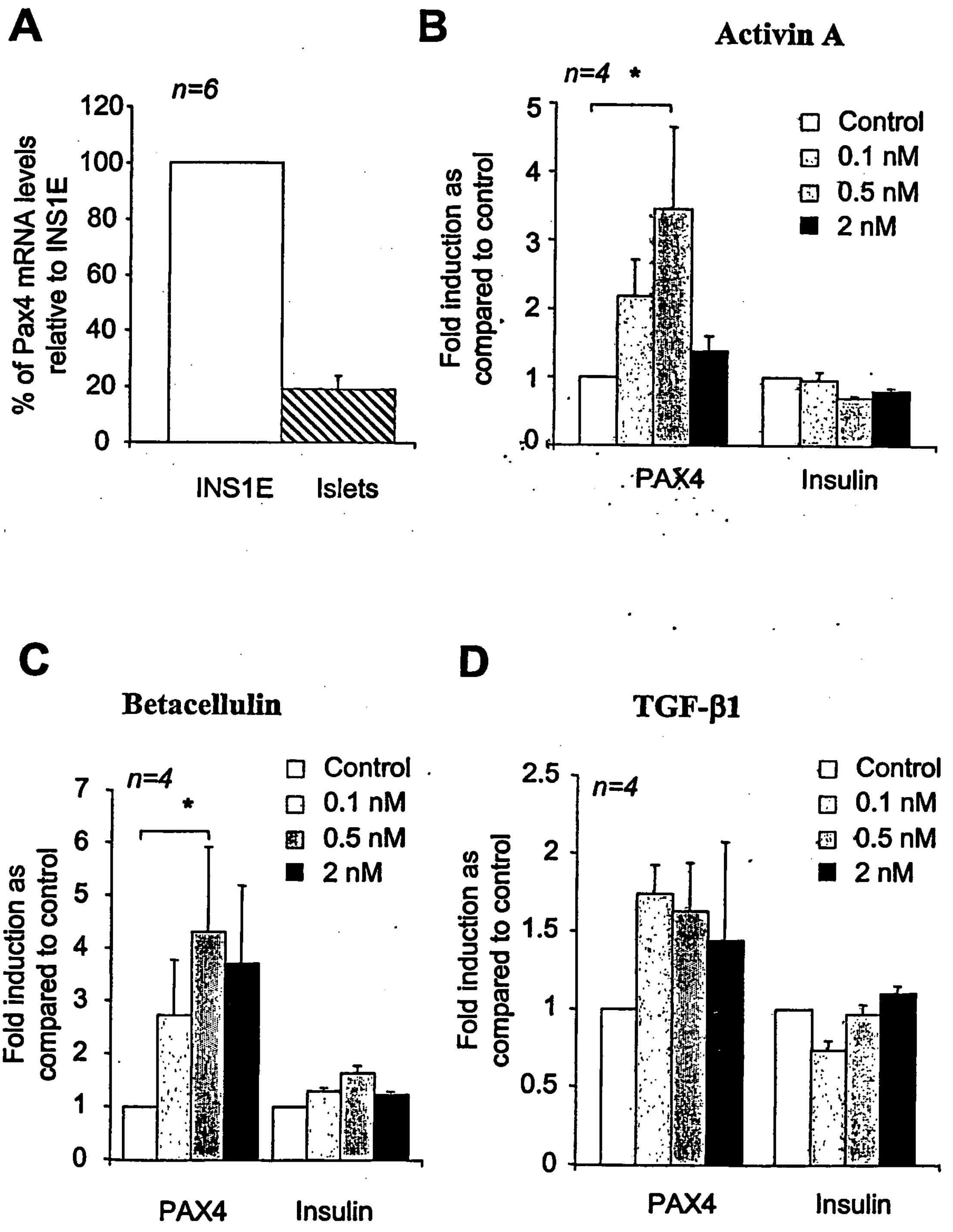
A
n=6
% of Pax4 mRNA levels relative to INS1E
120
100
80
60
40
20
0
INS1E
Islets
B
Activin A
n=4
*
Fold induction as compared to control
5
4
3
2
1
0
Control
0.1 nM
0.5 nM
2 nM
PAX4
Insulin
C
Betacellulin
n=4
*
Fold induction as compared to control
7
6
5
4
3
2
1
0
Control
0.1 nM
0.5 nM
2 nM
PAX4
Insulin
D
TGF-β1
n=4
Fold induction as compared to control
2.5
2
1.5
1
0.5
0
Control
0.1 nM
0.5 nM
2 nM
PAX4
Insulin

# Figure 2

A

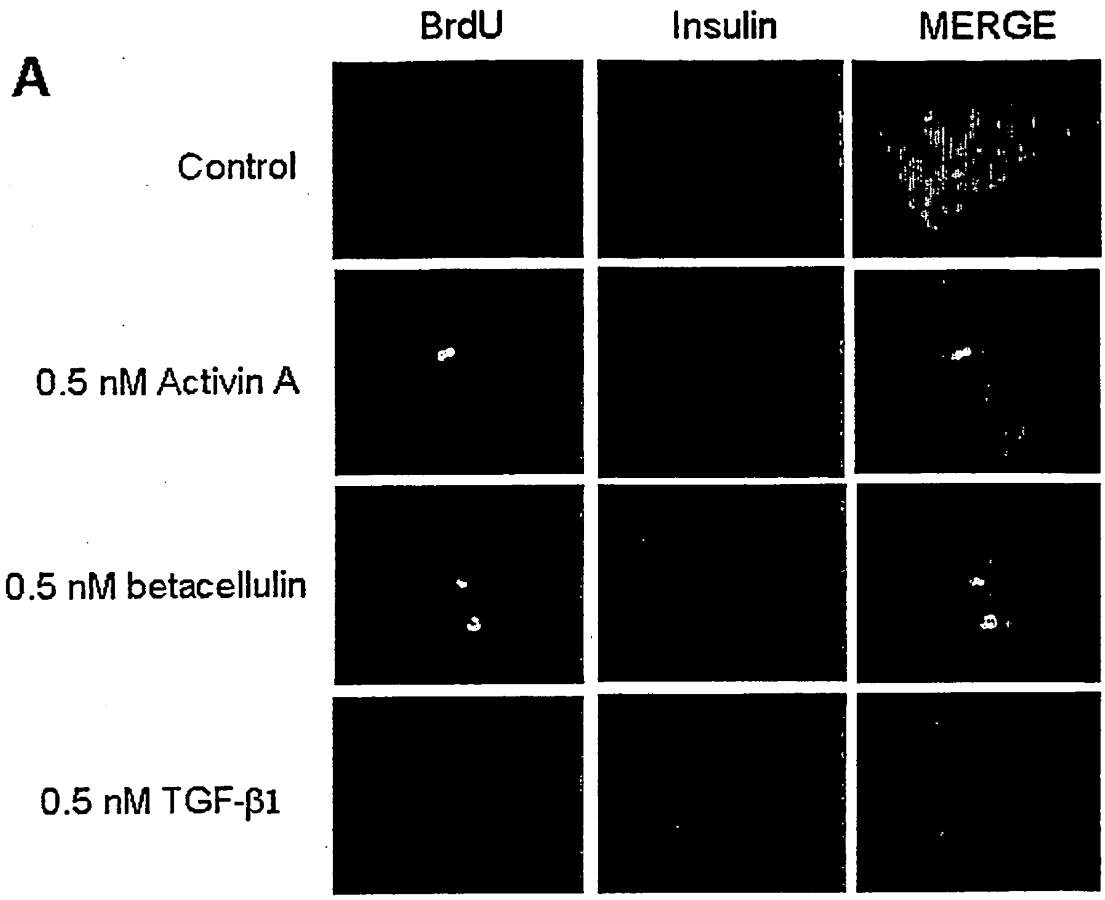
BrdU
Insulin
MERGE
Control
0.5 nM Activin A
0.5 nM betacellulin
0.5 nM TGF-β1

B

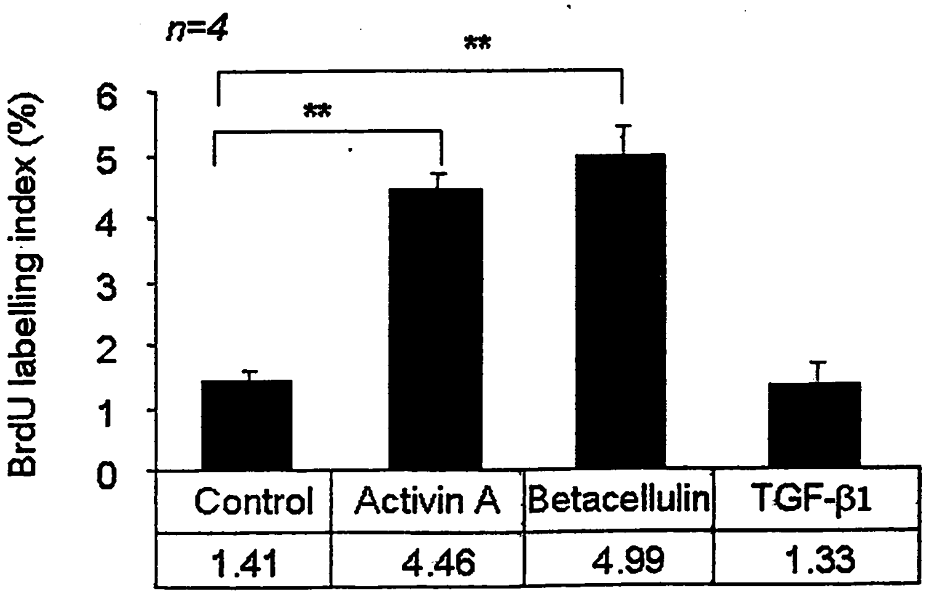
n=4
**
**
BrdU labelling index (%)
6
5
4
3
2
1
0
Control
Activin A
Betacellulin
TGF-β1
1.41
4.46
4.99
1.33

# Figure 3

## A

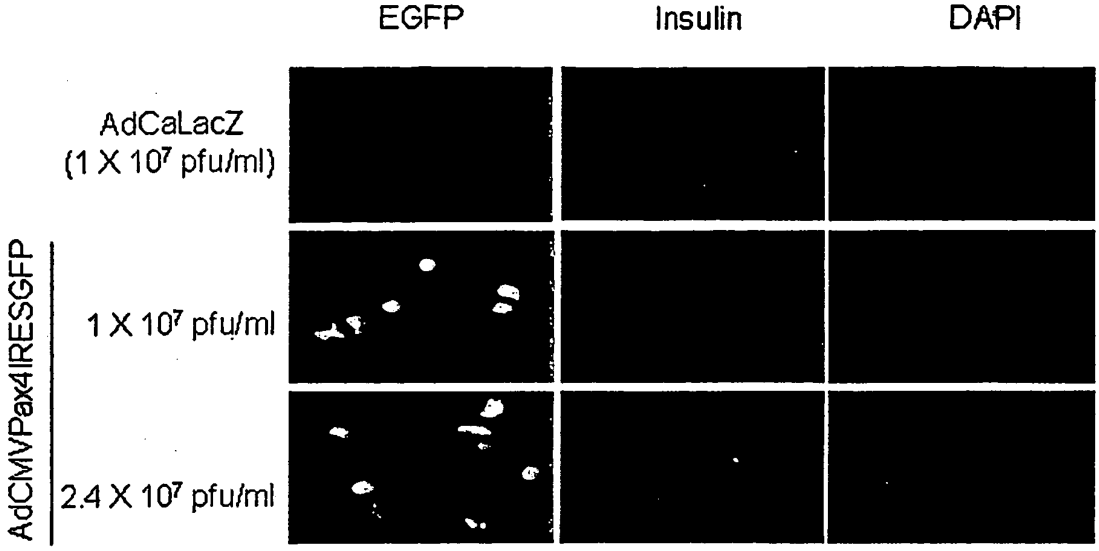
EGFP
Insulin
DAPI
AdCaLacZ
(1 X 10⁷ pfu/ml)
AdCMVPax4IRESGFP
1 X 10⁷ pfu/ml
2.4 X 10⁷ pfu/ml

## B

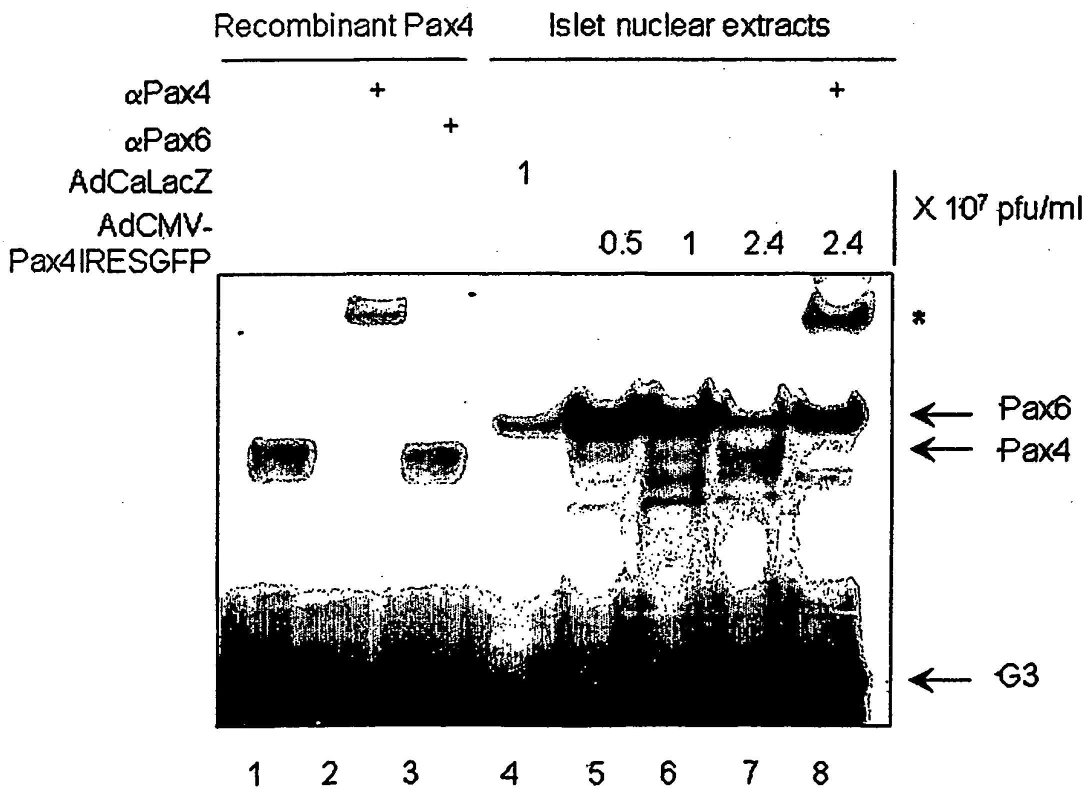
Recombinant Pax4
Islet nuclear extracts
αPax4
αPax6
AdCaLacZ
AdCMV-Pax4IRESGFP
+
+
1
0.5
1
2.4
2.4
+
X 10⁷ pfu/ml
*
Pax6
Pax4
G3
1 2 3 4 5 6 7 8

# Figure 4

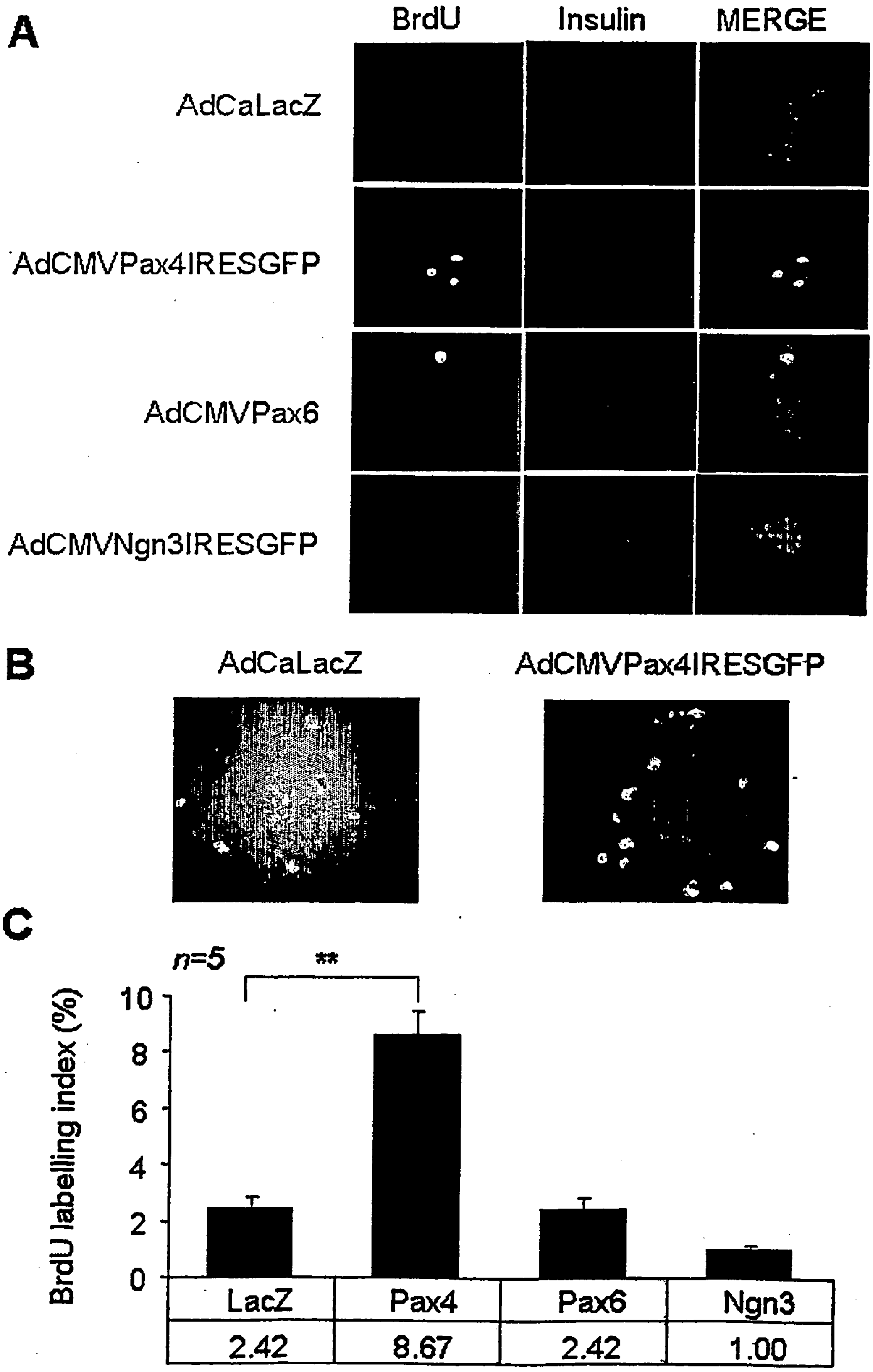
A
BrdU
Insulin
MERGE
AdCaLacZ
AdCMVPax4IRESGFP
AdCMVPax6
AdCMVNgn3IRESGFP
B
AdCaLacZ
AdCMVPax4IRESGFP
C
n=5
**
BrdU labelling index (%)
10
8
6
4
2
0
LacZ
Pax4
Pax6
Ngn3
2.42
8.67
2.42
1.00

# Figure 5

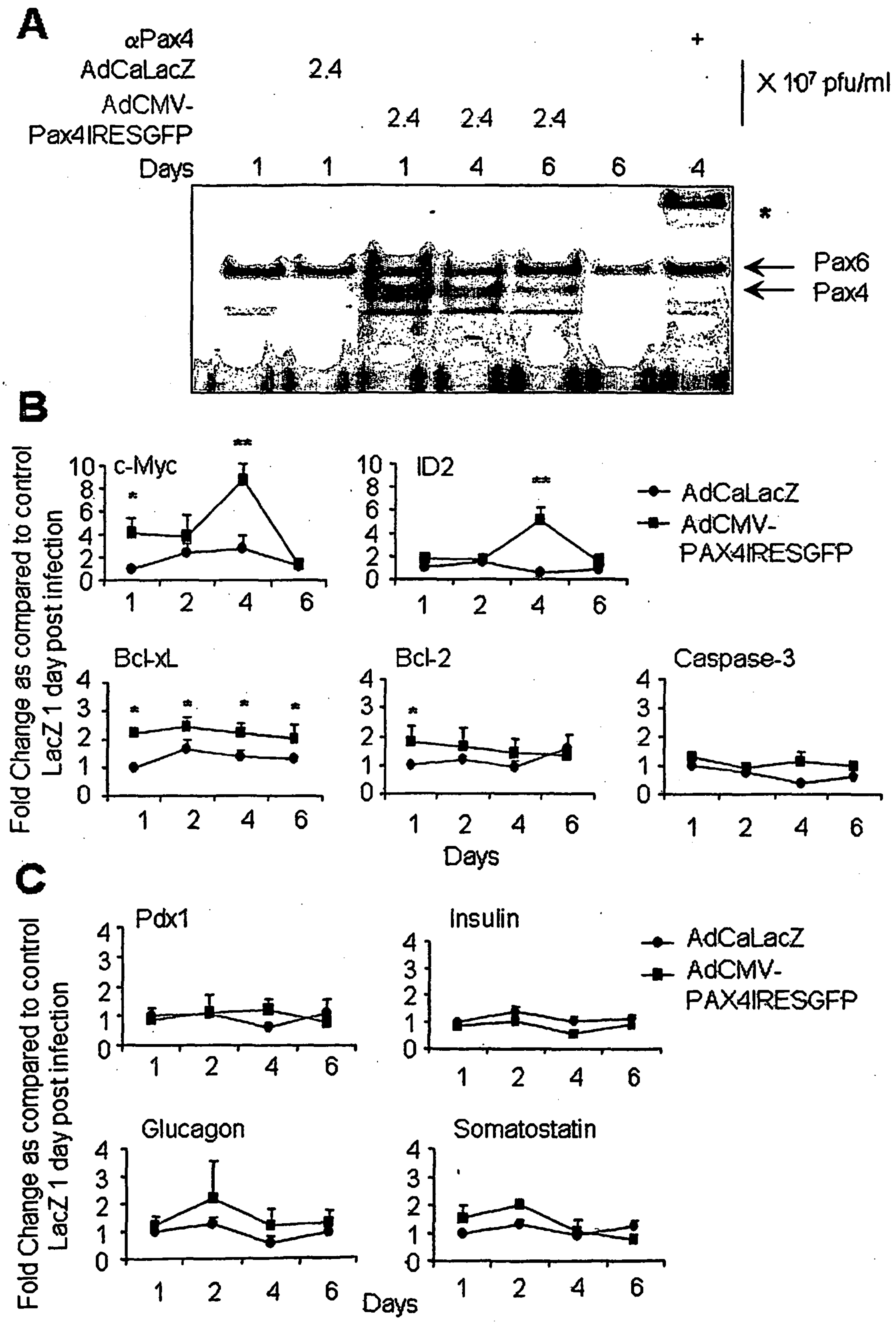
A
αPax4
AdCaLacZ
AdCMV-Pax4IRESGFP
Days
2.4
2.4 2.4 2.4
+
1 1 1 4 6 6 4
X $10^7$ pfu/ml
*
Pax6
Pax4
B
Fold Change as compared to control LacZ 1 day post infection
c-Myc
ID2
Bcl-xL
Bcl-2
Caspase-3
AdCaLacZ
AdCMV-PAX4IRESGFP
Days
C
Pdx1
Insulin
Glucagon
Somatostatin
AdCaLacZ
AdCMV-PAX4IRESGFP
Days

# Figure 6

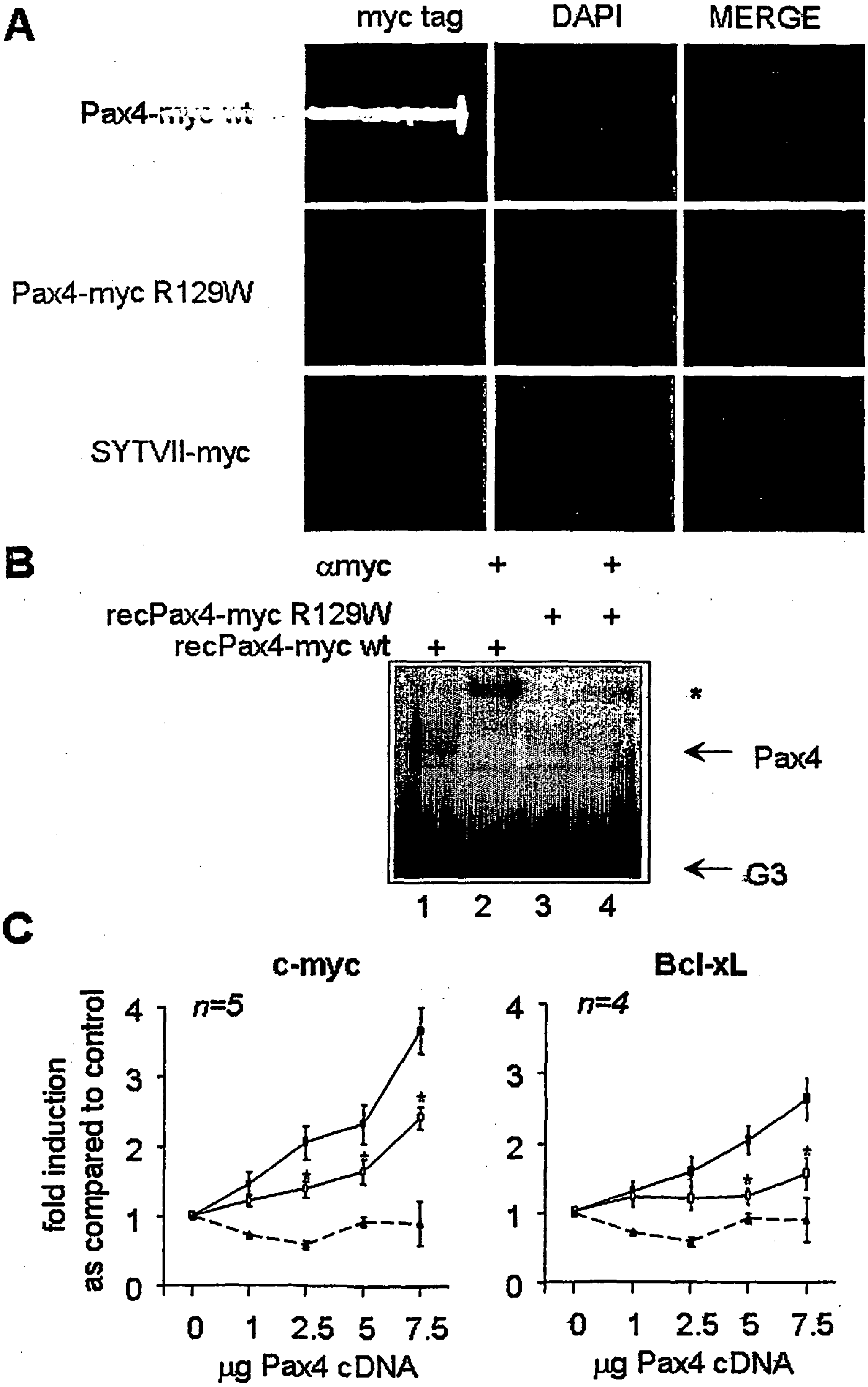
A
myc tag
DAPI
MERGE
Pax4-myc wt
Pax4-myc R129W
SYTVII-myc
B
αmyc + +
recPax4-myc R129W + +
recPax4-myc wt + +
*
Pax4
G3
1 2 3 4
C
c-myc
Bcl-xL
fold induction as compared to control
n=5
n=4
0 1 2.5 5 7.5
µg Pax4 cDNA

# Figure 7

## A

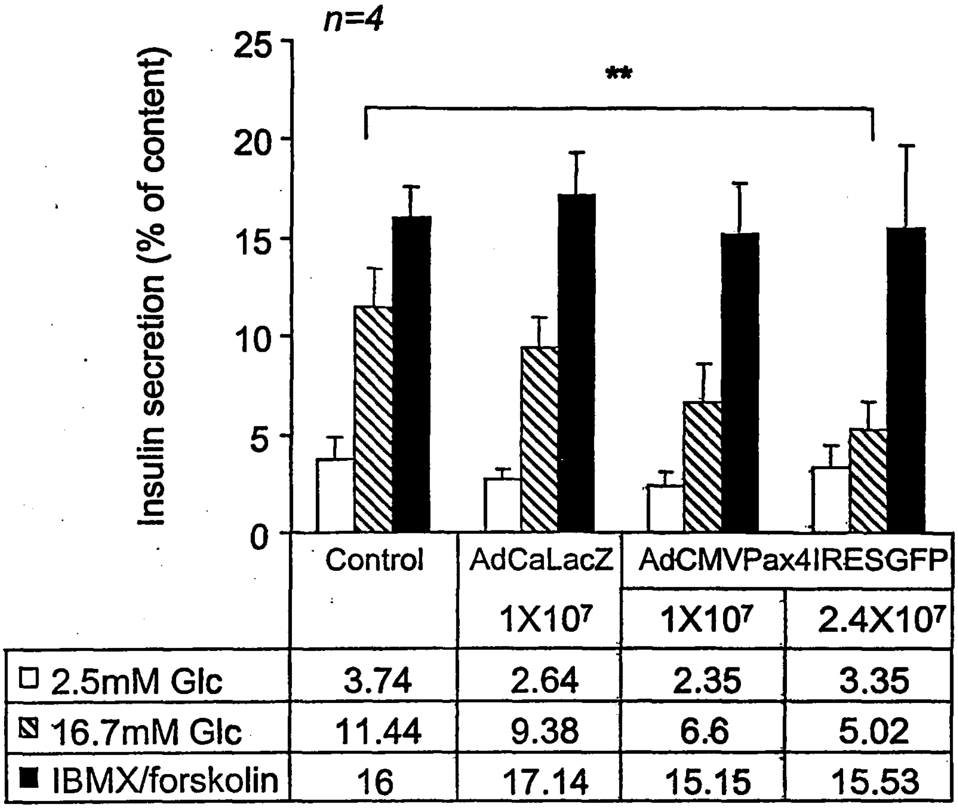


| | Control | AdCaLacZ | AdCMVPax4IRESGFP | |
|---|---|---|---|---|
| | | $1X10^7$ | $1X10^7$ | $2.4X10^7$ |
| □ 2.5mM Glc | 3.74 | 2.64 | 2.35 | 3.35 |
| ▧ 16.7mM Glc | 11.44 | 9.38 | 6.6 | 5.02 |
| ■ IBMX/forskolin | 16 | 17.14 | 15.15 | 15.53 |

## B

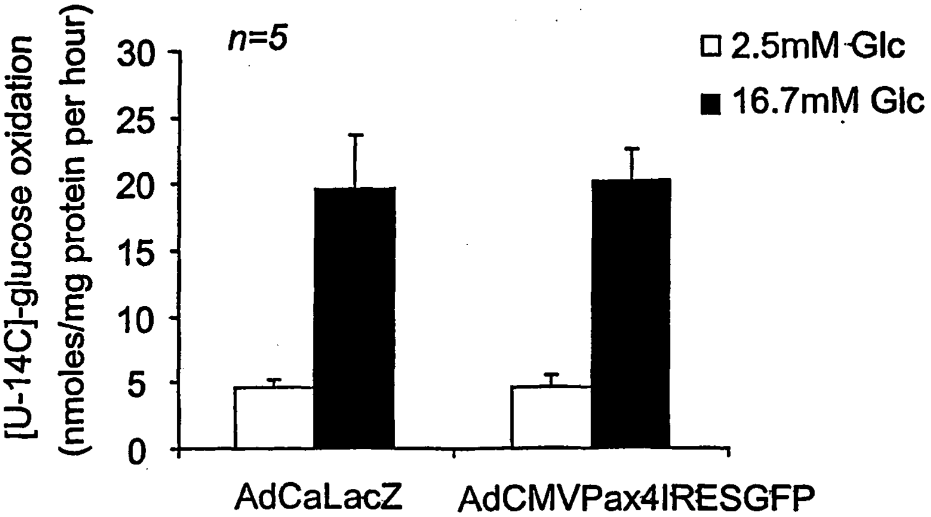

# Figure 8

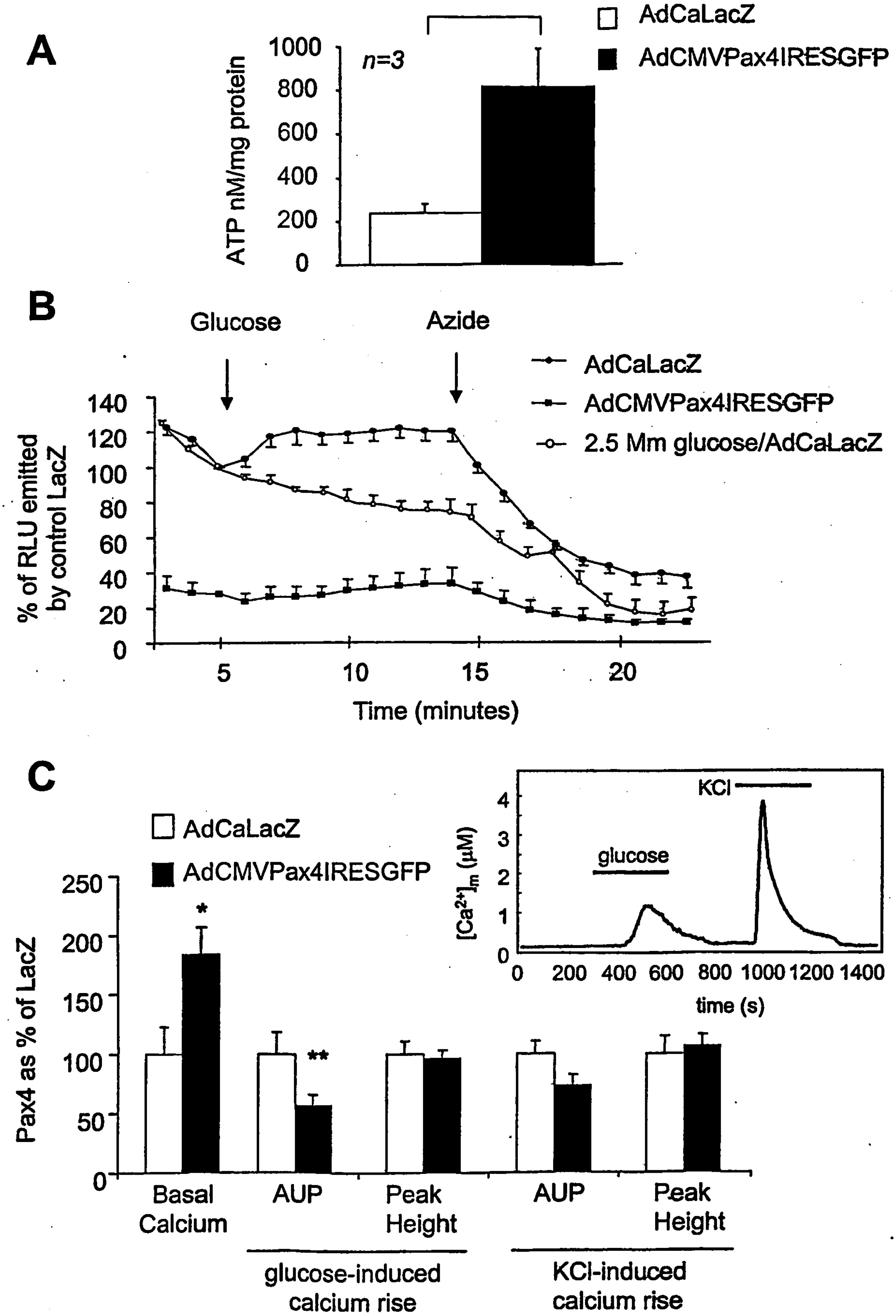
A
**
n=3
AdCaLacZ
AdCMVPax4IRESGFP
ATP nM/mg protein
1000
800
600
400
200
0
B
Glucose
Azide
AdCaLacZ
AdCMVPax4IRESGFP
2.5 Mm glucose/AdCaLacZ
% of RLU emitted by control LacZ
140
120
100
80
60
40
20
0
5
10
15
20
Time (minutes)
C
AdCaLacZ
AdCMVPax4IRESGFP
Pax4 as % of LacZ
250
200
150
100
50
0
*
**
Basal Calcium
AUP
Peak Height
AUP
Peak Height
glucose-induced calcium rise
KCl-induced calcium rise
KCl
glucose
$[Ca^{2+}]_m$ (μM)
4
3
2
1
0
0 200 400 600 800 1000 1200 1400
time (s)

# Figure 9

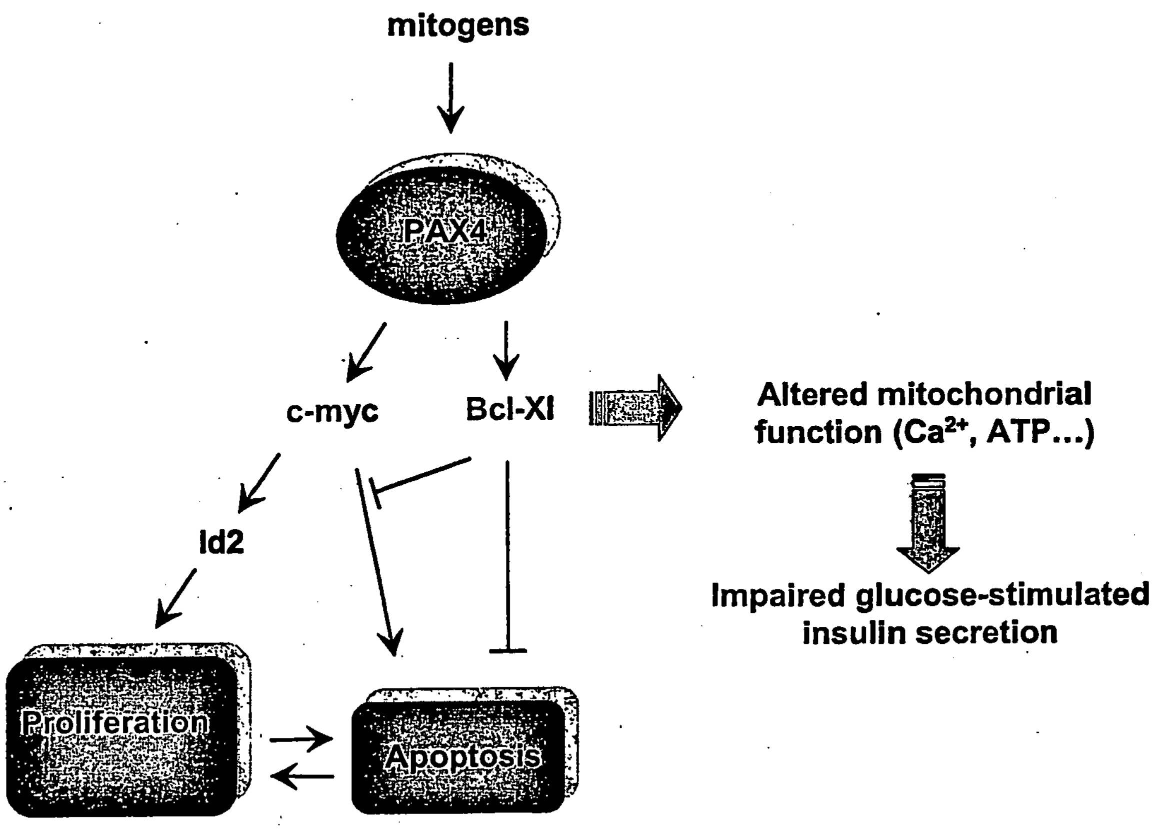
mitogens
PAX4
c-myc
Bcl-Xl
Altered mitochondrial function ($Ca^{2+}$, ATP...)
Impaired glucose-stimulated insulin secretion
Id2
Proliferation
Apoptosis

# Figure 10

| | Insulin (ng/islet) | Glucagon (ng/islet) |
|---|---|---|
| Control | 58.3 ± 2.4 | 1.0 ± 0.3 |
| AdCALacZ | 42.3 ± 0.8 | 1.1 ± 0.1 |
| AdCMVPax4IRESGFP | | |
| $1X10^7$ | 59.5 ± 2.2 | 1.0 ± 0.2 |
| $2.4X10^7$ | 74.9 ± 3.3 | 1.1 ± 0.2 |

# Figure 11

A

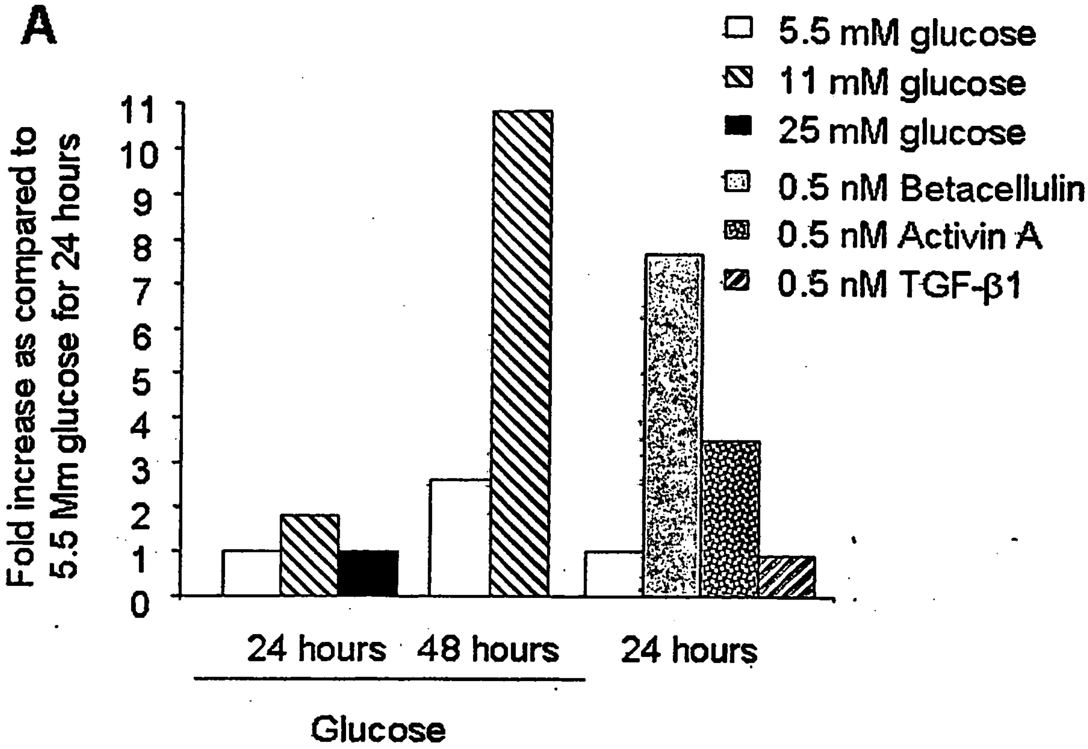
5.5 mM glucose
11 mM glucose
25 mM glucose
0.5 nM Betacellulin
0.5 nM Activin A
0.5 nM TGF-β1
Fold increase as compared to 5.5 Mm glucose for 24 hours
0
1
2
3
4
5
6
7
8
9
10
11
24 hours
48 hours
24 hours
Glucose

B

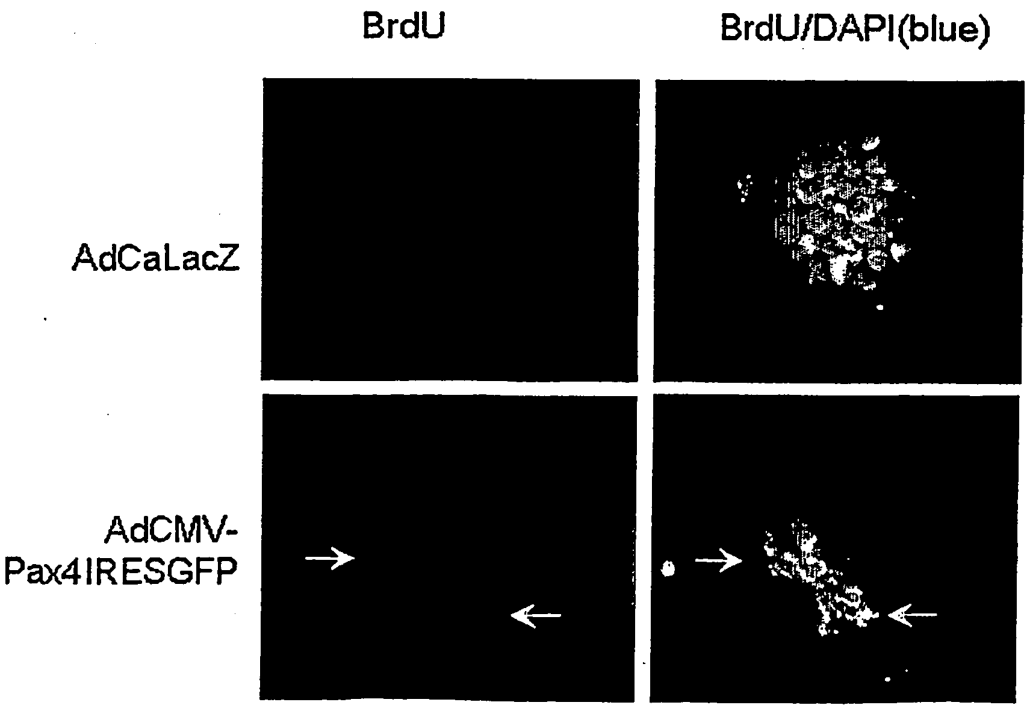
BrdU
BrdU/DAPI(blue)
AdCaLacZ
AdCMV-Pax4IRESGFP

# Figure 12

A

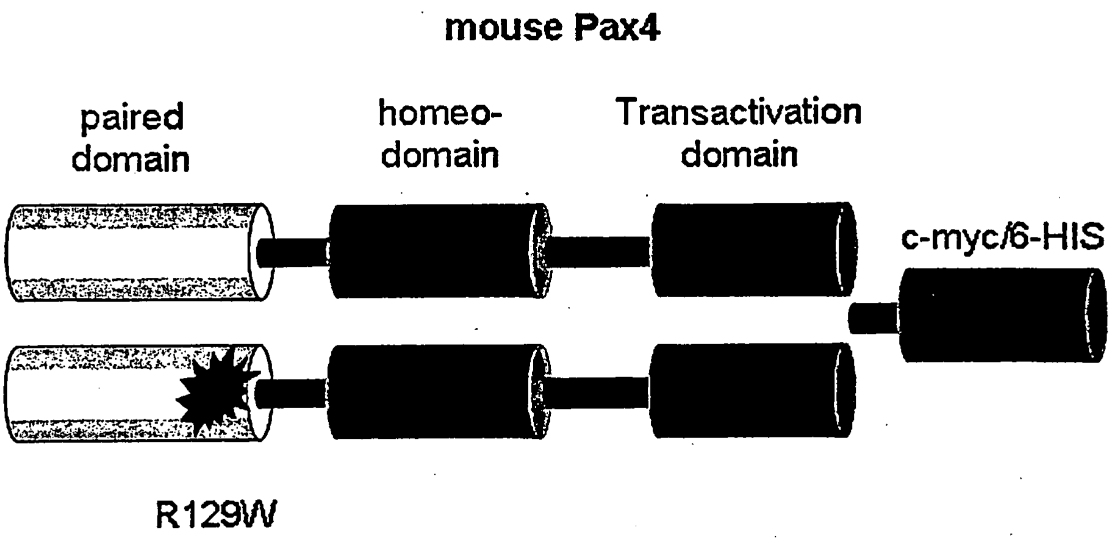
mouse Pax4
paired domain
homeo-domain
Transactivation domain
c-myc/6-HIS
R129W

B

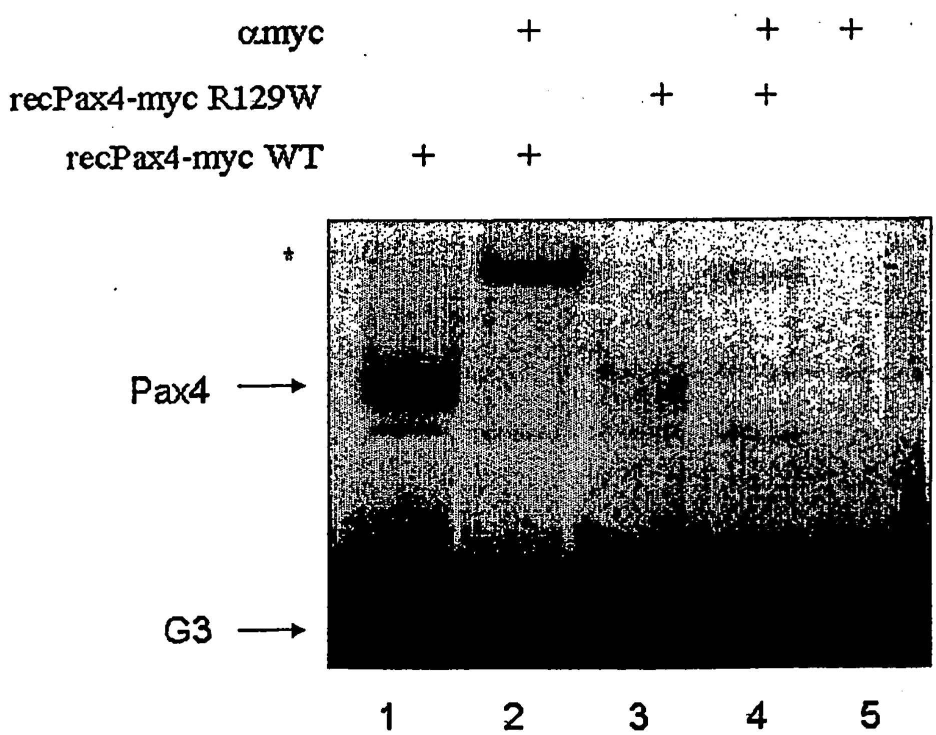
αmyc
recPax4-myc R129W
recPax4-myc WT
+
*
Pax4
G3
1
2
3
4
5

# Figure 13

## A

**Ad-X Tet-On / Ad-mPax4-myc WT**

Pax4 DAPI Merge

- doxycycline

+ doxycycline (0.5 ug/ml)

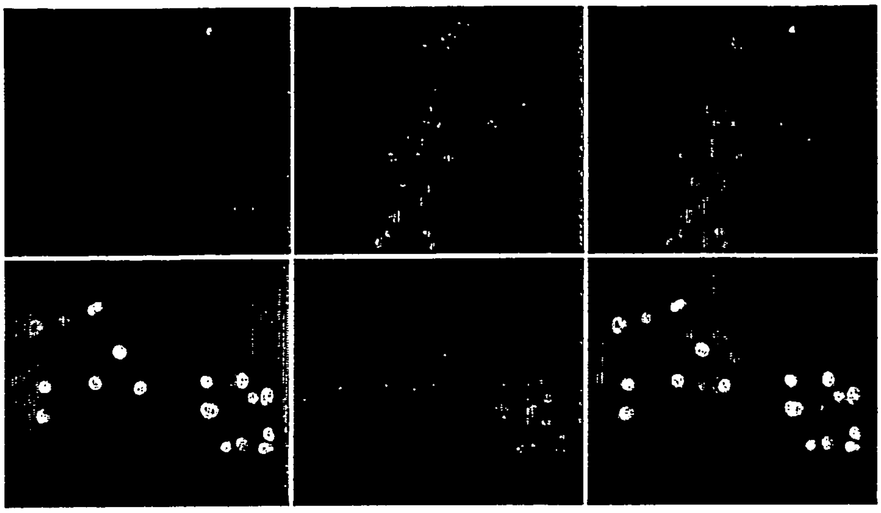

## B

BrdU DAPI Merge

- doxycycline

+ doxycycline (0.5 ug/ml)

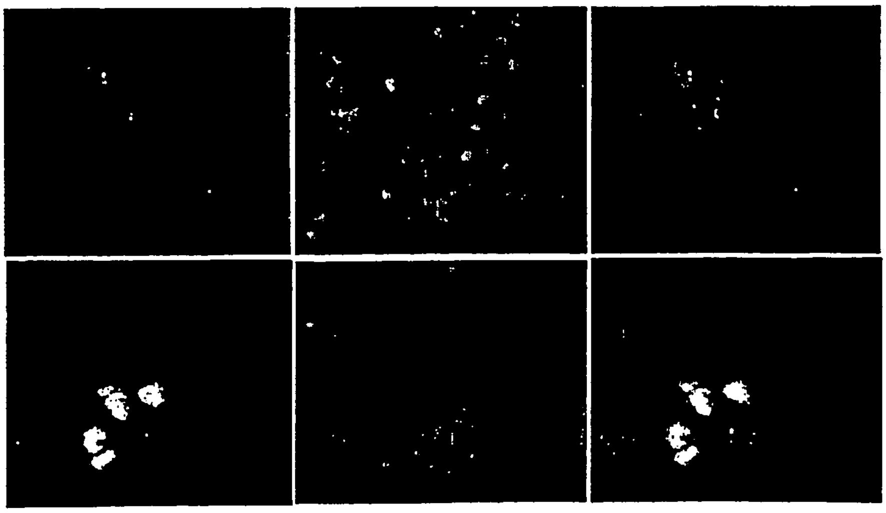

# Figure 14

A

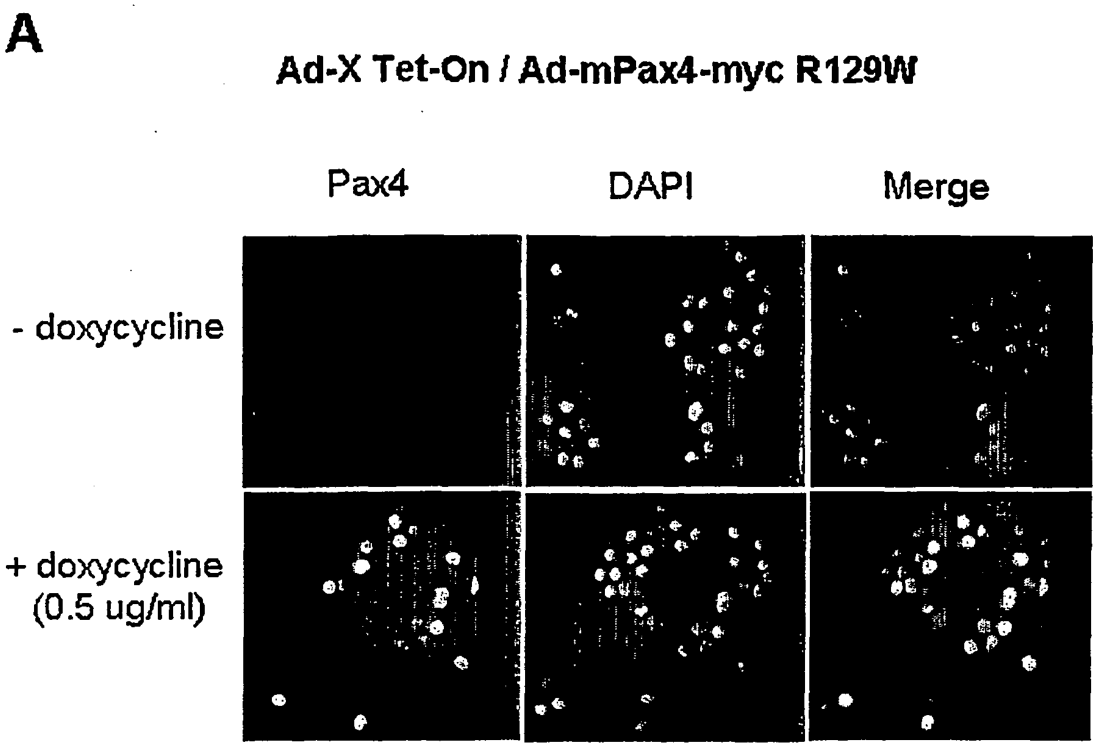
Ad-X Tet-On / Ad-mPax4-myc R129W
Pax4
DAPI
Merge
- doxycycline
+ doxycycline
(0.5 ug/ml)

B

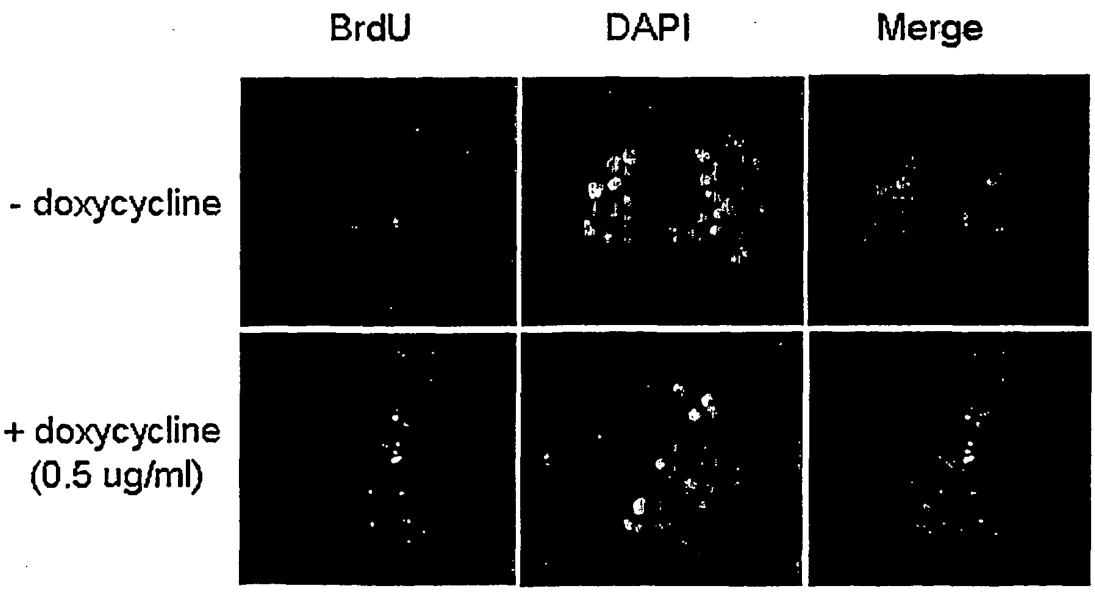
BrdU
DAPI
Merge
- doxycycline
+ doxycycline
(0.5 ug/ml)

# Figure 15

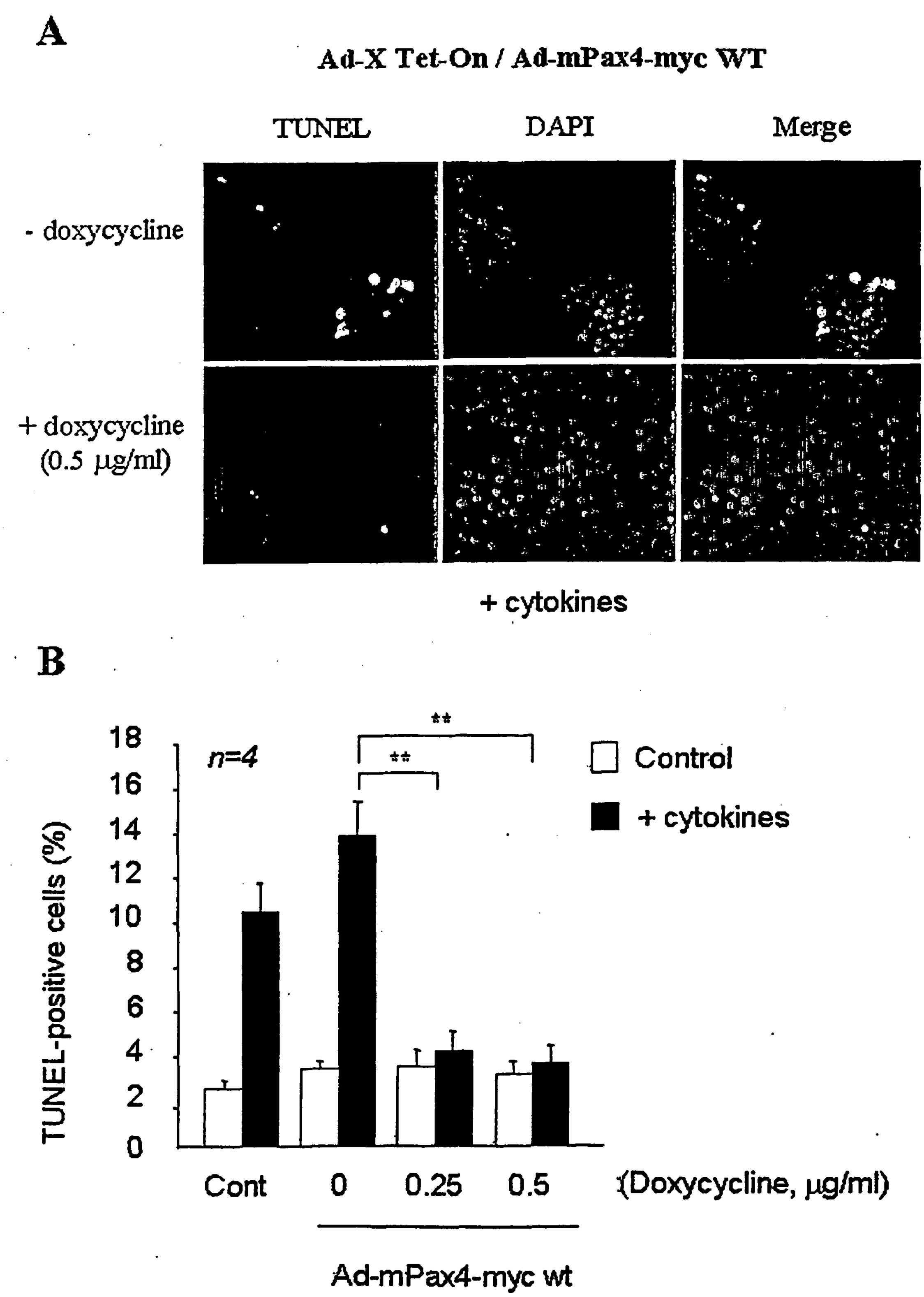
A
Ad-X Tet-On / Ad-mPax4-myc WT
TUNEL
DAPI
Merge
- doxycycline
+ doxycycline
(0.5 μg/ml)
+ cytokines
B
TUNEL-positive cells (%)
18
16
14
12
10
8
6
4
2
0
n=4
**
**
Control
+ cytokines
Cont
0
0.25
0.5
(Doxycycline, μg/ml)
Ad-mPax4-myc wt

# Figure 16

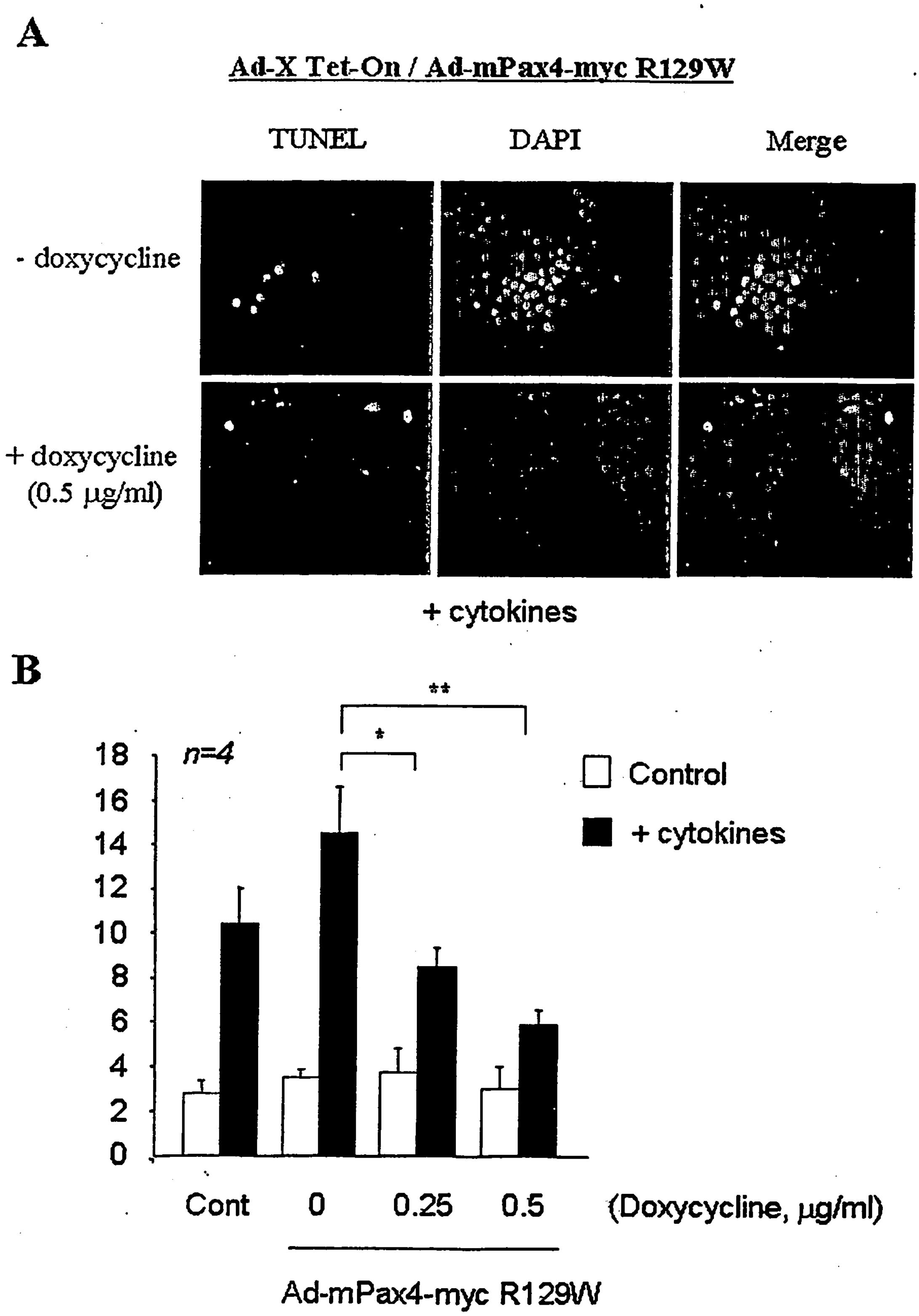
A
Ad-X Tet-On / Ad-mPax4-myc R129W
TUNEL
DAPI
Merge
- doxycycline
+ doxycycline
(0.5 μg/ml)
+ cytokines
B
n=4
**
*
18
16
14
12
10
8
6
4
2
0
Control
+ cytokines
Cont
0
0.25
0.5
(Doxycycline, μg/ml)
Ad-mPax4-myc R129W

# Figure 17

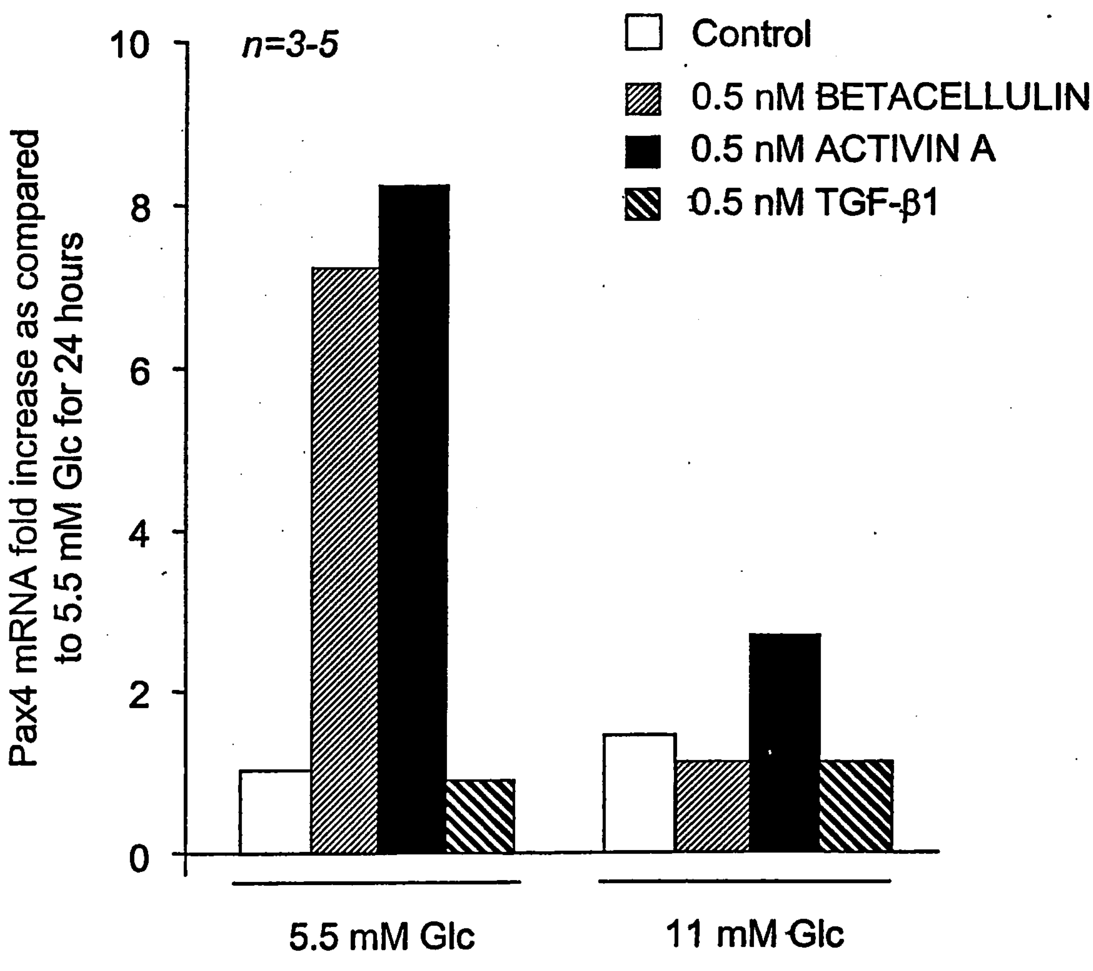
10
8
6
4
2
0
n=3-5
Pax4 mRNA fold increase as compared to 5.5 mM Glc for 24 hours
Control
0.5 nM BETACELLULIN
0.5 nM ACTIVIN A
0.5 nM TGF-β1
5.5 mM Glc
11 mM Glc

# Figure 18

**A**

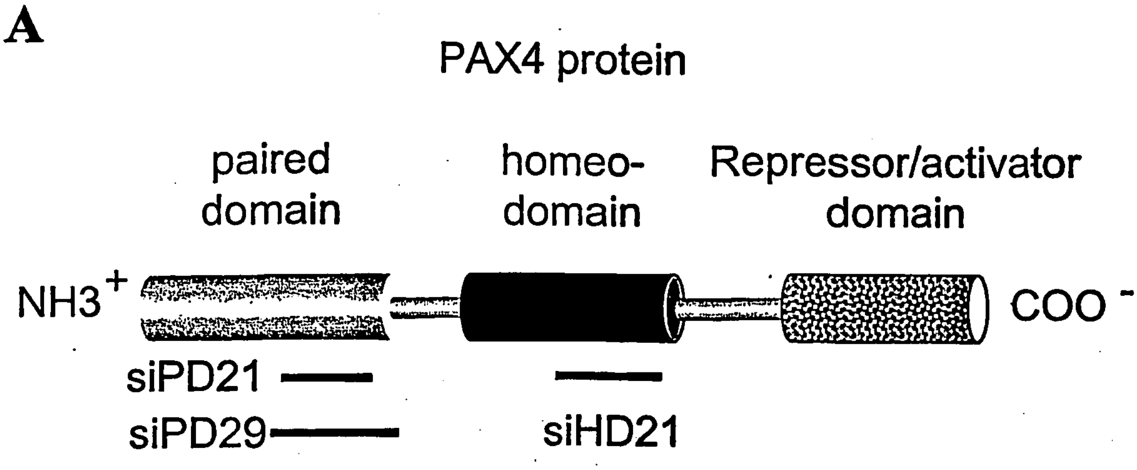
PAX4 protein
paired domain
homeo-domain
Repressor/activator domain
NH3+
COO-
siPD21
siPD29
siHD21

**B**

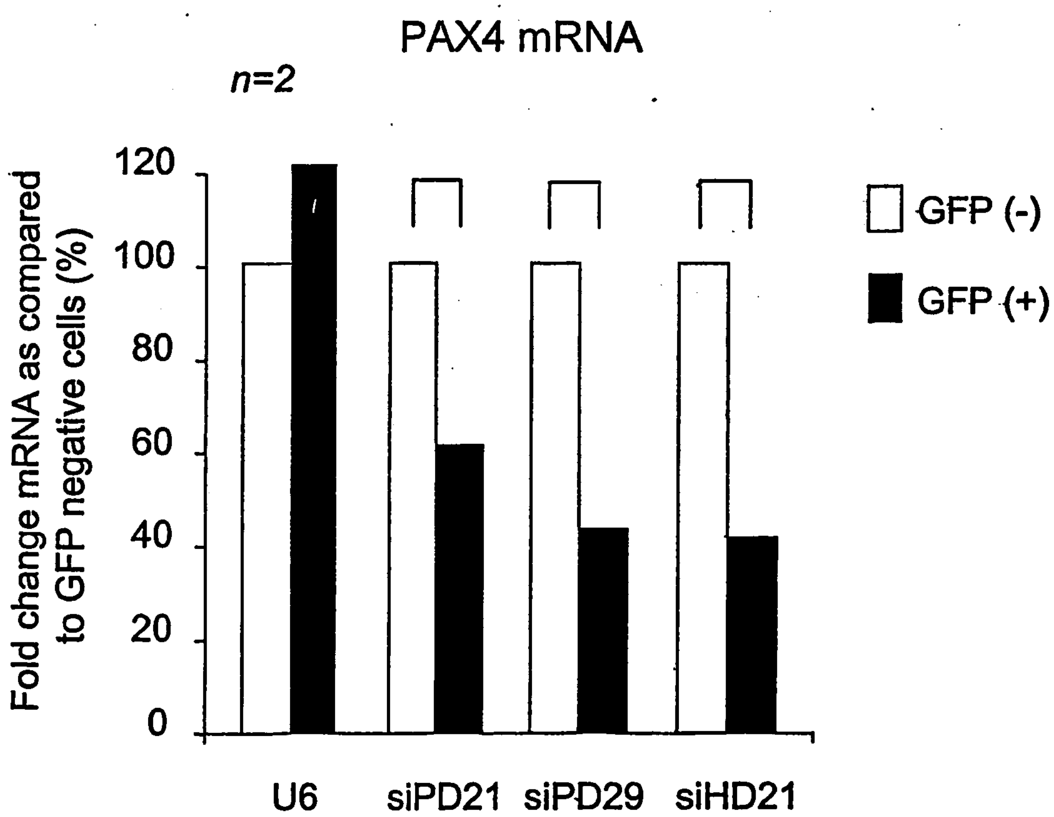
PAX4 mRNA
n=2
Fold change mRNA as compared to GFP negative cells (%)
120
100
80
60
40
20
0
U6
siPD21
siPD29
siHD21
GFP (-)
GFP (+)

# Figure 18

**C**

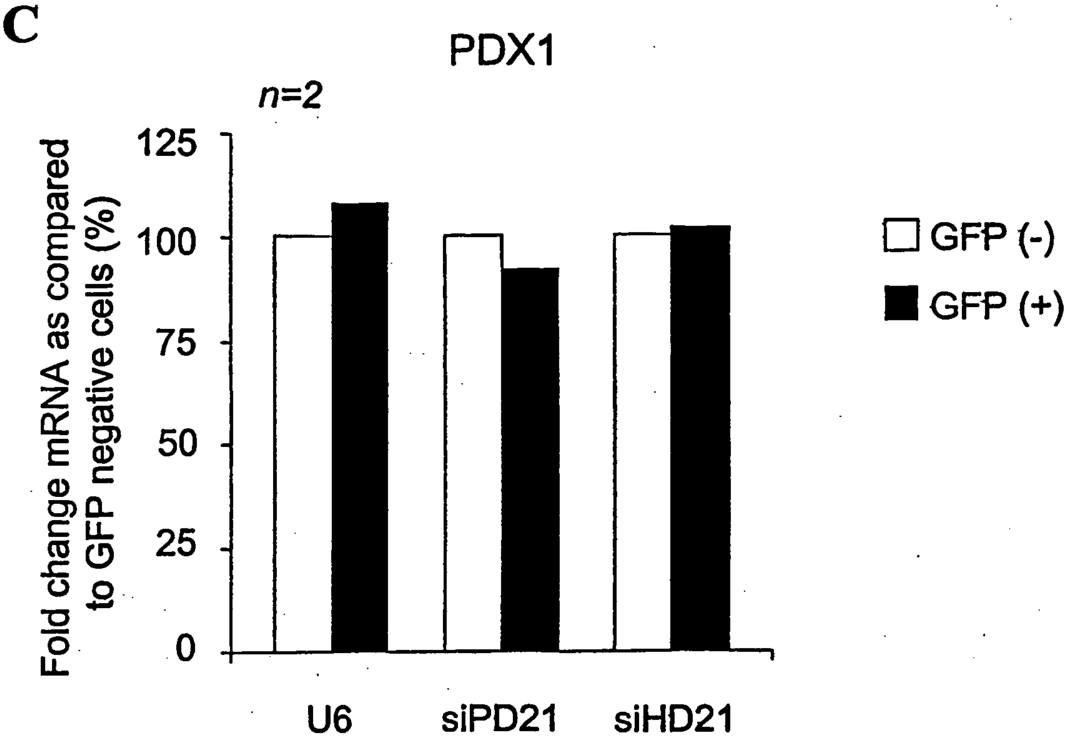
PDX1
n=2
Fold change mRNA as compared to GFP negative cells (%)
125
100
75
50
25
0
U6
siPD21
siHD21
GFP (-)
GFP (+)

**D**

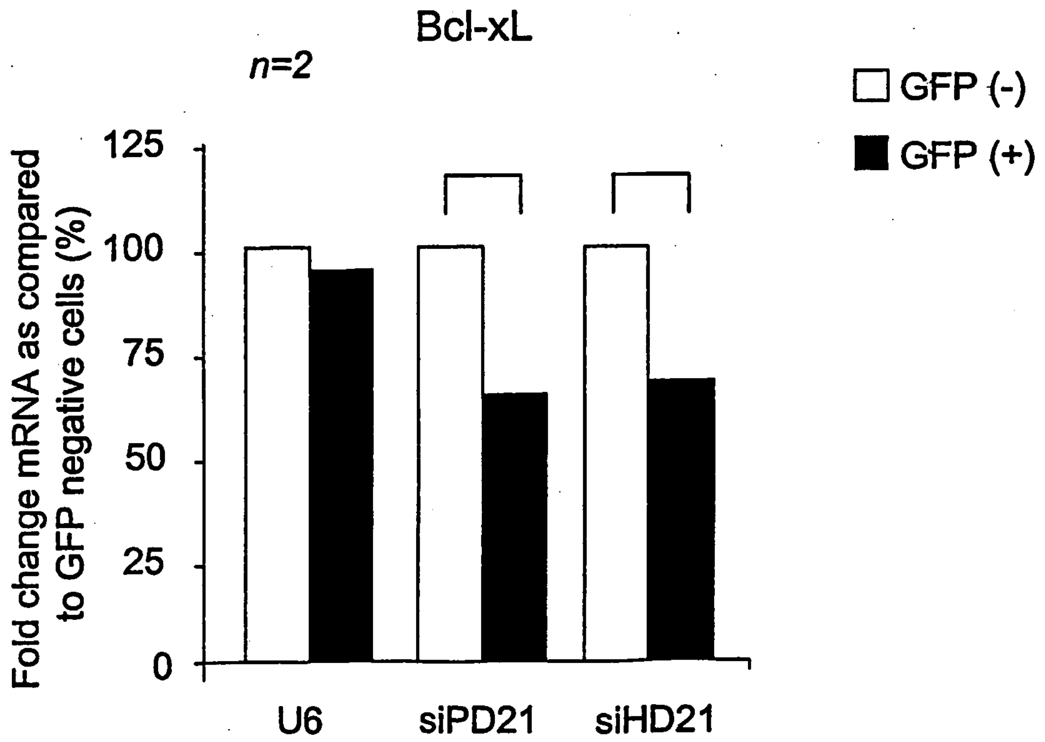
Bcl-xL
n=2
Fold change mRNA as compared to GFP negative cells (%)
125
100
75
50
25
0
U6
siPD21
siHD21
GFP (-)
GFP (+)

# Figure 19

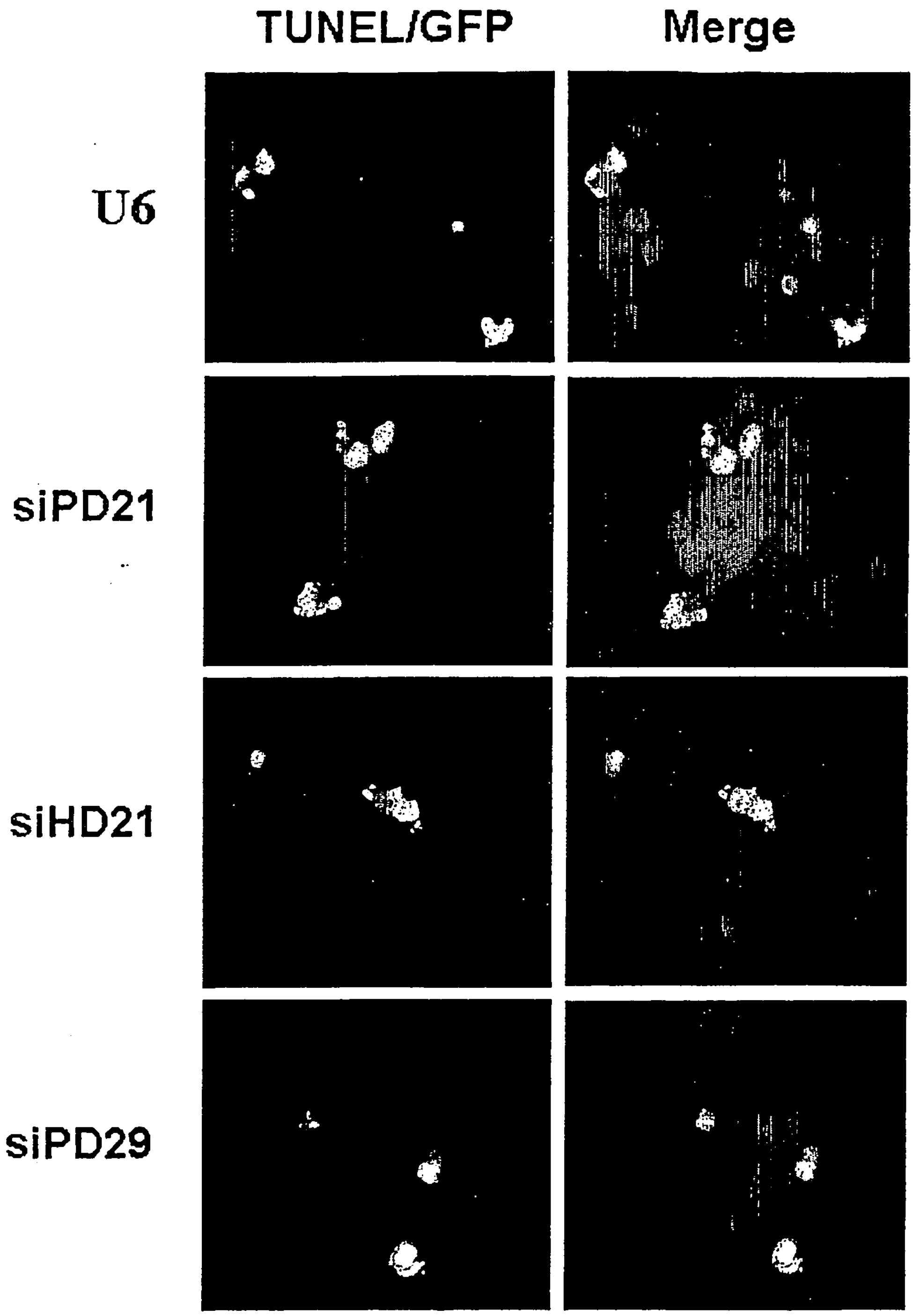

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9829566 A
- US 5928944 A
- US 6099831 A
- US 6013638 A
- US 6071697 A
- US 4683202 A
- US 4682195 A
- WO 9109939 A
- EP O0273085 A, Myers
- US 5139941 A
- US 4797368 A
- WO 9529989 A
- US 9900553 W
- WO 9110470 A
- WO 9110425 A
- WO 9015637 A
- WO 9002580 A
- US 5011472 A
- US 4892538 A
- WO 8901967 A
- US 5626561 A
- US 5787900 A
- US 5843069 A


### Non-patent literature cited in the description


- **Sosa-Pineda.** *Mol Cells,* 2004, vol. 18, 289-94
- **Biason-Lauber.** *Diabetologia,* 2005, vol. 48, 900-905
- **Holm.** *Diabetes,* 2004, vol. 53, 1584-91
- **Mauvais-Jarvis.** *Hum. Mol. Genet.,* 2004, vol. 13, 3151-3159
- **Shimajiri.** *Biochem. Biophys. Res. Commun.,* 2003, vol. 282, 34-40
- **Asfari.** *Endocrinology,* 1992, vol. 130, 167-78
- **Merglen.** *Endocrinology,* 2004, vol. 148, 667-78
- **Miyamoto.** *Biochem. Biophys. Res. Commun.,* 2001, vol. 282, 34-40
- **Siolas.** *Nat Biotechnol.,* 2005, vol. 23, 227-31
- **Sledz.** *Nat. Cell Biol.,* 2003, vol. 5, 834-9
- **Altman et al.** *Diabetes,* 1986, vol. 35 (6), 625-633
- **Altschul.** *J. Mol. Biol.,* 1990, vol. 215, 403-410
- **Altschul.** *J. Mol. Evol.,* 1993, vol. 36, 290-300
- **Altschul.** *Nucl. Acids Res.,* 1997, vol. 25, 3389-3402
- **Ausubel et al.** Current Protocols in Molecular Biology. John, Wiley & Sons, Inc, 1996
- **Baichwal ; Sugden.** Gene Transfer. Plenum Press, 1986, 117-148
- **Bauer et al.** *Blood,* 1995, vol. 86 (6), 2379-2387
- **Becker et al.** *Meth. Cell. Biol.,* 1994, vol. 43, 161-189
- **Becker et al.** *Methods Cell Biol.,* 1994, vol. 43 (A), 161-189
- **Becker et al.** *J. Biol. Chem.,* 1994, vol. 269 (33), 21234-212338
- **Becker et al.** *J. Biol. Chem.,* 1996, vol. 271 (1), 390-394
- **Bell.** *Nature,* 2001, vol. 414, 788-791
- **Bestwick et al.** *Proc. Natl. Acad. Sci. USA,* 1988, vol. 85 (15), 5404-5408
- **Blysczuk.** *PNAS,* 2003, vol. 100, 908-1003
- **Bonner-Weir.** *Endocrinology,* 2000, vol. 141, 1926-1929
- **Bonner-Weir.** *Trends Endocrinol Metab,* 2000, vol. 11, 375-378
- **Brandle.** *Diabetes Care,* 2001, vol. 24, 1253-1258
- **Brink.** *Mech. Dev.,* 2001, vol. 100, 37-43
- **Brutlag.** *Comp. App. Biosci.,* 1990, vol. 6, 237-245
- **Buteau.** *Diabetes,* 2003, vol. 52, 124-132
- **Butler.** *Diabetes,* 2003, vol. 52, 102-110
- **Campell.** *FEBS,* 1999, vol. 463, 53-57
- **Challet.** *J. Biol. Chem.,* 2001, vol. 276, 3791-3797
- **Chan.** *Diabetes,* 1999, vol. 48, 997-1005
- **Chang et al.** *Hepatology,* 1991, vol. 14, 134A
- **Chen ; Okayama.** *Mol. Cell Biol.,* 1987, vol. 7 (8), 2745-2752
- **Clark et al.** *Hum. Gene Ther.,* 1995, vol. 6 (10), 1329-1341
- **Clark et al.** *Am. J. Clin. Pathol.,* 1990, vol. 93 (1), 58-69
- **Coffin et al.** Virology. Raven Press, 1990, 1437-1500

- **Collins.** *Biotechniques,* 1998, vol. 24, 803-808
- **Collombat.** *Genes Dev.,* 2003, vol. 17, 2591-2603
- **Cotten et al.** *Proc. Natl. Acad. Sci. USA,* 1992, vol. 89 (13), 6094-6098
- **Couch et al.** *Am. Rev. Resp. Dis.,* 1963, vol. 88, 394-403
- **Coupar et al.** *Gene,* 1988, vol. 68, 1-10
- **Curiel.** *Nat. Immun.,* 1994, vol. 13 (2-3), 141-164
- **Davis.** *Cancer Res.,* 1994, vol. 54, 2869-2872
- **Demeterco.** *J. Clin. Endocrinol. Metab.,* 2000, vol. 85, 3892-3896
- **Detimary.** *J. Clin. Invest.,* 1995, vol. 96, 1738-1745
- **Dohrmann.** *Mech. Dev.,* 2000, vol. 92, 47-54
- **Dor.** *Nature,* 2004, vol. 429, 41-46
- **Drucker.** *Endocrinology,* 2001, vol. 142, 521-527
- **Dunbar.** *Int. J. Biochem. Cell Biol.,* 2000, vol. 32, 805-815
- **Dupont.** *Diabetologica,* 1999, vol. 42, 480-484
- **Efrat et al.** *Proc. Natl. Acad. Sci. USA,* 1988, vol. 85 (23), 9037-9041
- **Fechheimer et al.** *Proc Natl. Acad Sci. USA,* 1987, vol. 84, 8463-8467
- **Ferkol et al.** *FASEB J.,* 1993, vol. 7, 1081-1091
- **Flotte ; Carter.** *Gene Ther.,* 1995, vol. 2 (6), 357-62
- **Flotte et al.** *Am. J. Respir. Cell Mol. Biol.,* 1992, vol. 7 (3), 349-356
- **Flotte et al.** *Proc. Natl. Acad. Sci. USA,* 1993, vol. 90 (22), 10613-10617
- **Fournier.** *Transplant Proc.,* 1996, vol. 28, 2866-2868
- **Fraley et al.** *Proc. Natl. Acad. Sci. USA,* 1979, vol. 76, 3348-3352
- **Friedmann.** *Science,* 1989, vol. 244, 1275-1281
- **Fritschy et al.** *Diabetes,* 1991, vol. 40 (1), 37-43
- **Froguel.** *N. Engl. J. Med.,* 1993, vol. 328, 697-702
- **Fujitani.** *Mol. Cell Biol.,* 1999, vol. 19, 8281-8291
- **Gainer et al.** *Transplantation,* 1996, vol. 61 (11), 1567-1571
- **Galili.** *Nat. Genet.,* 1993, vol. 5, 230-235
- **Gallichan et al.** *Hum. Gene Ther.,* 1998, vol. 9 (18), 2717-2726
- **Gauthier.** *Atherosclerosis,* 1999, vol. 142, 301-307
- **Gauthier.** *J. Lipid Res.,* 1999, vol. 40, 1284-1293
- **Gauthier.** *Mol. Endocrinol.,* 2002, vol. 16, 170-183
- **German et al.** *Mol. Cell Biol.,* 1992, vol. 12 (4), 1777-1788
- Liver Diseases, Targeted Diagnosis and Therapy Using Specific Receptors and Ligands. Marcel Dekker, 87-104
- **Ghosh-Choudhury et al.** *EMBO J,* 1987, vol. 6, 1733-1739
- **Gittes.** *Proc. Natl. Acad. Sci. USA,* 1992, vol. 89, 1128-1132
- **Gomez-Foix et al.** *J. Biol. Chem,* 1992, vol. 267, 25129-25134
- **Gopal.** *Mol. Cell Biol.,* 1985, vol. 5, 1188-1190
- **Graham ; Prevec.** Methods in Molecular Biology: Gene Transfer and Expression Protocol. Humana Press, 1991, vol. 7, 109-128
- **Graham ; Van Der Eb.** *Virology,* 1973, vol. 52, 456-467
- **Graham et al.** *J. General Virology,* 1977, vol. 36, 59-74
- **Grunhaus ; Horwitz.** *Seminar in Virology,* 1992, vol. 3, 237-252
- **Hagenfeldt-Johansson.** *Endocrinology,* 2001, vol. 142, 5311-5320
- **Hansen.** *J. Clin. Endocrinol. Metab.,* 2000, vol. 85, 323-1326
- **Harland ; Weintraub.** *J. Cell Biol.,* 1985, vol. 101 (3), 1094-1099
- **Hellman.** *Ann. NY Acad. Sci.,* 1965, vol. 131, 541-558
- **Heremans.** *J. Cell Biol.,* 2002, vol. 159, 303-312
- **Hermonat ; Muzycska.** *Proc. Natl. Acad. Sci. USA,* 1984, vol. 81, 6466-6470
- **Hersdorffer et al.** *DNA Cell Biol.,* 1990, vol. 9, 713-723
- **Herz ; Gerard.** *Proc. Natl. Acad. Sci. USA,* 1993, vol. 90, 2812-2816
- **Higgins ; Hames.** Nucleic acid hybridization, a practical approach. IRL Press, 1985
- **Holm et al.** *Diabetes,* 2004, vol. 53, 1584-1591
- **Horwich et al.** *J. Virol.,* 1990, vol. 64, 642-650
- **Ishihara.** *J. Clin. Invest.,* 1999, vol. 104, 1621-1629
- **Ishihara.** *Nat. Cell Biol.,* 2003, vol. 5, 330-335
- **Janjic.** *Pancreas,* 1996, vol. 13, 166-172
- **Jones ; Shenk.** *Cell,* 1978, vol. 13, 181-188
- **Kafri et al.** *J. Virol.,* 1999, vol. 73 (1), 576-584
- **Kahan.** *Diabetes,* 2003, vol. 52, 2016-2024
- **Kanatsuka.** *Metabolism,* 2002, vol. 51, 1161-1165
- **Kaneda et al.** *Science,* 1989, vol. 243, 375-378
- **Kaplitt et al.** *Nat Genet.,* 1994, vol. 8 (2), 148-154

- **Karlsson et al.** *EMBO J.,* 1986, vol. 5, 2377-2385
- **Kashyap.** *Diabetes,* 2003, vol. 52, 2461-2474
- **Kato et al.** *J. Biol. Chem.,* 1991, vol. 266, 3361-3364
- **Kelleher ; Vos.** *Biotechniques,* 1994, vol. 17 (6), 1110-7
- **Kennedy.** *J. Clin. Invest.,* 1996, vol. 98, 2524-2538
- **Kiem et al.** *Blood,* 1994, vol. 83 (6), 1467-1473
- **Klein et al.** *Nature,* 1987, vol. 327, 70-73
- **Kloppel.** *Surv. Synth. Pathol. Res.,* 1985, vol. 4, 110-125
- **Kojima.** *Nat. Med.,* 2003, vol. 9, 596-603
- **Kotin et al.** *Proc. Natl. Acad. Sci. USA,* 1990, vol. 87 (6), 2211-2215
- **Kozmik.** *Proc. Natl. Acad. Sci. USA,* 1995, vol. 92, 5709-5713
- **Kristinsson.** *Diabetologia,* 2001, vol. 44, 2098-2103
- **Lacy et al.** *Science,* 1991, vol. 254 (5039), 1782-1784
- **LaFace et al.** *Virology,* 1988, vol. 162 (2), 483-486
- **Lasorella.** *Nature,* 2000, vol. 407, 592-598
- **Laughlin et al.** *J. Virol.,* 1986, vol. 60 (2), 515-524
- **Le Gal La Salle et al.** *Science,* 1993, vol. 259, 988-990
- **Lebkowski et al.** *Mol. Cell. Biol.,* 1988, vol. 8 (10), 3988-3996
- **Levrero et al.** *Gene,* 1991, vol. 101, 195-202
- **Luo et al.** *Proc. Natl. Acad. Sci. USA,* 1996, vol. 93, 8907-8912
- **Maassen.** *Exp. Clin. Endocrinol. Diabetes,* 2001, vol. 109, 127-134
- **Madsen et al.** *Proc. Natl. Acad. Sci. USA,* 1988, vol. 85 (18), 6652-6656
- **Maechler.** *Nature,* 1999, vol. 402, 685-689
- **Maechler.** *Nature,* 2001, vol. 414, 807-812
- **Maedler.** *Diabeetes,* 2001, vol. 50, 1683-1690
- **Malecki.** *Nature Genet.,* 1999, vol. 23, 323-328
- **Mann et al.** *Cell,* 1983, vol. 33, 153-159
- **Margue.** *Oncogene,* 2000, vol. 19, 2921-2929
- **Markowitz et al.** *J. Viro/.,* 1988, vol. 62, 1120-1124
- **McCarty et al.** *J. Virol.,* 1991, vol. 65 (6), 2936-2945
- **McLaughlin et al.** *J. Virol.,* 1988, vol. 62 (6), 1963-1973
- **Melloul et al.** *Proc. Natl. Acad Sci. USA,* 1993, vol. 90 (9), 3865-3869
- **Merglen.** *Endocrinology,* 2004, vol. 145, 667-678
- **Michael.** *Mol. Cell,* 2000, vol. 6, 87-97
- **Miller ; Rosman.** *Biotechniques,* 1989, vol. 7 (9), 980-982984-986989-990
- **Miller et al.** *Am. J. Clin. Oncol.,* 1992, vol. 15 (3), 216-221
- **Miller et al.** *J. Pharmacol. Exp. Ther.,* 1993, vol. 264, 11-16
- **Mitchell.** *Mol. Genet. Metab.,* 2002, vol. 77, 35-43
- **Miyoshi et al.** *J Virol.,* 1998, vol. 72 (10), 8150-8157
- **Muratovska.** *Oncogene,* 2003, vol. 22, 6045-6053
- **Muzyczka.** *Curr. Topics Microbiol. Immunol.,* 1992, vol. 158, 97-129
- **Nicolas ; Rubenstein.** Vectors: A survey of molecular cloning vectors and their uses. 1988, 494-513
- **Nicolau ; Sene.** *Biochim. Biophys. Acta,* 1982, vol. 721, 185-190
- **Nicolau et al.** *Methods Enzymol.,* 1987, vol. 149, 157-176
- **Ohi et al.** *Gene,* 1990, vol. 89 (2), 279-282
- **Osborne et al.** *Hum. Gene Ther.,* 1990, vol. 1 (1), 31-41
- **O'Shea ; Sun.** *Diabetes,* 1986, vol. 35 (8), 943-946
- **Paris.** *Endocrinology,* 2003, vol. 144, 2717-2727
- **Paskind et al.** *Virology,* 1975, vol. 67, 242-248
- **Pelengaris.** *Cell,* 2002, vol. 109, 321-334
- **Perales et al.** *Proc. Natl. Acad. Sci. USA,* 1994, vol. 91, 4086-4090
- **Potter et al.** *Proc. Natl. Acad Sci. USA,* 1984, vol. 81, 7161-7165
- **Racher et al.** *Biotechnology Techniques,* 1995, vol. 9, 169-174
- **Ragot et al.** *Nature,* 1993, vol. 361, 647-650
- **Renan.** *Radiother. Oncol.,* 1990, vol. 19, 197-218
- **Rich et al.** *Hum. Gene Ther.,* 1993, vol. 4, 461-476
- **Ridgeway.** Vectors: A survey of molecular cloning vectors and their uses. 1988, 467-492
- **Rippe et al.** *Mol. Cell Biol.,* 1990, vol. 10, 689-695
- **Ritz-Laser.** *Diabetologia,* 2002, vol. 45, 97-107
- **Rosenfeld et al.** *Science,* 1991, vol. 252, 431-434
- **Rosenfeld et al.** *Cell,* 1992, vol. 68, 143-155
- **Roux et al.** *Proc. Natl. Acad. Sci. USA,* 1989, vol. 86, 9079-9083
- **Ryan.** *Diabetes,* 2001, vol. 50, 710-719
- **Ryan.** *Diabetes,* 2002, vol. 51, 2148-2157
- **Ryffel.** *J. Mol. Endocrinol.,* 2001, vol. 27, 11-29
- **Salehi.** *J. Physiol.,* 1999, vol. 15, 579-591

- **Saltiel.** *Nature,* 2001, vol. 414, 99-806
- **Sambrook et al.** Molecular cloning. Cold Spring Harbor Laboratory Press, 2001
- **Samulski et al.** *EMBO J,* 1991, vol. 10, 3941-3950
- **Samulski et al.** *J. Virol,* 1989, vol. 63, 3822-3828
- **Sato et al.** *Proc. Natl. Acad. Sci. U.S.A,* 1962, vol. 48, 1184-1190
- **Schreiber.** *EMBO J.,* 1988, vol. 7, 4221-4229
- **Schwitzgebel.** *Mol. Cell Endocrinol.,* 2001, vol. 185, 99-108
- **Shapiro.** *N. Engl. J. Med.,* 2001, vol. 343, 230-238
- **Shelling ; Smith.** *Gene Therapy,* 1994, vol. 1, 165-169
- **Shih.** *Diabetes,* 2001, vol. 50, 2472-2480
- **Shimajiri.** *Biochem. Biophys. Res. Commun.,* 2003, vol. 302, 342-344
- **Shimajiri.** *Diabetes,* 2001, vol. 50, 2864-2869
- **Smith.** *J. Biol. Chem.,* 2000, vol. 275, 36910-36919
- **Smith.** *Mol. Endocrinol.,* 2004, vol. 18, 142-149
- **Soldevila et al.** *J. Autoimmun.,* 1991, vol. 4 (2), 291-306
- **Sosa-Pineda.** *Nature,* 1997, vol. 386, 399-402
- **Stockschlaeder et al.** *Hum. Gene Ther.,* 1991, vol. 2 (1), 33-39
- **Stoffers.** *J. Clin. Invest.,* 1998, vol. 102, 232-241
- **Stratford-Perricaudet ; Perricaudet.** Human Gene Transfer. John Libbey Eurotext, 1991, 51-61
- **Stratford-Perricaudet et al.** *Hum. Gene. Ther.,* 1990, vol. 1, 241-256
- **Street.** *Int. J. Biochem. Cell Biol.,* 2004, vol. 3 (6), 667-683
- **Sullivan et al.** *J. Infect. Dis.,* 1991, vol. 164 (4), 781-784
- **Tao.** *Diabetes,* 1998, vol. 47, 1650-1653
- **Temin.** Gene Transfer. Plenum Press, 1986, 149-188
- **Thompson.** *Nucl. Acids Res.,* 1994, vol. 2, 4673-4680
- **Top et al.** *J. Infect. Dis.,* 1971, vol. 124, 155-160
- **Tratschin et al.** *Mol. Cell. Biol.,* 1984, vol. 4, 2072-2081
- **Tratschin et al.** *Mol. Cell. Biol.,* 1985, vol. 5, 3258-3260
- **Tur-Kaspa et al.** *Mol. Cell Biol.,* 1986, vol. 6, 716-718
- **Ueda.** *FEBS Lett.,* 2000, vol. 480, 101-105
- **Ueda.** *J. Virol.,* 1996, vol. 70, 4714-472
- **Van der Laan et al.** *Nature,* 2000, vol. 407, 90-94
- **Wagner et al.** *Proc. Natl. Acad. Sci. USA,* 1990, vol. 87 (9), 3410-3414
- **Walsh et al.** *J. Clin. Invest,* 1994, vol. 94, 1440-1448
- **Wang.** *Dev. Biol.,* vol. 266, 178-189
- **Wei et al.** *Gene Therapy,* 1994, vol. 1, 261-268
- **Weir.** *Semin. Cell. Dev. Biol.,* 2004, vol. 15, 347-357
- **Weng.** *Diabetologia,* 2001, vol. 44, 249-258
- **Wong et al.** *Gene,* 1980, vol. 10, 87-94
- **Wu ; Wu.** *Adv. Drug Delivery Rev.,* 1993, vol. 12, 159-167
- **Wu ; Wu.** *Biochemistry,* 1988, vol. 27, 887-892
- **Wu ; Wu.** *J. Biol. Chem.,* 1987, vol. 262, 4429-4432
- **Xu.** *Diabetes,* 1999, vol. 48, 2270-2276
- **Xu.** *Mol. Cell Endocrinol.,* 2000, vol. 170, 79-89
- **Yamagata.** *Diabetes,* 2002, vol. 51, 114-123
- **Yang et al.** *J. Virol.,* 1994, vol. 68, 4847-4856
- **Yang et al.** *Proc. Natl. Acad Sci. USA,* 1990, vol. 87, 9568-9572
- **Yoder et al.** *Blood,* 1994, vol. 82 (1), 347A
- **Zalzman.** *Proc. Natl. Acad. Sci. USA,* 2003, vol. 100, 2426-2431
- **Zhang.** *Diabetes,* 2001, vol. 50 (1), 10-14
- **Zhou et al.** *Exp. Hematol,* 1993, vol. 21, 928-933
- **Zhou et al.** *J. Exp. Med.,* 1994, vol. 179, 1867-1875
- **Zhou.** *Am. J. Physiol Endocrinol. Metab.,* 2000, vol. 278, E340-351
- **Zimmet.** *Nature,* 2001, vol. 414, 782-787

SEQUENCE LISTING

SEQUENCE LISTING

<110> University of Geneva
DeveloGen Aktiengesellschaft

<120> Use of Pax4 in pancreatic cell proliferation

<130> K1759 PCT S3

<160> 23

<170> PatentIn Ver. 2.1

<210> 1
<211> 1050
<212> DNA
<213> Rattus norvegicus

<400> 1

```
atgcagcagg acggtctcag cagtgtgaat cagctggggg gactctttgt gaatggccgg 60
ccccttccac tggacacccg acagcagatt gtgcagctag cgataagagg gatgcgaccc 120
tgtgacatct cacggagcct taaggtatct aacggctgtg tgagtaagat cctaggacgc 180
tactaccgca caggagtctt ggaacccaag ggtattgggg gaagcaaacc acgtctggcc 240
acacctgctg tggtggctcg aattgcccag ctaaaggatg agtatccagc tctttttgcc 300
tgggagatcc aacgccagct ttgtgctgaa gggctttgta cccaggacaa ggctcccagt 360
gtatcctcta tcaatcgagt ccttcgggca ctacaggaag accagaggtt acactggaca 420
caacttagat ctccagctgt tttggctcca gctcttccta gtccccacag taactgtgag 480
gctccccgag gtccccaccc ggggaccagc cacaggaatc ggactatctt ctccccgggc 540
caagctgagg cactggagaa agagttccag cgtgggcagt atccagattc agttgtccgt 600
ggaaagctgg ctgctgccac ctctctgcct gaagacacag tgagggtctg gttttccaac 660
agaagagcca aatggcgcag acaagagaag ttgaaatggg aaacacagat gccaggtgct 720
tctcaggacc tgatggtacc aaaagattct ccagggatca tctctgcaca gcagtcccct 780
ggcagtgtac cctcagcagc cctgcctgtg ctggaacaat tgaatccttc cttctgtcag 840
ctgtgctggg gggcagtacc agacagatgt tccagtgaca ccacatccca agcctgtctc 900
caaccctact gggaatgcca ctccctcctt cctgtggctt cttcctcata catggaattt 960
gcctggccct gcctcaccac ccatcctgtg catcatctga ttggaggccc aggacaagcg 1020
ccatcaacct attacttaca ctggccataa                                 1050
```

<210> 2
<211> 349
<212> PRT
<213> Rattus norvegicus

<400> 2

```
Met Gln Gln Asp Gly Leu Ser Ser Val Asn Gln Leu Gly Gly Leu Phe
1               5                   10                  15

Val Asn Gly Arg Pro Leu Pro Leu Asp Thr Arg Gln Gln Ile Val Gln
            20                  25                  30

Leu Ala Ile Arg Gly Met Arg Pro Cys Asp Ile Ser Arg Ser Leu Lys
        35                  40                  45

Val Ser Asn Gly Cys Val Ser Lys Ile Leu Gly Arg Tyr Tyr Arg Thr
    50                  55                  60

Gly Val Leu Glu Pro Lys Gly Ile Gly Gly Ser Lys Pro Arg Leu Ala
65                  70                  75                  80

Thr Pro Ala Val Val Ala Arg Ile Ala Gln Leu Lys Asp Glu Tyr Pro
                85                  90                  95

Ala Leu Phe Ala Trp Glu Ile Gln Arg Gln Leu Cys Ala Glu Gly Leu
            100                 105                 110

Cys Thr Gln Asp Lys Ala Pro Ser Val Ser Ser Ile Asn Arg Val Leu
        115                 120                 125

Arg Ala Leu Gln Glu Asp Gln Arg Leu His Trp Thr Gln Leu Arg Ser
    130                 135                 140

Pro Ala Val Leu Ala Pro Ala Leu Pro Ser Pro His Ser Asn Cys Glu
145                 150                 155                 160

Ala Pro Arg Gly Pro His Pro Gly Thr Ser His Arg Asn Arg Thr Ile
                165                 170                 175

Phe Ser Pro Gly Gln Ala Glu Ala Leu Glu Lys Glu Phe Gln Arg Gly
            180                 185                 190

Gln Tyr Pro Asp Ser Val Val Arg Gly Lys Leu Ala Ala Ala Thr Ser
        195                 200                 205

Leu Pro Glu Asp Thr Val Arg Val Trp Phe Ser Asn Arg Arg Ala Lys
    210                 215                 220
```

Trp Arg Arg Gln Glu Lys Leu Lys Trp Glu Thr Gln Met Pro Gly Ala
225 230 235 240

Ser Gln Asp Leu Met Val Pro Lys Asp Ser Pro Gly Ile Ile Ser Ala
245 250 255

Gln Gln Ser Pro Gly Ser Val Pro Ser Ala Ala Leu Pro Val Leu Glu
260 265 270

Gln Leu Asn Pro Ser Phe Cys Gln Leu Cys Trp Gly Ala Val Pro Asp
275 280 285

Arg Cys Ser Ser Asp Thr Thr Ser Gln Ala Cys Leu Gln Pro Tyr Trp
290 295 300

Glu Cys His Ser Leu Leu Pro Val Ala Ser Ser Ser Tyr Met Glu Phe
305 310 315 320

Ala Trp Pro Cys Leu Thr Thr His Pro Val His His Leu Ile Gly Gly
325 330 335

Pro Gly Gln Ala Pro Ser Thr Tyr Tyr Leu His Trp Pro
340 345

<210> 3
<211> 1050
<212> DNA
<213> Mus musculus

<400> 3

atgcagcagg acggactcag cagtgtgaat cagctagggg gactctttgt gaatggccgg 60
ccccttcctc tggacaccag gcagcagatt gtgcagctag caataagagg gatgcgaccc 120
tgtgacattt cacggagcct taaggtatct aatggctgtg tgagcaagat cctaggacgc 180
tactaccgca caggtgtctt ggaacccaag tgtattgggg gaagcaaacc acgtctggcc 240
acacctgctg tggtggctcg aattgcccag ctaaaggatg agtaccctgc tctttttgcc 300
tgggagatcc aacaccagct ttgcactgaa gggctttgta cccaggacaa ggctcccagt 360
gtgtcctcta tcaatcgagt acttcgggca cttcaggaag accagagctt gcactggact 420
caactcagat caccagctgt gttggctcca gttcttccca gtccccacag taactgtggg 480
gctccccgag gtccccaccc aggaaccagc cacaggaatc ggactatctt ctccccggga 540
caagccgagg cactggagaa agagtttcag cgtgggcagt atccagattc agtggcccgt 600
gggaagctgg ctgctgccac ctctctgcct gaagacacgg tgagggtttg gttttctaac 660

agaagagcca aatggcgcag gcaagagaag ctgaaatggg aagcacagct gccaggtgct 720
tcccaggacc tgacagtacc aaaaaattct ccagggatca tctctgcaca gcagtccccc 780
ggcagtgtac cctcagctgc cttgcctgtg ctggaaccat tgagtccttc cttctgtcag 840
ctatgctgtg ggacagcacc aggcagatgt tccagtgaca cctcatccca ggcctatctc 900
caaccctact gggactgcca atccctcctt cctgtggctt cctcctcata tgtggaattt 960
gcctggccct gcctcaccac ccatcctgtg catcatctga ttggaggccc aggacaagtg 1020
ccatcaaccc attgctcaaa ctggccataa 1050

<210> 4
<211> 349
<212> PRT
<213> Mus musculus

<400> 4

Met Gln Gln Asp Gly Leu Ser Ser Val Asn Gln Leu Gly Gly Leu Phe
1 5 10 15

Val Asn Gly Arg Pro Leu Pro Leu Asp Thr Arg Gln Gln Ile Val Gln
20 25 30

Leu Ala Ile Arg Gly Met Arg Pro Cys Asp Ile Ser Arg Ser Leu Lys
35 40 45

Val Ser Asn Gly Cys Val Ser Lys Ile Leu Gly Arg Tyr Tyr Arg Thr
50 55 60

Gly Val Leu Glu Pro Lys Cys Ile Gly Gly Ser Lys Pro Arg Leu Ala
65 70 75 80

Thr Pro Ala Val Val Ala Arg Ile Ala Gln Leu Lys Asp Glu Tyr Pro
85 90 95

Ala Leu Phe Ala Trp Glu Ile Gln His Gln Leu Cys Thr Glu Gly Leu
100 105 110

Cys Thr Gln Asp Lys Ala Pro Ser Val Ser Ser Ile Asn Arg Val Leu
115 120 125

Arg Ala Leu Gln Glu Asp Gln Ser Leu His Trp Thr Gln Leu Arg Ser
130 135 140

Pro Ala Val Leu Ala Pro Val Leu Pro Ser Pro His Ser Asn Cys Gly

145 150 155 160

Ala Pro Arg Gly Pro His Pro Gly Thr Ser His Arg Asn Arg Thr Ile
165 170 175

Phe Ser Pro Gly Gln Ala Glu Ala Leu Glu Lys Glu Phe Gln Arg Gly
180 185 190

Gln Tyr Pro Asp Ser Val Ala Arg Gly Lys Leu Ala Ala Ala Thr Ser
195 200 205

Leu Pro Glu Asp Thr Val Arg Val Trp Phe Ser Asn Arg Arg Ala Lys
210 215 220

Trp Arg Arg Gln Glu Lys Leu Lys Trp Glu Ala Gln Leu Pro Gly Ala
225 230 235 240

Ser Gln Asp Leu Thr Val Pro Lys Asn Ser Pro Gly Ile Ile Ser Ala
245 250 255

Gln Gln Ser Pro Gly Ser Val Pro Ser Ala Ala Leu Pro Val Leu Glu
260 265 270

Pro Leu Ser Pro Ser Phe Cys Gln Leu Cys Cys Gly Thr Ala Pro Gly
275 280 285

Arg Cys Ser Ser Asp Thr Ser Ser Gln Ala Tyr Leu Gln Pro Tyr Trp
290 295 300

Asp Cys Gln Ser Leu Leu Pro Val Ala Ser Ser Ser Tyr Val Glu Phe
305 310 315 320

Ala Trp Pro Cys Leu Thr Thr His Pro Val His His Leu Ile Gly Gly
325 330 335

Pro Gly Gln Val Pro Ser Thr His Cys Ser Asn Trp Pro
340 345

<210> 5
<211> 1049
<212> DNA
<213> Homo sapiens

<400> 5

```
agggatcagc agcatgaacc agcttggggg gctctttgtg aatggccggc ccctgcctct 60
ggatacccgg cagcagattg tgcggctagc agtcagtgga atgcggccct gtgacatctc 120
acggatcctt aaggtatcta atggctgtgt gagcaagatc ctagggcgtt actaccgcac 180
aggtgtcttg gagccaaagg gcattggggg aagcaagcca cggctggcta caccccctgt 240
ggtggctcga attgcccagc tgaagggtga gtgtccagcc ctctttgcct gggaaatcca 300
acgccagctt tgtgctgaag ggctttgcac ccaggacaag actcccagtg tctcctccat 360
caaccgagtc ctgcgggcat tacaggagga ccagggacta ccgtgcacac ggctcaggtc 420
accagctgtt ttggctccag ctgtcctcac tccccatagt ggctctgaga ctccccgggg 480
tacccaccca gggaccggcc accggaatcg gactatcttc tccccaagcc aagcagaggc 540
actggagaaa gagttccagc gtgggcagta tcctgattca gtggcccgtg gaaagctggc 600
tactgccacc tctctgcctg aggacacggt gagggtctgg ttttccaaca gaagagccaa 660
atggcgtcgg caagagaagc tcaagtggga aatgcagctg ccaggtgctt cccaggggct 720
gactgtacca agggttgccc caggaatcat ctctgcacag cagtcccctg gcagtgtgcc 780
cacagcagcc ctgcctgccc tggaaccact gggtccctcc tgctatcagc tgtgctgggc 840
aacagcacca gaaaggtgtc tgagtgacac cccacctaaa gcctgtctca agccctgctg 900
gggccacttg cccccacagc cgaattccct ggactcagga ctgctttgcc ttccttgccc 960
ttcctcccac tgtcccctgg ccagtcttag tggctctcag gccctgctct ggcctggctg 1020
cccactactg tatggcttgg aatgaggca                                   1049
```

<210> 6
<211> 343
<212> PRT
<213> Homo sapiens

<400> 6

```
Met Asn Gln Leu Gly Gly Leu Phe Val Asn Gly Arg Pro Leu Pro Leu
1               5                   10                  15

Asp Thr Arg Gln Gln Ile Val Arg Leu Ala Val Ser Gly Met Arg Pro
            20                  25                  30

Cys Asp Ile Ser Arg Ile Leu Lys Val Ser Asn Gly Cys Val Ser Lys
        35                  40                  45

Ile Leu Gly Arg Tyr Tyr Arg Thr Gly Val Leu Glu Pro Lys Gly Ile
    50                  55                  60

Gly Gly Ser Lys Pro Arg Leu Ala Thr Pro Pro Val Val Ala Arg Ile
65                  70                  75                  80
```

Ala Gln Leu Lys Gly Glu Cys Pro Ala Leu Phe Ala Trp Glu Ile Gln
85 90 95

Arg Gln Leu Cys Ala Glu Gly Leu Cys Thr Gln Asp Lys Thr Pro Ser
100 105 110

Val Ser Ser Ile Asn Arg Val Leu Arg Ala Leu Gln Glu Asp Gln Gly
115 120 125

Leu Pro Cys Thr Arg Leu Arg Ser Pro Ala Val Leu Ala Pro Ala Val
130 135 140

Leu Thr Pro His Ser Gly Ser Glu Thr Pro Arg Gly Thr His Pro Gly
145 150 155 160

Thr Gly His Arg Asn Arg Thr Ile Phe Ser Pro Ser Gln Ala Glu Ala
165 170 175

Leu Glu Lys Glu Phe Gln Arg Gly Gln Tyr Pro Asp Ser Val Ala Arg
180 185 190

Gly Lys Leu Ala Thr Ala Thr Ser Leu Pro Glu Asp Thr Val Arg Val
195 200 205

Trp Phe Ser Asn Arg Arg Ala Lys Trp Arg Arg Gln Glu Lys Leu Lys
210 215 220

Trp Glu Met Gln Leu Pro Gly Ala Ser Gln Gly Leu Thr Val Pro Arg
225 230 235 240

Val Ala Pro Gly Ile Ile Ser Ala Gln Gln Ser Pro Gly Ser Val Pro
245 250 255

Thr Ala Ala Leu Pro Ala Leu Glu Pro Leu Gly Pro Ser Cys Tyr Gln
260 265 270

Leu Cys Trp Ala Thr Ala Pro Glu Arg Cys Leu Ser Asp Thr Pro Pro
275 280 285

Lys Ala Cys Leu Lys Pro Cys Trp Gly His Leu Pro Pro Gln Pro Asn
290 295 300

Ser Leu Asp Ser Gly Leu Leu Cys Leu Pro Cys Pro Ser Ser His Cys

305 310 315 320

Pro Leu Ala Ser Leu Ser Gly Ser Gln Ala Leu Leu Trp Pro Gly Cys
325 330 335

Pro Leu Leu Tyr Gly Leu Glu
340

<210> 7
<211> 1050
<212> DNA
<213> Mus musculus

<400> 7

```
atgcagcagg acggactcag cagtgtgaat cagctagggg gactctttgt gaatggccgg 60
cccccttcctc tggacaccag gcagcagatt gtgcagctag caataagagg gatgcgaccc 120
tgtgacattt cacggagcct taaggtatct aatggctgtg tgagcaagat cctaggacgc 180
tactaccgca caggtgtctt ggaacccaag tgtattgggg gaagcaaacc acgtctggcc 240
acacctgctg tggtggctcg aattgcccag ctaaaggatg agtaccctgc tctttttgcc 300
tgggagatcc aacaccagct ttgcactgaa gggctttgta cccaggacaa ggctcccagt 360
gtgtcctcta tcaatcgagt actttgggca cttcaggaag accagagctt gcactggact 420
caactcagat caccagctgt gttggctcca gttcttccca gtccccacag taactgtggg 480
gctccccgag gtccccaccc aggaaccagc cacaggaatc ggactatctt ctccccggga 540
caagccgagg cactggagaa agagtttcag cgtgggcagt atccagattc agtggcccgt 600
gggaagctgg ctgctgccac ctctctgcct gaagacacgg tgagggtttg gttttctaac 660
agaagagcca aatggcgcag gcaagagaag ctgaaatggg aagcacagct gccaggtgct 720
tcccaggacc tgacagtacc aaaaaattct ccagggatca tctctgcaca gcagtccccc 780
ggcagtgtac cctcagctgc cttgcctgtg ctggaaccat tgagtccttc cttctgtcag 840
ctatgctgtg ggacagcacc aggcagatgt tccagtgaca cctcatccca ggcctatctc 900
caaccctact gggactgcca atccctcctt cctgtggctt cctcctcata tgtggaattt 960
gcctggccct gcctcaccac ccatcctgtg catcatctga ttggaggccc aggacaagtg 1020
ccatcaaccc attgctcaaa ctggccataa 1050
```

<210> 8
<211> 349
<212> PRT
<213> Mus musculus

<400> 8

Met Gln Gln Asp Gly Leu Ser Ser Val Asn Gln Leu Gly Gly Leu Phe
1 5 10 15

Val Asn Gly Arg Pro Leu Pro Leu Asp Thr Arg Gln Gln Ile Val Gln
20 25 30

Leu Ala Ile Arg Gly Met Arg Pro Cys Asp Ile Ser Arg Ser Leu Lys
35 40 45

Val Ser Asn Gly Cys Val Ser Lys Ile Leu Gly Arg Tyr Tyr Arg Thr
50 55 60

Gly Val Leu Glu Pro Lys Cys Ile Gly Gly Ser Lys Pro Arg Leu Ala
65 70 75 80

Thr Pro Ala Val Val Ala Arg Ile Ala Gln Leu Lys Asp Glu Tyr Pro
85 90 95

Ala Leu Phe Ala Trp Glu Ile Gln His Gln Leu Cys Thr Glu Gly Leu
100 105 110

Cys Thr Gln Asp Lys Ala Pro Ser Val Ser Ser Ile Asn Arg Val Leu
115 120 125

Trp Ala Leu Gln Glu Asp Gln Ser Leu His Trp Thr Gln Leu Arg Ser
130 135 140

Pro Ala Val Leu Ala Pro Val Leu Pro Ser Pro His Ser Asn Cys Gly
145 150 155 160

Ala Pro Arg Gly Pro His Pro Gly Thr Ser His Arg Asn Arg Thr Ile
165 170 175

Phe Ser Pro Gly Gln Ala Glu Ala Leu Glu Lys Glu Phe Gln Arg Gly
180 185 190

Gln Tyr Pro Asp Ser Val Ala Arg Gly Lys Leu Ala Ala Ala Thr Ser
195 200 205

Leu Pro Glu Asp Thr Val Arg Val Trp Phe Ser Asn Arg Arg Ala Lys
210 215 220

Trp Arg Arg Gln Glu Lys Leu Lys Trp Glu Ala Gln Leu Pro Gly Ala
225 230 235 240

Ser Gln Asp Leu Thr Val Pro Lys Asn Ser Pro Gly Ile Ile Ser Ala
245 250 255

Gln Gln Ser Pro Gly Ser Val Pro Ser Ala Ala Leu Pro Val Leu Glu
260 265 270

Pro Leu Ser Pro Ser Phe Cys Gln Leu Cys Cys Gly Thr Ala Pro Gly
275 280 285

Arg Cys Ser Ser Asp Thr Ser Ser Gln Ala Tyr Leu Gln Pro Tyr Trp
290 295 300

Asp Cys Gln Ser Leu Leu Pro Val Ala Ser Ser Ser Tyr Val Glu Phe
305 310 315 320

Ala Trp Pro Cys Leu Thr Thr His Pro Val His His Leu Ile Gly Gly
325 330 335

Pro Gly Gln Val Pro Ser Thr His Cys Ser Asn Trp Pro
340 345

<210> 9
<211> 1028
<212> DNA
<213> Homo sapiens

<400> 9

atgaaccagc ttggggggct ctttgtgaat ggccggcccc tgcctctgga tacccggcag 60
cagattgtgc ggctgcagtc agtggaatgc ggccctgtga catctcacgg atccttaagg 120
tatctaatgg ctgtgtgagc aagatcctag ggcgttacta ccgacaggtg tcttggagcc 180
aaagggcatt gggggaagca agccacggct ggctacaccc cctgtggtgg ctcgaattgc 240
ccagctgaag ggtgagtgtc cagccctctt tgcctgggaa atccaacgcc agctttgtgc 300
tgaagggctt tgcacccagg acaagactcc cagtgtctcc tccatcaacc gagtcctgtg 360
ggcattacag gaggaccagg gactaccgtg cacacggctc aggtcaccag ctgttttggc 420
tccagctgtc ctcactcccc atagtggctc tgagactccc cggggtaccc acccagggac 480
cggccaccgg aatcggacta tcttctcccc aagccaagca gaggcactgg agaaagagtt 540
ccagcgtggg catatcctga ttcagtggcc cgtggaaagc tggctactgc cacctctctg 600
cctgaggaca cggtgagggt ctggttttcc aacagaagag ccaaatggcg tcggcaagag 660
aagctcaagt gggaaatgca gctgccaggt gcttcccagg ggctgactgt accaagggtt 720
gccccaggaa tcatctctgc acagcagtcc cctggcagtg tgcccacagc agccctgcct 780
gccctggaac cctgggtccc tcctgctatc agctgtgctg ggcaacagca ccagaaaggt 840
gtctgagtga caccccacct aaagcctgtc tcaagccctg ctggggccac ttgcccccac 900

agccgaattc cctggactca ggactgcttt gccttccttg cccttcctcc cactgtcccc 960
tggccagtct tagtggctct caggccctgc tctggcctgg ctgcccacta ctgtatggct 1020
tggaatga 1028

<210> 10
<211> 343
<212> PRT
<213> Homo sapiens

<400> 10

Met Asn Gln Leu Gly Gly Leu Phe Val Asn Gly Arg Pro Leu Pro Leu
1 5 10 15

Asp Thr Arg Gln Gln Ile Val Arg Leu Ala Val Ser Gly Met Arg Pro
20 25 30

Cys Asp Ile Ser Arg Ile Leu Lys Val Ser Asn Gly Cys Val Ser Lys
35 40 45

Ile Leu Gly Arg Tyr Tyr Arg Thr Gly Val Leu Glu Pro Lys Gly Ile
50 55 60

Gly Gly Ser Lys Pro Arg Leu Ala Thr Pro Pro Val Val Ala Arg Ile
65 70 75 80

Ala Gln Leu Lys Gly Glu Cys Pro Ala Leu Phe Ala Trp Glu Ile Gln
85 90 95

Arg Gln Leu Cys Ala Glu Gly Leu Cys Thr Gln Asp Lys Thr Pro Ser
100 105 110

Val Ser Ser Ile Asn Arg Val Leu Trp Ala Leu Gln Glu Asp Gln Gly
115 120 125

Leu Pro Cys Thr Arg Leu Arg Ser Pro Ala Val Leu Ala Pro Ala Val
130 135 140

Leu Thr Pro His Ser Gly Ser Glu Thr Pro Arg Gly Thr His Pro Gly
145 150 155 160

Thr Gly His Arg Asn Arg Thr Ile Phe Ser Pro Ser Gln Ala Glu Ala
165 170 175

Leu Glu Lys Glu Phe Gln Arg Gly Gln Tyr Pro Asp Ser Val Ala Arg
180 185 190

Gly Lys Leu Ala Thr Ala Thr Ser Leu Pro Glu Asp Thr Val Arg Val
195 200 205

Trp Phe Ser Asn Arg Arg Ala Lys Trp Arg Arg Gln Glu Lys Leu Lys
210 215 220

Trp Glu Met Gln Leu Pro Gly Ala Ser Gln Gly Leu Thr Val Pro Arg
225 230 235 240

Val Ala Pro Gly Ile Ile Ser Ala Gln Gln Ser Pro Gly Ser Val Pro
245 250 255

Thr Ala Ala Leu Pro Ala Leu Glu Pro Leu Gly Pro Ser Cys Tyr Gln
260 265 270

Leu Cys Trp Ala Thr Ala Pro Glu Arg Cys Leu Ser Asp Thr Pro Pro
275 280 285

Lys Ala Cys Leu Lys Pro Cys Trp Gly His Leu Pro Pro Gln Pro Asn
290 295 300

Ser Leu Asp Ser Gly Leu Leu Cys Leu Pro Cys Pro Ser Ser His Cys
305 310 315 320

Pro Leu Ala Ser Leu Ser Gly Ser Gln Ala Leu Leu Trp Pro Gly Cys
325 330 335

Pro Leu Leu Tyr Gly Leu Glu
340

<210> 11
<211> 4437
<212> DNA
<213> Artificial Sequence

<220>
<223> Description of Artificial Sequence: Synthetic Primer

<400> 11

```
taactataac ggtcctaagg tagcgaaagc tcagatctgg atctcccgat cccctatggt 60
cgactctcag tacaatctgc tctgatgccg catagttaag ccagtatctg ctccctgctt 120
gtgtgttgga ggtcgctgag tagtgcgcga gcaaaattta agctacaaca aggcaaggct 180
tgaccgacaa ttgcatgaag aatctgctta gggttaggcg ttttgcgctg cttcgcgatg 240
tacgggccag atatacgcgt cgagtttacc actccctatc agtgatagag aaaagtgaaa 300
gtcgagttta ccactcccta tcagtgatag agaaaagtga aagtcgagtt taccactccc 360
tatcagtgat agagaaaagt gaaagtcgag tttaccactc cctatcagtg atagagaaaa 420
gtgaaagtcg agtttaccac tccctatcag tgatagagaa aagtgaaagt cgagtttacc 480
actccctatc agtgatagag aaaagtgaaa gtcgagttta ccactcccta tcagtgatag 540
agaaaagtga aagtcgagct cggtacccgg gtcgagtagg cgtgtacggt gggaggccta 600
tataagcaga gctcgtttag tgaaccgtca gatcgcctgg agacgccatc cacgctgttt 660
tgacctccat agaagacacc gggaccgatc cagcctccgc ggccccgaat tcgagctcgg 720
tacccgggga tcctctagtc agctgacgcg tgctagcgcg gccgcatcga taagcttgtc 780
gacgatatct ctagaggatc ataatcagcc ataccacatt tgtagaggtt ttacttgctt 840
taaaaaacct cccacacctc cccctgaacc tgaaacataa aatgaatgca attgttgttg 900
ttaacttgtt tattgcagct tataatggtt acaaataaag caatagcatc acaaatttca 960
caaataaagc atttttttca ctgcattcta gttgtggttt gtccaaactc atcaatgtat 1020
cttatcatgt ctggatcccc aggaagctcc tctgtgtcct cataaaccct aacctcctct 1080
acttgagagg acattccaat cataggctgc ccatccaccc tctgtgtcct cctgttaatt 1140
aggtcactta acaaaaagga aattgggtag gggtttttca cagaccgctt tctaagggta 1200
attttaaaat atctgggaag tcccttccac tgctgtgttc cagaagtgtt ggtaaacagc 1260
ccacaaatgt caacagcaga aacatacaag ctgtcagctt tgcacaaggg cccaacaccc 1320
tgctcatcaa gaagcactgt ggttgctgtg ttagtaatgt gcaaaacagg aggcacattt 1380
tcccccacctg tgtaggttcc aaaatatcta gtgttttcat ttttacttgg atcaggaacc 1440
cagcactcca ctggataagc attatcctta tccaaaacag ccttgtggtc agtgttcatc 1500
tgctgactgt caactgtagc atttttgggg gttacagttt gagcaggata tttggtcctg 1560
tagtttgcta acacaccctg cagatctgaa ttcatctatg tcgggtgcgg agaaagaggt 1620
aatgaaatgg cattatgggt attatgggtc tgcattaatg aatcggccaa cgcgcgggga 1680
gaggcggttt gcgtattggg cgctcttccg cttcctcgct cactgactcg ctgcgctcgg 1740
tcgttcggct gcggcgagcg gtatcagctc actcaaaggc ggtaatacgg ttatccacag 1800
aatcagggga taacgcagga aagaacatgt gagcaaaagg ccagcaaaag gccaggaacc 1860
gtaaaaaggc cgcgttgctg gcgtttttcc ataggctccg cccccctgac gagcatcaca 1920
aaaatcgacg ctcaagtcag aggtggcgaa acccgacagg actataaaga taccaggcgt 1980
ttccccctgg aagctccctc gtgcgctctc ctgttccgac cctgccgctt accggatacc 2040
tgtccgcctt tctcccttcg ggaagcgtgg cgctttctca atgctcacgc tgtaggtatc 2100
tcagttcggt gtaggtcgtt cgctccaagc tgggctgtgt gcacgaaccc cccgttcagc 2160
ccgaccgctg cgccttatcc ggtaactatc gtcttgagtc caacccggta agacacgact 2220
tatcgccact ggcagcagcc actggtaaca ggattagcag agcgaggtat gtaggcggtg 2280
ctacagagtt cttgaagtgg tggcctaact acggctacac tagaaggaca gtatttggta 2340
tctgcgctct gctgaagcca gttaccttcg gaaaaagagt tggtagctct tgatccggca 2400
aacaaaccac cgctggtagc ggtggttttt ttgtttgcaa gcagcagatt acgcgcagaa 2460
```

```
aaaaaggatc tcaagaagat cctttgatct tttctacggg gtctgacgct cagtggaacg 2520
aaaactcacg ttaagggatt ttggtcatga gattatcaaa aaggatcttc acctagatcc 2580
ttttgatcct ccggcgttca gcctgtgcca cagccgacag gatggtgacc accatttgcc 2640
ccatatcacc gtcggtactg atcccgtcgt caataaaccg aaccgctaca ccctgagcat 2700
caaactcttt tatcagttgg atcatgtcgg cggtgtcgcg gccaagacgg tcgagcttct 2760
tcaccagaat gacatcacct tcctccacct tcatcctcag caaatccagc ccttcccgat 2820
ctgttgaact gccggatgcc ttgtcggtaa agatgcggtt agcttttacc cctgcatctt 2880
tgagcgctga ggtctgcctc gtgaagaagg tgttgctgac tcataccagg cctgaatcgc 2940
cccatcatcc agccagaaag tgagggagcc acggttgatg agagctttgt tgtaggtgga 3000
ccagttggtg attttgaact tttgctttgc cacggaacgg tctgcgttgt cgggaagatg 3060
cgtgatctga tccttcaact cagcaaaagt tcgatttatt caacaaagcc gccgtcccgt 3120
caagtcagcg taatgctctg ccagtgttac aaccaattaa ccaattctga ttagaaaaac 3180
tcatcgagca tcaaatgaaa ctgcaattta ttcatatcag gattatcaat accatatttt 3240
tgaaaaagcc gtttctgtaa tgaaggagaa aactcaccga ggcagttcca taggatggca 3300
agatcctggt atcggtctgc gattccgact cgtccaacat caatacaacc tattaatttc 3360
ccctcgtcaa aaataaggtt atcaagtgag aaatcaccat gagtgacgac tgaatccggt 3420
gagaatggca aaagcttatg catttctttc cagacttgtt caacaggcca gccattacgc 3480
tcgtcatcaa aatcactcgc atcaaccaaa ccgttattca ttcgtgattg cgcctgagcg 3540
agacgaaata cgcgatcgct gttaaaagga caattacaaa caggaatcga atgcaaccgg 3600
cgcaggaaca ctgccagcgc atcaacaata ttttcacctg aatcaggata ttcttctaat 3660
acctggaatg ctgttttccc ggggatcgca gtggtgagta accatgcatc atcaggagta 3720
cggataaaat gcttgatggt cggaagaggc ataaattccg tcagccagtt tagtctgacc 3780
atctcatctg taacatcatt ggcaacgcta cctttgccat gtttcagaaa caactctggc 3840
gcatcgggct tcccatacaa tcgatagatt gtcgcacctg attgcccgac attatcgcga 3900
gcccatttat acccatataa atcagcatcc atgttggaat ttaatcgcgg cctcgagcaa 3960
gacgtttccc gttgaatatg gctcataaca ccccttgtat tactgtttat gtaagcagac 4020
agttttattg ttcatgatga tatattttta tcttgtgcaa tgtaacatca gagattttga 4080
gacacaacgt ggctttgttg aataaatcga acttttgctg agttgaagga tcagatcacg 4140
catcttcccg acaacgcaga ccgttccgtg gcaaagcaaa agttcaaaat caccaactgg 4200
tccacctaca acaaagctct catcaaccgt ggctccctca ctttctggct ggatgatggg 4260
gcgattcagg cctggtatga gtcagcaaca ccttcttcac gaggcagacc tcagcgctag 4320
attattgaag catttatcag ggttattgtc tcatgagcgg atacatattt gaatgtattt 4380
agaaaaataa acaaataggg gttccgcgca catttccccg aaaagtgcca cctgacg     4437
```

<210> 12
<211> 32705
<212> DNA
<213> Artificial Sequence

<220>
<223> Description of Artificial Sequence: Synthetic Primer

<400> 12

cgtaactata acggtcctaa ggtagcgaaa attatttaaa tatctatgtc gggtgcggag 60
aaagaggtaa tgaaatggca tcgactcgaa gatctgggcg tggttaaggg tgggaaagaa 120
tatataaggt gggggtctta tgtagttttg tatctgtttt gcagcagccg ccgccgccat 180
gagcaccaac tcgtttgatg gaagcattgt gagctcatat ttgacaacgc gcatgccccc 240
atgggccggg gtgcgtcaga atgtgatggg ctccagcatt gatggtcgcc ccgtcctgcc 300
cgcaaactct actaccttga cctacgagac cgtgtctgga acgccgttgg agactgcagc 360
ctccgccgcc gcttcagccg ctgcagccac cgcccgcggg attgtgactg actttgcttt 420
cctgagcccg cttgcaagca gtgcagcttc ccgttcatcc gcccgcgatg acaagttgac 480
ggctcttttg gcacaattgg attctttgac ccgggaactt aatgtcgttt ctcagcagct 540
gttggatctg cgccagcagg tttctgccct gaaggcttcc tcccctccca atgcggttta 600
aaacataaat aaaaaaccag actctgtttg gatttggatc aagcaagtgt cttgctgtct 660
ttatttaggg gttttgcgcg cgcggtaggc ccgggaccag cggtctcggt cgttgagggt 720
cctgtgtatt ttttccagga cgtggtaaag gtgactctgg atgttcagat acatgggcat 780
aagcccgtct ctggggtgga ggtagcacca ctgcagagct tcatgctgcg gggtggtgtt 840
gtagatgatc cagtcgtagc aggagcgctg ggcgtggtgc ctaaaaatgt ctttcagtag 900
caagctgatt gccaggggca ggcccttggt gtaagtgttt acaaagcggt taagctggga 960
tgggtgcata cgtggggata tgagatgcat cttggactgt atttttaggt tggctatgtt 1020
cccagccata tccctccggg gattcatgtt gtgcagaacc accagcacag tgtatccggt 1080
gcacttggga aatttgtcat gtagcttaga aggaaatgcg tggaagaact tggagacgcc 1140
cttgtgacct ccaagatttt ccatgcattc gtccataatg atggcaatgg gcccacgggc 1200
ggcggcctgg gcgaagatat ttctgggatc actaacgtca tagttgtgtt ccaggatgag 1260
atcgtcatag gccattttta caaagcgcgg gcggagggtg ccagactgcg gtataatggt 1320
tccatccggc ccaggggcgt agttaccctc acagatttgc atttcccacg ctttgagttc 1380
agatgggggg atcatgtcta cctgcggggc gatgaagaaa acggtttccg ggtaggggga 1440
gatcagctgg gaagaaagca ggttcctgag cagctgcgac ttaccgcagc cggtgggccc 1500
gtaaatcaca cctattaccg gctgcaactg gtagttaaga gagctgcagc tgccgtcatc 1560
cctgagcagg ggggccactt cgttaagcat gtccctgact cgcatgtttt ccctgaccaa 1620
atccgccaga aggcgctcgc cgcccagcga tagcagttct tgcaaggaag caaagttttt 1680
caacggtttg agaccgtccg ccgtaggcat gcttttgagc gtttgaccaa gcagttccag 1740
gcggtcccac agctcggtca cctgctctac ggcatctcga tccagcatat ctcctcgttt 1800
cgcgggttgg ggcggctttc gctgtacggc agtagtcggt gctcgtccag acgggccagg 1860
gtcatgtctt tccacgggcg cagggtcctc gtcagcgtag tctgggtcac ggtgaagggg 1920
tgcgctccgg gctgcgcgct ggccagggtg cgcttgaggc tggtcctgct ggtgctgaag 1980
cgctgccggt cttcgccctg cgcgtcggcc aggtagcatt tgaccatggt gtcatagtcc 2040
agcccctccg cggcgtggcc cttggcgcgc agcttgccct tggaggaggc gccgcacgag 2100
gggcagtgca gacttttgag ggcgtagagc ttgggcgcga gaaataccga ttccggggag 2160
taggcatccg cgccgcaggc cccgcagacg gtctcgcatt ccacgagcca ggtgagctct 2220
ggccgttcgg ggtcaaaaac caggtttccc ccatgctttt tgatgcgttt cttacctctg 2280
gtttccatga gccggtgtcc acgctcggtg acgaaaaggc tgtccgtgtc cccgtataca 2340
gacttgagag gcctgtcctc gagcggtgtt ccgcggtcct cctcgtatag aaactcggac 2400
cactctgaga caaaggctcg cgtccaggcc agcacgaagg aggctaagtg ggaggggtag 2460

cggtcgttgt ccactagggg gtccactcgc tccagggtgt gaagacacat gtcgccctct 2520
tcggcatcaa ggaaggtgat tggtttgtag gtgtaggcca cgtgaccggg tgttcctgaa 2580
ggggggctat aaaaggggt gggggcgcgt tcgtcctcac tctcttccgc atcgctgtct 2640
gcgagggcca gctgttgggg tgagtactcc ctctgaaaag cgggcatgac ttctgcgcta 2700
agattgtcag tttccaaaaa cgaggaggat ttgatattca cctggcccgc ggtgatgcct 2760
ttgagggtgg ccgcatccat ctggtcagaa aagacaatct ttttgttgtc aagcttggtg 2820
gcaaacgacc cgtagagggc gttggacagc aacttggcga tggagcgcag ggtttggttt 2880
ttgtcgcgat cggcgcgctc cttggccgcg atgtttagct gcacgtattc gcgcgcaacg 2940
caccgccatt cgggaaagac ggtggtgcgc tcgtcgggca ccaggtgcac gcgccaaccg 3000
cggttgtgca gggtgacaag gtcaacgctg gtggctacct ctccgcgtag gcgctcgttg 3060
gtccagcaga ggcggccgcc cttgcgcgag cagaatggcg gtagggggtc tagctgcgtc 3120
tcgtccgggg ggtctgcgtc cacggtaaag accccgggca gcaggcgcgc gtcgaagtag 3180
tctatcttgc atccttgcaa gtctagcgcc tgctgccatg cgcgggcggc aagcgcgcgc 3240
tcgtatgggt tgagtggggg accccatggc atggggtggg tgagcgcgga ggcgtacatg 3300
ccgcaaatgt cgtaaacgta gaggggctct ctgagtattc caagatatgt agggtagcat 3360
cttccaccgc ggatgctggc gcgcacgtaa tcgtatagtt cgtgcgaggg agcgaggagg 3420
tcgggaccga ggttgctacg ggcgggctgc tctgctcgga agactatctg cctgaagatg 3480
gcatgtgagt tggatgatat ggttggacgc tggaagacgt tgaagctggc gtctgtgaga 3540
cctaccgcgt cacgcacgaa ggaggcgtag gagtcgcgca gcttgttgac cagctcggcg 3600
gtgacctgca cgtctagggc gcagtagtcc agggtttcct tgatgatgtc atacttatcc 3660
tgtccctttt ttttccacag ctcgcggttg aggacaaact cttcgcggtc tttccagtac 3720
tcttggatcg gaaacccgtc ggcctccgaa cggtaagagc ctagcatgta gaactggttg 3780
acggcctggt aggcgcagca tccctttttct acgggtagcg cgtatgcctg cgcggccttc 3840
cggagcgagg tgtgggtgag cgcaaaggtg tccctgacca tgactttgag gtactggtat 3900
ttgaagtcag tgtcgtcgca tccgccctgc tcccagagca aaaagtccgt gcgctttttg 3960
gaacgcggat ttggcagggc gaaggtgaca tcgttgaaga gtatctttcc cgcgcgaggc 4020
ataaagttgc gtgtgatgcg gaagggtccc ggcacctcgg aacggttgtt aattacctgg 4080
gcggcgagca cgatctcgtc aaagccgttg atgttgtggc ccacaatgta aagttccaag 4140
aagcgcggga tgcccttgat ggaaggcaat tttttaagtt cctcgtaggt gagctcttca 4200
ggggagctga gcccgtgctc tgaaagggcc cagtctgcaa gatgagggtt ggaagcgacg 4260
aatgagctcc acaggtcacg ggccattagc atttgcaggt ggtcgcgaaa ggtcctaaac 4320
tggcgaccta tggccatttt ttctggggtg atgcagtaga aggtaagcgg gtcttgttcc 4380
cagcggtccc atccaaggtt cgcggctagg tctcgcgcgg cagtcactag aggctcatct 4440
ccgccgaact tcatgaccag catgaagggc acgagctgct tcccaaaggc ccccatccaa 4500
gtataggtct ctacatcgta ggtgacaaag agacgctcgg tgcgaggatg cgagccgatc 4560
gggaagaact ggatctcccg ccaccaattg gaggagtggc tattgatgtg gtgaaagtag 4620
aagtccctgc gacgggccga acactcgtgc tggcttttgt aaaaacgtgc gcagtactgg 4680
cagcggtgca cgggctgtac atcctgcacg aggttgacct gacgaccgcg cacaaggaag 4740
cagagtggga atttgagccc ctcgcctggc gggtttggct ggtggtcttc tacttcggct 4800
gcttgtcctt gaccgtctgg ctgctcgagg ggagttacgg tggatcggac caccacgccg 4860
cgcgagccca aagtccagat gtccgcgcgc ggcggtcgga gcttgatgac aacatcgcgc 4920
agatgggagc tgtccatggt ctggagctcc cgcggcgtca ggtcaggcgg gagctcctgc 4980
aggtttacct cgcatagacg ggtcagggcg cgggctagat ccaggtgata cctaatttcc 5040

aggggctggt tggtggcggc gtcgatggct tgcaagaggc cgcatccccg cggcgcgact 5100
acggtaccgc gcggcgggcg gtgggccgcg ggggtgtcct tggatgatgc atctaaaagc 5160
ggtgacgcgg gcgagccccc ggaggtaggg ggggctccgg acccgccggg agagggggca 5220
ggggcacgtc ggcgccgcgc gcgggcagga gctggtgctg cgcgcgtagg ttgctggcga 5280
acgcgacgac gcggcggttg atctcctgaa tctggcgcct ctgcgtgaag acgacgggcc 5340
cggtgagctt gaacctgaaa gagagttcga cagaatcaat ttcggtgtcg ttgacggcgg 5400
cctggcgcaa aatctcctgc acgtctcctg agttgtcttg ataggcgatc tcggccatga 5460
actgctcgat ctcttcctcc tggagatctc cgcgtccggc tcgctccacg gtggcggcga 5520
ggtcgttgga aatgcgggcc atgagctgcg agaaggcgtt gaggcctccc tcgttccaga 5580
cgcggctgta gaccacgccc ccttcggcat cgcgggcgcg catgaccacc tgcgcgagat 5640
tgagctccac gtgccgggcg aagacggcgt agtttcgcag gcgctgaaag aggtagttga 5700
gggtggtggc ggtgtgttct gccacgaaga agtacataac ccagcgtcgc aacgtggatt 5760
cgttgatatc ccccaaggcc tcaaggcgct ccatggcctc gtagaagtcc acggcgaagt 5820
tgaaaaactg ggagttgcgc gccgacacgg ttaactcctc ctccagaaga cggatgagct 5880
cggcgacagt gtcgcgcacc tcgcgctcaa aggctacagg ggcctcttct tcttcttcaa 5940
tctcctcttc cataagggcc tccccttctt cttcttctgg cggcggtggg ggaggggga 6000
cacggcggcg acgacggcgc accgggaggc ggtcgacaaa gcgctcgatc atctccccgc 6060
ggcgacggcg catggtctcg gtgacggcgc ggccgttctc gcgggggcgc agttggaaga 6120
cgccgcccgt catgtcccgg ttatgggttg gcggggggct gccatgcggc agggatacgg 6180
cgctaacgat gcatctcaac aattgttgtg taggtactcc gccgccgagg gacctgagcg 6240
agtccgcatc gaccggatcg gaaaacctct cgagaaaggc gtctaaccag tcacagtcgc 6300
aaggtaggct gagcaccgtg gcgggcggca gcgggcggcg gtcggggttg tttctggcgg 6360
aggtgctgct gatgatgtaa ttaaagtagg cggtcttgag acggcggatg gtcgacagaa 6420
gcaccatgtc cttgggtccg gcctgctgaa tgcgcaggcg gtcggccatg ccccaggctt 6480
cgttttgaca tcggcgcagg tctttgtagt agtcttgcat gagcctttct accggcactt 6540
cttcttctcc ttcctcttgt cctgcatctc ttgcatctat cgctgcggcg gcggcggagt 6600
ttggccgtag gtggcgccct cttcctccca tgcgtgtgac cccgaagccc ctcatcggct 6660
gaagcagggc taggtcggcg acaacgcgct cggctaatat ggcctgctgc acctgcgtga 6720
gggtagactg gaagtcatcc atgtccacaa agcggtggta tgcgcccgtg ttgatggtgt 6780
aagtgcagtt ggccataacg gaccagttaa cggtctggtg acccggctgc gagagctcgg 6840
tgtacctgag acgcgagtaa gccctcgagt caaatacgta gtcgttgcaa gtccgcacca 6900
ggtactggta tcccaccaaa aagtgcggcg gcggctggcg gtagaggggc cagcgtaggg 6960
tggccggggc tccgggggcg agatcttcca acataaggcg atgatatccg tagatgtacc 7020
tggacatcca ggtgatgccg gcggcggtgg tggaggcgcg cggaaagtcg cggacgcggt 7080
tccagatgtt gcgcagcggc aaaaagtgct ccatggtcgg gacgctctgg ccggtcaggc 7140
gcgcgcaatc gttgacgctc tagcgtgcaa aaggagagcc tgtaagcggg cactcttccg 7200
tggtctggtg gataaattcg caagggtatc atggcggacg accggggttc gagccccgta 7260
tccggccgtc cgccgtgatc catgcggtta ccgcccgcgt gtcgaaccca ggtgtgcgac 7320
gtcagacaac gggggagtgc tcctttggc ttccttccag gcgcggcggc tgctgcgcta 7380
gcttttttgg ccactggccg cgcgcagcgt aagcggttag gctggaaagc gaaagcatta 7440
agtggctcgc tccctgtagc cggagggtta ttttccaagg gttgagtcgc gggacccccg 7500
gttcgagtct cggaccggcc ggactgcggc gaacgggggt ttgcctcccc gtcatgcaag 7560
accccgcttg caaattcctc cggaaacagg gacgagcccc tttttgctt ttcccagatg 7620

catccggtgc tgcggcagat gcgcccccct cctcagcagc ggcaagagca agagcagcgg 7680
cagacatgca gggcaccctc ccctcctcct accgcgtcag gaggggcgac atccgcggtt 7740
gacgcggcag cagatggtga ttacgaaccc ccgcggcgcc gggcccggca ctacctggac 7800
ttggaggagg gcgagggcct ggcgcggcta ggagcgccct ctcctgagcg gcacccaagg 7860
gtgcagctga agcgtgatac gcgtgaggcg tacgtgccgc ggcagaacct gtttcgcgac 7920
cgcgagggag aggagcccga ggagatgcgg gatcgaaagt tccacgcagg gcgcgagctg 7980
cggcatggcc tgaatcgcga gcggttgctg cgcgaggagg actttgagcc cgacgcgcga 8040
accgggatta gtcccgcgcg cgcacacgtg gcggccgccg acctggtaac cgcatacgag 8100
cagacggtga accaggagat taactttcaa aaaagcttta acaaccacgt gcgtacgctt 8160
gtggcgcgcg aggaggtggc tataggactg atgcatctgt gggactttgt aagcgcgctg 8220
gagcaaaacc caaatagcaa gccgctcatg gcgcagctgt tccttatagt gcagcacagc 8280
agggacaacg aggcattcag ggatgcgctg ctaaacatag tagagcccga gggccgctgg 8340
ctgctcgatt tgataaacat cctgcagagc atagtggtgc aggagcgcag cttgagcctg 8400
gctgacaagg tggccgccat caactattcc atgcttagcc tgggcaagtt ttacgcccgc 8460
aagatatacc atacccctta cgttcccata gacaaggagg taaagatcga ggggttctac 8520
atgcgcatgg cgctgaaggt gcttaccttg agcgacgacc tgggcgttta tcgcaacgag 8580
cgcatccaca aggccgtgag cgtgagccgg cggcgcgagc tcagcgaccg cgagctgatg 8640
cacagcctgc aaagggccct ggctggcacg ggcagcggcg atagagaggc cgagtcctac 8700
tttgacgcgg gcgctgacct gcgctgggcc ccaagccgac gcgccctgga ggcagctggg 8760
gccggacctg ggctggcggt ggcacccgcg cgcgctggca acgtcggcgg cgtggaggaa 8820
tatgacgagg acgatgagta cgagccagag gacggcgagt actaagcggt gatgtttctg 8880
atcagatgat gcaagacgca acggacccgg cggtgcgggc ggcgctgcag agccagccgt 8940
ccggccttaa ctccacggac gactggcgcc aggtcatgga ccgcatcatg tcgctgactg 9000
cgcgcaatcc tgacgcgttc cggcagcagc cgcaggccaa ccggctctcc gcaattctgg 9060
aagcggtggt cccggcgcgc gcaaacccca cgcacgagaa ggtgctggcg atcgtaaacg 9120
cgctggccga aaacagggcc atccggcccg acgaggccgg cctggtctac gacgcgctgc 9180
ttcagcgcgt ggctcgttac aacagcggca acgtgcagac caacctggac cggctggtgg 9240
gggatgtgcg cgaggccgtg gcgcagcgtg agcgcgcgca gcagcagggc aacctgggct 9300
ccatggttgc actaaacgcc ttcctgagta cacagcccgc caacgtgccg cggggacagg 9360
aggactacac caactttgtg agcgcactgc ggctaatggt gactgagaca ccgcaaagtg 9420
aggtgtacca gtctgggcca gactattttt tccagaccag tagacaaggc ctgcagaccg 9480
taaacctgag ccaggctttc aaaaacttgc aggggctgtg gggggtgcgg gctcccacag 9540
gcgaccgcgc gaccgtgtct agcttgctga cgcccaactc gcgcctgttg ctgctgctaa 9600
tagcgccctt cacggacagt ggcagcgtgt cccgggacac atacctaggt cacttgctga 9660
cactgtaccg cgaggccata ggtcaggcgc atgtggacga gcatactttc caggagatta 9720
caagtgtcag ccgcgcgctg gggcaggagg acacgggcag cctggaggca accctaaact 9780
acctgctgac caaccggcgg cagaagatcc cctcgttgca cagtttaaac agcgaggagg 9840
agcgcatttt gcgctacgtg cagcagagcg tgagccttaa cctgatgcgc gacggggtaa 9900
cgcccagcgt ggcgctggac atgaccgcgc gcaacatgga accgggcatg tatgcctcaa 9960
accggccgtt tatcaaccgc ctaatggact acttgcatcg cgcggccgcc gtgaaccccg 10020
agtatttcac caatgccatc ttgaacccgc actggctacc gccccctggt ttctacaccg 10080
ggggattcga ggtgcccgag ggtaacgatg gattcctctg ggacgacata gacgacagcg 10140
tgttttcccc gcaaccgcag accctgctag agttgcaaca gcgcgagcag gcagaggcgg 10200

cgctgcgaaa ggaaagcttc cgcaggccaa gcagcttgtc cgatctaggc gctgcggccc 10260
cgcggtcaga tgctagtagc ccatttccaa gcttgatagg gtctcttacc agcactcgca 10320
ccacccgccc gcgcctgctg ggcgaggagg agtacctaaa caactcgctg ctgcagccgc 10380
agcgcgaaaa aaacctgcct ccggcatttc ccaacaacgg gatagagagc ctagtggaca 10440
agatgagtag atggaagacg tacgcgcagg agcacaggga cgtgccaggc ccgcgcccgc 10500
ccacccgtcg tcaaaggcac gaccgtcagc ggggtctggt gtgggaggac gatgactcgg 10560
cagacgacag cagcgtcctg gatttgggag ggagtggcaa cccgtttgcg caccttcgcc 10620
ccaggctggg gagaatgttt taaaaaaaaa aaaagcatga tgcaaaataa aaaactcacc 10680
aaggccatgg caccgagcgt tggttttctt gtattcccct tagtatgcgg cgcgcggcga 10740
tgtatgagga aggtcctcct ccctcctacg agagtgtggt gagcgcggcg ccagtggcgg 10800
cggcgctggg ttctcccttc gatgctcccc tggacccgcc gtttgtgcct ccgcggtacc 10860
tgcggcctac cggggggaga aacagcatcc gttactctga gttggcaccc ctattcgaca 10920
ccacccgtgt gtacctggtg gacaacaagt caacggatgt ggcatccctg aactaccaga 10980
acgaccacag caactttctg accacggtca ttcaaaacaa tgactacagc ccgggggagg 11040
caagcacaca gaccatcaat cttgacgacc ggtcgcactg ggcggcgac ctgaaaacca 11100
tcctgcatac caacatgcca aatgtgaacg agttcatgtt taccaataag tttaaggcgc 11160
gggtgatggt gtcgcgcttg cctactaagg acaatcaggt ggagctgaaa tacgagtggg 11220
tggagttcac gctgcccgag ggcaactact ccgagaccat gaccatagac cttatgaaca 11280
acgcgatcgt ggagcactac ttgaaagtgg gcagacagaa cggggttctg gaaagcgaca 11340
tcggggtaaa gtttgacacc cgcaacttca gactggggtt tgaccccgtc actggtcttg 11400
tcatgcctgg ggtatataca aacgaagcct tccatccaga catcattttg ctgccaggat 11460
gcggggtgga cttcacccac agccgcctga gcaacttgtt gggcatccgc aagcggcaac 11520
ccttccagga gggctttagg atcacctacg atgatctgga gggtggtaac attcccgcac 11580
tgttggatgt ggacgcctac caggcgagct tgaaagatga caccgaacag ggcgggggtg 11640
gcgcaggcgg cagcaacagc agtggcagcg gcgcggaaga gaactccaac gcggcagccg 11700
cggcaatgca gccggtggag gacatgaacg atcatgccat tcgcggcgac acctttgcca 11760
cacgggctga ggagaagcgc gctgaggccg aagcagcggc cgaagctgcc gcccccgctg 11820
cgcaacccga ggtcgagaag cctcagaaga aaccggtgat caaacccctg acagaggaca 11880
gcaagaaacg cagttacaac ctaataagca atgacagcac cttcacccag taccgcagct 11940
ggtaccttgc atacaactac ggcgaccctc agaccggaat ccgctcatgg accctgcttt 12000
gcactcctga cgtaacctgc ggctcggagc aggtctactg gtcgttgcca gacatgatgc 12060
aagaccccgt gaccttccgc tccacgcgcc agatcagcaa ctttccggtg gtgggcgccg 12120
agctgttgcc cgtgcactcc aagagcttct acaacgacca ggccgtctac tcccaactca 12180
tccgccagtt tacctctctg acccacgtgt tcaatcgctt tcccgagaac cagattttgg 12240
cgcgcccgcc agcccccacc atcaccaccg tcagtgaaaa cgttcctgct ctcacagatc 12300
acgggacgct accgctgcgc aacagcatcg gaggagtcca gcgagtgacc attactgacg 12360
ccagacgccg cacctgcccc tacgtttaca aggccctggg catagtctcg ccgcgcgtcc 12420
tatcgagccg cactttttga gcaagcatgt ccatccttat atcgcccagc aataacacag 12480
gctggggcct gcgcttccca agcaagatgt ttggcggggc caagaagcgc tccgaccaac 12540
acccagtgcg cgtgcgcggg cactaccgcg cgccctgggg cgcgcacaaa cgcggccgca 12600
ctgggcgcac caccgtcgat gacgccatcg acgcggtggt ggaggaggcg cgcaactaca 12660
cgcccacgcc gccaccagtg tccacagtgg acgcggccat tcagaccgtg gtgcgcggag 12720
cccggcgcta tgctaaaatg aagagacggc ggaggcgcgt agcacgtcgc caccgccgcc 12780

gacccggcac tgccgcccaa cgcgcggcgg cggccctgct taaccgcgca cgtcgcaccg 12840
gccgacgggc ggccatgcgg gccgctcgaa ggctggccgc gggtattgtc actgtgcccc 12900
ccaggtccag gcgacgagcg gccgccgcag cagccgcggc cattagtgct atgactcagg 12960
gtcgcagggg caacgtgtat tgggtgcgcg actcggttag cggcctgcgc gtgcccgtgc 13020
gcacccgccc cccgcgcaac tagattgcaa gaaaaaacta cttagactcg tactgttgta 13080
tgtatccagc ggcggcggcg cgcaacgaag ctatgtccaa gcgcaaaatc aaagaagaga 13140
tgctccaggt catcgcgccg gagatctatg gccccccgaa gaaggaagag caggattaca 13200
agccccgaaa gctaaagcgg gtcaaaaaga aaaagaaaga tgatgatgat gaacttgacg 13260
acgaggtgga actgctgcac gctaccgcgc ccaggcgacg ggtacagtgg aaaggtcgac 13320
gcgtaaaacg tgttttgcga cccggcacca ccgtagtctt tacgcccggt gagcgctcca 13380
cccgcaccta caagcgcgtg tatgatgagg tgtacggcga cgaggacctg cttgagcagg 13440
ccaacgagcg cctcggggag tttgcctacg gaaagcggca taaggacatg ctggcgttgc 13500
cgctggacga gggcaaccca acacctagcc taaagcccgt aacactgcag caggtgctgc 13560
ccgcgcttgc accgtccgaa gaaaagcgcg gcctaaagcg cgagtctggt gacttggcac 13620
ccaccgtgca gctgatggta cccaagcgcc agcgactgga agatgtcttg gaaaaaatga 13680
ccgtggaacc tgggctggag cccgaggtcc gcgtgcggcc aatcaagcag gtggcgccgg 13740
gactgggcgt gcagaccgtg gacgttcaga tacccactac cagtagcacc agtattgcca 13800
ccgccacaga gggcatggag acacaaacgt ccccggttgc ctcagcggtg gcggatgccg 13860
cggtgcaggc ggtcgctgcg gccgcgtcca agacctctac ggaggtgcaa acggacccgt 13920
ggatgtttcg cgtttcagcc ccccggcgcc cgcgccgttc gaggaagtac ggcgccgcca 13980
gcgcgctact gcccgaatat gccctacatc cttccattgc gcctaccccc ggctatcgtg 14040
gctacaccta ccgcccccaga agacgagcaa ctacccgacg ccgaaccacc actggaaccc 14100
gccgccgccg tcgccgtcgc cagcccgtgc tggccccgat ttccgtgcgc agggtggctc 14160
gcgaaggagg caggaccctg gtgctgccaa cagcgcgcta ccaccccagc atcgtttaaa 14220
agccggtctt tgtggttctt gcagatatgg ccctcacctg ccgcctccgt ttcccggtgc 14280
cgggattccg aggaagaatg caccgtagga ggggcatggc cggccacggc ctgacgggcg 14340
gcatgcgtcg tgcgcaccac cggcggcggc gcgcgtcgca ccgtcgcatg cgcggcggta 14400
tcctgcccct ccttattcca ctgatcgccg cggcgattgg cgccgtgccc ggaattgcat 14460
ccgtggcctt gcaggcgcag agacactgat taaaaacaag ttgcatgtgg aaaaatcaaa 14520
ataaaaagtc tggactctca cgctcgcttg gtcctgtaac tattttgtag aatggaagac 14580
atcaactttg cgtctctggc ccccgcgacac ggctcgcgcc cgttcatggg aaactggcaa 14640
gatatcggca ccagcaatat gagcggtggc gccttcagct ggggctcgct gtggagcggc 14700
attaaaaatt tcggttccac cgttaagaac tatggcagca aggcctggaa cagcagcaca 14760
ggccagatgc tgagggataa gttgaaagag caaaatttcc aacaaaaggt ggtagatggc 14820
ctggcctctg gcattagcgg ggtggtggac ctggccaacc aggcagtgca aaataagatt 14880
aacagtaagc ttgatccccg ccctcccgta gaggagcctc caccggccgt ggagacagtg 14940
tctccagagg ggcgtggcga aaagcgtccg cgccccgaca gggaagaaac tctggtgacg 15000
caaatagacg agcctccctc gtacgaggag gcactaaagc aaggcctgcc caccacccgt 15060
cccatcgcgc ccatggctac cggagtgctg ggccagcaca caccccgtaac gctggacctg 15120
cctcccccccg ccgacaccca gcagaaacct gtgctgccag gcccgaccgc cgttgttgta 15180
acccgtccta gccgcgcgtc cctgcgccgc gccgccagcg gtccgcgatc gttgcggccc 15240
gtagccagtg gcaactggca aagcacactg aacagcatcg tgggtctggg ggtgcaatcc 15300
ctgaagcgcc gacgatgctt ctgatagcta acgtgtcgta tgtgtgtcat gtatgcgtcc 15360

atgtcgccgc cagaggagct gctgagccgc cgcgcgcccg ctttccaaga tggctacccc 15420
ttcgatgatg ccgcagtggt cttacatgca catctcgggc caggacgcct cggagtacct 15480
gagccccggg ctggtgcagt ttgcccgcgc caccgagacg tacttcagcc tgaataacaa 15540
gtttagaaac cccacggtgg cgcctacgca cgacgtgacc acagaccggt cccagcgttt 15600
gacgctgcgg ttcatccctg tggaccgtga ggatactgcg tactcgtaca aggcgcggtt 15660
caccctagct gtgggtgata accgtgtgct ggacatggct tccacgtact ttgacatccg 15720
cggcgtgctg gacaggggcc ctacttttaa gccctactct ggcactgcct acaacgccct 15780
ggctcccaag ggtgccccaa atccttgcga atgggatgaa gctgctactg ctcttgaaat 15840
aaacctagaa gaagaggacg atgacaacga agacgaagta gacgagcaag ctgagcagca 15900
aaaaactcac gtatttgggc aggcgcctta ttctggtata aatattacaa aggagggtat 15960
tcaaataggt gtcgaaggtc aaacacctaa atatgccgat aaaacatttc aacctgaacc 16020
tcaaatagga gaatctcagt ggtacgaaac agaaattaat catgcagctg ggagagtcct 16080
aaaaaagact accccaatga aaccatgtta cggttcatat gcaaaaccca caaatgaaaa 16140
tggagggcaa ggcattcttg taaagcaaca aaatggaaag ctagaaagtc aagtggaaat 16200
gcaatttttc tcaactactg aggcagccgc aggcaatggt gataacttga ctcctaaagt 16260
ggtattgtac agtgaagatg tagatataga aaccccagac actcatattt cttacatgcc 16320
cactattaag gaaggtaact cacgagaact aatgggccaa caatctatgc ccaacaggcc 16380
taattacatt gcttttaggg acaattttat tggtctaatg tattacaaca gcacgggtaa 16440
tatgggtgtt ctggcgggcc aagcatcgca gttgaatgct gttgtagatt tgcaagacag 16500
aaacacagag ctttcatacc agcttttgct tgattccatt ggtgatagaa ccaggtactt 16560
ttctatgtgg aatcaggctg ttgacagcta tgatccagat gttagaatta ttgaaaatca 16620
tggaactgaa gatgaacttc caaattactg ctttccactg ggaggtgtga ttaatacaga 16680
gactcttacc aaggtaaaac ctaaaacagg tcaggaaaat ggatgggaaa aagatgctac 16740
agaattttca gataaaaatg aaataagagt tggaaataat tttgccatgg aaatcaatct 16800
aaatgccaac ctgtggagaa atttcctgta ctccaacata gcgctgtatt tgcccgacaa 16860
gctaaagtac agtccttcca acgtaaaaat ttctgataac ccaaacacct acgactacat 16920
gaacaagcga gtggtggctc ccgggctagt ggactgctac attaaccttg gagcacgctg 16980
gtcccttgac tatatggaca acgtcaaccc atttaaccac caccgcaatg ctggcctgcg 17040
ctaccgctca atgttgctgg gcaatggtcg ctatgtgccc ttccacatcc aggtgcctca 17100
gaagttcttt gccattaaaa acctccttct cctgccgggc tcatacacct acgagtggaa 17160
cttcaggaag gatgttaaca tggttctgca gagctcccta ggaaatgacc taagggttga 17220
cggagccagc attaagtttg atagcatttg cctttacgcc accttcttcc ccatggccca 17280
caacaccgcc tccacgcttg aggccatgct tagaaacgac accaacgacc agtcctttaa 17340
cgactatctc tccgccgcca acatgctcta ccctataccc gccaacgcta ccaacgtgcc 17400
catatccatc ccctcccgca actgggcggc tttccgcggc tgggccttca cgcgccttaa 17460
gactaaggaa accccatcac tgggctcggg ctacgaccct tattacacct actctggctc 17520
tataccctac ctagatggaa ccttttacct caaccacacc tttaagaagg tggccattac 17580
ctttgactct tctgtcagct ggcctggcaa tgaccgcctg cttaccccca acgagtttga 17640
aattaagcgc tcagttgacg gggagggtta caacgttgcc cagtgtaaca tgaccaaaga 17700
ctggttcctg gtacaaatgc tagctaacta taacattggc taccagggct tctatatccc 17760
agagagctac aaggaccgca tgtactcctt ctttagaaac ttccagccca tgagccgtca 17820
ggtggtggat gatactaaat acaaggacta ccaacaggtg ggcatcctac accaacacaa 17880
caactctgga tttgttggct accttgcccc caccatgcgc gaaggacagg cctaccctgc 17940

taacttcccc tatccgctta taggcaagac cgcagttgac agcattaccc agaaaaagtt 18000
tctttgcgat cgcacccttt ggcgcatccc attctccagt aactttatgt ccatgggcgc 18060
actcacagac ctgggccaaa accttctcta cgccaactcc gcccacgcgc tagacatgac 18120
ttttgaggtg gatcccatgg acgagcccac ccttctttat gttttgtttg aagtctttga 18180
cgtggtccgt gtgcaccagc cgcaccgcgg cgtcatcgaa accgtgtacc tgcgcacgcc 18240
cttctcggcc ggcaacgcca caacataaag aagcaagcaa catcaacaac agctgccgcc 18300
atgggctcca gtgagcagga actgaaagcc attgtcaaag atcttggttg tgggccatat 18360
tttttgggca cctatgacaa gcgctttcca ggctttgttt ctccacacaa gctcgcctgc 18420
gccatagtca atacggccgg tcgcgagact gggggcgtac actggatggc ctttgcctgg 18480
aacccgcact caaaaacatg ctacctcttt gagccctttg gcttttctga ccagcgactc 18540
aagcaggttt accagtttga gtacgagtca ctcctgcgcc gtagcgccat tgcttcttcc 18600
cccgaccgct gtataacgct ggaaaagtcc acccaaagcg tacaggggcc caactcggcc 18660
gcctgtggac tattctgctg catgtttctc cacgcctttg ccaactggcc ccaaactccc 18720
atggatcaca accccaccat gaaccttatt accggggtac ccaactccat gctcaacagt 18780
ccccaggtac agcccaccct gcgtcgcaac caggaacagc tctacagctt cctggagcgc 18840
cactcgccct acttccgcag ccacagtgcg cagattagga gcgccacttc tttttgtcac 18900
ttgaaaaaca tgtaaaaata atgtactaga gacactttca ataaaggcaa atgcttttat 18960
ttgtacactc tcgggtgatt atttaccccc acccttgccg tctgcgccgt ttaaaaatca 19020
aaggggttct gccgcgcatc gctatgcgcc actggcaggg acacgttgcg atactggtgt 19080
ttagtgctcc acttaaactc aggcacaacc atccgcggca gctcggtgaa gttttcactc 19140
cacaggctgc gcaccatcac caacgcgttt agcaggtcgg gcgccgatat cttgaagtcg 19200
cagttggggc ctccgccctg cgcgcgcgag ttgcgataca cagggttgca gcactggaac 19260
actatcagcg ccgggtggtg cacgctggcc agcacgctct tgtcggagat cagatccgcg 19320
tccaggtcct ccgcgttgct cagggcgaac ggagtcaact ttggtagctg ccttcccaaa 19380
aagggcgcgt gcccaggctt tgagttgcac tcgcaccgta gtggcatcaa aaggtgaccg 19440
tgcccggtct gggcgttagg atacagcgcc tgcataaaag ccttgatctg cttaaaagcc 19500
acctgagcct ttgcgccttc agagaagaac atgccgcaag acttgccgga aaactgattg 19560
gccggacagg ccgcgtcgtg cacgcagcac cttgcgtcgg tgttggagat ctgcaccaca 19620
tttcggcccc accggttctt cacgatcttg gccttgctag actgctcctt cagcgcgcgc 19680
tgcccgtttt cgctcgtcac atccatttca atcacgtgct ccttatttat cataatgctt 19740
ccgtgtagac acttaagctc gccttcgatc tcagcgcagc ggtgcagcca caacgcgcag 19800
cccgtgggct cgtgatgctt gtaggtcacc tctgcaaacg actgcaggta cgcctgcagg 19860
aatcgcccca tcatcgtcac aaaggtcttg ttgctggtga aggtcagctg caacccgcgg 19920
tgctcctcgt tcagccaggt cttgcatacg gccgccagag cttccacttg gtcaggcagt 19980
agtttgaagt tcgcctttag atcgttatcc acgtggtact tgtccatcag cgcgcgcgca 20040
gcctccatgc ccttctccca cgcagacacg atcggcacac tcagcgggtt catcaccgta 20100
atttcacttt ccgcttcgct gggctcttcc tcttcctctt gcgtccgcat accacgcgcc 20160
actgggtcgt cttcattcag ccgccgcact gtgcgcttac ctcctttgcc atgcttgatt 20220
agcaccggtg ggttgctgaa acccaccatt tgtagcgcca catcttctct ttcttcctcg 20280
ctgtccacga ttacctctgg tgatggcggg cgctcgggct tgggagaagg gcgcttcttt 20340
ttcttcttgg gcgcaatggc caaatccgcc gccgaggtcg atggccgcgg gctgggtgtg 20400
cgcggcacca gcgcgtcttg tgatgagtct tcctcgtcct cggactcgat acgccgcctc 20460
atccgctttt ttgggggcgc ccggggaggc ggcggcgacg gggacgggga cgacacgtcc 20520

tccatggttg ggggacgtcg cgccgcaccg cgtccgcgct cggggggtggt ttcgcgctgc 20580
tcctcttccc gactggccat ttccttctcc tataggcaga aaaagatcat ggagtcagtc 20640
gagaagaagg acagcctaac cgccccctct gagttcgcca ccaccgcctc caccgatgcc 20700
gccaacgcgc ctaccacctt ccccgtcgag gcacccccgc ttgaggagga ggaagtgatt 20760
atcgagcagg acccaggttt tgtaagcgaa gacgacgagg accgctcagt accaacagag 20820
gataaaaagc aagaccagga caacgcagag gcaaacgagg aacaagtcgg gcggggggac 20880
gaaaggcatg gcgactacct agatgtggga gacgacgtgc tgttgaagca tctgcagcgc 20940
cagtgcgcca ttatctgcga cgcgttgcaa gagcgcagcg atgtgcccct cgccatagcg 21000
gatgtcagcc ttgcctacga acgccaccta ttctcaccgc gcgtaccccc caaacgccaa 21060
gaaaacggca catgcgagcc caacccgcgc ctcaacttct accccgtatt tgccgtgcca 21120
gaggtgcttg ccacctatca catctttttc caaaactgca agatacccct atcctgccgt 21180
gccaaccgca gccgagcgga caagcagctg gccttgcggc agggcgctgt catacctgat 21240
atcgcctcgc tcaacgaagt gccaaaaatc tttgagggtc ttggacgcga cgagaagcgc 21300
gcggcaaacg ctctgcaaca ggaaaacagc gaaaatgaaa gtcactctgg agtgttggtg 21360
gaactcgagg gtgacaacgc gcgcctagcc gtactaaaac gcagcatcga ggtcacccac 21420
tttgcctacc cggcacttaa cctacccccc aaggtcatga gcacagtcat gagtgagctg 21480
atcgtgcgcc gtgcgcagcc cctggagagg gatgcaaatt tgcaagaaca aacagaggag 21540
ggcctacccg cagttggcga cgagcagcta gcgcgctggc ttcaaacgcg cgagcctgcc 21600
gacttggagg agcgacgcaa actaatgatg gccgcagtgc tcgttaccgt ggagcttgag 21660
tgcatgcagc ggttctttgc tgacccggag atgcagcgca agctagagga aacattgcac 21720
tacacctttc gacagggcta cgtacgccag gcctgcaaga tctccaacgt ggagctctgc 21780
aacctggtct cctaccttgg aattttgcac gaaaaccgcc ttgggcaaaa cgtgcttcat 21840
tccacgctca agggcgaggc gcgccgcgac tacgtccgcg actgcgttta cttatttcta 21900
tgctacacct ggcagacggc catgggcgtt tggcagcagt gcttggagga gtgcaacctc 21960
aaggagctgc agaaactgct aaagcaaaac ttgaaggacc tatggacggc cttcaacgag 22020
cgctccgtgg ccgcgcacct ggcggacatc attttccccg aacgcctgct taaaaccctg 22080
caacagggtc tgccagactt caccagtcaa agcatgttgc agaactttag gaactttatc 22140
ctagagcgct caggaatctt gcccgccacc tgctgtgcac ttcctagcga ctttgtgccc 22200
attaagtacc gcgaatgccc tccgccgctt tggggccact gctaccttct gcagctagcc 22260
aactaccttg cctaccactc tgacataatg gaagacgtga gcggtgacgg tctactggag 22320
tgtcactgtc gctgcaacct atgcaccccg caccgctccc tggtttgcaa ttcgcagctg 22380
cttaacgaaa gtcaaattat cggtaccttt gagctgcagg gtccctcgcc tgacgaaaag 22440
tccgcggctc cggggttgaa actcactccg ggctgtggac cgtcggctta ccttcgcaaa 22500
tttgtacctg aggactacca cgcccacgag attaggttct acgaagacca atcccgcccg 22560
cctaatgcgg agcttaccgc ctgcgtcatt acccagggcc acattcttgg ccaattgcaa 22620
gccatcaaca aagcccgcca agagtttctg ctacgaaagg gacggggggt ttacttggac 22680
ccccagtccg gcgaggagct caacccaatc ccccgccgc cgcagcccta tcagcagcag 22740
ccgcgggccc ttgcttccca ggatggcacc caaaaagaag ctgcagctgc cgccgccacc 22800
cacggacgag gaggaatact gggacagtca ggcagaggag gttttggacg aggaggagga 22860
ggacatgatg gaagactggg agagcctaga cgaggaagct tccgaggtcg aagaggtgtc 22920
agacgaaaca ccgtcaccct cggtcgcatt cccctcgccg gcgccccaga aatcggcaac 22980
cggttccagc atggctacaa cctccgctcc tcaggcgccg ccggcactgc ccgttcgccg 23040
acccaaccgt agatgggaca ccactggaac cagggccggt aagtccaagc agccgccgcc 23100

gttagcccaa gagcaacaac agcgccaagg ctaccgctca tggcgcgggc acaagaacgc 23160
catagttgct tgcttgcaag actgtggggg caacatctcc ttcgcccgcc gctttcttct 23220
ctaccatcac ggcgtggcct tcccccgtaa catcctgcat tactaccgtc atctctacag 23280
cccatactgc accggcggca gcggcagcaa cagcagcggc cacacagaag caaaggcgac 23340
cggatagcaa gactctgaca aagcccaaga aatccacagc ggcggcagca gcaggaggag 23400
gagcgctgcg tctggcgccc aacgaacccg tatcgacccg cgagcttaga aacaggattt 23460
ttcccactct gtatgctata tttcaacaga gcaggggcca agaacaagag ctgaaaataa 23520
aaaacaggtc tctgcgatcc ctcacccgca gctgcctgta tcacaaaagc gaagatcagc 23580
ttcggcgcac gctggaagac gcggaggctc tcttcagtaa atactgcgcg ctgactctta 23640
aggactagtt tcgcgccctt tctcaaattt aagcgcgaaa actacgtcat ctccagcggc 23700
cacacccggc gccagcacct gttgtcagcg ccattatgag caaggaaatt cccacgccct 23760
acatgtggag ttaccagcca caaatgggac ttgcggctgg agctgcccaa gactactcaa 23820
cccgaataaa ctacatgagc gcgggacccc acatgatatc ccgggtcaac ggaatacgcg 23880
cccaccgaaa ccgaattctc ctggaacagg cggctattac caccacacct cgtaataacc 23940
ttaatccccg tagttggccc gctgccctgg tgtaccagga aagtcccgct cccaccactg 24000
tggtacttcc cagagacgcc caggccgaag ttcagatgac taactcaggg gcgcagcttg 24060
cgggcggctt tcgtcacagg gtgcggtcgc ccgggcaggg tataactcac ctgacaatca 24120
gagggcgagg tattcagctc aacgacgagt cggtgagctc ctcgcttggt ctccgtccgg 24180
acgggacatt tcagatcggc ggcgccggcc gctcttcatt cacgcctcgt caggcaatcc 24240
taactctgca gacctcgtcc tctgagccgc gctctggagg cattggaact ctgcaattta 24300
ttgaggagtt tgtgccatcg gtctacttta accccttctc gggacctccc ggccactatc 24360
cggatcaatt tattcctaac tttgacgcgg taaaggactc ggcggacggc tacgactgaa 24420
tgttataagt tcctgtccat ccgcacccac tatcttcatg ttgttgcaga tgaagcgcgc 24480
aagaccgtct gaagatacct tcaaccccgt gtatccatat gacacggaaa ccggtcctcc 24540
aactgtgcct ttttcttactc ctccctttgt atcccccaat gggtttcaag agagtccccc 24600
tggggtactc tctttgcgcc tatccgaacc tctagttacc tccaatggca tgcttgcgct 24660
caaaatgggc aacggcctct ctctggacga ggccggcaac cttacctccc aaaatgtaac 24720
cactgtgagc ccacctctca aaaaaaccaa gtcaaacata aacctggaaa tatctgcacc 24780
cctcacagtt acctcagaag ccctaactgt ggctgccgcc gcacctctaa tggtcgcggg 24840
caacacactc accatgcaat cacaggcccc gctaaccgtg cacgactcca aacttagcat 24900
tgccacccaa ggacccctca cagtgtcaga aggaaagcta gccctgcaaa catcaggccc 24960
cctcaccacc accgatagca gtacccttac tatcactgcc tcacccccctc taactactgc 25020
cactggtagc ttgggcattg acttgaaaga gcccatttat acacaaaatg gaaaactagg 25080
actaaagtac ggggctcctt tgcatgtaac agacgaccta aacactttga ccgtagcaac 25140
tggtccaggt gtgactatta ataatacttc cttgcaaact aaagttactg gagccttggg 25200
ttttgattca caaggcaata tgcaacttaa tgtagcagga ggactaagga ttgattctca 25260
aaacagacgc cttatacttg atgttagtta tccgtttgat gctcaaaacc aactaaatct 25320
aagactagga cagggccctc tttttataaa ctcagcccac aacttggata ttaactacaa 25380
caaaggcctt tacttgttta cagcttcaaa caattccaaa aagcttgagg ttaacctaag 25440
cactgccaag gggttgatgt ttgacgctac agccatagcc attaatgcag gagatgggct 25500
tgaatttggt tcacctaatg caccaaacac aaatcccctc aaaacaaaaa ttggccatgg 25560
cctagaattt gattcaaaca aggctatggt tcctaaacta ggaactggcc ttagttttga 25620
cagcacaggt gccattacag taggaaacaa aaataatgat aagctaactt tgtggaccac 25680

accagctcca tctcctaact gtagactaaa tgcagagaaa gatgctaaac tcactttggt 25740
cttaacaaaa tgtggcagtc aaatacttgc tacagtttca gttttggctg ttaaaggcag 25800
tttggctcca atatctggaa cagttcaaag tgctcatctt attataagat ttgacgaaaa 25860
tggagtgcta ctaaacaatt ccttcctgga cccagaatat tggaacttta gaaatggaga 25920
tcttactgaa ggcacagcct atacaaacgc tgttggattt atgcctaacc tatcagctta 25980
tccaaaatct cacggtaaaa ctgccaaaag taacattgtc agtcaagttt acttaaacgg 26040
agacaaaact aaacctgtaa cactaaccat tacactaaac ggtacacagg aaacaggaga 26100
cacaactcca agtgcatact ctatgtcatt ttcatgggac tggtctggcc acaactacat 26160
taatgaaata tttgccacat cctcttacac tttttcatac attgcccaag aataaagaat 26220
cgtttgtgtt atgtttcaac gtgtttattt ttcaattgca gaaaatttca agtcattttt 26280
cattcagtag tatagcccca ccaccacata gcttatacag atcaccgtac cttaatcaaa 26340
ctcacagaac cctagtattc aacctgccac ctccctccca acacacagag tacacagtcc 26400
tttctccccg gctggcctta aaaagcatca tatcatgggt aacagacata ttcttaggtg 26460
ttatattcca cacggtttcc tgtcgagcca aacgctcatc agtgatatta ataaactccc 26520
cgggcagctc acttaagttc atgtcgctgt ccagctgctg agccacaggc tgctgtccaa 26580
cttgcggttg cttaacgggc ggcgaaggag aagtccacgc ctacatgggg gtagagtcat 26640
aatcgtgcat caggataggg cggtggtgct gcagcagcgc gcgaataaac tgctgccgcc 26700
gccgctccgt cctgcaggaa tacaacatgg cagtggtctc ctcagcgatg attcgcaccg 26760
cccgcagcat aaggcgcctt gtcctccggg cacagcagcg caccctgatc tcacttaaat 26820
cagcacagta actgcagcac agcaccacaa tattgttcaa aatcccacag tgcaaggcgc 26880
tgtatccaaa gctcatggcg gggaccacag aacccacgtg gccatcatac cacaagcgca 26940
ggtagattaa gtggcgaccc ctcataaaca cgctggacat aaacattacc tcttttggca 27000
tgttgtaatt caccacctcc cggtaccata taaacctctg attaaacatg gcgccatcca 27060
ccaccatcct aaaccagctg gccaaaacct gcccgccggc tatacactgc agggaaccgg 27120
gactggaaca atgacagtgg agagcccagg actcgtaacc atggatcatc atgctcgtca 27180
tgatatcaat gttggcacaa cacaggcaca cgtgcataca cttcctcagg attacaagct 27240
cctcccgcgt tagaaccata tcccagggaa caacccattc ctgaatcagc gtaaatccca 27300
cactgcaggg aagacctcgc acgtaactca cgttgtgcat tgtcaaagtg ttacattcgg 27360
gcagcagcgg atgatcctcc agtatggtag cgcgggtttc tgtctcaaaa ggaggtagac 27420
gatccctact gtacggagtg cgccgagaca accgagatcg tgttggtcgt agtgtcatgc 27480
caaatggaac gccggacgta gtcatatttc ctgaagcaaa accaggtgcg ggcgtgacaa 27540
acagatctgc gtctccggtc tcgccgctta gatcgctctg tgtagtagtt gtagtatatc 27600
cactctctca aagcatccag gcgccccctg gcttcgggtt ctatgtaaac tccttcatgc 27660
gccgctgccc tgataacatc caccaccgca gaataagcca cacccagcca acctacacat 27720
tcgttctgcg agtcacacac gggaggagcg ggaagagctg gaagaaccat gtttttttt 27780
ttattccaaa agattatcca aaacctcaaa atgaagatct attaagtgaa cgcgctcccc 27840
tccggtggcg tggtcaaact ctacagccaa agaacagata atggcatttg taagatgttg 27900
cacaatggct tccaaaaggc aaacggccct cacgtccaag tggacgtaaa ggctaaaccc 27960
ttcagggtga atctcctcta taaacattcc agcaccttca accatgccca aataattctc 28020
atctcgccac cttctcaata tatctctaag caaatcccga atattaagtc cggccattgt 28080
aaaaatctgc tccagagcgc cctccacctt cagcctcaag cagcgaatca tgattgcaaa 28140
aattcaggtt cctcacagac ctgtataaga ttcaaaagcg gaacattaac aaaaataccg 28200
cgatcccgta ggtcccttcg cagggccagc tgaacataat cgtgcaggtc tgcacggacc 28260

agcgcggcca cttccccgcc aggaaccatg acaaaagaac ccacactgat tatgacacgc 28320
atactcggag ctatgctaac cagcgtagcc ccgatgtaag cttgttgcat gggcggcgat 28380
ataaaatgca aggtgctgct caaaaaatca ggcaaagcct cgcgcaaaaa agaaagcaca 28440
tcgtagtcat gctcatgcag ataaaggcag gtaagctccg gaaccaccac agaaaaagac 28500
accattttc tctcaaacat gtctgcgggt ttctgcataa acacaaaata aaataacaaa 28560
aaaacattta aacattagaa gcctgtctta caacaggaaa aacaaccctt ataagcataa 28620
gacggactac ggccatgccg gcgtgaccgt aaaaaaactg gtcaccgtga ttaaaaagca 28680
ccaccgacag ctcctcggtc atgtccggag tcataatgta agactcggta aacacatcag 28740
gttgattcac atcggtcagt gctaaaaagc gaccgaaata gcccggggga atacataccc 28800
gcaggcgtag agacaacatt acagccccca taggaggtat aacaaaatta ataggagaga 28860
aaaacacata aacacctgaa aaaccctcct gcctaggcaa aatagcaccc tcccgctcca 28920
gaacaacata cagcgcttcc acagcggcag ccataacagt cagccttacc agtaaaaaag 28980
aaaacctatt aaaaaaacac cactcgacac ggcaccagct caatcagtca cagtgtaaaa 29040
aagggccaag tgcagagcga gtatatatag gactaaaaaa tgacgtaacg gttaaagtcc 29100
acaaaaaaca cccagaaaac cgcacgcgaa cctacgccca gaaacgaaag ccaaaaaacc 29160
cacaacttcc tcaaatcgtc acttccgttt tcccacgtta cgtcacttcc catttaaga 29220
aaactacaat tcccaacaca tacaagttac tccgccctaa aacctacgtc acccgccccg 29280
ttcccacgcc ccgcgccacg tcacaaactc cacccctca ttatcatatt ggcttcaatc 29340
caaaataagg tatattattg atgatgttac atcgttaatt aacgatttcg aacccggggt 29400
accgaattcc tcgagtctag aggagcatgc gacgtcgcaa ttcgccctat agtgagtcgt 29460
attacaattc actggccgtc gttttacaac gtcgtgactg ggaaaaccct ggcgttaccc 29520
aacttaatcg ccttgcagca catcccctt tcgccagctg gcgtaatagc gaagaggccc 29580
gcaccgatcg cccttcccaa cagttgcgca gcctgaatgg cgaatggaaa ttgtaagcgt 29640
taatattttg ttaaaattcg cgttaaattt ttgttaaatc agctcatttt ttaaccaata 29700
ggccgaaatc ggcaaaatcc cttataaatc aaaagaatag accgagatag ggttgagtgt 29760
tgttccagtt tggaacaaga gtccactatt aaagaacgtg gactccaacg tcaaagggcg 29820
aaaaaccgtc tatcagggcg atggcccact acgtgaacca tcaccctaat caagtttttt 29880
ggggtcgagg tgccgtaaag cactaaatcg gaaccctaaa gggagccccc gatttagagc 29940
ttgacgggga aagccggcga acgtggcgag aaaggaaggg aagaaagcga aaggagcggg 30000
cgctagggcg ctggcaagtg tagcggtcac gctgcgcgta accaccacac ccgccgcgct 30060
taatgcgccg ctacagggcg cgtcctgatg cggtattttc tccttacgca tctgtgcggt 30120
atttcacacc gcatacaggt ggcacttttc ggggaaatgt gcgcggaacc cctatttgtt 30180
tatttttcta aatacattca aatatgtatc cgctcatgag acaataaccc tgataaatgc 30240
ttcaataata ttgaaaaagg aagagtatga gtattcaaca tttccgtgtc gcccttattc 30300
ccttttttgc ggcattttgc cttcctgttt ttgctcaccc agaaacgctg gtgaaagtaa 30360
aagatgctga agatcagttg ggtgcacgag tgggttacat cgaactggat ctcaacagcg 30420
gtaagatcct tgagagtttt cgccccgaag aacgttttcc aatgatgagc acttttaaag 30480
ttctgctatg tggcgcggta ttatcccgta ttgacgccgg gcaagagcaa ctcggtcgcc 30540
gcatacacta ttctcagaat gacttggttg agtactcacc agtcacagaa aagcatctta 30600
cggatggcat gacagtaaga gaattatgca gtgctgccat aaccatgagt gataacactg 30660
cggccaactt acttctgaca acgatcggag gaccgaagga gctaaccgct tttttgcaca 30720
acatggggga tcatgtaact cgccttgatc gttgggaacc ggagctgaat gaagccatac 30780
caaacgacga gcgtgacacc acgatgcctg tagcaatggc aacaacgttg cgcaaactat 30840

taactggcga actacttact ctagcttccc ggcaacaatt aatagactgg atggaggcgg 30900
ataaagttgc aggaccactt ctgcgctcgg cccttccggc tggctggttt attgctgata 30960
aatctggagc cggtgagcgt gggtctcgcg gtatcattgc agcactgggg ccagatggta 31020
agccctcccg tatcgtagtt atctacacga cggggagtca ggcaactatg gatgaacgaa 31080
atagacagat cgctgagata ggtgcctcac tgattaagca ttggtaactg tcagaccaag 31140
tttactcata tatactttag attgatttaa aacttcattt ttaatttaaa aggatctagg 31200
tgaagatcct ttttgataat ctcatgacca aaatccctta acgtgagttt tcgttccact 31260
gagcgtcaga ccccgtagaa aagatcaaag gatcttcttg agatcctttt tttctgcgcg 31320
taatctgctg cttgcaaaca aaaaaaccac cgctaccagc ggtggtttgt ttgccggatc 31380
aagagctacc aactcttttt ccgaaggtaa ctggcttcag cagagcgcag ataccaaata 31440
ctgttcttct agtgtagccg tagttaggcc accacttcaa gaactctgta gcaccgccta 31500
catacctcgc tctgctaatc ctgttaccag tggctgctgc cagtggcgat aagtcgtgtc 31560
ttaccgggtt ggactcaaga cgatagttac cggataaggc gcagcggtcg ggctgaacgg 31620
ggggttcgtg cacacagccc agcttggagc gaacgaccta caccgaactg agatacctac 31680
agcgtgagct atgagaaagc gccacgcttc ccgaagggag aaaggcggac aggtatccgg 31740
taagcggcag ggtcggaaca ggagagcgca cgagggagct tccaggggga aacgcctggt 31800
atctttatag tcctgtcggg tttcgccacc tctgacttga gcgtcgattt ttgtgatgct 31860
cgtcaggggg gcggagccta tggaaaaacg ccagcaacgc ggccttttta cggttcctgg 31920
ccttttgctg gccttttgct cacatgttct ttcctgcgtt atcccctgat tctgtggata 31980
accgtattac cgcctttgag tgagctgata ccgctcgccg cagccgaacg accgagcgca 32040
gcgagtcagt gagcgaggaa gcggaagagc gcccaatacg caaaccgcct ctccccgcgc 32100
gttggccgat tcattaatgc agctggcacg acaggtttcc cgactggaaa gcgggcagtg 32160
agcgcaacgc aattaatgtg agttagctca ctcattaggc accccaggct ttacacttta 32220
tgcttccggc tcgtatgttg tgtggaattg tgagcggata acaatttcac acaggaaaca 32280
gctatgacca tgattacgcc aagctattta ggtgacacta tagaatactc aagctagtta 32340
attaacgtta attaacatca tcaataatat accttatttt ggattgaagc caatatgata 32400
atgagggggt ggagtttgtg acgtggcgcg gggcgtggga acggggcggg tgacgtagta 32460
gtgtggcgga agtgtgatgt tgcaagtgtg gcggaacaca tgtaagcgac ggatgtggca 32520
aaagtgacgt ttttggtgtg cgccggtgta cacaggaagt gacaattttc gcgcggtttt 32580
aggcggatgt tgtagtaaat ttgggcgtaa ccgagtaaga tttggccatt ttcgcgggaa 32640
aactgaataa gaggaagtga aatctgaata attttgtgtt actcatagcg cgtaatctct 32700
agcat 32705

<210> 13
<211> 736
<212> DNA
<213> Artificial Sequence

<220>
<223> Description of Artificial Sequence: Synthetic Primer

<400> 13

atgaaccagc ttgggggct ctttgtgaat ggccggcccc tgcctctgga tacccggcag 60
cagattgtgc ggctagcagt cagtggaatg cggccctgtg acatctcacg gatccttaag 120
gtatctaatg gctgtgtgag caagatccta gggcgttact accgcacagg tgtcttggag 180
ccaaagggca ttgggggaag caagccacgg ctggctacac cccctgtggt ggctcgaatt 240
gcccagctga agggtgagtg tccagccctc tttgcctggg aaatccaacg ccagctttgt 300
gctgaagggc tttgcaccca ggacaagact cccagtgtct cctccatcaa ccgagtcctg 360
cgggcattac aggaggacca gggactaccg tgcacacggc tcaggtcacc agctgttttg 420
gctccagctg tcctcactcc ccatagtggc tctgagactc cccggggtac ccacccaggg 480
accggccacc ggaatcggac tatcttctcc ccaagccaag cagaggcact ggagaaagag 540
ttccagcgtg ggcagtatcc tgattcagtg gcccgtggaa agctggctac tgccacctct 600
ctgcctgagg acacggtgag ggtctggttt tccaacagaa gagccaaatg gcgtcggcaa 660
gagaagctca agtgggaaat gcagctgcca ggtgcttccc aggggctgac tgtaccaagg 720
gttgccccag gaatca 736

<210> 14
<211> 4714
<212> DNA
<213> Artificial Sequence

<220>
<223> Description of Artificial Sequence: Synthetic Primer

<400> 14

tcccttccag ctctctgccc cttttggatt gaagccaata tgataatgag gggtggagt 60
ttgtgacgtg gcgcggggcg tgggaacggg gcgggtgacg tagtagtgtg gcggaagtgt 120
gatgttgcaa gtgtggcgga acacatgtaa gcgacggatg tggcaaaagt gacgtttttg 180
gtgtgcgccg gtgtacacag gaagtgacaa ttttcgcgcg gttttaggcg gatgttgtag 240
taaatttggg cgtaaccgag taagatttgg ccattttcgc gggaaaactg aataagagga 300
agtgaaatct gaataatttt gtgttactca tagcgcgtaa tctctagcat cgatgtcgag 360
gatccctcga gtctagagat atcgaattca agcttgtcga ctcgaagatc tgggcgtggt 420
taagggtggg aaagaatata taaggtgggg gtcttatgta gttttgtatc tgttttgcag 480
cagccgccgc cgccatgagc accaactcgt ttgatggaag cattgtgagc tcatatttga 540
caacgcgcat gcccccatgg gccggggtgc gtcagaatgt gatgggctcc agcattgatg 600
gtcgccccgt cctgcccgca aactctacta ccttgaccta cgagaccgtg tctggaacgc 660
cgttggagac tgcagcctcc gccgccgctt cagccgctgc agccaccgcc cgcgggattg 720
tgactgactt tgctttcctg agcccgcttg caagcagtgc agcttcccgt tcatccgccc 780
gcgatgacaa gttgacggct cttttggcac aattggattc tttgacccgg gaacttaatg 840
tcgtttctca gcagctgttg gatctgcgcc agcaggtttc tgccctgaag gcttcctccc 900
ctcccaatgc ggtttaaaac ataaataaaa aaccagactc tgtttggatt tggatcaagc 960
aagtgtcttg ctgtctttat ttaggggttt tgcgcgcgcg gtaggcccgg gaccagcggt 1020

ctcggtcgtt gagggtcctg tgtatttttt ccaggacgtg gtaaaggtga ctctggatgt 1080
tcagatacat gggcataagc ccgtctctgg ggtggaggta gcaccactgc agagcttcat 1140
gctgcggggt ggtgttgtag atgatccagt cgtagcagga gcgctgggcg tggtgcctaa 1200
aaatgtcttt cagtagcaag ctgattgcca ggggcaggcc cttggtgtaa gtgtttacaa 1260
agcggttaag ctgggatggg tgcatacgtg gggatatgag atgcatcttg gactgtattt 1320
ttaggttggc tatgttccca gccatatccc tccggggatt catgttgtgc agaaccacca 1380
gcacagtgta tccggtgcac ttgggaaatt tgtcatgtag cttagaagga aatgcgtgga 1440
agaacttgga gacgcccttg tgacctccaa gattttccat gcattcgtcc ataatgatgg 1500
caatgggccc acgggcggcg gcctgggcga agatatttct gggatcacta acgtcatagt 1560
tgtgttccag gatgagatcg tcataggcca tttttacaaa gcgcgggcgg agggtgccag 1620
actgcggtat aatggttcca tccggcccag gggcgtagtt accctcacag atttgcattt 1680
cccacgcttt gagttcagat gggggggatca tgtctacctg cggggcgatg aagaaaacgg 1740
tttccggggt aggggagatc agctgggaag aaagcaggtt cctgagcagc tgcgacttac 1800
cgcagccggt gggcccgtaa atcacaccta ttaccgggtg caactggtag ttaagagagc 1860
tgcagctgcc gtcatccctg agcagggggg ccacttcgtt aagcatgtcc ctgactcgca 1920
tgttttccct gaccaaatcc gccagaaggc gctcgccgcc cagcgatagc agttcttgca 1980
aggaagcaaa gtttttcaac ggtttgagac cgtccgccgt aggcatgctt ttgagcgttt 2040
gaccaagcag ttccaggcgg tcccacagct cggtcacctg ctctacggca tctcgatcca 2100
gcatatctcc tcgtttcgcg ggttggggcg gctttcgctg tacggcagta gtcggtgctc 2160
gtccagacgg gccagggtca tgtctttcca cgggcgcagg gtcctcgtca gcgtagtctg 2220
ggtcacggtg aaggggtgcg ctccgggctg cgcgctggcc agggtgcgct tgaggctggt 2280
cctgctggtg ctgaagcgct gccggtcttc gccctgcgcg tcggccaggt agcatttgac 2340
catggtgtca tagtccagcc cctccgcggc gtggcccttg gcgcgcagct tgcccttgga 2400
ggaggcgccg cacgaggggc agtgcagact tttgagggcg tagagcttgg gcgcgagaaa 2460
taccgattcc ggggagtagg catccgcgcc gcaggccccg cagacggtct cgcattccac 2520
gagccaggtg agctctggcc gttcggggtc aaaaaccagg tttcccccat gctttttgat 2580
gcgtttctta cctctggttt ccatgagccg gtgtccacgc tcggtgacga aaaggctgtc 2640
cgtgtccccg tatacagact tgagaggcct ctcgatcgag gcggccgcag atctcgcttc 2700
ctcgctcact gactcgctgc gctcggtcgt tcggctgcgg cgagcggtat cagctcactc 2760
aaaggcggta atacggttat ccacagaatc aggggataac gcaggaaaga acatgtgagc 2820
aaaaggccag caaaaggcca ggaaccgtaa aaaggccgcg ttgctggcgt ttttccatag 2880
gctccgcccc cctgacgagc atcacaaaaa tcgacgctca agtcagaggt ggcgaaaccc 2940
gacaggacta taaagatacc aggcgtttcc ccctggaagc tccctcgtgc gctctcctgt 3000
tccgaccctg ccgcttaccg gatacctgtc cgcctttctc ccttcgggaa gcgtggcgct 3060
ttctcaatgc tcacgctgta ggtatctcag ttcggtgtag gtcgttcgct ccaagctggg 3120
ctgtgtgcac gaaccccccg ttcagcccga ccgctgcgcc ttatccggta actatcgtct 3180
tgagtccaac ccggtaagac acgacttatc gccactggca gcagccactg gtaacaggat 3240
tagcagagcg aggtatgtag gcggtgctac agagttcttg aagtggtggc ctaactacgg 3300
ctacactaga aggacagtat ttggtatctg cgctctgctg aagccagtta ccttcggaaa 3360
aagagttggt agctcttgat ccggcaaaca aaccaccgct ggtagcggtg gtttttttgt 3420
ttgcaagcag cagattacgc gcagaaaaaa aggatctcaa gaagatcctt tgatcttttc 3480
tacggggtct gacgctcagt ggaacgaaaa ctcacgttaa gggattttgg tcatgagatt 3540
atcaaaaagg atcttcacct agatcctttt aaattaaaaa tgaagtttta aatcaatcta 3600

aagtatatat gagtaaactt ggtctgacag ttaccaatgc ttaatcagtg aggcacctat 3660
ctcagcgatc tgtctatttc gttcatccat agttgcctga ctccccgtcg tgtagataac 3720
tacgatacgg gagggcttac catctggccc cagtgctgca atgataccgc gagacccacg 3780
ctcaccggct ccagatttat cagcaataaa ccagccagcc ggaagggccg agcgcagaag 3840
tggtcctgca actttatccg cctccatcca gtctattaat tgttgccggg aagctagagt 3900
aagtagttcg ccagttaata gtttgcgcaa cgttgttgcc attgctacag gcatcgtggt 3960
gtcacgctcg tcgtttggta tggcttcatt cagctccggt tcccaacgat caaggcgagt 4020
tacatgatcc cccatgttgt gcaaaaaagc ggttagctcc ttcggtcctc cgatcgttgt 4080
cagaagtaag ttggccgcag tgttatcact catggttatg gcagcactgc ataattctct 4140
tactgtcatg ccatccgtaa gatgcttttc tgtgactggt gagtactcaa ccaagtcatt 4200
ctgagaatag tgtatgcggc gaccgagttg ctcttgcccg gcgtcaatac gggataatac 4260
cgcgccacat agcagaactt taaaagtgct catcattgga aaacgttctt cggggcgaaa 4320
actctcaagg atcttaccgc tgttgagatc cagttcgatg taacccactc gtgcacccaa 4380
ctgatcttca gcatctttta ctttcaccag cgtttctggg tgagcaaaaa caggaaggca 4440
aaatgccgca aaaaagggaa taagggcgac acggaaatgt tgaatactca tactcttcct 4500
ttttcaatat tattgaagca tttatcaggg ttattgtctc atgagcggat acatatttga 4560
atgtatttag aaaaataaac aaataggggt tccgcgcaca tttccccgaa aagtgccacc 4620
tgacgtctaa gaaaccatta ttatcatgac attaacctat aaaaataggc gtatcacgag 4680
gccctttcgt ctcgcgggat caattcttaa ttaa 4714

<210> 15
<211> 7064
<212> DNA
<213> Artificial Sequence

<220>
<223> Description of Artificial Sequence: Synthetic Primer

<400> 15

tcccttccag ctctctgccc cttttggatt gaagccaata tgataatgag gggtggagt 60
ttgtgacgtg gcgcggggcg tgggaacggg gcgggtgacg tagtagtgtg gcggaagtgt 120
gatgttgcaa gtgtggcgga acacatgtaa gcgacggatg tggcaaaagt gacgtttttg 180
gtgtgcgccg gtgtacacag gaagtgacaa ttttcgcgcg gttttaggcg gatgttgtag 240
taaatttggg cgtaaccgag taagatttgg ccattttcgc gggaaaactg aataagagga 300
agtgaaatct gaataatttt gtgttactca tagcgcgtaa tctctagcat cgatgtcgag 360
gatccctcga gtctagagat ggccgcgatc tatacattga atcaatattg gcaattagcc 420
atattagtca ttggttatat agcataaatc aatattggct attggccatt gcatacgttg 480
tatctatatc ataatatgta catttatatt ggctcatgtc caatatgacc gccatgttga 540
cattgattat tgactagtta ttaatagtaa tcaattacgg ggtcattagt tcatagccca 600
tatatggagt tccgcgttac ataacttacg gtaaatggcc cgcctcgtga ccgcccaacg 660
acccccgccc attgacgtca ataatgacgt atgttcccat agtaacgcca atagggactt 720

tccattgacg tcaatgggtg gagtatttac ggtaaactgc ccacttggca gtacatcaag 780
tgtatcatat gccaagtccg ccccctattg acgtcaatga cggtaaatgg cccgcctggc 840
attatgccca gtacatgacc ttacgggact ttcctacttg gcagtacatc tacgtattag 900
tcatcgctat taccatggtg atgcggtttt ggcagtacac caatgggcgt ggatagcggt 960
ttgactcacg gggatttcca agtctccacc ccattgacgt caatgggagt ttgttttggc 1020
accaaaatca acgggacttt ccaaaatgtc gtaataaccc cgccccgttg acgcaaatgg 1080
gcggtaggcg tgtacggtgg gaggtctata taagcagagc tcgtttagtg aaccgtcaga 1140
tccggtcgcg cgaattcgag ctcggtaccc ggggatcccc cgatcgcgac tgtgaggagt 1200
accagtgtga agcatgcagc aggacggact cagcagtgtg aatcagctag gggactctt 1260
tgtgaatggc cggccccttc ctctggacac caggcagcag attgtgcagc tagcaataag 1320
agggatgcga ccctgtgaca tttcacggag ccttaaggta tctaatggct gtgtgagcaa 1380
gatcctagga cgctactacc gcacaggtgt cttggaaccc aagtgtattg ggggaagcaa 1440
accacgtctg gccacacctg ctgtggtggc tcgaattgcc cagctaaagg atgagtaccc 1500
tgctcttttt gcctgggaga tccaacacca gctttgcact gaagggcttt gtacccagga 1560
caaggctccc agtgtgtcct ctatcaatcg agtacttcgg gcacttcagg aagaccagag 1620
cttgcactgg actcaactca gatcaccagc tgtgttggct ccagttcttc ccagtcccca 1680
cagtaactgt ggggctcccc gaggccccca cccaggaacc agccacagga atcgggctat 1740
cttctccccg ggacaagccg aggcactgga gaaagagttt cagcgtgggc agtatccaga 1800
ttcagtggcc cgtgggaagc tggctgctgc cacctctctg cctgaagaca cggtgagggt 1860
ttggttttct aacagaagag ccaaatggcg caggcaagag aagctgaaat gggaagcaca 1920
gctgccaggt gcttcccagg acctgacagt accaaaaaat tctccaggga tcatctctgc 1980
acagcagtcc cccggcagtg taccctcagc tgccttgcct gtgctggaac cattgagtcc 2040
ttccttctgt cagctatgct gtgggacagc accaggcaga tgttccagtg acacctcatc 2100
ccaggcctat ctccaaccct actgggactg ccaatccctc cttcctgtgg cttcctcctc 2160
atatgtggaa tttgcctggc cctgcctcac cacccatcct gtgcatcatc tgattggagg 2220
cccaggacaa gtgccatcaa cccattgctc aaactggcca taaccgcgga attcgatatc 2280
aagcttggga tctttgtgaa ggaaccttac ttctgtggtg tgacataatt ggacaaacta 2340
cctacagaga tttaaagctc taaggtaaat ataaaatttt taagtgtata atgtgttaaa 2400
ctactgattc taattgtttg tgtattttag attcacagtc ccaaggctca tttcaggccc 2460
ctcagtcctc acagtctgtt catgatcata atcagccata ccacatttgt agaggtttta 2520
cttgctttaa aaaacctccc acacctcccc ctgaacctga aacataaaat gaatgcaatt 2580
gttgttgtta acttgtttat tgcagcttat aatggttaca aataaagcaa tagcatcaca 2640
aatttcacaa ataaagcatt tttttcactg cattctagtt gtggtttgtc caaactcatc 2700
aatgtatctt atcatgtctg gatcgcggcc atcgaattca agcttgtcga ctcgaagatc 2760
tgggcgtggt taagggtggg aaagaatata taaggtgggg gtcttatgta gttttgtatc 2820
tgttttgcag cagccgccgc cgccatgagc accaactcgt ttgatggaag cattgtgagc 2880
tcatatttga caacgcgcat gcccccatgg gccggggtgc gtcagaatgt gatgggctcc 2940
agcattgatg gtcgccccgt cctgcccgca aactctacta ccttgaccta cgagaccgtg 3000
tctggaacgc cgttggagac tgcagcctcc gccgccgctt cagccgctgc agccaccgcc 3060
cgcgggattg tgactgactt tgctttcctg agcccgcttg caagcagtgc agcttcccgt 3120
tcatccgccc gcgatgacaa gttgacggct cttttggcac aattggattc tttgacccgg 3180
gaacttaatg tcgtttctca gcagctgttg gatctgcgcc agcaggtttc tgccctgaag 3240
gcttcctccc ctcccaatgc ggtttaaaac ataaataaaa aaccagactc tgtttggatt 3300

tggatcaagc aagtgtcttg ctgtctttat ttaggggttt tgcgcgcgcg gtaggcccgg 3360
gaccagcggt ctcggtcgtt gagggtcctg tgtatttttt ccaggacgtg gtaaaggtga 3420
ctctggatgt tcagatacat gggcataagc ccgtctctgg ggtggaggta gcaccactgc 3480
agagcttcat gctgcggggt ggtgttgtag atgatccagt cgtagcagga gcgctgggcg 3540
tggtgcctaa aaatgtcttt cagtagcaag ctgattgcca ggggcaggcc cttggtgtaa 3600
gtgtttacaa agcggttaag ctgggatggg tgcatacgtg gggatatgag atgcatcttg 3660
gactgtattt ttaggttggc tatgttccca gccatatccc tccggggatt catgttgtgc 3720
agaaccacca gcacagtgta tccggtgcac ttgggaaatt tgtcatgtag cttagaagga 3780
aatgcgtgga agaacttgga gacgcccttg tgacctccaa gattttccat gcattcgtcc 3840
ataatgatgg caatgggccc acgggcggcg gcctgggcga agatatttct gggatcacta 3900
acgtcatagt tgtgttccag gatgagatcg tcataggcca tttttacaaa gcgcgggcgg 3960
agggtgccag actgcggtat aatggttcca tccggcccag gggcgtagtt accctcacag 4020
atttgcattt cccacgcttt gagttcagat ggggggatca tgtctacctg cggggcgatg 4080
aagaaaacgg tttccggggt aggggagatc agctgggaag aaagcaggtt cctgagcagc 4140
tgcgacttac cgcagccggt gggcccgtaa atcacaccta ttaccgggtg caactggtag 4200
ttaagagagc tgcagctgcc gtcatccctg agcagggggg ccacttcgtt aagcatgtcc 4260
ctgactcgca tgttttccct gaccaaatcc gccagaaggc gctcgccgcc cagcgatagc 4320
agttcttgca aggaagcaaa gtttttcaac ggtttgagac cgtccgccgt aggcatgctt 4380
ttgagcgttt gaccaagcag ttccaggcgg tcccacagct cggtcacctg ctctacggca 4440
tctcgatcca gcatatctcc tcgtttcgcg ggttggggcg gctttcgctg tacggcagta 4500
gtcggtgctc gtccagacgg gccagggtca tgtctttcca cgggcgcagg gtcctcgtca 4560
gcgtagtctg ggtcacggtg aaggggtgcg ctccgggctg cgcgctggcc agggtgcgct 4620
tgaggctggt cctgctggtg ctgaagcgct gccggtcttc gccctgcgcg tcggccaggt 4680
agcatttgac catggtgtca tagtccagcc cctccgcggc gtggcccttg gcgcgcagct 4740
tgcccttgga ggaggcgccg cacgaggggc agtgcagact tttgagggcg tagagcttgg 4800
gcgcgagaaa taccgattcc ggggagtagg catccgcgcc gcaggccccg cagacggtct 4860
cgcattccac gagccaggtg agctctggcc gttcggggtc aaaaaccagg tttcccccat 4920
gctttttgat gcgtttctta cctctggttt ccatgagccg gtgtccacgc tcggtgacga 4980
aaaggctgtc cgtgtccccg tatacagact tgagaggcct ctcgatcgag gcggccgcag 5040
atctcgcttc ctcgctcact gactcgctgc gctcggtcgt tcggctgcgg cgagcggtat 5100
cagctcactc aaaggcggta atacggttat ccacagaatc aggggataac gcaggaaaga 5160
acatgtgagc aaaaggccag caaaaggcca ggaaccgtaa aaaggccgcg ttgctggcgt 5220
ttttccatag gctccgcccc cctgacgagc atcacaaaaa tcgacgctca agtcagaggt 5280
ggcgaaaccc gacaggacta taaagatacc aggcgtttcc ccctggaagc tccctcgtgc 5340
gctctcctgt tccgaccctg ccgcttaccg gatacctgtc cgcctttctc ccttcgggaa 5400
gcgtggcgct ttctcaatgc tcacgctgta ggtatctcag ttcggtgtag gtcgttcgct 5460
ccaagctggg ctgtgtgcac gaaccccccg ttcagcccga ccgctgcgcc ttatccggta 5520
actatcgtct tgagtccaac ccggtaagac acgacttatc gccactggca gcagccactg 5580
gtaacaggat tagcagagcg aggtatgtag gcggtgctac agagttcttg aagtggtggc 5640
ctaactacgg ctacactaga aggacagtat ttggtatctg cgctctgctg aagccagtta 5700
ccttcggaaa aagagttggt agctcttgat ccggcaaaca aaccaccgct ggtagcggtg 5760
gtttttttgt ttgcaagcag cagattacgc gcagaaaaaa aggatctcaa gaagatcctt 5820
tgatcttttc tacggggtct gacgctcagt ggaacgaaaa ctcacgttaa gggattttgg 5880

tcatgagatt atcaaaaagg atcttcacct agatcctttt aaattaaaaa tgaagtttta 5940
aatcaatcta aagtatatat gagtaaactt ggtctgacag ttaccaatgc ttaatcagtg 6000
aggcacctat ctcagcgatc tgtctatttc gttcatccat agttgcctga ctccccgtcg 6060
tgtagataac tacgatacgg gagggcttac catctggccc cagtgctgca atgataccgc 6120
gagacccacg ctcaccggct ccagatttat cagcaataaa ccagccagcc ggaagggccg 6180
agcgcagaag tggtcctgca actttatccg cctccatcca gtctattaat tgttgccggg 6240
aagctagagt aagtagttcg ccagttaata gtttgcgcaa cgttgttgcc attgctacag 6300
gcatcgtggt gtcacgctcg tcgtttggta tggcttcatt cagctccggt tcccaacgat 6360
caaggcgagt tacatgatcc cccatgttgt gcaaaaaagc ggttagctcc ttcggtcctc 6420
cgatcgttgt cagaagtaag ttggccgcag tgttatcact catggttatg gcagcactgc 6480
ataattctct tactgtcatg ccatccgtaa gatgcttttc tgtgactggt gagtactcaa 6540
ccaagtcatt ctgagaatag tgtatgcggc gaccgagttg ctcttgcccg gcgtcaatac 6600
gggataatac cgcgccacat agcagaactt taaaagtgct catcattgga aaacgttctt 6660
cggggcgaaa actctcaagg atcttaccgc tgttgagatc cagttcgatg taacccactc 6720
gtgcacccaa ctgatcttca gcatctttta ctttcaccag cgtttctggg tgagcaaaaa 6780
caggaaggca aaatgccgca aaaaagggaa taagggcgac acggaaatgt tgaatactca 6840
tactcttcct ttttcaatat tattgaagca tttatcaggg ttattgtctc atgagcggat 6900
acatatttga atgtatttag aaaaataaac aaataggggt tccgcgcaca tttccccgaa 6960
aagtgccacc tgacgtctaa gaaaccatta ttatcatgac attaacctat aaaaataggc 7020
gtatcacgag gccctttcgt ctcgcgggat caattcttaa ttaa 7064

<210> 16
<211> 35474
<212> DNA
<213> Artificial Sequence

<220>
<223> Description of Artificial Sequence: Synthetic Primer

<400> 16

cgtaactata acggtcctaa ggtagcgaaa gctcagatct ggatctcccg atcccctatg 60
gtcgactctc agtacaatct gctctgatgc cgcatagtta agccagtatc tgctccctgc 120
ttgtgtgttg gaggtcgctg agtagtgcgc gagcaaaatt taagctacaa caaggcaagg 180
cttgaccgac aattgcatga agaatctgct tagggttagg cgttttgcgc tgcttcgcga 240
tgtacgggcc agatatacgc gtcgagttta ccactcccta tcagtgatag agaaaagtga 300
aagtcgagtt taccactccc tatcagtgat agagaaaagt gaaagtcgag tttaccactc 360
cctatcagtg atagagaaaa gtgaaagtcg agtttaccac tccctatcag tgatagagaa 420
aagtgaaagt cgagtttacc actccctatc agtgatagag aaaagtgaaa gtcgagttta 480
ccactcccta tcagtgatag agaaaagtga aagtcgagtt taccactccc tatcagtgat 540
agagaaaagt gaaagtcgag ctcggtaccc gggtcgagta ggcgtgtacg gtgggaggcc 600
tatataagca gagctcgttt agtgaaccgt cagatcgcct ggagacgcca tccacgctgt 660

tttgacctcc atagaagaca ccgggaccga tccagcctcc gcggccccga attcgagctc 720
ggtaccoggg gatcctctag tcagctgacg cgtgctagcg cggccgcatc gataagcttg 780
tcgacgatgc ttggtaccga gctcggatcc actagtccag tgtggtggaa ttcgatttga 840
agatgaacca gcttgggggg ctctttgtga atggccggcc cctgcctctg gatacccggc 900
agcagattgt gcggctagca gtcagtggaa tgcggccctg tgacatctca cggatcctta 960
aggtatctaa tggctgtgtg agcaagatcc tagggcgtta ctaccgcaca ggtgtcttgg 1020
agccaaaggg cattggggga agcaagccac ggctggctac accoctgtg gtggctcgaa 1080
ttgcccagct gaagggtgag tgtccagccc tctttgcctg ggaaatccaa cgccagcttt 1140
gtgctgaagg gctttgcacc caggacaaga ctcccagtgt ctcctccatc aaccgagtcc 1200
tgcgggcatt acaggaggac cagggactac cgtgcacacg gctcaggtca ccagctgttt 1260
tggctccagc tgtcctcact ccccatagtg gctctgagac tccccggggt acccacccag 1320
ggaccggcca ccggaatcgg actatcttct ccccaagcca ggcagaggca ctggagaaag 1380
agttccagcg tgggcagtat cctgattcag tggcccgtgg aaagctggct actgccacct 1440
ctctgcctga ggacacggtg agggtctggt tttccaacag aagagccaaa tggcgtcggc 1500
aagagaagct caagtgggaa atgcagctgc caggtgcttc ccaggggctg actgtaccaa 1560
gggttgcccc aggaatcatc tctgcacagc agtcccctgg cagtgtgccc acagcagccc 1620
tgcctgccct ggaaccactg ggtccctcct gctatcagct gtgctgggca acagcaccag 1680
aaaggtgtct gagtgacacc ccacctaaag cctgtctcaa gccctgctgg ggccacttgc 1740
ccccacagcc gaattccctg gactcaggac tgctttgcct tccttgccct tcctcccact 1800
gtcccctggc cagtcttagt ggctctcagg ccctgctctg gcctggctgc ccactactgt 1860
atggcttgga aatcactagt gaattctgca gatatccagc acagtggcgg ccgctcgagt 1920
ctagagggcc cttcgaacaa aaactcatct cagaagagga tctgaatatg cataccggtc 1980
atcatcacca tcaccattga gttatctcta gaggatcata atcagccata ccacatttgt 2040
agaggtttta cttgctttaa aaaacctccc acacctcccc ctgaacctga aacataaaat 2100
gaatgcaatt gttgttgtta acttgtttat tgcagcttat aatggttaca aataaagcaa 2160
tagcatcaca aatttcacaa ataaagcatt tttttcactg cattctagtt gtggtttgtc 2220
caaactcatc aatgtatctt atcatgtctg gatccccagg aagctcctct gtgtcctcat 2280
aaaccctaac ctcctctact tgagaggaca ttccaatcat aggctgccca tccaccctct 2340
gtgtcctcct gttaattagg tcacttaaca aaaaggaaat tgggtagggg tttttcacag 2400
accgctttct aagggtaatt ttaaaatatc tgggaagtcc cttccactgc tgtgttccag 2460
aagtgttggt aaacagccca caaatgtcaa cagcagaaac atacaagctg tcagctttgc 2520
acaagggccc aacaccctgc tcatcaagaa gcactgtggt tgctgtgtta gtaatgtgca 2580
aaacaggagg cacattttcc ccacctgtgt aggttccaaa atatctagtg ttttcatttt 2640
tacttggatc aggaacccag cactccactg gataagcatt atccttatcc aaaacagcct 2700
tgtggtcagt gttcatctgc tgactgtcaa ctgtagcatt ttttggggtt acagtttgag 2760
caggatattt ggtcctgtag tttgctaaca caccctgcag atctgaattc atctatgtcg 2820
ggtgcggaga aagaggtaat gaaatggcat cgactcgaag atctgggcgt ggttaagggt 2880
gggaaagaat atataaggtg ggggtcttat gtagttttgt atctgttttg cagcagccgc 2940
cgccgccatg agcaccaact cgtttgatgg aagcattgtg agctcatatt tgacaacgcg 3000
catgccccca tgggccgggg tgcgtcagaa tgtgatgggc tccagcattg atggtcgccc 3060
cgtcctgccc gcaaactcta ctaccttgac ctacgagacc gtgtctggaa cgccgttgga 3120
gactgcagcc tccgccgccg cttcagccgc tgcagccacc gcccgcggga ttgtgactga 3180
ctttgctttc ctgagcccgc ttgcaagcag tgcagcttcc cgttcatccg cccgcgatga 3240

caagttgacg gctcttttgg cacaattgga ttctttgacc cgggaactta atgtcgtttc 3300
tcagcagctg ttggatctgc gccagcaggt ttctgccctg aaggcttcct cccctcccaa 3360
tgcggtttaa aacataaata aaaaaccaga ctctgtttgg atttggatca agcaagtgtc 3420
ttgctgtctt tatttagggg ttttgcgcgc gcggtaggcc cgggaccagc ggtctcggtc 3480
gttgagggtc ctgtgtattt tttccaggac gtggtaaagg tgactctgga tgttcagata 3540
catgggcata agcccgtctc tggggtggag gtagcaccac tgcagagctt catgctgcgg 3600
ggtggtgttg tagatgatcc agtcgtagca ggagcgctgg gcgtggtgcc taaaaatgtc 3660
tttcagtagc aagctgattg ccaggggcag gcccttggtg taagtgttta caaagcggtt 3720
aagctgggat gggtgcatac gtggggatat gagatgcatc ttggactgta tttttaggtt 3780
ggctatgttc ccagccatat ccctccgggg attcatgttg tgcagaacca ccagcacagt 3840
gtatccggtg cacttgggaa atttgtcatg tagcttagaa ggaaatgcgt ggaagaactt 3900
ggagacgccc ttgtgacctc caagattttc catgcattcg tccataatga tggcaatggg 3960
cccacgggcg gcggcctggg cgaagatatt tctgggatca ctaacgtcat agttgtgttc 4020
caggatgaga tcgtcatagg ccatttttac aaagcgcggg cggagggtgc cagactgcgg 4080
tataatggtt ccatccggcc caggggcgta gttaccctca cagatttgca tttcccacgc 4140
tttgagttca gatgggggga tcatgtctac ctgcggggcg atgaagaaaa cggtttccgg 4200
ggtaggggag atcagctggg aagaaagcag gttcctgagc agctgcgact taccgcagcc 4260
ggtgggcccg taaatcacac ctattaccgg ctgcaactgg tagttaagag agctgcagct 4320
gccgtcatcc ctgagcaggg gggccacttc gttaagcatg tccctgactc gcatgttttc 4380
cctgaccaaa tccgccagaa ggcgctcgcc gcccagcgat agcagttctt gcaaggaagc 4440
aaagtttttc aacggtttga gaccgtccgc cgtaggcatg cttttgagcg tttgaccaag 4500
cagttccagg cggtcccaca gctcggtcac ctgctctacg gcatctcgat ccagcatatc 4560
tcctcgtttc gcgggttggg gcggctttcg ctgtacggca gtagtcggtg ctcgtccaga 4620
cgggccaggg tcatgtcttt ccacgggcgc agggtcctcg tcagcgtagt ctgggtcacg 4680
gtgaaggggt gcgctccggg ctgcgcgctg gccagggtgc gcttgaggct ggtcctgctg 4740
gtgctgaagc gctgccggtc ttcgccctgc gcgtcggcca ggtagcattt gaccatggtg 4800
tcatagtcca gcccctccgc ggcgtggccc ttggcgcgca gcttgccctt ggaggaggcg 4860
ccgcacgagg ggcagtgcag acttttgagg gcgtagagct tgggcgcgag aaataccgat 4920
tccggggagt aggcatccgc gccgcaggcc ccgcagacgg tctcgcattc cacgagccag 4980
gtgagctctg gccgttcggg gtcaaaaacc aggtttcccc catgcttttt gatgcgtttc 5040
ttacctctgg tttccatgag ccggtgtcca cgctcggtga cgaaaaggct gtccgtgtcc 5100
ccgtatacag acttgagagg cctgtcctcg agcggtgttc cgcggtcctc ctcgtataga 5160
aactcggacc actctgagac aaaggctcgc gtccaggcca gcacgaagga ggctaagtgg 5220
gaggggtagc ggtcgttgtc cactaggggg tccactcgct ccagggtgtg aagacacatg 5280
tcgccctctt cggcatcaag gaaggtgatt ggtttgtagg tgtaggccac gtgaccgggt 5340
gttcctgaag gggggctata aaagggggtg ggggcgcgtt cgtcctcact ctcttccgca 5400
tcgctgtctg cgagggccag ctgttggggt gagtactccc tctgaaaagc gggcatgact 5460
tctgcgctaa gattgtcagt ttccaaaaac gaggaggatt tgatattcac ctggcccgcg 5520
gtgatgcctt tgagggtggc cgcatccatc tggtcagaaa agacaatctt tttgttgtca 5580
agcttggtgg caaacgaccc gtagagggcg ttggacagca acttggcgat ggagcgcagg 5640
gtttggtttt tgtcgcgatc ggcgcgctcc ttggccgcga tgtttagctg cacgtattcg 5700
cgcgcaacgc accgccattc gggaaagacg gtggtgcgct cgtcgggcac caggtgcacg 5760
cgccaaccgc ggttgtgcag ggtgacaagg tcaacgctgg tggctacctc tccgcgtagg 5820

cgctcgttgg tccagcagag gcggccgccc ttgcgcgagc agaatggcgg taggggtct 5880
agctgcgtct cgtccggggg gtctgcgtcc acggtaaaga ccccgggcag caggcgcgcg 5940
tcgaagtagt ctatcttgca tccttgcaag tctagcgcct gctgccatgc gcgggcggca 6000
agcgcgcgct cgtatgggtt gagtggggga ccccatggca tggggtgggt gagcgcggag 6060
gcgtacatgc cgcaaatgtc gtaaacgtag aggggctctc tgagtattcc aagatatgta 6120
gggtagcatc ttccaccgcg gatgctggcg cgcacgtaat cgtatagttc gtgcgaggga 6180
gcgaggaggt cgggaccgag gttgctacgg gcgggctgct ctgctcggaa gactatctgc 6240
ctgaagatgg catgtgagtt ggatgatatg gttggacgct ggaagacgtt gaagctggcg 6300
tctgtgagac ctaccgcgtc acgcacgaag gaggcgtagg agtcgcgcag cttgttgacc 6360
agctcggcgg tgacctgcac gtctagggcg cagtagtcca gggtttcctt gatgatgtca 6420
tacttatcct gtcccttttt tttccacagc tcgcggttga ggacaaactc ttcgcggtct 6480
ttccagtact cttggatcgg aaacccgtcg gcctccgaac ggtaagagcc tagcatgtag 6540
aactggttga cggcctggta ggcgcagcat cccttttcta cgggtagcgc gtatgcctgc 6600
gcggccttcc ggagcgaggt gtgggtgagc gcaaaggtgt ccctgaccat gactttgagg 6660
tactggtatt tgaagtcagt gtcgtcgcat ccgccctgct cccagagcaa aaagtccgtg 6720
cgctttttgg aacgcggatt tggcagggcg aaggtgacat cgttgaagag tatctttccc 6780
gcgcgaggca taaagttgcg tgtgatgcgg aagggtcccg gcacctcgga acggttgtta 6840
attacctggg cggcgagcac gatctcgtca aagccgttga tgttgtggcc cacaatgtaa 6900
agttccaaga agcgcgggat gcccttgatg gaaggcaatt ttttaagttc ctcgtaggtg 6960
agctcttcag gggagctgag cccgtgctct gaaagggccc agtctgcaag atgagggttg 7020
gaagcgacga atgagctcca caggtcacgg gccattagca tttgcaggtg gtcgcgaaag 7080
gtcctaaact ggcgacctat ggccattttt tctggggtga tgcagtagaa ggtaagcggg 7140
tcttgttccc agcggtccca tccaaggttc gcggctaggt ctcgcgcggc agtcactaga 7200
ggctcatctc cgccgaactt catgaccagc atgaagggca cgagctgctt cccaaaggcc 7260
cccatccaag tataggtctc tacatcgtag gtgacaaaga gacgctcggt gcgaggatgc 7320
gagccgatcg ggaagaactg gatctcccgc caccaattgg aggagtggct attgatgtgg 7380
tgaaagtaga agtccctgcg acgggccgaa cactcgtgct ggcttttgta aaaacgtgcg 7440
cagtactggc agcggtgcac gggctgtaca tcctgcacga ggttgacctg acgaccgcgc 7500
acaaggaagc agagtgggaa tttgagcccc tcgcctggcg ggtttggctg gtggtcttct 7560
acttcggctg cttgtccttg accgtctggc tgctcgaggg gagttacggt ggatcggacc 7620
accacgccgc gcgagcccaa agtccagatg tccgcgcgcg gcggtcggag cttgatgaca 7680
acatcgcgca gatgggagct gtccatggtc tggagctccc gcggcgtcag gtcaggcggg 7740
agctcctgca ggtttacctc gcatagacgg gtcagggcgc gggctagatc caggtgatac 7800
ctaatttcca ggggctggtt ggtggcggcg tcgatggctt gcaagaggcc gcatccccgc 7860
ggcgcgacta cggtaccgcg cggcgggcgg tgggccgcgg gggtgtcctt ggatgatgca 7920
tctaaaagcg gtgacgcggg cgagcccccg gaggtagggg gggctccgga cccgccggga 7980
gagggggcag gggcacgtcg gcgccgcgcg cgggcaggag ctggtgctgc gcgcgtaggt 8040
tgctggcgaa cgcgacgacg cggcggttga tctcctgaat ctggcgcctc tgcgtgaaga 8100
cgacgggccc ggtgagcttg aacctgaaag agagttcgac agaatcaatt tcggtgtcgt 8160
tgacggcggc ctggcgcaaa atctcctgca cgtctcctga gttgtcttga taggcgatct 8220
cggccatgaa ctgctcgatc tcttcctcct ggagatctcc gcgtccggct cgctccacgg 8280
tggcggcgag gtcgttggaa atgcgggcca tgagctgcga gaaggcgttg aggcctccct 8340
cgttccagac gcggctgtag accacgcccc cttcggcatc gcgggcgcgc atgaccacct 8400

gcgcgagatt gagctccacg tgccgggcga agacggcgta gtttcgcagg cgctgaaaga 8460
ggtagttgag ggtggtggcg gtgtgttctg ccacgaagaa gtacataacc cagcgtcgca 8520
acgtggattc gttgatatcc cccaaggcct caaggcgctc catggcctcg tagaagtcca 8580
cggcgaagtt gaaaaactgg gagttgcgcg ccgacacggt taactcctcc tccagaagac 8640
ggatgagctc ggcgacagtg tcgcgcacct cgcgctcaaa ggctacaggg gcctcttctt 8700
cttcttcaat ctcctcttcc ataagggcct ccccttcttc ttcttctggc ggcggtgggg 8760
gaggggggac acggcggcga cgacggcgca ccgggaggcg gtcgacaaag cgctcgatca 8820
tctccccgcg gcgacggcgc atggtctcgg tgacggcgcg gccgttctcg cgggggcgca 8880
gttggaagac gccgcccgtc atgtcccggt tatgggttgg cggggggctg ccatgcggca 8940
gggatacggc gctaacgatg catctcaaca attgttgtgt aggtactccg ccgccgaggg 9000
acctgagcga gtccgcatcg accggatcgg aaaacctctc gagaaaggcg tctaaccagt 9060
cacagtcgca aggtaggctg agcaccgtgg cgggcggcag cgggcggcgg tcggggttgt 9120
ttctggcgga ggtgctgctg atgatgtaat taaagtaggc ggtcttgaga cggcggatgg 9180
tcgacagaag caccatgtcc ttgggtccgg cctgctgaat gcgcaggcgg tcggccatgc 9240
cccaggcttc gttttgacat cggcgcaggt ctttgtagta gtcttgcatg agcctttcta 9300
ccggcacttc ttcttctcct tcctcttgtc ctgcatctct tgcatctatc gctgcggcgg 9360
cggcggagtt tggccgtagg tggcgccctc ttcctcccat gcgtgtgacc ccgaagcccc 9420
tcatcggctg aagcagggct aggtcggcga caacgcgctc ggctaatatg gcctgctgca 9480
cctgcgtgag ggtagactgg aagtcatcca tgtccacaaa gcggtggtat gcgcccgtgt 9540
tgatggtgta agtgcagttg gccataacgg accagttaac ggtctggtga cccggctgcg 9600
agagctcggt gtacctgaga cgcgagtaag ccctcgagtc aaatacgtag tcgttgcaag 9660
tccgcaccag gtactggtat cccaccaaaa agtgcggcgg cggctggcgg tagaggggcc 9720
agcgtagggt ggccggggct ccgggggcga gatcttccaa cataaggcga tgatatccgt 9780
agatgtacct ggacatccag gtgatgccgg cggcggtggt ggaggcgcgc ggaaagtcgc 9840
ggacgcggtt ccagatgttg cgcagcggca aaaagtgctc catggtcggg acgctctggc 9900
cggtcaggcg cgcgcaatcg ttgacgctct agcgtgcaaa aggagagcct gtaagcgggc 9960
actcttccgt ggtctggtgg ataaattcgc aagggtatca tggcggacga ccggggttcg 10020
agccccgtat ccggccgtcc gccgtgatcc atgcggttac cgcccgcgtg tcgaacccag 10080
gtgtgcgacg tcagacaacg ggggagtgct cctttggct tccttccagg cgcggcggct 10140
gctgcgctag ctttttggc cactggccgc gcgcagcgta agcggttagg ctggaaagcg 10200
aaagcattaa gtggctcgct ccctgtagcc ggagggttat ttccaaggg ttgagtcgcg 10260
ggacccccgg ttcgagtctc ggaccggccg gactgcggcg aacgggggtt tgcctccccg 10320
tcatgcaaga ccccgcttgc aaattcctcc ggaaacaggg acgagcccct tttttgcttt 10380
tcccagatgc atccggtgct gcggcagatg cgcccccctc ctcagcagcg gcaagagcaa 10440
gagcagcggc agacatgcag ggcaccctcc cctcctccta ccgcgtcagg aggggcgaca 10500
tccgcggttg acgcggcagc agatggtgat tacgaacccc cgcggcgccg ggcccggcac 10560
tacctggact tggaggaggg cgagggcctg gcgcggctag gagcgccctc tcctgagcgg 10620
cacccaaggg tgcagctgaa gcgtgatacg cgtgaggcgt acgtgccgcg gcagaacctg 10680
tttcgcgacc gcgagggaga ggagcccgag gagatgcggg atcgaaagtt ccacgcaggg 10740
cgcgagctgc ggcatggcct gaatcgcgag cggttgctgc gcgaggagga ctttgagccc 10800
gacgcgcgaa ccgggattag tcccgcgcgc gcacacgtgg cggccgccga cctggtaacc 10860
gcatacgagc agacggtgaa ccaggagatt aactttcaaa aaagctttaa caaccacgtg 10920
cgtacgcttg tggcgcgcga ggaggtggct ataggactga tgcatctgtg ggactttgta 10980

agcgcgctgg agcaaaaccc aaatagcaag ccgctcatgg cgcagctgtt ccttatagtg 11040
cagcacagca gggacaacga ggcattcagg gatgcgctgc taaacatagt agagcccgag 11100
ggccgctggc tgctcgattt gataaacatc ctgcagagca tagtggtgca ggagcgcagc 11160
ttgagcctgg ctgacaaggt ggccgccatc aactattcca tgcttagcct gggcaagttt 11220
tacgcccgca agatatacca taccccttac gttcccatag acaaggaggt aaagatcgag 11280
gggttctaca tgcgcatggc gctgaaggtg cttaccttga gcgacgacct gggcgtttat 11340
cgcaacgagc gcatccacaa ggccgtgagc gtgagccggc ggcgcgagct cagcgaccgc 11400
gagctgatgc acagcctgca aagggccctg gctggcacgg gcagcggcga tagagaggcc 11460
gagtcctact ttgacgcggg cgctgacctg cgctgggccc caagccgacg cgccctggag 11520
gcagctgggg ccggacctgg gctggcggtg gcacccgcgc gcgctggcaa cgtcggcggc 11580
gtggaggaat atgacgagga cgatgagtac gagccagagg acggcgagta ctaagcggtg 11640
atgtttctga tcagatgatg caagacgcaa cggacccggc ggtgcgggcg gcgctgcaga 11700
gccagccgtc cggccttaac tccacggacg actggcgcca ggtcatggac cgcatcatgt 11760
cgctgactgc gcgcaatcct gacgcgttcc ggcagcagcc gcaggccaac cggctctccg 11820
caattctgga agcggtggtc ccggcgcgcg caaaccccac gcacgagaag gtgctggcga 11880
tcgtaaacgc gctggccgaa aacagggcca tccggcccga cgaggccggc ctggtctacg 11940
acgcgctgct tcagcgcgtg gctcgttaca acagcggcaa cgtgcagacc aacctggacc 12000
ggctggtggg ggatgtgcgc gaggccgtgg cgcagcgtga gcgcgcgcag cagcagggca 12060
acctgggctc catggttgca ctaaacgcct tcctgagtac acagcccgcc aacgtgccgc 12120
ggggacagga ggactacacc aactttgtga gcgcactgcg gctaatggtg actgagacac 12180
cgcaaagtga ggtgtaccag tctgggccag actatttttt ccagaccagt agacaaggcc 12240
tgcagaccgt aaacctgagc caggctttca aaaacttgca ggggctgtgg ggggtgcggg 12300
ctcccacagg cgaccgcgcg accgtgtcta gcttgctgac gcccaactcg cgcctgttgc 12360
tgctgctaat agcgcccttc acggacagtg gcagcgtgtc ccgggacaca tacctaggtc 12420
acttgctgac actgtaccgc gaggccatag gtcaggcgca tgtggacgag catactttcc 12480
aggagattac aagtgtcagc cgcgcgctgg ggcaggagga cacgggcagc ctggaggcaa 12540
ccctaaacta cctgctgacc aaccggcggc agaagatccc ctcgttgcac agtttaaaca 12600
gcgaggagga gcgcattttg cgctacgtgc agcagagcgt gagccttaac ctgatgcgcg 12660
acggggtaac gcccagcgtg gcgctggaca tgaccgcgcg caacatggaa ccgggcatgt 12720
atgcctcaaa ccggccgttt atcaaccgcc taatggacta cttgcatcgc gcggccgccg 12780
tgaaccccga gtatttcacc aatgccatct tgaacccgca ctggctaccg cccctggtt 12840
tctacaccgg gggattcgag gtgcccgagg gtaacgatgg attcctctgg gacgacatag 12900
acgacagcgt gttttccccg caaccgcaga ccctgctaga gttgcaacag cgcgagcagg 12960
cagaggcggc gctgcgaaag gaaagcttcc gcaggccaag cagcttgtcc gatctaggcg 13020
ctgcggcccc gcggtcagat gctagtagcc catttccaag cttgataggg tctcttacca 13080
gcactcgcac cacccgcccg cgcctgctgg gcgaggagga gtacctaaac aactcgctgc 13140
tgcagccgca gcgcgaaaaa aacctgcctc cggcatttcc caacaacggg atagagagcc 13200
tagtggacaa gatgagtaga tggaagacgt acgcgcagga gcacagggac gtgccaggcc 13260
cgcgcccgcc cacccgtcgt caaaggcacg accgtcagcg gggtctggtg tgggaggacg 13320
atgactcggc agacgacagc agcgtcctgg atttgggagg gagtggcaac ccgtttgcgc 13380
accttcgccc caggctgggg agaatgtttt aaaaaaaaaa aaagcatgat gcaaaataaa 13440
aaactcacca aggccatggc accgagcgtt ggttttcttg tattcccctt agtatgcggc 13500
gcgcggcgat gtatgaggaa ggtcctcctc cctcctacga gagtgtggtg agcgcggcgc 13560

cagtggcggc ggcgctgggt tctcccttcg atgctcccct ggacccgccg tttgtgcctc 13620
cgcggtacct gcggcctacc gggggggagaa acagcatccg ttactctgag ttggcacccc 13680
tattcgacac cacccgtgtg tacctggtgg acaacaagtc aacggatgtg gcatccctga 13740
actaccagaa cgaccacagc aactttctga ccacggtcat tcaaaacaat gactacagcc 13800
cgggggaggc aagcacacag accatcaatc ttgacgaccg gtcgcactgg ggcggcgacc 13860
tgaaaaccat cctgcatacc aacatgccaa atgtgaacga gttcatgttt accaataagt 13920
ttaaggcgcg ggtgatggtg tcgcgcttgc ctactaagga caatcaggtg gagctgaaat 13980
acgagtgggt ggagttcacg ctgcccgagg gcaactactc cgagaccatg accatagacc 14040
ttatgaacaa cgcgatcgtg gagcactact tgaaagtggg cagacagaac ggggttctgg 14100
aaagcgacat cggggtaaag tttgacaccc gcaacttcag actggggttt gaccccgtca 14160
ctggtcttgt catgcctggg gtatatacaa acgaagcctt ccatccagac atcattttgc 14220
tgccaggatg cggggtggac ttcacccaca gccgcctgag caacttgttg ggcatccgca 14280
agcggcaacc cttccaggag ggctttagga tcacctacga tgatctggag ggtggtaaca 14340
ttcccgcact gttggatgtg gacgcctacc aggcgagctt gaaagatgac accgaacagg 14400
gcgggggtgg cgcaggcggc agcaacagca gtggcagcgg cgcggaagag aactccaacg 14460
cggcagccgc ggcaatgcag ccggtggagg acatgaacga tcatgccatt cgcggcgaca 14520
cctttgccac acgggctgag gagaagcgcg ctgaggccga agcagcggcc gaagctgccg 14580
cccccgctgc gcaacccgag gtcgagaagc ctcagaagaa accggtgatc aaacccctga 14640
cagaggacag caagaaacgc agttacaacc taataagcaa tgacagcacc ttcacccagt 14700
accgcagctg gtaccttgca tacaactacg gcgaccctca gaccggaatc cgctcatgga 14760
ccctgctttg cactcctgac gtaacctgcg gctcggagca ggtctactgg tcgttgccag 14820
acatgatgca agaccccgtg accttccgct ccacgcgcca gatcagcaac tttccggtgg 14880
tgggcgccga gctgttgccc gtgcactcca agagcttcta caacgaccag gccgtctact 14940
cccaactcat ccgccagttt acctctctga cccacgtgtt caatcgcttt cccgagaacc 15000
agattttggc gcgcccgcca gcccccacca tcaccaccgt cagtgaaaac gttcctgctc 15060
tcacagatca cgggacgcta ccgctgcgca acagcatcgg aggagtccag cgagtgacca 15120
ttactgacgc cagacgccgc acctgcccct acgtttacaa ggccctgggc atagtctcgc 15180
cgcgcgtcct atcgagccgc actttttgag caagcatgtc catccttata tcgcccagca 15240
ataacacagg ctggggcctg cgcttcccaa gcaagatgtt tggcggggcc aagaagcgct 15300
ccgaccaaca cccagtgcgc gtgcgcgggc actaccgcgc gccctggggc gcgcacaaac 15360
gcggccgcac tgggcgcacc accgtcgatg acgccatcga cgcggtggtg gaggaggcgc 15420
gcaactacac gcccacgccg ccaccagtgt ccacagtgga cgcggccatt cagaccgtgg 15480
tgcgcggagc ccggcgctat gctaaaatga agagacggcg gaggcgcgta gcacgtcgcc 15540
accgccgccg acccggcact gccgcccaac gcgcggcggc ggccctgctt aaccgcgcac 15600
gtcgcaccgg ccgacgggcg gccatgcggg ccgctcgaag gctggccgcg ggtattgtca 15660
ctgtgccccc caggtccagg cgacgagcgg ccgccgcagc agccgcggcc attagtgcta 15720
tgactcaggg tcgcaggggc aacgtgtatt gggtgcgcga ctcggttagc ggcctgcgcg 15780
tgcccgtgcg cacccgcccc ccgcgcaact agattgcaag aaaaaactac ttagactcgt 15840
actgttgtat gtatccagcg gcggcggcgc gcaacgaagc tatgtccaag cgcaaaatca 15900
aagaagagat gctccaggtc atcgcgccgg agatctatgg ccccccgaag aaggaagagc 15960
aggattacaa gccccgaaag ctaaagcggg tcaaaaagaa aaagaaagat gatgatgatg 16020
aacttgacga cgaggtggaa ctgctgcacg ctaccgcgcc caggcgacgg gtacagtgga 16080
aaggtcgacg cgtaaaacgt gttttgcgac ccggcaccac cgtagtcttt acgcccggtg 16140

agcgctccac ccgcacctac aagcgcgtgt atgatgaggt gtacggcgac gaggacctgc 16200
ttgagcaggc caacgagcgc ctcggggagt ttgcctacgg aaagcggcat aaggacatgc 16260
tggcgttgcc gctggacgag ggcaacccaa cacctagcct aaagcocgta acactgcagc 16320
aggtgctgcc cgcgcttgca ccgtccgaag aaaagcgcgg cctaaagcgc gagtctggtg 16380
acttggcacc caccgtgcag ctgatggtac ccaagcgcca gcgactggaa gatgtcttgg 16440
aaaaaatgac cgtggaacct gggctggagc ccgaggtccg cgtgcggcca atcaagcagg 16500
tggcgccggg actgggcgtg cagaccgtgg acgttcagat acccactacc agtagcacca 16560
gtattgccac cgccacagag ggcatggaga cacaaacgtc cccggttgcc tcagcggtgg 16620
cggatgccgc ggtgcaggcg gtcgctgcgg ccgcgtccaa gacctctacg gaggtgcaaa 16680
cggacccgtg gatgtttcgc gtttcagccc cccggcgccc gcgccgttcg aggaagtacg 16740
gcgccgccag cgcgctactg cccgaatatg ccctacatcc ttccattgcg cctacccccg 16800
gctatcgtgg ctacacctac cgccccagaa gacgagcaac tacccgacgc cgaaccacca 16860
ctggaacccg ccgccgccgt cgccgtcgcc agcccgtgct ggccccgatt tccgtgcgca 16920
gggtggctcg cgaaggaggc aggaccctgg tgctgccaac agcgcgctac caccccagca 16980
tcgtttaaaa gccggtcttt gtggttcttg cagatatggc cctcacctgc cgcctccgtt 17040
tcccggtgcc gggattccga ggaagaatgc accgtaggag gggcatggcc ggccacggcc 17100
tgacgggcgg catgcgtcgt gcgcaccacc ggcggcggcg cgcgtcgcac cgtcgcatgc 17160
gcggcggtat cctgcccctc cttattccac tgatcgccgc ggcgattggc gccgtgcccg 17220
gaattgcatc cgtggccttg caggcgcaga gacactgatt aaaaacaagt tgcatgtgga 17280
aaaatcaaaa taaaaagtct ggactctcac gctcgcttgg tcctgtaact attttgtaga 17340
atggaagaca tcaactttgc gtctctggcc ccgcgacacg gctcgcgccc gttcatggga 17400
aactggcaag atatcggcac cagcaatatg agcggtggcg ccttcagctg ggctcgctg 17460
tggagcggca ttaaaaattt cggttccacc gttaagaact atggcagcaa ggcctggaac 17520
agcagcacag gccagatgct gagggataag ttgaaagagc aaaatttcca acaaaaggtg 17580
gtagatggcc tggcctctgg cattagcggg gtggtggacc tggccaacca ggcagtgcaa 17640
aataagatta acagtaagct tgatccccgc cctcccgtag aggagcctcc accggccgtg 17700
gagacagtgt ctccagaggg gcgtggcgaa aagcgtccgc gccccgacag ggaagaaact 17760
ctggtgacgc aaatagacga gcctccctcg tacgaggagg cactaaagca aggcctgccc 17820
accacccgtc ccatcgcgcc catggctacc ggagtgctgg gccagcacac acccgtaacg 17880
ctggacctgc ctccccccgc cgacacccag cagaaacctg tgctgccagg cccgaccgcc 17940
gttgttgtaa cccgtcctag ccgcgcgtcc ctgcgccgcg ccgccagcgg tccgcgatcg 18000
ttgcggcccg tagccagtgg caactggcaa agcacactga acagcatcgt gggtctgggg 18060
gtgcaatccc tgaagcgccg acgatgcttc tgatagctaa cgtgtcgtat gtgtgtcatg 18120
tatgcgtcca tgtcgccgcc agaggagctg ctgagccgcc gcgcgcccgc tttccaagat 18180
ggctacccct tcgatgatgc cgcagtggtc ttacatgcac atctcgggcc aggacgcctc 18240
ggagtacctg agccccgggc tggtgcagtt tgcccgcgcc accgagacgt acttcagcct 18300
gaataacaag tttagaaacc ccacggtggc gcctacgcac gacgtgacca cagaccggtc 18360
ccagcgtttg acgctgcggt tcatccctgt ggaccgtgag gatactgcgt actcgtacaa 18420
ggcgcggttc accctagctg tgggtgataa ccgtgtgctg gacatggctt ccacgtactt 18480
tgacatccgc ggcgtgctgg acaggggccc tacttttaag ccctactctg gcactgccta 18540
caacgccctg gctcccaagg gtgccccaaa tccttgcgaa tgggatgaag ctgctactgc 18600
tcttgaaata aacctagaag aagaggacga tgacaacgaa gacgaagtag acgagcaagc 18660
tgagcagcaa aaaactcacg tatttgggca ggcgccttat tctggtataa atattacaaa 18720

ggagggtatt caaataggtg tcgaaggtca aacacctaaa tatgccgata aaacatttca 18780
acctgaacct caaataggag aatctcagtg gtacgaaaca gaaattaatc atgcagctgg 18840
gagagtccta aaaaagacta ccccaatgaa accatgttac ggttcatatg caaaacccac 18900
aaatgaaaat ggagggcaag gcattcttgt aaagcaacaa aatggaaagc tagaaagtca 18960
agtggaaatg caatttttct caactactga ggcagccgca ggcaatggtg ataacttgac 19020
tcctaaagtg gtattgtaca gtgaagatgt agatatagaa accccagaca ctcatatttc 19080
ttacatgccc actattaagg aaggtaactc acgagaacta atgggccaac aatctatgcc 19140
caacaggcct aattacattg cttttaggga caattttatt ggtctaatgt attacaacag 19200
cacgggtaat atgggtgttc tggcgggcca agcatcgcag ttgaatgctg ttgtagattt 19260
gcaagacaga aacacagagc tttcatacca gcttttgctt gattccattg gtgatagaac 19320
caggtacttt tctatgtgga atcaggctgt tgacagctat gatccagatg ttagaattat 19380
tgaaaatcat ggaactgaag atgaacttcc aaattactgc tttccactgg gaggtgtgat 19440
taatacagag actcttacca aggtaaaacc taaaacaggt caggaaaatg gatgggaaaa 19500
agatgctaca gaattttcag ataaaaatga aataagagtt ggaaataatt ttgccatgga 19560
aatcaatcta aatgccaacc tgtggagaaa tttcctgtac tccaacatag cgctgtattt 19620
gcccgacaag ctaaagtaca gtccttccaa cgtaaaaatt tctgataacc caaacaccta 19680
cgactacatg aacaagcgag tggtggctcc cgggctagtg gactgctaca ttaaccttgg 19740
agcacgctgg tcccttgact atatggacaa cgtcaaccca tttaaccacc accgcaatgc 19800
tggcctgcgc taccgctcaa tgttgctggg caatggtcgc tatgtgccct tccacatcca 19860
ggtgcctcag aagttctttg ccattaaaaa cctccttctc ctgccgggct catacaccta 19920
cgagtggaac ttcaggaagg atgttaacat ggttctgcag agctccctag gaaatgacct 19980
aagggttgac ggagccagca ttaagtttga tagcatttgc ctttacgcca ccttcttccc 20040
catggcccac aacaccgcct ccacgcttga ggccatgctt agaaacgaca ccaacgacca 20100
gtcctttaac gactatctct ccgccgccaa catgctctac cctatacccg ccaacgctac 20160
caacgtgccc atatccatcc cctcccgcaa ctgggcggct ttccgcggct gggccttcac 20220
gcgccttaag actaaggaaa ccccatcact gggctcgggc tacgaccctt attacaccta 20280
ctctggctct ataccctacc tagatggaac cttttacctc aaccacacct ttaagaaggt 20340
ggccattacc tttgactctt ctgtcagctg gcctggcaat gaccgcctgc ttacccccaa 20400
cgagtttgaa attaagcgct cagttgacgg ggagggttac aacgttgccc agtgtaacat 20460
gaccaaagac tggttcctgg tacaaatgct agctaactat aacattggct accagggctt 20520
ctatatccca gagagctaca aggaccgcat gtactccttc tttagaaact tccagcccat 20580
gagccgtcag gtggtggatg atactaaata caaggactac caacaggtgg gcatcctaca 20640
ccaacacaac aactctggat ttgttggcta ccttgccccc accatgcgcg aaggacaggc 20700
ctaccctgct aacttcccct atccgcttat aggcaagacc gcagttgaca gcattaccca 20760
gaaaaagttt ctttgcgatc gcaccctttg gcgcatccca ttctccagta actttatgtc 20820
catgggcgca ctcacagacc tgggccaaaa ccttctctac gccaactccg cccacgcgct 20880
agacatgact tttgaggtgg atcccatgga cgagcccacc cttctttatg ttttgtttga 20940
agtctttgac gtggtccgtg tgcaccagcc gcaccgcggc gtcatcgaaa ccgtgtacct 21000
gcgcacgccc ttctcggccg gcaacgccac aacataaaga agcaagcaac atcaacaaca 21060
gctgccgcca tgggctccag tgagcaggaa ctgaaagcca ttgtcaaaga tcttggttgt 21120
gggccatatt ttttgggcac ctatgacaag cgctttccag gctttgtttc tccacacaag 21180
ctcgcctgcg ccatagtcaa tacggccggt cgcgagactg gggcgtaca ctggatggcc 21240
tttgcctgga acccgcactc aaaaacatgc tacctctttg agccctttgg cttttctgac 21300

cagcgactca agcaggttta ccagtttgag tacgagtcac tcctgcgccg tagcgccatt 21360
gcttcttccc ccgaccgctg tataacgctg gaaaagtcca cccaaagcgt acaggggccc 21420
aactcggccg cctgtggact attctgctgc atgtttctcc acgcctttgc caactggccc 21480
caaactccca tggatcacaa ccccaccatg aaccttatta ccggggtacc caactccatg 21540
ctcaacagtc cccaggtaca gcccaccctg cgtcgcaacc aggaacagct ctacagcttc 21600
ctggagcgcc actcgcccta cttccgcagc cacagtgcgc agattaggag cgccacttct 21660
ttttgtcact tgaaaaacat gtaaaaataa tgtactagag acactttcaa taaaggcaaa 21720
tgcttttatt tgtacactct cgggtgatta tttaccccca cccttgccgt ctgcgccgtt 21780
taaaaatcaa aggggttctg ccgcgcatcg ctatgcgcca ctggcaggga cacgttgcga 21840
tactggtgtt tagtgctcca cttaaactca ggcacaacca tccgcggcag ctcggtgaag 21900
ttttcactcc acaggctgcg caccatcacc aacgcgttta gcaggtcggg cgccgatatc 21960
ttgaagtcgc agttggggcc tccgccctgc gcgcgcgagt tgcgatacac agggttgcag 22020
cactggaaca ctatcagcgc cgggtggtgc acgctggcca gcacgctctt gtcggagatc 22080
agatccgcgt ccaggtcctc cgcgttgctc agggcgaacg gagtcaactt tggtagctgc 22140
cttcccaaaa agggcgcgtg cccaggcttt gagttgcact cgcaccgtag tggcatcaaa 22200
aggtgaccgt gcccggtctg ggcgttagga tacagcgcct gcataaaagc cttgatctgc 22260
ttaaaagcca cctgagcctt tgcgccttca gagaagaaca tgccgcaaga cttgccggaa 22320
aactgattgg ccggacaggc cgcgtcgtgc acgcagcacc ttgcgtcggt gttggagatc 22380
tgcaccacat ttcggcccca ccggttcttc acgatcttgg ccttgctaga ctgctccttc 22440
agcgcgcgct gcccgttttc gctcgtcaca tccatttcaa tcacgtgctc cttatttatc 22500
ataatgcttc cgtgtagaca cttaagctcg ccttcgatct cagcgcagcg gtgcagccac 22560
aacgcgcagc ccgtgggctc gtgatgcttg taggtcacct ctgcaaacga ctgcaggtac 22620
gcctgcagga atcgccccat catcgtcaca aaggtcttgt tgctggtgaa ggtcagctgc 22680
aacccgcggt gctcctcgtt cagccaggtc ttgcatacgg ccgccagagc ttccacttgg 22740
tcaggcagta gtttgaagtt cgcctttaga tcgttatcca cgtggtactt gtccatcagc 22800
gcgcgcgcag cctccatgcc cttctcccac gcagacacga tcggcacact cagcgggttc 22860
atcaccgtaa tttcactttc cgcttcgctg ggctcttcct cttcctcttg cgtccgcata 22920
ccacgcgcca ctgggtcgtc ttcattcagc cgccgcactg tgcgcttacc tcctttgcca 22980
tgcttgatta gcaccggtgg gttgctgaaa cccaccattt gtagcgccac atcttctctt 23040
tcttcctcgc tgtccacgat tacctctggt gatggcgggc gctcgggctt gggagaaggg 23100
cgcttctttt tcttcttggg cgcaatggcc aaatccgccg ccgaggtcga tggccgcggg 23160
ctgggtgtgc gcggcaccag cgcgtcttgt gatgagtctt cctcgtcctc ggactcgata 23220
cgccgcctca tccgcttttt tgggggcgcc cggggaggcg gcggcgacgg ggacggggac 23280
gacacgtcct ccatggttgg gggacgtcgc gccgcaccgc gtccgcgctc gggggtggtt 23340
tcgcgctgct cctcttcccg actggccatt tccttctcct ataggcagaa aaagatcatg 23400
gagtcagtcg agaagaagga cagcctaacc gccccctctg agttcgccac caccgcctcc 23460
accgatgccg ccaacgcgcc taccaccttc cccgtcgagg cacccccgct tgaggaggag 23520
gaagtgatta tcgagcagga cccaggtttt gtaagcgaag acgacgagga ccgctcagta 23580
ccaacagagg ataaaaagca agaccaggac aacgcagagg caaacgagga acaagtcggg 23640
cggggggacg aaaggcatgg cgactaccta gatgtgggag acgacgtgct gttgaagcat 23700
ctgcagcgcc agtgcgccat tatctgcgac gcgttgcaag agcgcagcga tgtgcccctc 23760
gccatagcgg atgtcagcct tgcctacgaa cgccacctat tctcaccgcg cgtacccccc 23820
aaacgccaag aaaacggcac atgcgagccc aacccgcgcc tcaacttcta ccccgtattt 23880

gccgtgccag aggtgcttgc cacctatcac atctttttcc aaaactgcaa gataccccta 23940
tcctgccgtg ccaaccgcag ccgagcggac aagcagctgg ccttgcggca gggcgctgtc 24000
atacctgata tcgcctcgct caacgaagtg ccaaaaatct ttgagggtct tggacgcgac 24060
gagaagcgcg cggcaaacgc tctgcaacag gaaaacagcg aaaatgaaag tcactctgga 24120
gtgttggtgg aactcgaggg tgacaacgcg cgcctagccg tactaaaacg cagcatcgag 24180
gtcacccact ttgcctaccc ggcacttaac ctacccccca aggtcatgag cacagtcatg 24240
agtgagctga tcgtgcgccg tgcgcagccc ctggagaggg atgcaaattt gcaagaacaa 24300
acagaggagg gcctacccgc agttggcgac gagcagctag cgcgctggct tcaaacgcgc 24360
gagcctgccg acttggagga gcgacgcaaa ctaatgatgg ccgcagtgct cgttaccgtg 24420
gagcttgagt gcatgcagcg gttctttgct gacccggaga tgcagcgcaa gctagaggaa 24480
acattgcact acacctttcg acagggctac gtacgccagg cctgcaagat ctccaacgtg 24540
gagctctgca acctggtctc ctaccttgga attttgcacg aaaaccgcct tgggcaaaac 24600
gtgcttcatt ccacgctcaa gggcgaggcg cgccgcgact acgtccgcga ctgcgtttac 24660
ttatttctat gctacacctg gcagacggcc atgggcgttt ggcagcagtg cttggaggag 24720
tgcaacctca aggagctgca gaaactgcta aagcaaaact tgaaggacct atggacggcc 24780
ttcaacgagc gctccgtggc cgcgcacctg gcggacatca ttttccccga acgcctgctt 24840
aaaaccctgc aacagggtct gccagacttc accagtcaaa gcatgttgca gaactttagg 24900
aactttatcc tagagcgctc aggaatcttg cccgccacct gctgtgcact tcctagcgac 24960
tttgtgccca ttaagtaccg cgaatgccct ccgccgcttt ggggccactg ctaccttctg 25020
cagctagcca actaccttgc ctaccactct gacataatgg aagacgtgag cggtgacggt 25080
ctactggagt gtcactgtcg ctgcaaccta tgcaccccgc accgctccct ggtttgcaat 25140
tcgcagctgc ttaacgaaag tcaaattatc ggtacctttg agctgcaggg tccctcgcct 25200
gacgaaaagt ccgcggctcc ggggttgaaa ctcactccgg ggctgtggac gtcggcttac 25260
cttcgcaaat ttgtacctga ggactaccac gcccacgaga ttaggttcta cgaagaccaa 25320
tcccgcccgc ctaatgcgga gcttaccgcc tgcgtcatta cccagggcca cattcttggc 25380
caattgcaag ccatcaacaa agcccgccaa gagtttctgc tacgaaaggg acgggggtt 25440
tacttggacc cccagtccgg cgaggagctc aacccaatcc ccccgccgcc gcagccctat 25500
cagcagcagc cgcgggccct tgcttcccag gatggcaccc aaaaagaagc tgcagctgcc 25560
gccgccaccc acggacgagg aggaatactg ggacagtcag gcagaggagg ttttggacga 25620
ggaggaggag gacatgatgg aagactggga gagcctagac gaggaagctt ccgaggtcga 25680
agaggtgtca gacgaaacac cgtcaccctc ggtcgcattc ccctcgccgg cgccccagaa 25740
atcggcaacc ggttccagca tggctacaac ctccgctcct caggcgccgc cggcactgcc 25800
cgttcgccga cccaaccgta gatgggacac cactggaacc agggccggta agtccaagca 25860
gccgccgccg ttagcccaag agcaacaaca gcgccaaggc taccgctcat ggcgcgggca 25920
caagaacgcc atagttgctt gcttgcaaga ctgtgggggc aacatctcct tcgcccgccg 25980
ctttcttctc taccatcacg gcgtggcctt cccccgtaac atcctgcatt actaccgtca 26040
tctctacagc ccatactgca ccggcggcag cggcagcaac agcagcggcc acacagaagc 26100
aaaggcgacc ggatagcaag actctgacaa agcccaagaa atccacagcg gcggcagcag 26160
caggaggagg agcgctgcgt ctggcgccca acgaacccgt atcgacccgc gagcttagaa 26220
acaggatttt tcccactctg tatgctatat ttcaacagag caggggccaa gaacaagagc 26280
tgaaaataaa aaacaggtct ctgcgatccc tcacccgcag ctgcctgtat cacaaaagcg 26340
aagatcagct tcggcgcacg ctggaagacg cggaggctct cttcagtaaa tactgcgcgc 26400
tgactcttaa ggactagttt cgcgcccttt ctcaaattta agcgcgaaaa ctacgtcatc 26460

tccagcggcc acacccggcg ccagcacctg ttgtcagcgc cattatgagc aaggaaattc 26520
ccacgcccta catgtggagt taccagccac aaatgggact tgcggctgga gctgcccaag 26580
actactcaac ccgaataaac tacatgagcg cgggacccca catgatatcc cgggtcaacg 26640
gaatacgcgc ccaccgaaac cgaattctcc tggaacaggc ggctattacc accacacctc 26700
gtaataacct taatccccgt agttggcccg ctgccctggt gtaccaggaa agtcccgctc 26760
ccaccactgt ggtacttccc agagacgccc aggccgaagt tcagatgact aactcagggg 26820
cgcagcttgc gggcggcttt cgtcacaggg tgcggtcgcc cgggcagggt ataactcacc 26880
tgacaatcag agggcgaggt attcagctca acgacgagtc ggtgagctcc tcgcttggtc 26940
tccgtccgga cgggacattt cagatcggcg gcgccggccg ctcttcattc acgcctcgtc 27000
aggcaatcct aactctgcag acctcgtcct ctgagccgcg ctctggaggc attggaactc 27060
tgcaatttat tgaggagttt gtgccatcgg tctactttaa ccccttctcg ggacctcccg 27120
gccactatcc ggatcaattt attcctaact ttgacgcggt aaaggactcg gcggacggct 27180
acgactgaat gttataagtt cctgtccatc cgcacccact atcttcatgt tgttgcagat 27240
gaagcgcgca agaccgtctg aagatacctt caaccccgtg tatccatatg acacggaaac 27300
cggtcctcca actgtgcctt ttcttactcc tccctttgta tcccccaatg ggtttcaaga 27360
gagtcccccct ggggtactct ctttgcgcct atccgaacct ctagttacct ccaatggcat 27420
gcttgcgctc aaaatgggca acggcctctc tctggacgag gccggcaacc ttacctccca 27480
aaatgtaacc actgtgagcc cacctctcaa aaaaaccaag tcaaacataa acctggaaat 27540
atctgcaccc ctcacagtta cctcagaagc cctaactgtg gctgccgccg cacctctaat 27600
ggtcgcgggc aacacactca ccatgcaatc acaggccccg ctaaccgtgc acgactccaa 27660
acttagcatt gccacccaag gacccctcac agtgtcagaa ggaaagctag ccctgcaaac 27720
atcaggcccc ctcaccacca ccgatagcag taccccttact atcactgcct caccccctct 27780
aactactgcc actggtagct tgggcattga cttgaaagag cccatttata cacaaaatgg 27840
aaaactagga ctaaagtacg gggctccttt gcatgtaaca gacgacctaa acactttgac 27900
cgtagcaact ggtccaggtg tgactattaa taatacttcc ttgcaaacta aagttactgg 27960
agccttgggt tttgattcac aaggcaatat gcaacttaat gtagcaggag gactaaggat 28020
tgattctcaa aacagacgcc ttatacttga tgttagttat ccgtttgatg ctcaaaacca 28080
actaaatcta agactaggac agggccctct tttataaac tcagcccaca acttggatat 28140
taactacaac aaaggccttt acttgtttac agcttcaaac aattccaaaa agcttgaggt 28200
taacctaagc actgccaagg ggttgatgtt tgacgctaca gccatagcca ttaatgcagg 28260
agatgggctt gaatttggtt cacctaatgc accaaacaca aatcccctca aaacaaaaat 28320
tggccatggc ctagaatttg attcaaacaa ggctatggtt cctaaactag gaactggcct 28380
tagttttgac agcacaggtg ccattacagt aggaaacaaa aataatgata agctaacttt 28440
gtggaccaca ccagctccat ctcctaactg tagactaaat gcagagaaag atgctaaact 28500
cactttggtc ttaacaaaat gtggcagtca aatacttgct acagtttcag ttttggctgt 28560
taaaggcagt ttggctccaa tatctggaac agttcaaagt gctcatctta ttataagatt 28620
tgacgaaaat ggagtgctac taaacaattc cttcctggac ccagaatatt ggaactttag 28680
aaatggagat cttactgaag gcacagccta tacaaacgct gttggattta tgcctaacct 28740
atcagcttat ccaaaatctc acggtaaaac tgccaaaagt aacattgtca gtcaagttta 28800
cttaaacgga gacaaaacta aacctgtaac actaaccatt acactaaacg gtacacagga 28860
aacaggagac acaactccaa gtgcatactc tatgtcattt tcatgggact ggtctggcca 28920
caactacatt aatgaaatat ttgccacatc ctcttacact ttttcataca ttgcccaaga 28980
ataaagaatc gtttgtgtta tgtttcaacg tgtttatttt tcaattgcag aaaatttcaa 29040

gtcatttttc attcagtagt atagccccac caccacatag cttatacaga tcaccgtacc 29100
ttaatcaaac tcacagaacc ctagtattca acctgccacc tccctcccaa cacacagagt 29160
acacagtcct ttctccccgg ctggccttaa aaagcatcat atcatgggta acagacatat 29220
tcttaggtgt tatattccac acggtttcct gtcgagccaa acgctcatca gtgatattaa 29280
taaactcccc gggcagctca cttaagttca tgtcgctgtc cagctgctga gccacaggct 29340
gctgtccaac ttgcggttgc ttaacgggcg gcgaaggaga agtccacgcc tacatggggg 29400
tagagtcata atcgtgcatc aggatagggc ggtggtgctg cagcagcgcg cgaataaact 29460
gctgccgccg ccgctccgtc ctgcaggaat acaacatggc agtggtctcc tcagcgatga 29520
ttcgcaccgc ccgcagcata aggcgccttg tcctccgggc acagcagcgc accctgatct 29580
cacttaaatc agcacagtaa ctgcagcaca gcaccacaat attgttcaaa atcccacagt 29640
gcaaggcgct gtatccaaag ctcatggcgg ggaccacaga acccacgtgg ccatcatacc 29700
acaagcgcag gtagattaag tggcgacccc tcataaacac gctggacata aacattacct 29760
cttttggcat gttgtaattc accacctccc ggtaccatat aaacctctga ttaaacatgg 29820
cgccatccac caccatccta aaccagctgg ccaaaacctg cccgccggct atacactgca 29880
gggaaccggg actggaacaa tgacagtgga gagcccagga ctcgtaacca tggatcatca 29940
tgctcgtcat gatatcaatg ttggcacaac acaggcacac gtgcatacac ttcctcagga 30000
ttacaagctc ctcccgcgtt agaaccatat cccagggaac aacccattcc tgaatcagcg 30060
taaatcccac actgcaggga agacctcgca cgtaactcac gttgtgcatt gtcaaagtgt 30120
tacattcggg cagcagcgga tgatcctcca gtatggtagc gcgggtttct gtctcaaaag 30180
gaggtagacg atccctactg tacggagtgc gccgagacaa ccgagatcgt gttggtcgta 30240
gtgtcatgcc aaatggaacg ccggacgtag tcatatttcc tgaagcaaaa ccaggtgcgg 30300
gcgtgacaaa cagatctgcg tctccggtct cgccgcttag atcgctctgt gtagtagttg 30360
tagtatatcc actctctcaa agcatccagg cgccccctgg cttcgggttc tatgtaaact 30420
ccttcatgcg ccgctgccct gataacatcc accaccgcag aataagccac acccagccaa 30480
cctacacatt cgttctgcga gtcacacacg ggaggagcgg gaagagctgg aagaaccatg 30540
tttttttttt tattccaaaa gattatccaa aacctcaaaa tgaagatcta ttaagtgaac 30600
gcgctcccct ccggtggcgt ggtcaaactc tacagccaaa gaacagataa tggcatttgt 30660
aagatgttgc acaatggctt ccaaaaggca aacggccctc acgtccaagt ggacgtaaag 30720
gctaaaccct tcagggtgaa tctcctctat aaacattcca gcaccttcaa ccatgcccaa 30780
ataattctca tctcgccacc ttctcaatat atctctaagc aaatcccgaa tattaagtcc 30840
ggccattgta aaaatctgct ccagagcgcc ctccaccttc agcctcaagc agcgaatcat 30900
gattgcaaaa attcaggttc ctcacagacc tgtataagat tcaaaagcgg aacattaaca 30960
aaaataccgc gatcccgtag gtcccttcgc agggccagct gaacataatc gtgcaggtct 31020
gcacggacca gcgcggccac ttccccgcca ggaaccatga caaaagaacc cacactgatt 31080
atgacacgca tactcggagc tatgctaacc agcgtagccc cgatgtaagc ttgttgcatg 31140
ggcggcgata taaaatgcaa ggtgctgctc aaaaaatcag gcaaagcctc gcgcaaaaaa 31200
gaaagcacat cgtagtcatg ctcatgcaga taaaggcagg taagctccgg aaccaccaca 31260
gaaaaagaca ccatttttct ctcaaacatg tctgcgggtt tctgcataaa cacaaaataa 31320
aataacaaaa aaacatttaa acattagaag cctgtcttac aacaggaaaa acaaccctta 31380
taagcataag acggactacg gccatgccgg cgtgaccgta aaaaaactgg tcaccgtgat 31440
taaaaagcac caccgacagc tcctcggtca tgtccggagt cataatgtaa gactcggtaa 31500
acacatcagg ttgattcaca tcggtcagtg ctaaaaagcg accgaaatag cccgggggaa 31560
tacatacccg caggcgtaga gacaacatta cagcccccat aggaggtata acaaaattaa 31620

taggagagaa aaacacataa acacctgaaa aaccctcctg cctaggcaaa atagcaccct 31680
cccgctccag aacaacatac agcgcttcca cagcggcagc cataacagtc agccttacca 31740
gtaaaaaaga aaacctatta aaaaaacacc actcgacacg gcaccagctc aatcagtcac 31800
agtgtaaaaa agggccaagt gcagagcgag tatatatagg actaaaaaat gacgtaacgg 31860
ttaaagtcca caaaaaacac ccagaaaacc gcacgcgaac ctacgcccag aaacgaaagc 31920
caaaaaaccc acaacttcct caaatcgtca cttccgtttt cccacgttac gtcacttccc 31980
attttaagaa aactacaatt cccaacacat acaagttact ccgccctaaa acctacgtca 32040
cccgccccgt tcccacgccc cgcgccacgt cacaaactcc accccctcat tatcatattg 32100
gcttcaatcc aaaataaggt atattattga tgatgttaca tcgttaatta acgatttcga 32160
acccggggta ccgaattcct cgagtctaga ggagcatgcg acgtcgcaat tcgccctata 32220
gtgagtcgta ttacaattca ctggccgtcg ttttacaacg tcgtgactgg gaaaaccctg 32280
gcgttaccca acttaatcgc cttgcagcac atcccccttt cgccagctgg cgtaatagcg 32340
aagaggcccg caccgatcgc ccttcccaac agttgcgcag cctgaatggc gaatggaaat 32400
tgtaagcgtt aatattttgt taaaattcgc gttaaatttt tgttaaatca gctcattttt 32460
taaccaatag gccgaaatcg gcaaaatccc ttataaatca aaagaataga ccgagatagg 32520
gttgagtgtt gttccagttt ggaacaagag tccactatta aagaacgtgg actccaacgt 32580
caaagggcga aaaaccgtct atcagggcga tggcccacta cgtgaaccat caccctaatc 32640
aagttttttg gggtcgaggt gccgtaaagc actaaatcgg aaccctaaag ggagcccccg 32700
atttagagct tgacggggaa agccggcgaa cgtggcgaga aaggaaggga agaaagcgaa 32760
aggagcgggc gctagggcgc tggcaagtgt agcggtcacg ctgcgcgtaa ccaccacacc 32820
cgcccgcgctt aatgcgccgc tacagggcgc gtcctgatgc ggtattttct ccttacgcat 32880
ctgtgcggta tttcacaccg catacaggtg gcacttttcg gggaaatgtg cgcggaaccc 32940
ctatttgttt atttttctaa atacattcaa atatgtatcc gctcatgaga caataaccct 33000
gataaatgct tcaataatat tgaaaaagga agagtatgag tattcaacat ttccgtgtcg 33060
cccttattcc cttttttgcg gcattttgcc ttcctgtttt tgctcaccca gaaacgctgg 33120
tgaaagtaaa agatgctgaa gatcagttgg gtgcacgagt gggttacatc gaactggatc 33180
tcaacagcgg taagatcctt gagagttttc gccccgaaga acgttttcca atgatgagca 33240
cttttaaagt tctgctatgt ggcgcggtat tatcccgtat tgacgccggg caagagcaac 33300
tcggtcgccg catacactat tctcagaatg acttggttga gtactcacca gtcacagaaa 33360
agcatcttac ggatggcatg acagtaagag aattatgcag tgctgccata accatgagtg 33420
ataacactgc ggccaactta cttctgacaa cgatcggagg accgaaggag ctaaccgctt 33480
ttttgcacaa catgggggat catgtaactc gccttgatcg ttgggaaccg gagctgaatg 33540
aagccatacc aaacgacgag cgtgacacca cgatgcctgt agcaatggca acaacgttgc 33600
gcaaactatt aactggcgaa ctacttactc tagcttcccg gcaacaatta atagactgga 33660
tggaggcgga taaagttgca ggaccacttc tgcgctcggc ccttccggct ggctggttta 33720
ttgctgataa atctggagcc ggtgagcgtg ggtctcgcgg tatcattgca gcactggggc 33780
cagatggtaa gccctcccgt atcgtagtta tctacacgac ggggagtcag gcaactatgg 33840
atgaacgaaa tagacagatc gctgagatag gtgcctcact gattaagcat tggtaactgt 33900
cagaccaagt ttactcatat atactttaga ttgatttaaa acttcatttt taatttaaaa 33960
ggatctaggt gaagatcctt tttgataatc tcatgaccaa aatcccttaa cgtgagtttt 34020
cgttccactg agcgtcagac cccgtagaaa agatcaaagg atcttcttga gatccttttt 34080
ttctgcgcgt aatctgctgc ttgcaaacaa aaaaaccacc gctaccagcg gtggtttgtt 34140
tgccggatca agagctacca actctttttc cgaaggtaac tggcttcagc agagcgcaga 34200

taccaaatac tgttcttcta gtgtagccgt agttaggcca ccacttcaag aactctgtag 34260
caccgcctac atacctcgct ctgctaatcc tgttaccagt ggctgctgcc agtggcgata 34320
agtcgtgtct taccgggttg gactcaagac gatagttacc ggataaggcg cagcggtcgg 34380
gctgaacggg gggttcgtgc acacagccca gcttggagcg aacgacctac accgaactga 34440
gatacctaca gcgtgagcta tgagaaagcg ccacgcttcc cgaagggaga aaggcggaca 34500
ggtatccggt aagcggcagg gtcggaacag gagagcgcac gagggagctt ccagggggaa 34560
acgcctggta tctttatagt cctgtcgggt ttcgccacct ctgacttgag cgtcgatttt 34620
tgtgatgctc gtcagggggg cggagcctat ggaaaaacgc cagcaacgcg gcctttttac 34680
ggttcctggc cttttgctgg ccttttgctc acatgttctt tcctgcgtta tcccctgatt 34740
ctgtggataa ccgtattacc gcctttgagt gagctgatac cgctcgccgc agccgaacga 34800
ccgagcgcag cgagtcagtg agcgaggaag cggaagagcg cccaatacgc aaaccgcctc 34860
tccccgcgcg ttggccgatt cattaatgca gctggcacga caggtttccc gactggaaag 34920
cgggcagtga gcgcaacgca attaatgtga gttagctcac tcattaggca ccccaggctt 34980
tacactttat gcttccggct cgtatgttgt gtggaattgt gagcggataa caatttcaca 35040
caggaaacag ctatgaccat gattacgcca agctatttag gtgacactat agaatactca 35100
agctagttaa ttaacgttaa ttaacatcat caataatata ccttattttg gattgaagcc 35160
aatatgataa tgagggggtg gagtttgtga cgtggcgcgg ggcgtgggaa cggggcgggt 35220
gacgtagtag tgtggcggaa gtgtgatgtt gcaagtgtgg cggaacacat gtaagcgacg 35280
gatgtggcaa aagtgacgtt tttggtgtgc gccggtgtac acaggaagtg acaattttcg 35340
cgcggtttta ggcggatgtt gtagtaaatt tgggcgtaac cgagtaagat ttggccattt 35400
tcgcgggaaa actgaataag aggaagtgaa atctgaataa ttttgtgtta ctcatagcgc 35460
gtaatctcta gcat 35474

<210> 17
<211> 19
<212> DNA
<213> Artificial Sequence

<220>
<223> Description of Artificial Sequence: Synthetic Primer

<400> 17
cgagtacttt gggcacttc 19

<210> 18
<211> 19
<212> DNA
<213> Artificial Sequence

<220>

<223> Description of Artificial Sequence: Synthetic Primer

<400> 18
gaagtgccca aagtactcg 19

<210> 19
<211> 30

<212> DNA
<213> Artificial Sequence

<220>
<223> Description of Artificial Sequence: Synthetic Primer

<400> 19
gaacaaaaac tcatctcaga agaggatctg 30

<210> 20
<211> 33
<212> DNA
<213> Artificial Sequence

<220>
<223> Description of Artificial Sequence: Synthetic Primer

<400> 20
aatatgcata ccggtcatca tcaccatcac cat 33

<210> 21
<211> 21
<212> DNA
<213> artificial sequence

<220>
<223> siRNA: siPD21

<400> 21
gcaggcaaga gaagctgaaa t 21

<210> 22
<211> 21
<212> DNA

<213> artificial sequence

<220>
<223> siRNA: siHD21

<400> 22
ggctcgaatt gcccagctaa a 21

<210> 23
<211> 29
<212> DNA
<213> artificial sequence

<220>
<223> siRNA: siPD29

<400> 23
ggctcgaatt gcccagctaa aggatgagt 29